# EUROPÄISCHE PATENTSCHRIFT

(11) **EP 2 611 802 B1**
(45) Veröffentlichungstag und Bekanntmachung des Hinweises auf die Patenterteilung: **24.02.2016**
(21) Anmeldenummer: 11752194.8
(22) Anmeldetag: 31.08.2011
(51) Int. Cl.: C07D 471/04, C07D 487/04, A61K 31/437, A61K 31/519, A61P 9/00, A61P 9/04, A61P 9/12, A61P 9/14, A61P 9/10, A61P 7/02, A61P 13/12

(54) **BICYCLISCHE AZA-HETEROCYCLEN UND IHRE VERWENDUNG**
BICYCLIC AZA-HETEROCYCLES AND THEIR USE
HÉTÉROCYCLES BICYCLIQUES AZA ET LEUR UTILISATION

(30) Priorität: 03.09.2010 DE 102010040233
(43) Veröffentlichungstag der Anmeldung: 10.07.2013
(73) Patentinhaber: Bayer Intellectual Property GmbH, 40789 Monheim (DE)
(72) Erfinder: FOLLMANN, Markus, 50859 Köln (DE); STASCH, Johannes-Peter, 42651 Solingen (DE); REDLICH, Gorden, 44799 Bochum (DE); ACKERSTAFF, Jens, 10437 Berlin (DE); GRIEBENOW, Nils, 41541 Dormagen (DE); KNORR, Andreas, 40699 Erkrath (DE); WUNDER, Frank, 42117 Wuppertal (DE); LI, Volkhart Min-Jian, 42553 Velbert (DE); BÄRFACKER, Lars, 46047 Oberhausen (DE); WEIGAND, Stefan, 82377 Penzberg (DE)
(86) Internationale Anmeldenummer: PCT/EP2011/065006
(87) Internationale Veröffentlichungsnummer: WO 2012/028647

(56) Entgegenhaltungen:
- WO-A1-00/06569
- WO-A1-2010/065275

## Beschreibung

Die vorliegende Anmeldung betrifft neue, bicyclische Aza-Heterocyclen, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-l-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Als Stimulatoren der löslichen Guanylatcyclase werden in WO 00/06569 annellierte Pyrazol-Derivate und in WO 01/083490 ein annelliertes Aminopyrimidin-Derivate beschrieben. WO 2010/065275 offenbart Pyrrolopyrimidone als Aktivatoren der löslichen Guanylatcyclase.

Aufgabe der vorliegenden Erfindung war die Bereitstellung neuer Substanzen, die als sehr potente Stimulatoren der löslichen Guanylatcyclase wirken und sich daher zur Behandlung und /oder Prophylaxe von kardiovaskulären Erkrankungen eignen.

Gegenstand der vorliegenden Erfindung sind Verbindungen der allgemeinen Formel (I) 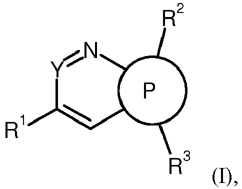 in welcher
- der Ring P: für eine Gruppe der Formel 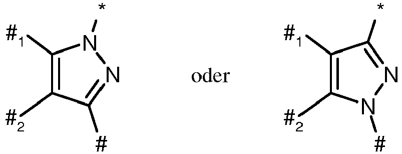 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
- Y: für CH oder N steht,
- R¹: für Wasserstoff oder Fluor steht,
- R²: für (C₁-C₆)-Alkyl oder Benzyl steht,
wobei (C₁-C₆)-Alkyl mit einem Substituenten Trifluormethyl substituiert ist,
wobei (C₁-C₆)-Alkyl mit 1 bis 3 Substituenten Fluor substituiert sein kann,
und
wobei Benzyl mit 1 bis 3 Substituenten Fluor substituiert ist,
- R³: für eine Gruppe der Formel 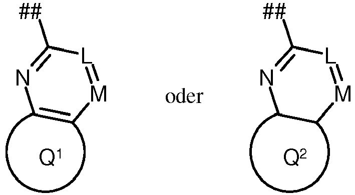 steht, wobei
## für die Anknüpfstelle an den Ring P steht,
L für CH oder N steht
M für CR⁴ oder N steht,
worin
R⁴ für Wasserstoff, Halogen, Cyano, (C₁-C₄)-Alkyl, (C₂-C₄)-Alkinyl, Hydroxy, (C₁-C₄)-Alkoxy, (C₁-C₄)-Alkylcarbonyl, (C₁-C₄)-Alkoxy-carbonyl, Amino, Mono-(C₁-C₄)-alkylamino, Di-(C₁-C₄)-Alkylamino oder Azetidinyl steht,
worin (C₁-C₄)-Alkyl, (C₂-C₄)-Alkinyl, (C₁-C₄)-Alkoxy, Mono-(C₁-C₄)-alkylamino und Di-(C₁-C₄)-Alkylamino ihrerseits mit 1 oder 2 Substituenten unabhängig voneinander ausgewählt aus der Gruppe Fluor, Hydroxy und Amino substituiert sein können,
der Ring Q¹ für 5- bis 7-gliedriges Heterocyclyl, Phenyl oder 5- oder 6-gliedriges Heteroaryl steht,
worin 5- bis 7-gliedriges Heterocyclyl, Phenyl und 5- oder 6-gliedriges Heteroaryl mit 1 bis 3 Substituenten unabhängig voneinander ausgewählt aus der Gruppe Halogen, Difluormethyl, Trifluormethyl, Trideuteromethyl, (C₁-C₆)-Alkyl, (C₂-C₆)-Alkenyl, (C₃-C₇)-Cycloalkyl, Oxo, Hydroxy, (C₁-C₄)-Alkoxy, (C₁-C₄)-Alkoxycarbonyl, (C₃-C₇)-Cycloalkoxy-carbonyl, Amino, Mono-(C₁-C₄)-alkylamino, Di-(C₁-C₄)-Alkylamino, Thiooxo, (C₁-C₄)-Alkylthio, Aminosulfonyl, Mono-(C1-C₄)-alkylaminosulfonyl, Di-(C₁-C₄)-alkylaminosulfonyl, 4- bis 7-gliedriges Heterocyclyl, Phenyl und Benzyl substituiert sein können,
worin (C₁-C₆)-Alkyl, (C₃-C₇)-Cycloalkyl, (C₁-C₄)-Alkoxy, (C₁-C₄)-Alkoxycarbonyl und (C₁-C₄)-Alkylthio ihrerseits mit 1 bis 3 Substituenten unabhängig voneinander ausgewählt aus der Gruppe Halogen, Trifluormethyl, (C₃-C₇)-Cycloalkyl, Hydroxy, (C₁-C₄)-Alkoxy, (C₁-C₄)-Alkylsulfonyl und 4- bis 7-gliedriges Heterocyclyl substituiert sein können,
   worin 4- bis 7-gliedriges Heterocyclyl wiederum seinerseits mit 1 oder 2 Substituenten unabhängig voneinander ausgewählt aus der Gruppe Halogen, Trifluormethyl, (C₁-C₄)-Alkyl und (C₃-C₇)-Cycloalkyl substituiert sein kann,
      worin (C₁-C₄)-Alkyl weiterhin seinerseits mit 1 oder 2 Substituenten unabhängig voneinander ausgewählt aus der Gruppe Fluor, Trifluormethyl, (C₃-C₇)-Cycloalkyl, Hydroxy und (C₁-C₄)-Alkoxy substituiert sein kann,
   worin 4- bis 7-gliedriges Heterocyclyl seinerseits mit 1 oder 2 Substituenten unabhängig voneinander ausgewählt aus der Gruppe Halogen, Trifluormethyl, (C₁-C₄)-Alkyl und (C₃-C₇)-Cycloalkyl substituiert sein kann,
      worin (C₁-C₄)-Alkyl wiederum seinerseits mit 1 oder 2 Substituenten unabhängig voneinander ausgewählt aus der Gruppe Fluor, Trifluormethyl, (C₃-C₇)-Cycloalkyl, Hydroxy und (C₁-C₄)-Alkoxy substituiert sein kann,
   und
   worin Phenyl und Benzyl ihrerseits mit 1 bis 3 Substituenten Halogen, Cyano, Trifluormethyl, (C₁-C₄)-Alkyl, (C₁-C₄)-Alkoxy und (C₁-C₄)-Alkylsulfonyl substituiert sein können,

der Ring Q² für 5-gliedriges Heteroaryl steht,
worin 5-gliedriges Heteroaryl mit 1 bis 3 Substituenten unabhängig voneinander ausgewählt aus der Gruppe Halogen, Difluormethyl, Trifluormethyl, Trideuteromethyl, (C₁-C₆)-Alkyl, (C₂-C₆)-Alkenyl, (C₃-C₇)-Cycloalkyl, Hydroxy, (C₁-C₄)-Alkoxy, (C₁-C₄)-Alkoxycarbonyl, (C₃-C₇)-Cycloalkoxycarbonyl, Amino, Mono-(C₁-C₄)-alkylamino, Di-(C₁-C₄)-Alkylamino, (C₁-C₄)-Alkylthio, Aminosulfonyl, Mono-(C₁-C₄)-alkylaminosulfonyl, Di-(C₁-C₄)-alkylaminosulfonyl, Phenyl oder Benzyl substituiert sein kann,
worin (C₁-C₆)-Alkyl, (C₃-C₇)-Cycloalkyl, (C₁-C₄)-Alkoxy, (C₁-C₄)-Alkoxycarbonyl und (C₁-C₄)-Alkylthio ihrerseits mit 1 bis 3 Substituenten unabhängig voneinander ausgewählt aus der Gruppe Halogen, Trifluormethyl, (C₃-C₇)-Cycloalkyl, Hydroxy, (C₁-C₄)-Alkoxy, (C₁-C₄)-Alkylsulfonyl und 4- bis 7-gliedriges Heterocyclyl substituiert sein können,
und
worin Phenyl und Benzyl ihrerseits mit 1 bis 3 Substituenten Halogen, Trifluormethyl, (C₁-C₄)-Alkyl und (C₁-C₄)-Alkoxy substituiert sein können,
sowie ihre *N*-Oxide, Salze, Solvate, Salze der *N*-Oxide und Solvate der *N*-Oxide und Salze,
mit Ausnahme der Verbindungen:
2-[1-(2-Fluorbenzyl)-1H-pyrazolo[3,4-b]pyridin-3-yl]-8-methyl-9H-purin,
2-[1-(2-Fluorbenzyl)-1H-pyrazolo[3,4-b]pyridin-3-yl]-8-methyl-9H-purin-6-amin_{,}
N-Butyl-2-[1-(2-fluorbenzyl)-1H-pyrazolo[3,4-b]pyridin-3-yl]-8-methyl-9H-purin-6-amin.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O ¹⁸O, ³²P, ³³P, ³³S, ³⁴S, ³⁵S, ³⁶S, ¹⁸F ³⁶Cl, ⁸²Br, ¹²³I, ¹²⁴I ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem offenbart die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl, 1,4-Dimethylpentyl, 4,4-Dimethylpentyl und 1,4,4-Trimethylpentyl.
Cycloalkyl steht in Rahmen der Erfindung für einen monocyclischen, gesättigten Alkylrest mit 3 bis 7 Kohlenstoffatomen. Beispielhaft und vorzugsweise seien genannt: Cyclopropyl, Cyclobutyl, Cyclopentyl, Cyclohexyl und Cycloheptyl.
Alkenyl steht im Rahmen der Erfindung für einen linearen oder verzweigten Alkenylrest mit 2 bis 6 Kohlenstoffatomen und einer oder zwei Doppelbindungen. Bevorzugt ist ein geradkettiger oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Alkin^{y}l steht im Rahmen der Erfindung für einen linearen oder verzweigten Alkinylrest mit 2 bis 4 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.
Alkylcarbonyl steht im Rahmen der Erfindung für einen linearen oder verzweigten Alkylrest mit 1 bis 6 bzw. 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.
Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
Alkoxycarbonyl stehen im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Bevorzugt ist ein linearer oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Cycloalkoxycarbonyl steht im Rahmen der Erfindung für einen monocyclischen, gesättigten Cycloalkoxyrest mit 3 bis 7 Kohlenstoffatomen und einer am Sauerstoffatom angebundenen Carbonylgruppe. Beispielhaft und vorzugsweise seien genannt: Cyclopropyloxycarbonyl, Cyclobutyloxycarbonyl, Cyclopentyloxycarbonyl, Cyclohexyloxycarbonyl und Cycloheptyloxycarbonyl.
Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.
Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino und tert.-Butylamino.
Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-propylamino, N-tert.-Butyl-*N*-methylamino, *N*-Ethyl-*N*-n-pentylamino und *N*-n-Hexyl-*N*-methylamino.
Mono-alkylaminosulfonyl steht im Rahmen der Erfindung für eine Amino-Gruppe, die über eine Sulfonylgruppe verknüpft ist und die einen linearen oder verzweigten Alkylsubstituenten mit 1 bis 6 Kohlenstoffatomen aufweist. Beispielhaft und vorzugsweise seien genannt: Methylaminosulfonyl, Ethylaminosulfonyl, n-Propylaminosulfonyl, Isopropylaminosulfonyl, n-Butylaminosulfonyl und tert.-Butylaminosulfonyl.
Di-alkylaminosulfonyl steht im Rahmen der Erfindung für eine Amino-Gruppe, die über eine Sulfonylgruppe verknüpft ist und die zwei gleiche oder verschiedene lineare oder verzweigte Alkylsubstituenten mit jeweils 1 bis 6 Kohlenstoffatomen aufweist. Beispielhaft und vorzugsweise seien genannt: *N*,*N*-Dimethylaminosulfonyl, *N,N*-Diethylaminosulfonyl, *N*-Ethyl-*N-*methylaminosulfonyl, *N*-Methyl-*N*-n-propylaminosulfonyl, *N*-n-Butyl-*N*-methylaminosulfonyl und *N*-tert*.-*Butyl-*N*-methylaminosulfonyl.
Alkylthio steht im Rahmen der Erfindung für eine Thio-Gruppe mit einem linearen oder verzweigten Alkylsubstituenten, der 1 bis 4 Kohlenstoffatome aufweist. Beispielhaft und vorzugsweise seien genannt: Methylthio, Ethylthio, n-Propylthio, Isopropylthio, n-Butylthio und *tert.-*Butylthio.
5- bis 7-gliedriges Heterocyclyl steht im Rahmen der Erfindung für einen teilweise ungesättigten Heterocyclus mit insgesamt 5 bis 7 Ringatomen und 1 oder 2 Doppelbindungen, der 1 RingStickstoffatom enthält, und der 1 oder 2 weitere Ring-Heteroatome aus der Reihe N, O und/oder S enthalten kann. Beispielhaft seien genannt: Dihydropyrazolyl, Dihydroimidazolyl, Dihydrooxazolyl, Dihydrothiazolyl, Dihydroisooxazolyl, Dihydroisothiazolyl, Dihydrothiadiazolyl, Dihydropyridinyl, Tetrahydropyridinyl, Tetrahydropyridazinyl, Dihydropyrimidinyl, Tetrahydropyrimidinyl, Dihydropyrazinyl, Tetrahydropyrazinyl, Dihydrotriazinyl, Tetrahydrotriazinyl, Dihydrooxazinyl, Thiadiazinanyl, Dihydrodiazepinyl und Tetrahydrodiazepinyl. Bevorzugt sind: Dihydroimidazolyl, Dihydrooxazolyl, Dihydropyrazinyl, Tetrahydropyrazinyl, Dihydrotriazinyl und Dihydrodiazepinyl.
4- bis 7-gliedriges Heterocyclyl steht im Rahmen der Erfindung für einen gesättigten Heterocyclus mit insgesamt 4 bis 7 Ringatomen, der 1 bis 3 Ring-Heteroatome aus der Reihe N, O und/oder S enthält. Beispielhaft seien genannt: Azetidinyl, Pyrrolidinyl, Tetrahydrofuranyl, Piperidinyl, Tetrahydropyranyl, Piperazinyl und Morpholinyl.
Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt seien genannt: Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl und Pyrazinyl.
Eine Oxo-Gruppe steht im Rahmen der Erfindung für ein Sauerstoffatom, das über eine Doppelbindung an ein Kohlenstoffatom gebunden ist.
Eine Thiooxo-Gruppe steht im Rahmen der Erfindung für ein Schwefelatom, das über eine Doppelbindung an ein Kohlenstoffatom gebunden ist.
Halogen steht im Rahmen der Erfindung für Fluor, Chlor, Brom und Iod.

In der Formel der Gruppe, für die P bzw. R³ stehen kann, steht der Endpunkt der Linie, an dem das Zeichen *, #, #₁, #₂ bzw. ## steht, nicht für ein Kohlenstoffatom beziehungsweise eine CH₂-Gruppe, sondern ist Bestandteil der Bindung zu dem jeweils bezeichneten Atom, an das P bzw. R³ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Bevorzugt sind im Rahmen der vorliegenden Erfindung Verbindungen der Formel (I), in welcher
- der Ring P: für eine Gruppe der Formel 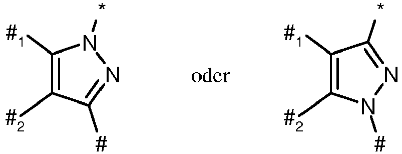 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
- Y: für CH oder N steht,
- R¹: für Wasserstoff oder Fluor steht,
- R²: für (C₁-C₆)-Alkyl oder Benzyl steht,
wobei (C₁-C₆)-Alkyl mit einem Substituenten Trifluormethyl substituiert ist,
wobei (C₁-C₆)-Alkyl mit 1 bis 3 Substituenten Fluor substituiert sein kann,
und
wobei Benzyl mit 1 bis 3 Substituenten Fluor substituiert ist,
- R³: für eine Gruppe der Formel 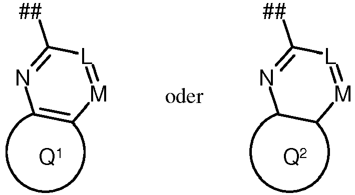 steht, wobei
## für die Anlcnüpfstelle an den Ring P steht,
L für CH oder N steht
M für CR⁴ oder N steht,
worin
R⁴ für Wasserstoff, Halogen, Cyano, (C₁-C₄)-Alkyl, (C₂-C₄)-Alkinyl, Hydroxy, (C₁-C₄)-Alkoxy, (C₁-C₄)-Alkylcarbonyl, (C₁-C₄)-Alkoxy-carbonyl, Amino, Mono-(C₁-C₄)-alkylamino, Di-(C₁-C₄)-Alkylamino oder Azetidinyl steht,
worin (C₁-C₄)-Alkyl, (C₂-C₄)-Alkinyl, (C₁-C₄)-Alkoxy, Mono-(C₁-C₄)-alkylamino und Di-(C₁-C₄)-Alkylamino ihrerseits mit 1 oder 2 Substituenten unabhängig voneinander ausgewählt aus der Gruppe Fluor, Hydroxy und Amino substituiert sein können,
der Ring Q¹ für 5- bis 7-gliedriges Heterocyclyl, Phenyl oder 5- oder 6-gliedriges Heteroaryl steht,
worin 5- bis 7-gliedriges Heterocyclyl, Phenyl und 5- oder 6-gliedriges Heteroaryl mit 1 bis 3 Substituenten unabhängig voneinander ausgewählt aus der Gruppe Halogen, Difluormethyl, Trifluormethyl, Trideuteromethyl, (C₁-C₆)-Alkyl, (C₂-C₆)-Alkenyl, (C₃-C₇)-Cycloalkyl, Oxo, Hydroxy, (C₁-C₄)-Alkoxy, (C₁-C₄)-Alkoxycarbonyl, (C₃-C₇)-Cycloalkoxycarbonyl, Amino, Mono-(C₁-C₄)-alkylamino, Di-(C₁-C₄)-Alkylamino, Thiooxo, (C₁-C₄)-Alkylthio, Aminosulfonyl, Mono-(C₁-C₄)-alkylaminosulfonyl, Di-(C₁-C₄)-alkylaminosulfonyl, 4- bis 7-gliedriges Heterocyclyl, Phenyl und Benzyl substituiert sein können,
worin (C₁-C₄)-Alkyl, (C₃-C₇)-Cycloalkyl, (C₁-C₄)-Alkoxy, (C₁-C₄)-Alkoxycarbonyl und (C₁-C₄)-Alkylthio ihrerseits mit 1 bis 3 Substituenten unabhängig voneinander ausgewählt aus der Gruppe Halogen, Trifluormethyl, (C₃-C₇)-Cycloalkyl, Hydroxy und (C₁-C₄)-Alkoxy substituiert sein können,
worin 4- bis 7-gliedriges Heterocyclyl seinerseits mit 1 oder 2 Substituenten unabhängig voneinander ausgewählt aus der Gruppe Halogen, Trifluormethyl, (C₁-C₄)-Alkyl und (C₃-C₇)-Cycloalkyl substituiert sein kann,
   worin (C₁-C₄)-Alkyl seinerseits mit 1 oder 2 Substituenten unabhängig voneinander ausgewählt aus der Gruppe Fluor, Trifluormethyl, (C₃-C₇)-Cycloalkyl, Hydroxy und (C₁-C₄)-Alkoxy substituiert sein kann,
und
worin Phenyl und Benzyl ihrerseits mit 1 bis 3 Substituenten Halogen, Cyano, Trifluormethyl, (C₁-C₄)-Alkyl, (C₁-C₄)-Alkoxy und (C₁-C₄)-Alkylsulfonyl substituiert sein können,
der Ring Q² für 5-gliedriges Heteroaryl steht,
worin 5-gliedriges Heteroaryl mit 1 bis 3 Substituenten unabhängig voneinander ausgewählt aus der Gruppe Halogen, Difluormethyl, Trifluormethyl, Trideuteromethyl, (C₁-C₆)-Alkyl, (C₂-C₆)-Alkenyl, (C₃-C₇)-Cycloalkyl, Hydroxy, (C₁-C₄)-Alkoxy, (C₁-C₄)-Alkoxycarbonyl, (C₃-C₇)-Cycloalkoxycarbonyl, Amino, Mono-(C₁-C₄)-alkylamino, Di-(C₁-C₄)-Alkylamino, (C₁-C₄)-Alkylthio, Aminosulfonyl, Mono-(C₁-C₄)-alkylaminosulfonyl, Di-(C₁-C₄)-alkylaminosulfonyl, Phenyl oder Benzyl substituiert sein kann,
worin (C₁-C₄)-Alkyl, (C₃-C₇)-Cycloalkyl, (C₁-C₄)-Alkoxy, (C₁-C₄)-Alkoxycarbonyl und (C₁-C₄)-Alkylthio ihrerseits mit 1 bis 3 Substituenten unabhängig voneinander ausgewählt aus der Gruppe Halogen, Trifluormethyl, (C₃-C₇)-Cycloalkyl, Hydroxy und (C₁-C₄)-Alkoxy substituiert sein können,
und
worin Phenyl und Benzyl ihrerseits mit 1 bis 3 Substituenten Halogen, Trifluormethyl, (C₁-C₄)-Alkyl und (C₁-C₄)-Alkoxy substituiert sein können,
sowie ihre *N*-Oxide, Salze, Solvate, Salze der *N*-Oxide und Solvate der *N*-Oxide und Salze,
mit Ausnahme der Verbindungen:
2-[1-(2-Fluorbenzyl)-1H-pyrazolo[3,4-b]pyridin-3-yl]-8-methyl-9H-purin,
2-[1-(2-Fluorbenzyl)-1H-pyrazolo[3,4-b]pyridin-3-yl]-8-methyl-9H-purin-6-amin,
N-Butyl-2-[1-(2-fluorbenzyl)-1H-pyrazolo[3,4-b]pyridin-3-yl]-8-methyl-9H-purin-6-amin.

Bevorzugt sind im Rahmen der vorliegenden Erfindung Verbindungen der Formel (I), in welcher
- der Ring P: für eine Gruppe der Formel 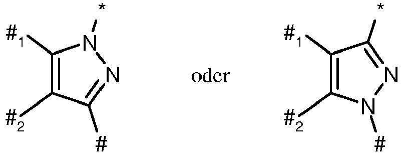 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
- Y: für CH steht,
- R¹: für Wasserstoff oder Fluor steht,
- R²: für 2,2,3,3,3-Pentafluorprop-1-yl oder Benzyl steht,
wobei Benzyl mit 1 oder 2 Substituenten Fluor substituiert ist,
- R³: für eine Gruppe der Formel 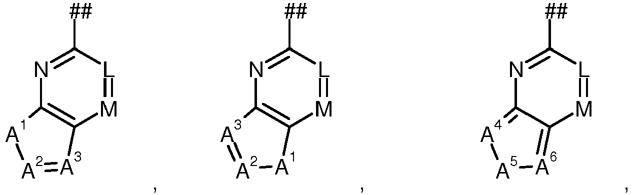 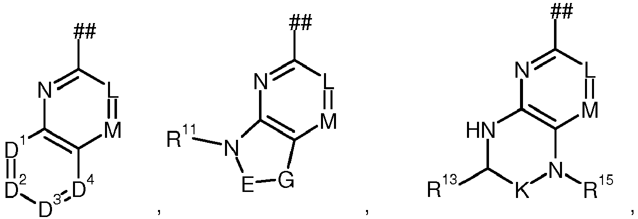 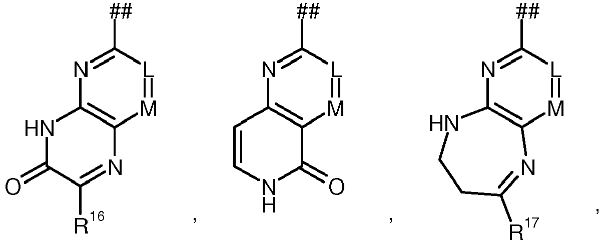 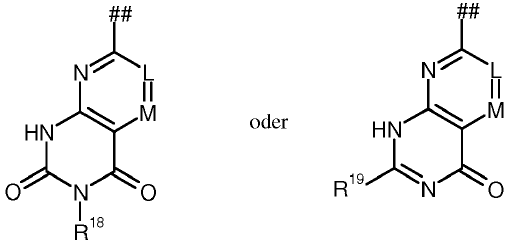 steht, wobei
## für die Anknüpfstelle an den Ring P steht,
L für CH oder N steht
M für CR⁴ oder N steht,
worin
R⁴ für Wasserstoff, Chlor Cyano, (C₁-C₄)-Alkyl, (C₂-C₄)-Alkinyl, Amino, Methylamino, Ethylamino, Dimethylamino, Diethylamino oder Azetidinyl steht,
worin (C₁-C₄)-Alkyl, (C₂-C₄)-Alkinyl, Ethylamino und Diethylamino ihrerseits mit 1 oder 2 Substituenten unanhangig voneinander ausgewählt aus der Gruppe Fluor, Hydroxy und Amino substituiert sein können,
A¹ für O, S oder NR⁵ steht,
worin
R⁵ für Wasserstoff, Trifluormethyl oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
A² für N steht,
A³ für N oder CR⁷ steht,
worin
R⁷ für Wasserstoff, Fluor, Trifluormethyl, (C₁-C₄)-Alkyl, Hydroxy, (C₁-C₄)-Alkoxy, Amino, Methylamino, Ethylamino, Dimethylamino oder Diethylamino steht,
worin (C₁-C₄)-Alkyl und (C₁-C₄)-Alkoxy ihrerseits mit 1 oder 2 Substituenten unabhängig voneinander ausgewählt aus der Gruppe Fluor, Trifluormethyl, Hydroxy, Methoxy und Ethoxy substituiert sein können,

A⁴ und A⁶ unabhängig voneinander jeweils für N oder CR⁸ stehen,
worin
R⁸ für Wasserstoff, Fluor, Chlor, Trifluormethyl, (C₁-C₄)-Alkyl, Hydroxy, (C₁-C₄)-Alkoxy, Methylamino, Ethylamino Dimethylamino oder Diethylamino steht,
worin (C₁-C₄)-Alkyl und (C₁-C₄)-Alkoxy ihrerseits mit 1 oder 2 Substituenten unabhängig voneinander ausgewählt aus der Gruppe Fluor, Trifluormethyl, Hydroxy, Methoxy und Ethoxy substituiert sein können,

A⁵ für NR⁹ steht,
worin
R⁹ für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
D¹, D², D³ und D⁴ unabhängig voneinander jeweils für N oder CR¹⁰ stehen,
worin
R¹⁰ für Wasserstoff, Fluor, Chlor oder Trifluormethyl steht,
mit der Maßgabe, dass maximal zwei der Gruppen D¹, D², D³ und D⁴ für Stickstoff stehen,
und
mit der Maßgabe, dass mindestens eine der Gruppen D¹, D², D³ und D⁴ für CH steht,
- E: für C=O, C=S oder S0₂ steht,
- G: für O oder NR¹² steht,
worin
R¹² für Wasserstoff, Trideuteromethyl, (C₁-C₆)-Alkyl, (C₂-C₆)-Alkenyl, (C₃-C₇)-Cycloalkyl, Azetidinyl, Pyrrolidinyl, Piperidinyl oder Benzyl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Methoxy, Ethoxy, Methylsulfonyl, Ethylsulfonyl, Azetidinyl, Oxetanyl, Pyrrolidinyl, Piperidinyl und Morpholinyl substituiert sein kann,
worin Azetidinyl, Oxetanyl, Pyrrolidinyl, Piperidinyl und Morpholinyl wiederum ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl substituiert sein können,
worin Azetidinyl, Pyrrolidinyl und Piperidinyl ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
worin Methyl und Ethyl wiederum ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
und
worin Benzyl seinerseits mit 1 oder 2 Substituenten Fluor, Chlor, Trifluormethyl, Methyl, Ethyl, Methylsulfonyl und Ethylsulfonyl substituiert sein kann,
K für N oder CR¹⁴ steht,
worin
R¹⁴ für Wasserstoff oder Oxo steht,
R¹¹ für Wasserstoff, Trideuteromethyl, (C₁-C₆)-Alkyl oder (C₂-C₆)-Alkenyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
R¹³ für Wasserstoff oder Oxo steht,
R¹⁵ für Wasserstoff, (C₁-C₃)-Alkoxycarbonyl oder Aminosulfonyl steht,
worin (C₁-C₃)-Alkoxycarbonyl mit 1 oder 2 Substituenten unabhängig voneinander ausgewählt aus der Gruppe Fluor, Trifluormethyl, Hydroxy, Methoxy und Ethoxy substituiert sein können,
R¹⁶ für Wasserstoff, Trifluormethyl, (C₁-C₄)-Alkyl, (C₃-C₆)-Cycloalkyl und Phenyl steht,
worin Phenyl mit 1 oder 2 Substituenten unabhängig voneinander ausgewählt aus der Gruppe Fluor, Chlor, Trifluormethyl, (C₁-C₄)-Alkyl, Methoxy und Ethoxy substituiert sein kann,
R¹⁷ für Wasserstoff, Trifluormethyl, (C₁-C₄)-Alkyl, Cyclopropyl, Cyclobutyl und Phenyl steht,
worin Phenyl mit 1 oder 2 Substituenten unabhängig voneinander ausgewählt aus der Gruppe Fluor, Chlor, Trifluormethyl, Methyl, Ethyl, Methoxy und Ethoxy substituiert sein kann,
R¹⁸ für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
R¹⁹ für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
sowie ihre Salze, Solvate und Solvate der Salze.

Bevorzugt sind im Rahmen der vorliegenden Erfindung Verbindungen der Formel (I), in welcher
- der Ring P: für eine Gruppe der Formel 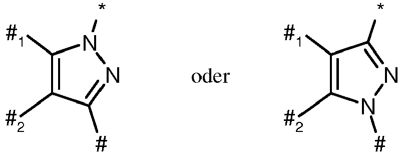 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
- Y: für CH steht,
- R¹: für Wasserstoff oder Fluor steht,
- R²: für 2,2,3,3,3-Pentafluorprop-1-yl oder Benzyl steht,
wobei Benzyl mit 1 oder 2 Substituenten Fluor substituiert ist,
R³ für eine Gruppe der Formel 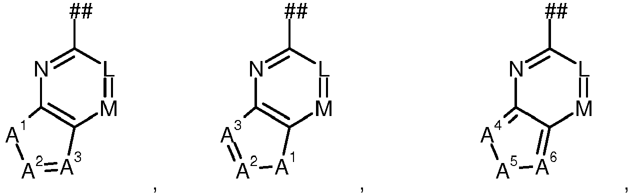 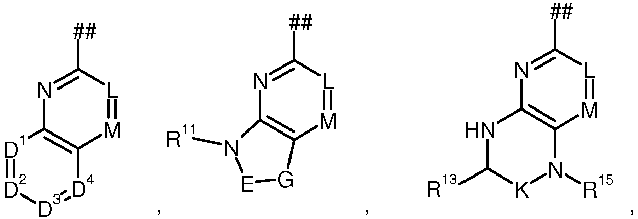 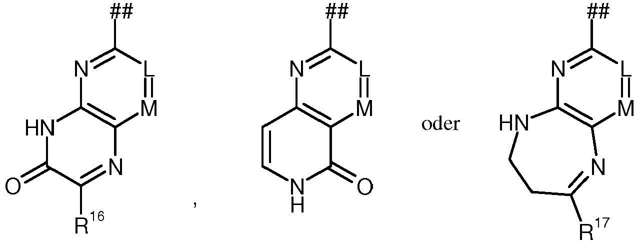 steht, wobei
## für die Anlcnüpfstelle an den Ring P steht,
L für CH oder N steht
M für CR⁴ oder N steht,
worin
R⁴ für Wasserstoff, Chlor Cyano, (C₁-C₄)-Alkyl, (C₂-C₄)-Alkinyl, Amino, Methylamino, Ethylamino, Dimethylamino, Diethylamino oder Azetidinyl steht,
worin (C₁-C₄)-Alkyl, (C₂-C₄)-Alkinyl, Ethylamino und Diethylamino ihrerseits mit 1 oder 2 Substituenten unanhangig voneinander ausgewählt aus der Gruppe Fluor, Hydroxy und Amino substituiert sein können,
A¹ für O, S oder NR⁵ steht,
worin
R⁵ für Wasserstoff, Trifluormethyl oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
A² für N oder CR⁶ steht,
worin
R⁶ für Wasserstoff, Fluor, Chlor, Trifluormethyl, 2,2,2-Trifluorethyl, (C₂-C₄)-Alkyl, Hydroxy, (C₁-C₄)-Alkoxy, Methylamino, Ethylamino, Dimethylamino oder Diethylamino steht,
worin (C₂-C₄)-Alkyl und (C₁-C₄)-Alkoxy ihrerseits mit 1 oder 2 Substituenten unabhängig voneinander ausgewählt aus der Gruppe Fluor, Trifluormethyl, Hydroxy, Methoxy und Ethoxy substituiert sein können,

A³ für N oder CR⁷ steht,
worin
R⁷ für Wasserstoff, Fluor, Trifluormethyl, (C₁-C₄)-Alkyl, Hydroxy, (C₁-C₄)-Alkoxy, Amino, Methylamino, Ethylamino Dimethylamino oder Diethylamino steht,
worin (C₁-C₄)-Alkyl und (C₁-C₄)-Alkoxy ihrerseits mit 1 oder 2 Substituenten unabhängig voneinander ausgewählt aus der Gruppe Fluor, Trifluormethyl, Hydroxy, Methoxy und Ethoxy substituiert sein können,

A⁴ und A⁶ unabhängig voneinander jeweils für N oder CR⁸ stehen,
worin
R⁸ für Wasserstoff, Fluor, Chlor, Trifluormethyl, (C₁-C₄)-Alkyl, Hydroxy, (C₁-C₄)-Alkoxy, Methylamino, Ethylamino Dimethylamino oder Diethylamino steht,
worin (C₁-C₄)-Alkyl und (C₁-C₄)-Alkoxy ihrerseits mit 1 oder 2 Substituenten unabhängig voneinander ausgewählt aus der Gruppe Fluor, Trifluormethyl, Hydroxy, Methoxy und Ethoxy substituiert sein können,

A⁵ für NR⁹ steht,
worin
R⁹ für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
D¹, D², D³ und D⁴ unabhängig voneinander jeweils für N oder CR¹⁰ stehen,
worin
R¹⁰ für Wasserstoff, Fluor, Chlor oder Trifluormethyl steht,
mit der Maßgabe, dass maximal zwei der Gruppen D¹, D², D³ und D⁴ für Stickstoff stehen,
- E: für C=O, C=S oder S0₂ steht,
- G: für O oder NR¹² steht,
worin
R¹² für Wasserstoff, Trideuteromethyl, (C₁-C₆)-Alkyl, (C₂-C₆)-Alkenyl, (C₃-C₇)-Cycloalkyl, Azetidinyl, Pyrrolidinyl, Piperidinyl oder Benzyl steht,
worin (C₁-C₆)-Alkyl seinerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
worin Azetidinyl, Pyrrolidinyl und Piperidinyl ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
worin Methyl und Ethyl ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
und
worin Benzyl seinerseits mit 1 oder 2 Substituenten Fluor, Chlor, Trifluormethyl, Methyl, Ethyl, Methylsulfonyl und Ethylsulfonyl substituiert sein kann,
K für N oder CR¹⁴ steht,
worin
R¹⁴ für Wasserstoff oder Oxo steht,
R¹¹ für Wasserstoff, Trideuteromethyl, (C₁-C₆)-Alkyl oder (C₂-C₆)-Alkenyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
R¹³ für Wasserstoff oder Oxo steht,
R¹⁵ für Wasserstoff, (C₁-C₃)-Alkoxycarbonyl oder Aminosulfonyl steht,
worin (C₁-C₃)-Alkoxycarbonyl mit 1 oder 2 Substituenten unabhängig voneinander ausgewählt aus der Gruppe Fluor, Trifluormethyl, Hydroxy, Methoxy und Ethoxy substituiert sein können,
R¹⁶ für Wasserstoff, Trifluormethyl, (C₁-C₄)-Alkyl, (C₃-C₆)-Cycloalkyl und Phenyl steht,
worin Phenyl mit 1 oder 2 Substituenten unabhängig voneinander ausgewählt aus der Gruppe Fluor, Chlor, Trifluormethyl, (C₁-C₄)-Alkyl, Methoxy und Ethoxy substituiert sein kann,
R¹⁷ für Wasserstoff, Trifluormethyl, (C₁-C₄)-Alkyl, Cyclopropyl, Cyclobutyl und Phenyl steht,
worin Phenyl mit 1 oder 2 Substituenten unabhängig voneinander ausgewählt aus der Gruppe Fluor, Chlor, Trifluormethyl, Methyl, Ethyl, Methoxy und Ethoxy substituiert sein kann,
sowie ihre Salze, Solvate und Solvate der Salze.

Besonders bevorzugt sind im Rahmen der vorliegenden Erfindung Verbindungen der Formel (I), in welcher
- der Ring P: für eine Gruppe der Formel 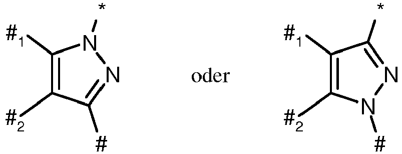 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
- Y: für CH steht,
- R¹: für Wasserstoff oder Fluor steht,
- R²: für 2-Fluorbenzyl steht,
- R³: für eine Gruppe der Formel 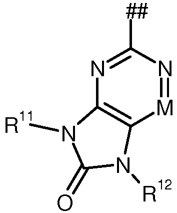 steht, wobei
## für die Anknüpfstelle an den Ring P steht,
M für CR⁴ oder N steht,
wobei
R⁴ für Wasserstoff oder Amino steht,
R¹¹ für Wasserstoff steht,
R¹² für Wasserstoff, Trideuteromethyl, (C₁-C₄)-Alkyl, Cyclopropyl, Cyclobutyl, Azetidinyl, Pyrrolidinyl oder Piperidinyl steht,
worin (C₁-C₄)-Alkyl seinerseits mit 1 oder 2 Substituenten unabhängig voneinander ausgewählt aus der Gruppe Fluor, Trifluormethyl, Cyclopropyl, Cyclobutyl und Hydroxy substituiert sein kann,
und
worin Azetidinyl, Pyrrolidinyl und Piperidinyl ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
sowie ihre Salze, Solvate und Solvate der Salze.

Bevorzugt sind im Rahmen der vorliegenden Erfindung Verbindungen der Formel (I), in welcher
- der Ring P: für eine Gruppe der Formel 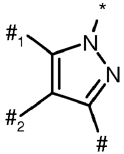 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
- Y: für CH steht,
- R¹: für Wasserstoff oder Fluor steht,
- R²: für 2-Fluorbenzyl steht,
- R³: für eine Gruppe der Formel 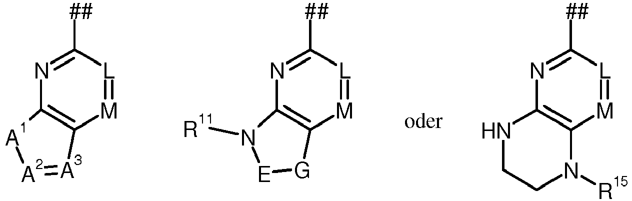 steht, wobei
## für die Anknüpfstelle an den Ring P steht,
L für N oder CH steht,
M für N oder CR⁴ steht,
worin
R⁴ für Wasserstoff oder Amino steht, mit der Maßgabe, dass nur eine der Gruppen L und M für N steht,
A¹ für NR⁵ steht,
worin
R⁵ für Wasserstoff steht,
A² für N steht,
A³ für N oder CR⁷ steht,
worin
R⁷ für Wasserstoff, Fluor, Trifluormethyl, (C₁-C₄)-Alkyl, (C₁-C₄)-Alkoxy, Amino, Methylamino, Ethylamino, Dimethylamino oder Diethylamino steht,
worin (C₁-C₄)-Alkyl und (C₁-C₄)-Alkoxy ihrerseits mit 1 oder 2 Substituenten unabhängig voneinander ausgewählt aus der Gruppe Fluor, Trifluormethyl, Hydroxy, Methoxy und Ethoxy substituiert sein können,

E für C=O steht,
G für NR¹² steht,
worin
R¹² für Trideuteromethyl, (C₁-C₆)-Alkyl, Cyclopropyl, Cyclobutyl, Azetidin-3-yl, Pyrrolidin-3-yl oder Piperidin-4-yl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-1-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl, Cyclopropyl und Cyclobutyl substituiert sind,
R¹¹ für Wasserstoff steht
R¹⁵ für Wasserstoff oder (C₁-C₃)-Alkoxycarbonyl steht,
worin (C₁-C₃)-Alkoxycarbonyl mit 1 oder 2 Substituenten unabhängig voneinander ausgewählt aus der Gruppe Fluor, Trifluormethyl, Hydroxy, Methoxy und Ethoxy substituiert sein können,
sowie ihre Salze, Solvate und Solvate der Salze.

Besonders bevorzugt sind im Rahmen der vorliegenden Erfindung auch Verbindungen der Formel (I), in welcher
- der Ring P: für eine Gruppe der Formel 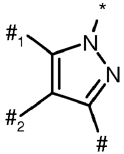 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
- Y: für CH steht,
- R¹: für Wasserstoff oder Fluor steht,
- R²: für 2-Fluorbenzyl steht,
- R³: für eine Gruppe der Formel 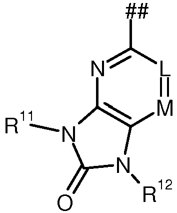 steht, wobei
## für die Anknüpfstelle an den Ring P steht,
L für CH oder N steht,
M für N oder CR⁴ steht,
wobei
R⁴ für Wasserstoff oder Amino steht, mit der Maßgabe, dass nur eine der Gruppen L und M für N steht,
R¹¹ für Wasserstoff steht,
R¹² für Trideuteromethyl, (C₁-C₆)-Alkyl, Cyclopropyl, Cyclobutyl, Azetidin-3-yl, Pyrrolidin-3-yl oder Piperidin-4-yl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-1-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl, Cyclopropyl und Cyclobutyl substituiert sind,
sowie ihre Salze, Solvate und Solvate der Salze.

Bevorzugt sind im Rahmen der vorliegenden Erfindung auch Verbindungen der Formel (I), in welcher
- der Ring P: für eine Gruppe der Formel 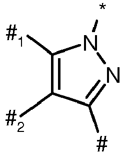 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
- Y: für CH steht,
- R¹: für Wasserstoff oder Fluor steht,
- R²: für 2-Fluorbenzyl steht,
- R³: für eine Gruppe der Formel 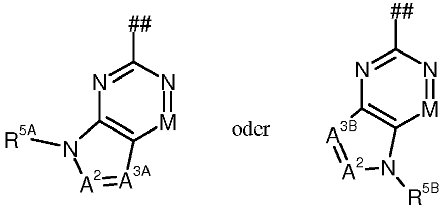 steht, wobei
## für die Anknüpfstelle an den Ring P steht,
M für CR⁴ oder N steht,
wobei
R⁴ für Wasserstoff oder Amino steht,
A² für N oder CR⁶ steht,
worin
R⁶ für Wasserstoff, Trifluormethyl oder 2,2,2-Trifluorethyl steht,
A^{3A} für N oder CR^{7A} steht,
worin
R^{7A} für Wasserstoff, Fluor, Trifluormethyl, 2,2,2-Trifluorethyl, (C₂-C₄)-Alkyl Hydroxy, (C₁-C₄)-Alkoxy, Amino, Methylamino, Ethylamino, Dimethyl-amino oder Diethylamino steht,
worin (C₂-C₄)-Alkyl und (C₁-C₄)-Alkoxy ihrerseits mit einem Substituenten ausgewählt aus der Gruppe Hydroxy und Methoxy substituiert sein können,

A^{3B} für N oder CH steht, mit der Maßgabe, dass mindestens eine der Gruppen A² und A^{3A} bzw. A^{3B} für N steht,
R^{5A} für Wasserstoff steht,
R^{5B} für Wasserstoff, 2,2,2-Trifluorethyl oder Methyl steht,
steht, sowie ihre Salze, Solvate und Solvate der Salze.

Bevorzugt sind im Rahmen der vorliegenden Erfindung auch Verbindungen der Formel (I), in welcher Y für CH steht, sowie ihre Salze, Solvate und Solvate der Salze.

Bevorzugt sind im Rahmen der vorliegenden Erfindung auch Verbindungen der Formel (I), in welcher R² für 2-Fluorbenzyl steht, sowie ihre Salze, Solvate und Solvate der Salze.

Bevorzugt sind im Rahmen der vorliegenden Erfindung auch Verbindungen der Formel (I), in welcher
- L: für N steht
und
- M: für N steht,
sowie ihre Salze, Solvate und Solvate der Salze.

Bevorzugt sind im Rahmen der vorliegenden Erfindung auch Verbindungen der Formel (I), in welcher
- L: für N steht
und
- M: für CH steht,
sowie ihre Salze, Solvate und Solvate der Salze.

Bevorzugt sind im Rahmen der vorliegenden Erfindung auch Verbindungen der Formel (I), in welcher
- L: für N steht
und
- M: für Cm-1 steht,
wobei
R⁴ für Amino steht,
sowie ihre Salze, Solvate und Solvate der Salze.

Bevorzugt sind im Rahmen der vorliegenden Erfindung auch Verbindungen der Formel (I), in welcher L für N steht, sowie ihre Salze, Solvate und Solvate der Salze.

Bevorzugt sind im Rahmen der vorliegenden Erfindung auch Verbindungen der Formel (I), in welcher M für N steht, sowie ihre Salze, Solvate und Solvate der Salze.

Bevorzugt sind im Rahmen der vorliegenden Erfindung auch Verbindungen der Formel (I), in welcher M für CH steht, sowie ihre Salze, Solvate und Solvate der Salze.

Bevorzugt sind im Rahmen der vorliegenden Erfindung auch Verbindungen der Formel (I), in welcher
- M: für CR⁴ steht,
wobei
R⁴ für Wasserstoff oder Amino steht,
sowie ihre Salze, Solvate und Solvate der Salze.

Bevorzugt sind im Rahmen der vorliegenden Erfindung auch Verbindungen der Formel (I), in welcher
- L: für CH oder N steht,
- M: für N oder CR⁴ steht,
wobei
- R⁴: für Wasserstoff oder Amino steht,
mit der Maßgabe, dass nur eine der Gruppen L und M für N steht,
sowie ihre Salze, Solvate und Solvate der Salze.

Bevorzugt sind im Rahmen der vorliegenden Erfindung auch Verbindungen der Formel (I), in welcher
- R³: für eine Gruppe der Formel 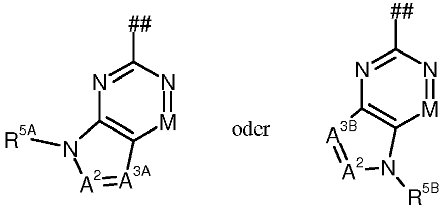 steht, wobei
## für die Anknüpfstelle an den Ring P steht,
M für CR⁴ oder N steht,
wobei
R⁴ für Wasserstoff oder Amino steht,
A² für N oder CR⁶ steht,
worin
R⁶ für Wasserstoff, Trifluormethyl, 2,2,2-Trifluorethyl oder Methyl steht,
A^{3A} für N oder CR^{7A} steht,
worin
R^{7A} für Wasserstoff, Fluor, Trifluormethyl, 2,2,2-Trifluorethyl, (C₂-C₄)-Alkyl Hydroxy, (C₁-C₄)-Alkoxy, Amino, Methylamino, Ethylamino, Dimethylamino oder Diethylamino steht,
worin (C₂-C₄)-Alkyl und (C₁-C₄)-Alkoxy ihrerseits mit einem Substituenten ausgewählt aus der Gruppe Hydroxy und Methoxy substituiert sein können,

A^{3B} für N oder CH steht,
mit der Maßgabe, dass mindestens eine der Gruppen A² und A^{3A} bzw. A^{3B} für N steht,
R^{5A} für Wasserstoff steht,
R^{5B} für Wasserstoff, 2,2,2-Trifluorethyl oder Methyl steht,
steht, sowie ihre Salze, Solvate und Solvate der Salze.

Bevorzugt sind im Rahmen der vorliegenden Erfindung auch Verbindungen der Formel (I), in welcher
- R³: für eine Gruppe der Formel 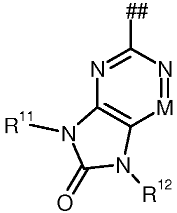 steht, wobei
## für die Anknüpfstelle an den Ring P steht,
M für CR⁴ oder N steht,
wobei
R⁴ für Wasserstoff oder Amino steht,
R¹¹ für Wasserstoff steht,
R¹² für Trideuteromethyl, (C₁-C₄)-Alkyl, Cyclopropyl, Cyclobutyl, Azetidin-3-yl, Pyrrolidin-3-yl oder Piperidin-4-yl steht,
worin (C₁-C₄)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-1-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl, Cyclopropyl und Cyclobutyl substituiert sind,
sowie ihre Salze, Solvate und Solvate der Salze.

Bevorzugt sind im Rahmen der vorliegenden Erfindung auch Verbindungen der Formel (I), in welcher
- der Ring P: für eine Gruppe der Formel 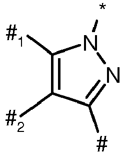 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
sowie ihre Salze, Solvate und Solvate der Salze.

Bevorzugt sind im Rahmen der vorliegenden Erfindung auch Verbindungen der Formel (I), in welcher
- der Ring P: für eine Gruppe der Formel 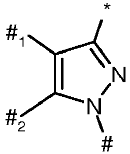 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II-1) bzw. (II-2) 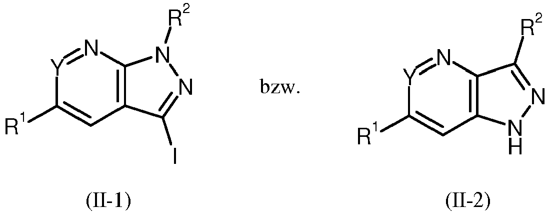 in welchen Y, R¹ und R² jeweils die oben angegebenen Bedeutungen haben,
   in einem inerten Lösungsmittel in Gegenwart eines geeigneten Übergangsmetall-Katalysators mit einer Verbindung der Formel (III)

   R³-X¹ (III),

   in welcher R³ die oben angegebene Bedeutung hat und
   - X¹: für eine geeignete Abgangsgruppe wie beispielsweise Halogen, Mesylat, Tosylat oder Triflat steht,
   zu einer Verbindung der Formel (I-A-1) bzw. (I-A-2) 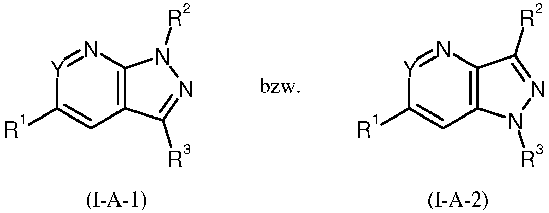 in welchen Y, R¹ und R² jeweils die oben angegebenen Bedeutungen haben,
   umsetzt,
   oder
[B] eine Verbindung der Formel (IV) 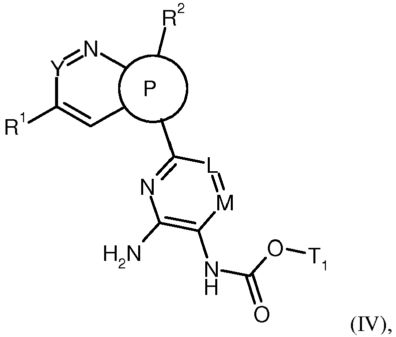 in welcher L, M, P, Y, R¹ und R² jeweils die oben angegebenen Bedeutungen haben und
   - T¹: für (C₁-C₄)-Alkyl steht,
   in einem inerten Lösungsmittel in Gegenwart einer geeigneten Base zu einer Verbindung der Formel (I-B) 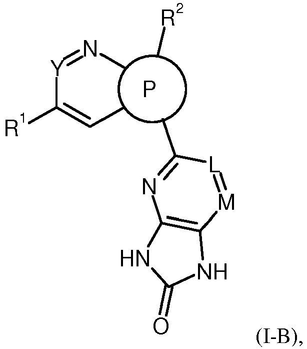 in welcher L, M, P, Y, R¹ und R² jeweils die oben angegebenen Bedeutungen haben,
   umsetzt,
   oder
[C] eine Verbindung der Formel (IV) zunächst in einem inerten Lösungsmittel in Gegenwart einer geeigneten Base mit einer Verbindung der Formel (V)

   R^{12A}-X² (V),

   in welcher
   - R^{12A}: für Trideuteromethyl, (C₁-C₆)-Alkyl, (C₂-C₆)-Alkenyl, (C₃-C₇)-Cycloalkyl, Azetidinyl, Pyrrolidinyl, Piperidinyl oder Benzyl steht,
   worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Methoxy, Ethoxy, Methylsulfonyl, Ethylsulfonyl, Azetidinyl, Oxetanyl, Pyrrolidinyl, Piperidinyl und Morpholinyl substituiert sein kann,
   worin Azetidinyl, Oxetanyl, Pyrrolidinyl, Piperidinyl und Morpholinyl wiederum ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl substituiert sein können,
   worin Azetidinyl, Pyrrolidinyl und Piperidinyl ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
   worin Methyl und Ethyl wiederum ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
   und
   worin Benzyl seinerseits mit 1 oder 2 Substituenten Fluor, Chlor, Trifluormethyl, Methyl, Ethyl, Methylsulfonyl und Ethylsulfonyl substituiert sein kann,
   und
   - x²: für eine geeignete Abgangsgruppe, wie beispielsweise Halogen, insbesondere Chlor oder Brom, Meyslat oder Tosylat steht
   zu einer Verbindung der Formel (VI) 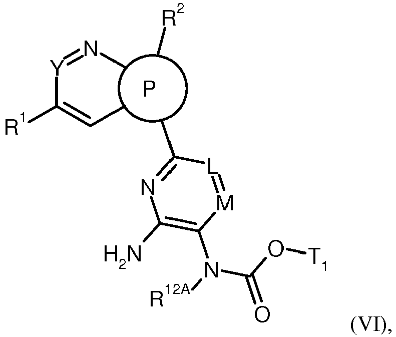 in welcher L, M, P, Y, R¹, R², R^{12A} und T¹ jeweils die oben angegebenen Bedeutungen haben,
   umsetzt, und diese anschliessend in einem inerten Lösungsmittel in Gegenwart einer geeigneten Base zu einer Verbindung der Formel (I-C) 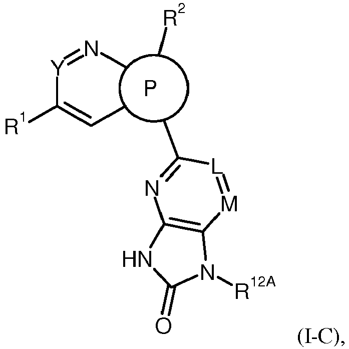 in welcher L, M, P, Y, R¹, R² und R^{12A} jeweils die oben angegebenen Bedeutungen haben,
   cyclisiert,
   oder
[D] eine Verbindung der Formel (VII) 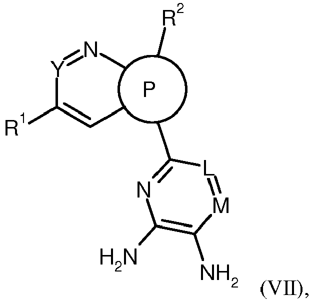 in welcher L, M, P, Y, R¹ und R² jeweils die oben angegebenen Bedeutungen haben,
   mit einer Verbindung der Formel (VIII) 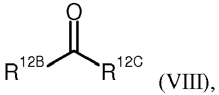 in welcher
   - R^{12B}: für Trifluormethyl, (C₁-C₅)-Alkyl, (C₂-C₆)-Alkenyl, Cyclopropyl, Cyclobutyl oder Phenyl steht,
   worin (C₁-C₅)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
   und
   worin Phenyl seinerseits mit 1 oder 2 Substituenten Fluor, Chlor, Trifluormethyl, Methyl, Ethyl, Methylsulfonyl und Ethylsulfonyl substituiert sein kann,
   - R^{12C}: für Wasserstoff oder (C₁-C₅)-Alkyl steht,
   worin (C₁-C₅)-Alkyl seinerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
   oder worin
   - R^{12B} und R^{12C}: zusammen mit dem Kohlenstoffatom, an das sie gebunden sind einen Cyclobutyl-, Azetidinyl-, Pyrrolidinyl- oder Piperidinyl-Ring bilden,
   worin der Azetidinyl-, Pyrrolidinyl- und Piperidinyl-Ring ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
   worin Methyl und Ethyl wiederum ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
   reduktiv zu einer Verbindung der Formel (IX) 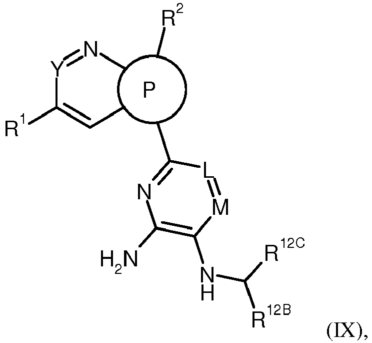 in welcher L, M, P, Y, R¹, R², R^{12B} und R^{12C} jeweils die oben angegebenen Bedeutungen haben,
   aminiert, und diese in einem inerten Lösungsmittel in Gegenwart einer geeigneten Base mit Phosgen, einem Phosgen-Derivat oder einem Phosgen-Äquivalent zu einer Verbindung der Formel (I-D) 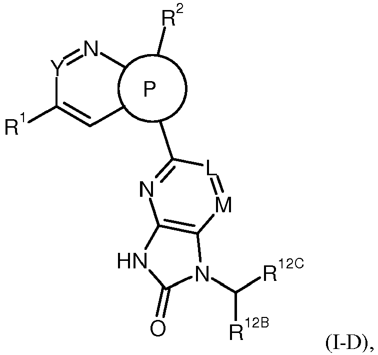 in welcher L, M, P, Y, R¹, R², R^{12B} und R^{12C} jeweils die oben angegebenen Bedeutungen haben,
   cyclisiert,
   oder
[E] eine Verbindung der Formel (X) 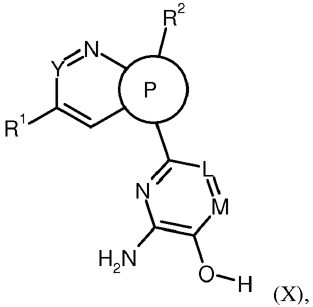 in welcher L, M, P, Y, R¹ und R² jeweils die oben angegebenen Bedeutungen haben,
   in einem inerten Lösungsmittel in Gegenwart einer geeigneten Base mit Phosgen, einem Phosgen-Derivat oder einem Phosgen-Äquivalent zu einer Verbindung der Formel (I-E) 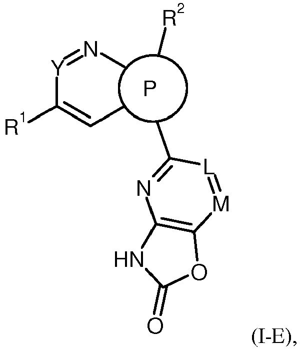 in welcher L, M, P, Y, R¹ und R² jeweils die oben angegebenen Bedeutungen haben,
   cyclisiert,
   oder
[F] eine Verbindung der Formel (VII) in einem inerten Lösungsmittel unter sauren Bedingungen mit einem geeigneten Nitrit zu einer Verbindung der Formel (I-F) 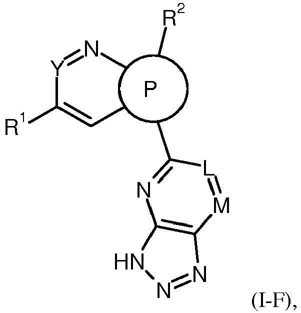 in welcher L, M, P, Y, R¹ und R² jeweils die oben angegebenen Bedeutungen haben,
   umsetzt,
   oder
[G] eine Verbindung der Formel (VII) in einem inerten Lösungsmittel mit einer Verbindung der Formel (XI)

   R^{5C}-X³ (XI),

   in welcher
   - R^{5C}: für Trifluormethyl oder (C₁-C₄)-Alkyl steht,
   worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
   und
   - X³: für eine geeignete Abgangsgruppe, wie beispielsweise Halogen, insbesondere Chlor oder Brom, Meyslat oder Tosylat steht,
   zu einer Verbindung der Formel (XII) 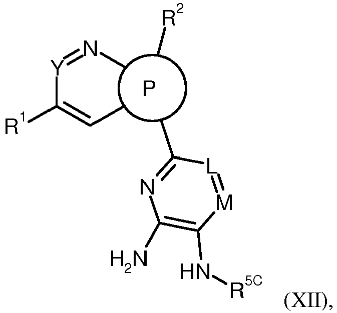 in welcher L, M, P, Y, R¹, R² und R^{5C} jeweils die oben angegebenen Bedeutungen haben,
   umsetzt, und diese anschliessend in einem inerten Lösungsmittel mit einer Verbindung der Formel (XIII) 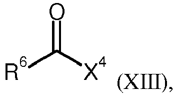 in welcher R⁶ die oben angegebene Bedeutung hat und
   - X⁴: für Chlor, Hydroxy, (C₁-C₄)-Alkoxycarbonyl oder eine Gruppe der Formel O(C=O)R⁶ steht,
   zu einer Verbindung der Formel (I-G) 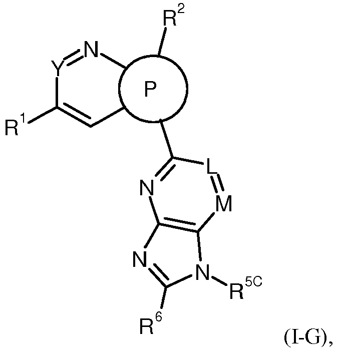 in welcher L, M, P, Y, R¹, R², R^{5C} und R⁶ jeweils die oben angegebenen Bedeutungen haben,
   cyclisiert,
   oder
[H] eine Verbindung der Formel (XIV) 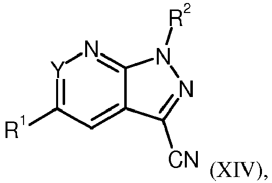 in welcher Y, R¹ und R² jeweils die oben angegebenen Bedeutungen haben,
   in einem inerten Lösungsmittel in Gegenwart einer geeigneten Base mit einer Verbindung der Formel (XV-1) bzw. (XV-2) 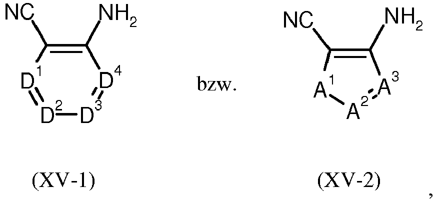 in welcher D¹, D², D³, D⁴, A¹, A² und A³ jeweils die oben angegebenen Bedeutungen haben,
   zu einer Verbindung der Formel (I-H-1) bzw- (I-H-2) 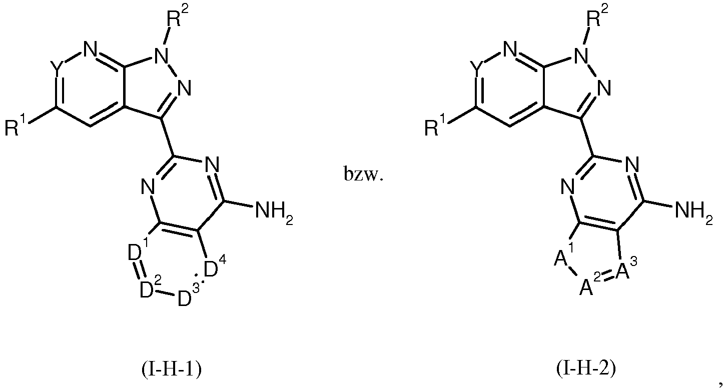 in welcher Y, R¹, R², D¹, D², D³, D⁴, A¹, A² und A³ jeweils die oben angegebenen Bedeutungen haben,
   umsetzt,
   oder
[I] eine Verbindung der Formel (I-I-1) oder (I-I-2) 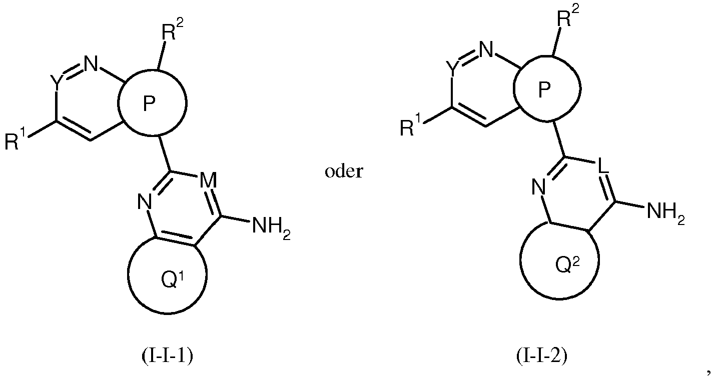 in welcher M, P, Y, R¹, R², Q¹ und Q² jeweils die oben angegebenen Bedeutungen haben,
   in einem inerten Lösungsmittel mit einem geeigneten Nitrit zu einer Verbindung der Formel (I-I-3) oder (I-I-4) 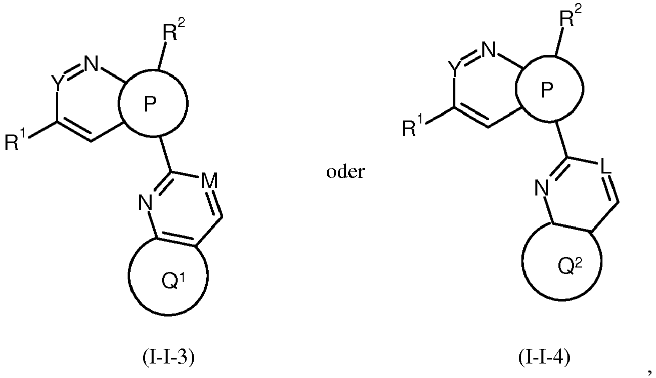 in welcher M, P, Y, R¹, R², Q¹ und Q² jeweils die oben angegebenen Bedeutungen haben,
   umsetzt,
   oder
[J] eine Verbindung der Formel (I-I-1) oder (1-1-2) in einem inerten Lösungsmittel mit Isopentylnitrit und Diiodmethan zu einer Verbindung der Formel (I-J-1) oder (I-J-2) 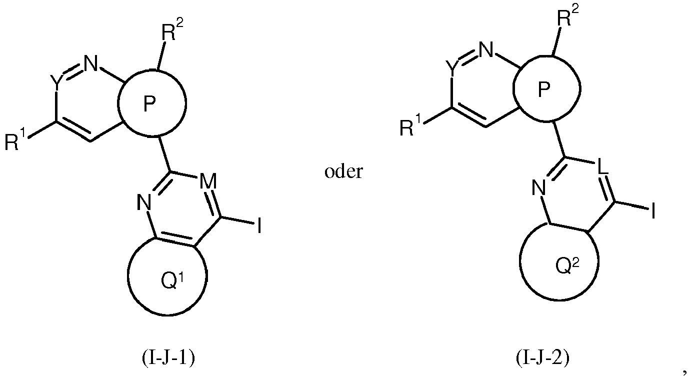 in welcher M, P, Y, R¹, R², Q¹ und Q² jeweils die oben angegebenen Bedeutungen haben,
   oder
[K] eine Verbindung der Formel (I-J-1) oder (I-J-2) in einem inerten Lösungsmittel mit einer Verbindung der Formel (XVI)

   R^{4A}-X⁵ (XVI),

   in welcher
   - R^{4A}: für Cyano, (C₁-C₄)-Alkyl, (C₂-C₄)-Alkinyl, Hydroxy, (C₁-C₄)-Alkoxy, (C₁-C₄)-Alkylcarbonyl, (C₁-C₄)-Alkoxycarbonyl, Amino, Mono-(C₁-C₄)-alkylamino, Di-(C₁-C₄)-Alkylamino oder Azetidinyl steht,
   worin (C₁-C₄)-Alkyl, (C₂-C₄)-Alkinyl, (C₁-C₄)-Alkoxy, Mono-(C₁-C₄)-alkylamino und Di-(C₁-C₄)-Alkylamino ihrerseits mit 1 oder 2 Substituenten unanhangig voneinander ausgewählt aus der Gruppe Fluor, Hydroxy und Amino substituiert sein können,
   und
   - X⁵: für Wasserstoff, Halogen, Tosylat, Mesylat, oder ein geeignetes Kation steht,
   zu einer Verbindung der Formel (I-K-1) oder (I-K-2) 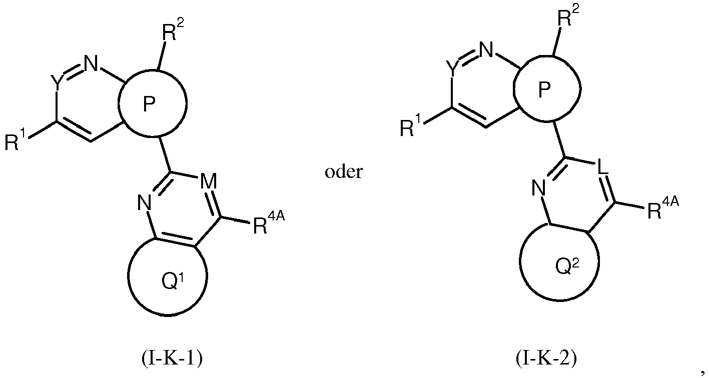 in welcher M, P, Y, R¹, R², R^{4A}, Q¹ und Q² jeweils die oben angegebenen Bedeutungen haben,
   umsetzt,
und gegebenenfalls vorhandene Schutzgruppen nach den dem Fachmann bekannten Methoden abspaltet, und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Inerte Lösungsmittel für den Verfahrensschritt (II-1) bzw. (II-2) + (III) → (I-A-1) bzw. (I-A-2) sind beispielsweise Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), Dimethylacetamid, *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist Dioxan.

Die Umsetzung (II-1) + (III) → (I-A-1) erfolgt in Gegenwart von Hexabutylzinn und einem geeigneten Palladiumkatalysator unter intermediärer Bildung einer Zinnspezies.

Als Palladium-Katalysator für den Verfahrensschritt (II-1) + (III) → (I-A-1) ist beispielsweise Palladium auf Aktivkohle, Palladium(II)-acetat, Tetrakis-(triphenylphosphin)-palladium(0), Bis-(triphenylphosphin)-palladium(II)-chlorid, Bis-(acetonitril)-palladium(II)-chlorid und [1,1'-Bis(diphenylphosphino)ferrocen]dichlorpalladium(II)-Dichlormethan-Komplex, gegebenenfalls in Verbindung mit zusätzlichen Phosphanliganden wie beispielsweise (2-Biphenyl)di-*tert.-*butylphosphin, Dicyclohexyl[2',4',6'-tris(1-methylethyl)biphenyl-2-yl]phosphan (XPHOS), Bis(2-phenylphosphinophenyl)ether (DPEphos) or 4,5-Bis(diphenylphosphino)-9,9-dimethylxanthen (Xantphos) [vgl. z.B. Hassan J. et al., Chem. Rev. 102, 1359-1469 (2002)] geeignet.

Die Reaktion (II-1) + (III) →(I-A-1) wird im Allgemeinen in einem Temperaturbereich von +20°C bis +180°C, bevorzugt bei +50°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Umsetzung (II-2) + (III) →(I-A-2) erfolgt in Gegenwart einer geeigneten Base. Geeignete Basen für diese Umsetzung sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N*-Methylmorpholin, *N*-Methylpiperidin, *N,N-*Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Natriumhydrid oder Cäsiumcarbonat verwendet.

Die Reaktion (II-2) + (III) →(I-A-2) wird im Allgemeinen in einem Temperaturbereich von 0°C bis +80°C, bevorzugt bei +10°C bis +40°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Gegebenenfalls kann die Umsetzung (II-2) + (III) →(I-A-2) in Gegenwart eines geeigneten Palladium- oder Kupferkatalysators erfolgen. Als Palladium-Katalysator ist beispielsweise Palladium auf Aktivkohle, Palladium(II)-acetat, Tetrakis-(triphenylphosphin)-palladium(0), Bis-(triphenylphosphin)-palladium(II)-chlorid, Bis-(acetonitril)-palladium(II)-chlorid und [1,1'-Bis(diphenylphosphino)ferrocen]dichlorpalladium(II)-Dichlormethan-Komplex, gegebenenfalls in Verbindung mit zusätzlichen Phosphanliganden wie beispielsweise (2-Biphenyl)di-*tert.-*butylphosphin, Dicyclohexyl[2',4',6'-tris(1-methylethyl)biphenyl-2-yl]phosphan (XPHOS), Bis(2-phenylphosphinophenyl)ether (DPEphos) or 4,5-Bis(diphenylphosphino)-9,9-dimethylxanthen (Xantphos) [vgl. z.B. Hassan J. et al., Chem. Rev. 102, 1359-1469 (2002)] geeignet. Als Kupfer-Katalysatoren eignen sich beispielsweise Kupferbronze, Kupfer-(I)-iodid oder Kupfer-(I)-bromid.

Die Reaktion (II-2) + (III) → (I-A-2) wird im Allgemeinen in einem Temperaturbereich von +20°C bis +180°C, bevorzugt bei +50°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Als inerte Lösungsmittel für die Cyclisierungen (IV) → (I-B) und (VI) → (I-C) eignen sich Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), Dimethylacetamid, *N*-Methylpyrrolidon (NMP), Pyridin, Acetonitril, oder Sulfolan. Ebenso ist es möglich, Gemische der genannten Lösungsmittel einzusetzen. Bevorzugt ist Tetrahydrofuran.

Geeignete Basen für die Cyclisierungen (IV) → (I-B) und (VI) → (I-C) sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N*-Methylmorpholin, *N*-Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Natrium-bis(trimethylsilyl)amid verwendet.

Die Reaktionen (IV) → (I-B) und (VI) → (I-C) werden im Allgemeinen in einem Temperaturbereich von -10°C bis +80°C, bevorzugt bei +10°C bis +30°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzungen (IV) + (V) → (VI) und (VII) + (XI) → (XII) sind Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), Dimethylacetamid, *N*-Methylpyrrolidon (NMP), Pyridin, Acetonitril, oder Sulfolan. Ebenso ist es möglich, Gemische der genannten Lösungsmittel einzusetzen. Bevorzugt ist Tetrahydrofuran.

Geeignete Basen für die Umsetzungen (IV) + (V) → (VI) und (VII) + (XI) → (XII) sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N*-Methylmorpholin, *N*-Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Natriumhydrid verwendet.

Die Reaktionen (IV) + (V) → (VI) und (VII) + (XI) → (XII) werden im Allgemeinen in einem Temperaturbereich von -10°C bis +80°C, bevorzugt bei +10°C bis +30°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Umsetzungen (IX) → (I-D) und (X) → (I-E) erfolgen mit Phosgen, einem Phosgen-Derivat wie Di- oder Triphosgen, oder einem Phosgen Äquivalent wie beispielsweise *N,N*-Carbonyldiimidazol oder einem Chlorameisensäureester.

Inerte Lösungsmittel für die Verfahrensschritte (IX) → (I-D) und (X) → (I-E) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N*-Dimethylformamid, Dimethylsulfoxid, *N,N'-*Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird DMF verwendet.

Als Basen für die Verfahrensschritte (IX) → (I-D) und (X) → (I-E) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N*-Methylmorpholin, *N*-Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Triethylamin verwendet.

Die Verfahrensschritte (IX) → (I-D) und (X) → (I-E) werden im Allgemeinen in einem Temperaturbereich von -10°C bis +50°C, bevorzugt bei 0°C bis +30°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die reduktive Aminierung (VII) + (VIII) → (IX) erfolgt in Gegenwart von Alkaliborhydriden, wie zum Beispiel Natriumtriacetoxyborhydrid, Natriumcyanoborhydrid oder Natriumborhydrid, unter Verwendung katalytischer Mengen einer geeigneten Säure wie beispielsweise Ameisensäure oder Essigsäure.

Inerte Lösungsmittel, für die Umsetzung (VII) + (VIII) → (IX) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), Dimethylacetamid, *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist Dichlorethan oder Methanol.

Die reduktive Aminierung (VII) + (VIII) → (IX) wird im Allgemeinen bei Temperaturen zwischen 0°C und +100°C, bevorzugt bei +10°C bis +40°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Geeignete Nitrite für die Umsetzungen (VII) → (I-F) und (I-I-1) bzw. (I-I-2) → (I-I-3) bzw. (I-I-4) eind beispielsweise Natriumnitrit, Isopentylnitrit oder tert.-Butylnitrit.

Inerte Lösungsmittel für die Umsetzungen (VII) → (I-F) und (I-I-1) bzw. (I-I-2) → (I-I-3) bzw. (I-I-4) sind beispielsweise Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Tetrahydrofuran oder DMF.

Die Reaktionen (VII) → (I-F) und *(I-I-1) bzw. (I-I-2) → (I-I-3) bzw. (I-I-4) werden im Allgemeinen in einem Temperaturbereich von 0°C bis +120°C, bevorzugt bei +40°C bis +80°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Als inerte Lösungsmittel für den Verfahrensschritt (XII) + (XIII) → (I-G) bei der Umsetzung von Carbonsäureanhydriden eignen sich Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N*,*N*-Dimethylformamid, *N*,*N*'-Dimethylpropylenharnstoff (DMPU) oder *N*-Methylpyrrolidon (NMP).

Die Umsetzung von Carbonsäureanhydriden im Verfahrensschritt (XII) + (XIII) → (I-G) erfolgt in Gegenwart einer geeignten Base wie beispielsweise organische Amine wie Triethylamin, *N*-Methylmorpholin, *N*-Methylpiperidin, *N*,*N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Triethylamin verwendet.

Der Verfahrensschritt (XII) + (XIII) → (I-G) mit Carbonsäureanhydriden wird im Allgemeinen in einem Temperaturbereich von +20°C bis +120°C, bevorzugt bei +50°C bis +80°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (XIV) + (XV-1) → (I-H-1) bzw. (XIV) + (XV-2) → (I-H-2) sind beispielsweise Halogenkohlenwasserstoffe wie Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N*-Dimethylformamid (DMF), Dimethylsulfoxid (DMSO), *N*,*N*'-Dimethylpropylenharnstoff (DMPU), *N*-Methylpyrrolidon (NMP), Sulfolan oder Pyridin. Ebenso ist es möglich, Gemische der genannten Lösungsmittel einzusetzen. Bevorzugt wird DMF verwendet.

Als Basen für den Verfahrensschritt (XIV) + (XV-1) → (I-H-1) bzw. (XIV) + (XV-2) → (I-H-2) eignen sich die üblichen anorganischen oder organischen Basen. Hierzu gehören bevorzugt AlkaliAlkoholate wie Natrium- oder Kaliummethanolat, Natrium- oder Kaliumethanolat oder Natrium-oder Kalium-tert.-butylat, Alkalihydride wie Natrium- oder Kaliumhydrid oder Amide wie Natriumamid, Lithium- oder Kalium-bis(trimethylsilyl)amid oder Lithiumdiisopropylamid, Bevorzugt wird Kalium-tert.-butylat verwendet.

Der Verfahrensschritt (XIV) + (XV-1) → (I-H-1) bzw. (XIV) + (XV-2) → (I-H-2) wird im Allgemeinen in einem Temperaturbereich von +100°C bis +200°C, bevorzugt bei +140°C bis +180°C durchgeführt, gegebenenfalls in einer Mikrowelle. Die Umsetzung kann bei normalem, erhöhtem oder bei erniedrigtem Druck erfolgen (z.B. von 0.5 bis 5 bar). Im Allgemeinen arbeitet man bei erhöhtem Druck oder Normaldruck.

Der Verfahrensschritt (I-I-1) → (I-J-1) bzw. (I-I-2) → (I-J-2) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (I-I-1) → (I-J-1) bzw. (I-I-2) → (I-J-2) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (I-I-1) → (I-J-1) bzw. (I-I-2) → (I-J-2) erfolgt im Allgemeinen mit einem Molverhältnis von 10 bis 30 Mol Isopentylnitrit und 10 bis 30 Mol des Iod-Äquivalents bezogen auf 1 Mol der Verbindung der Formel (IV).

Als Iod-Quelle bei der Umsetzung (I-I-1) → (I-J-1) bzw. (I-I-2) → (I-J-2) eignen sich beispielsweise Diiodmethan oder eine Mischung aus Cäsiumiodid, Iod und Kupfer-(I)-iodid.

Inerte Lösungsmittel für den Verfahrensschritt (I-I-1) → (I-I-3) bzw. (I-I-2) → (I-I-4) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist DMF.

Die Reduktion (I-I-1) → (I-I-3) bzw. (I-I-2) → (I-I-4) erfolgt mit Wasserstoff in Verbindung mit Übergangsmetallkatalysatoren wie beispielsweise Palladium (10% auf Aktivkohle), Raney-Nickel oder Palladiumhydroxid.

Die Reaktion (I-I-1) → (I-I-3) bzw. (I-I-2) → (I-I-4) erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Die Umsetzung (I-J-1) + (XVI) → (I-K-1) bzw. (I-J-2) + (XVI) → (I-K-2) erfolgt in einem Lösungsmittel, welches unter den Reaktionsbedingungen inert ist oder gegebenenfalls ohne Lösungsmittel. Inerte Lösungsmittel sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N-*Dimethylformamid, Dimethylsulfoxid, *N*,*N*'-Dimethylpropylenharnstoff (DMPU), N-Methylpyrrolidon (NMP), Sulfolan oder Pyridin. Bevorzugt ist NMP.

Die Umsetzung (I-J-1) + (XVI) → (I-K-1) bzw. (I-J-2) + (XVI) → (I-K-2) erfolgt im Allgemeinen in einem Temperaturbereich von +50°C bis +200°C, bevorzugt von +100°C bis +160°C, bevorzugt in einer Mikrowelle. Die Umsetzung kann bei normalem oder erhöhtem Druck durchgeführt werden (z.B. im Bereich von 0.5 bis 5 bar). Im Allgemeinen arbeitet man bei erhöhten Druck oder Normaldruck.

Wenn R^{4A} für gegebenenfalls im oben angegebenen Umfang substituiertes (C₁-C₄)-Alkoxy steht, erfolgt die Umsetzung (I-J-1) + (XVI) → (I-K-1) bzw. (I-J-2) + (XVI) → (I-K-2) in Gegenwart eines geeigneten Kupfer-Katalysators wie beispielsweise Kupfer-(I)-iodid, unter Zusatz von 3,4,7,8-Tetramethyl-l,10-Phenanthrolin, sowie einer geeigneten Base wie beispielsweise Erdalkalicarbonate wie Lithium-, Natrium-, Kalium-, Calcium- oder Cäsiumcarbonat, bevorzugt Cäsiumcarbonat.

Wenn R^{4A} für gegebenenfalls im oben angegebenen Umfang substituiertes (C₂-C₄)-Alkinyl steht, erfolgt die Umsetzung (I-J-1) + (XVI) → (I-K-1) bzw. (I-J-2) + (XVI) → (I-K-2) in Gegenwart eines geeigneten Palladium- und/oder Kupfer-Katalysators, wobei sich die für den Verfahrensschritt (II-2) + (III) → (I-A-2) genannten Verbindungen eignen.

Die weiteren Derivate R^{4A} werden nach den dem Fachmann bekannten Methoden hergestellt.

Die zuvor beschriebenen Herstellverfahren werden anhand der nachfolgenden Syntheseschemata (Schema 1 bis 9) beispielhaft erläutert:

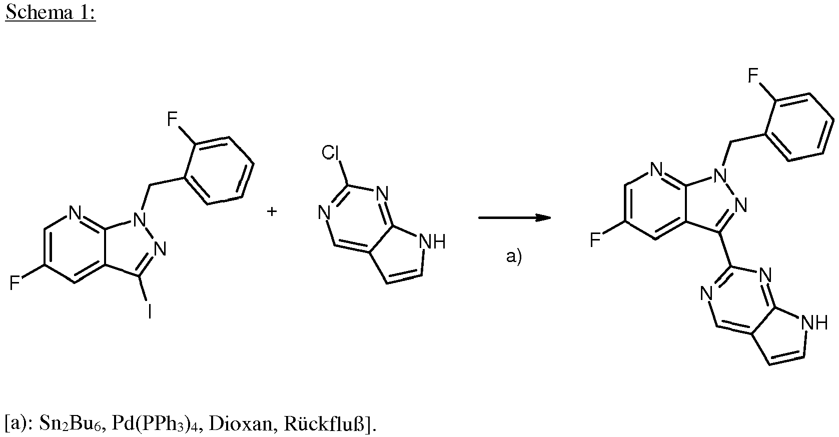

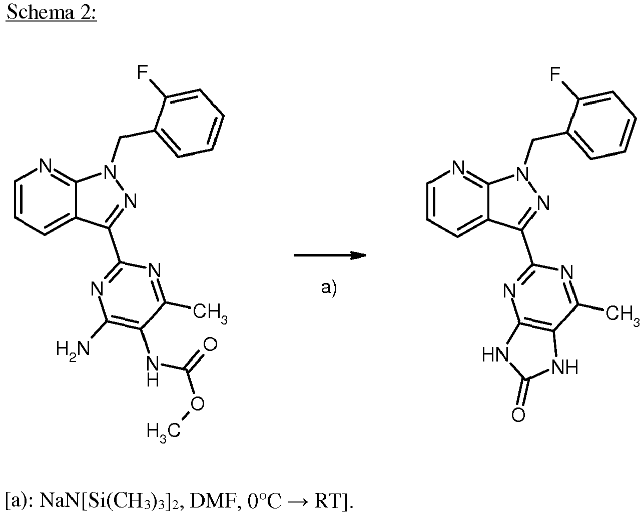

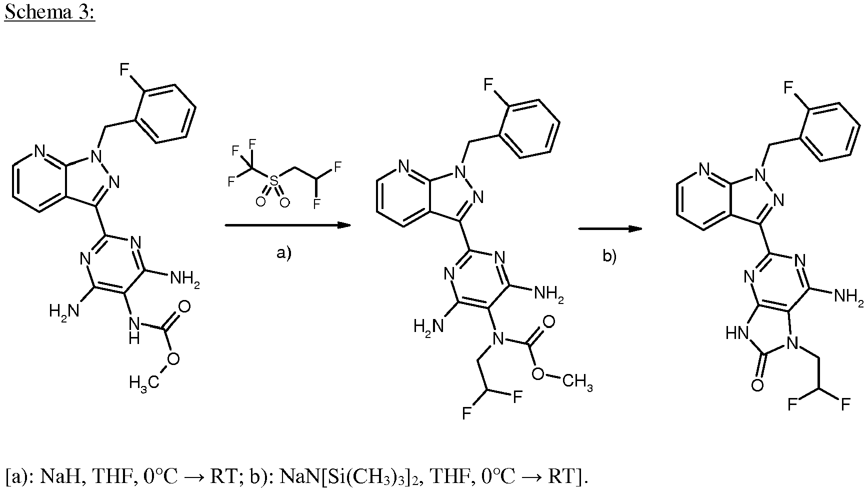

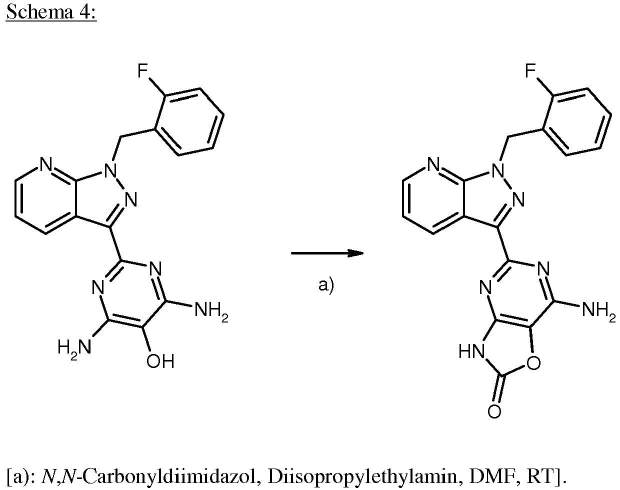

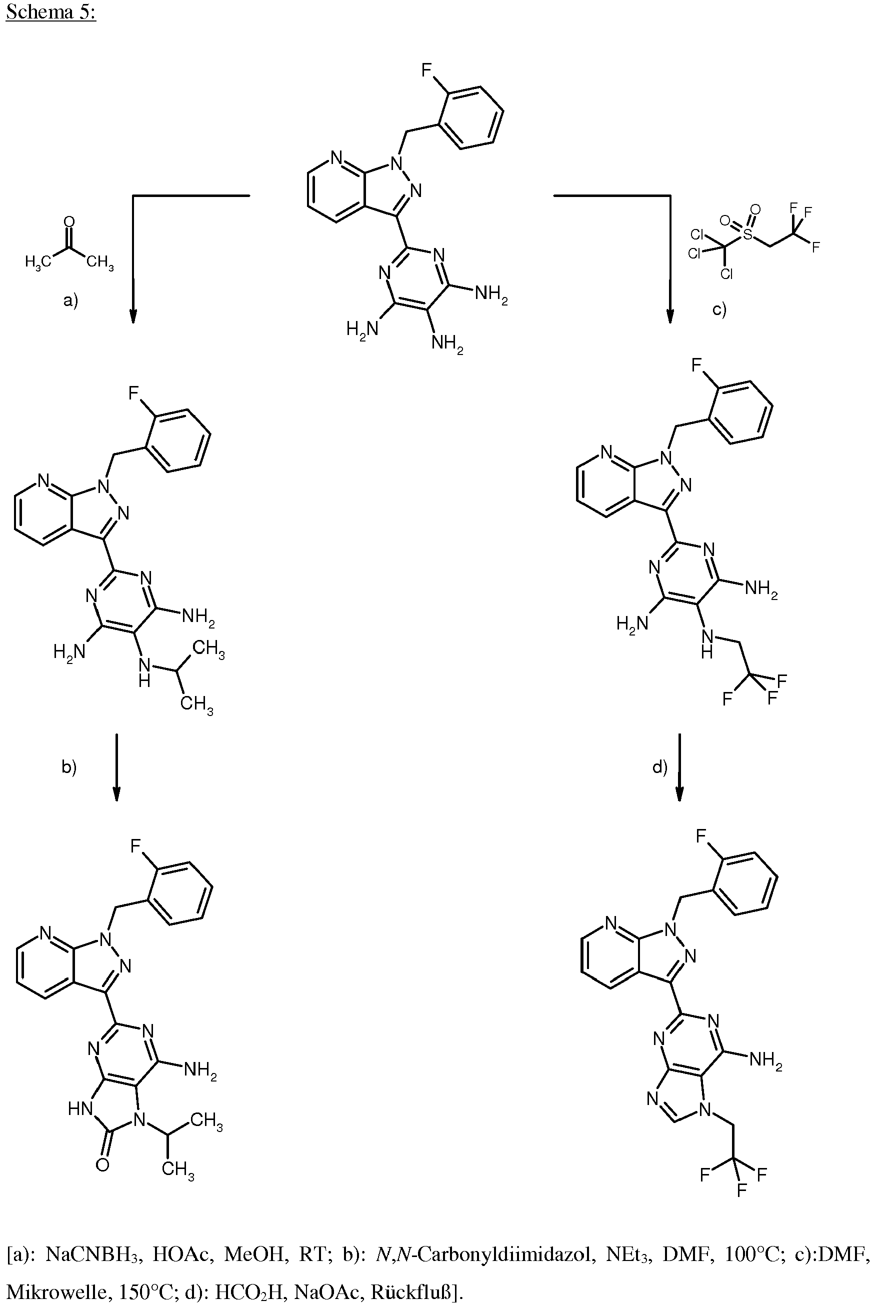

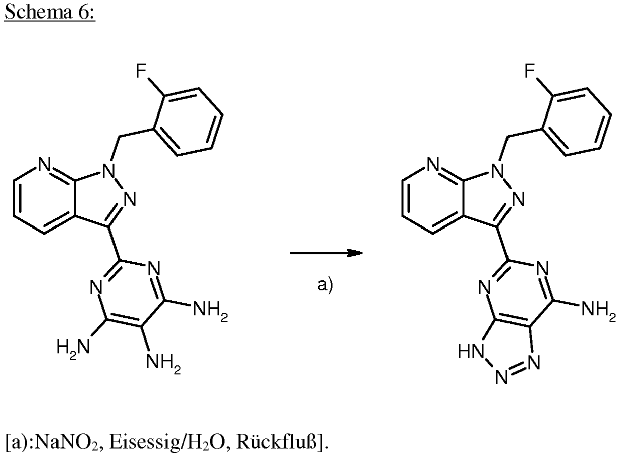

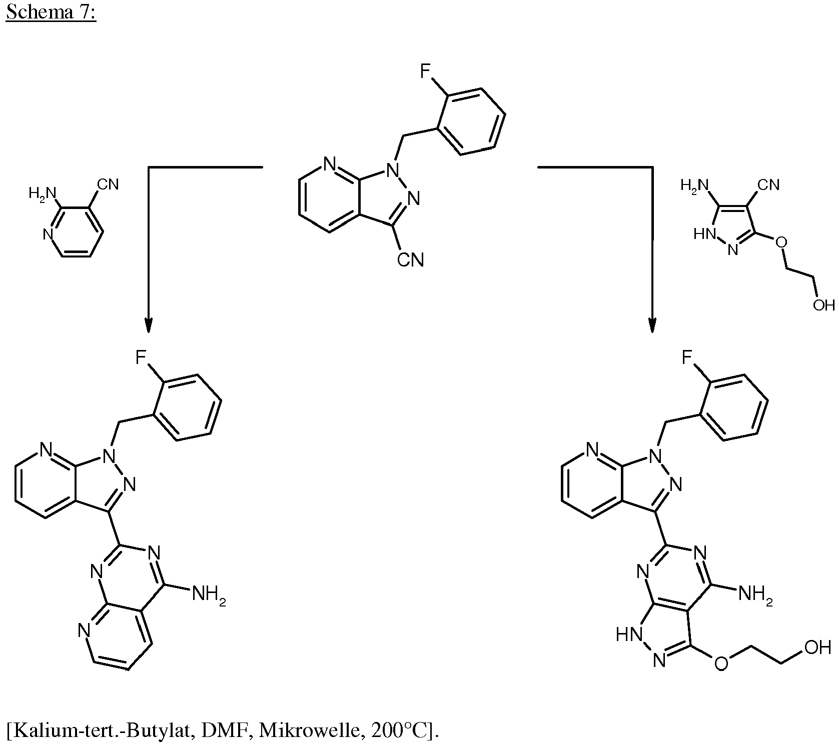

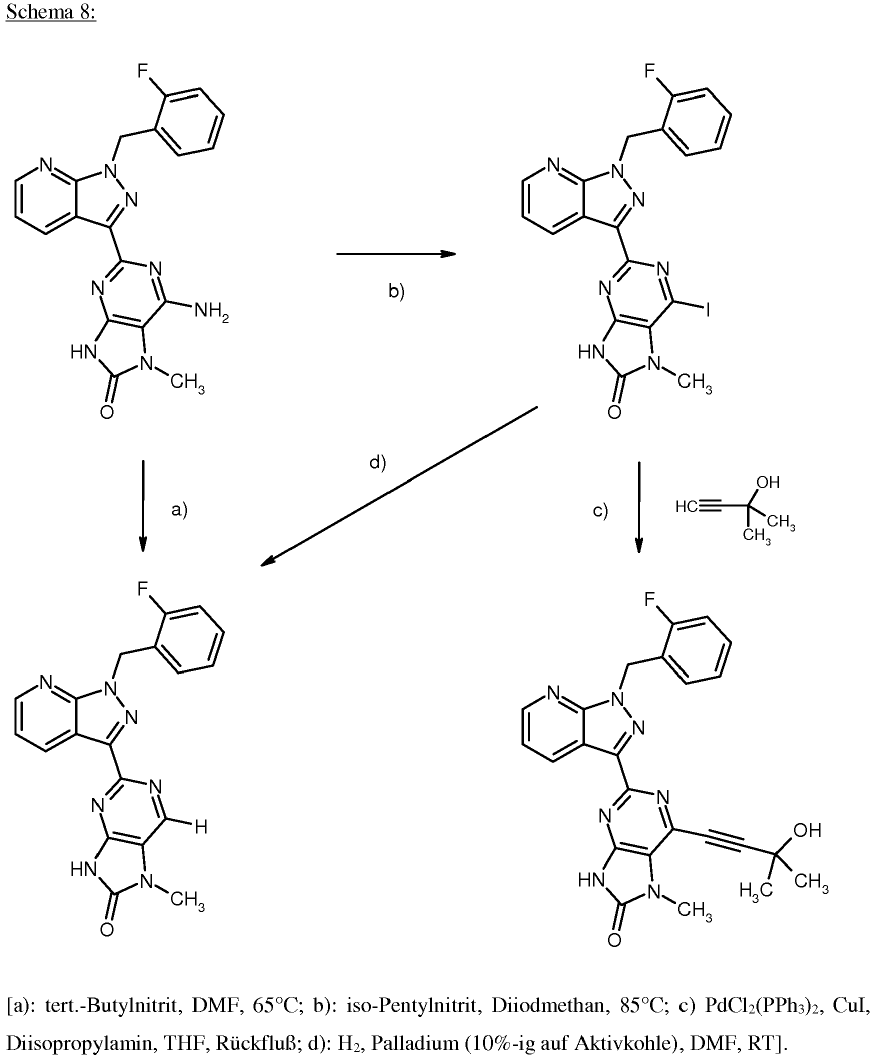

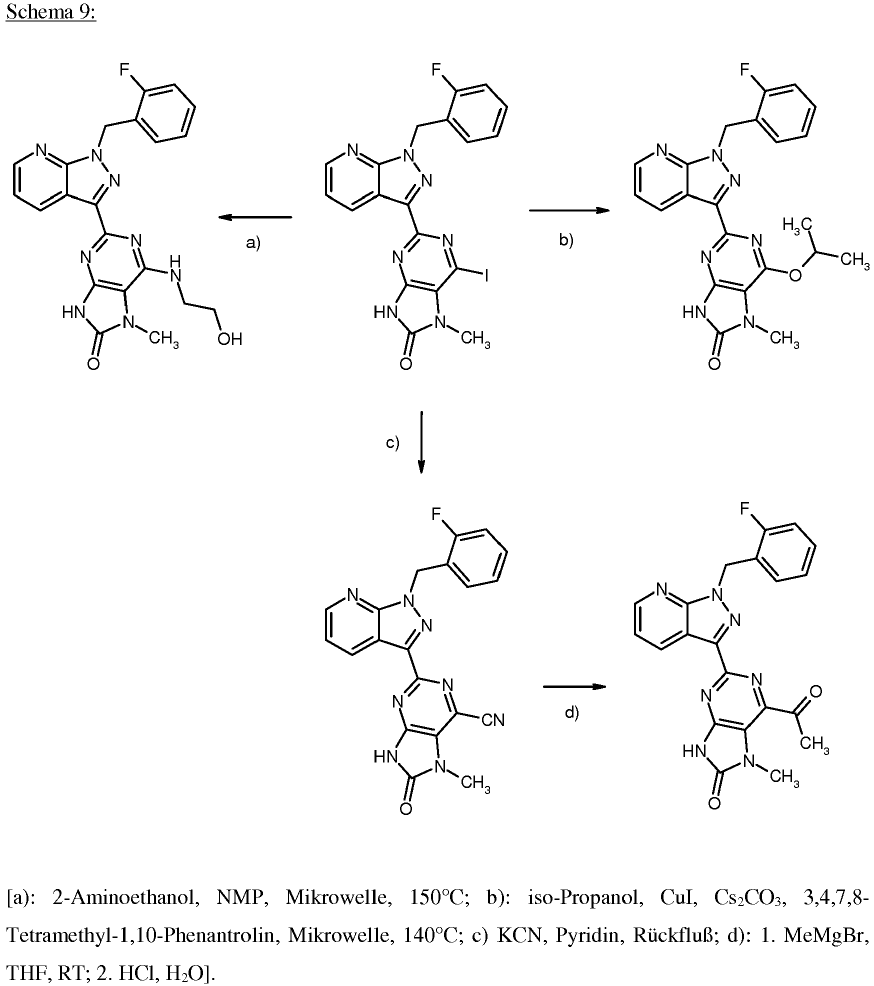

Weitere erfindungsgemäße Verbindungen können nach den dem Fachmann bekannten Methoden, in Analogie zu literaturbekannten Verfahren, wie im vorliegenden experimentellen Teil beschrieben und wie in den nachfolgenden Syntheseschemata (Schemata 10 bis 13, 17 und 18) gezeigt, hergestellt werden:

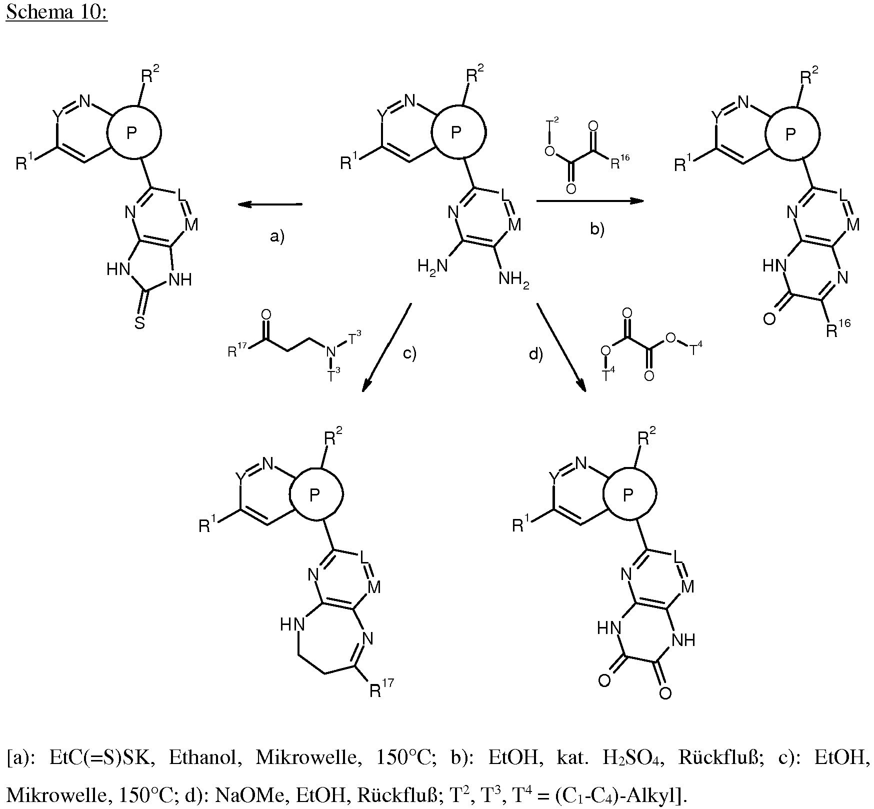

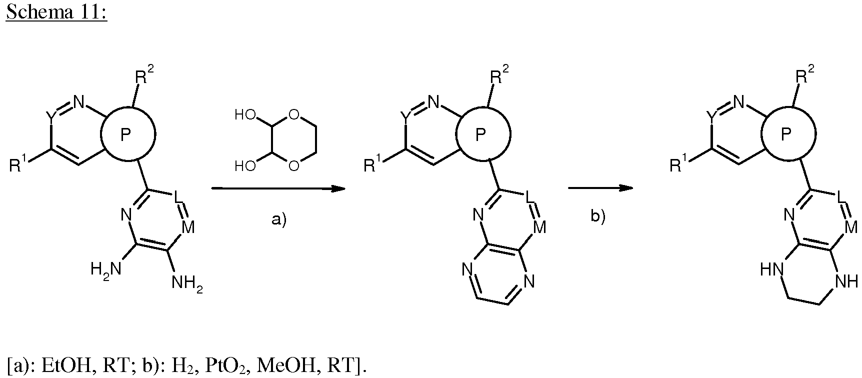

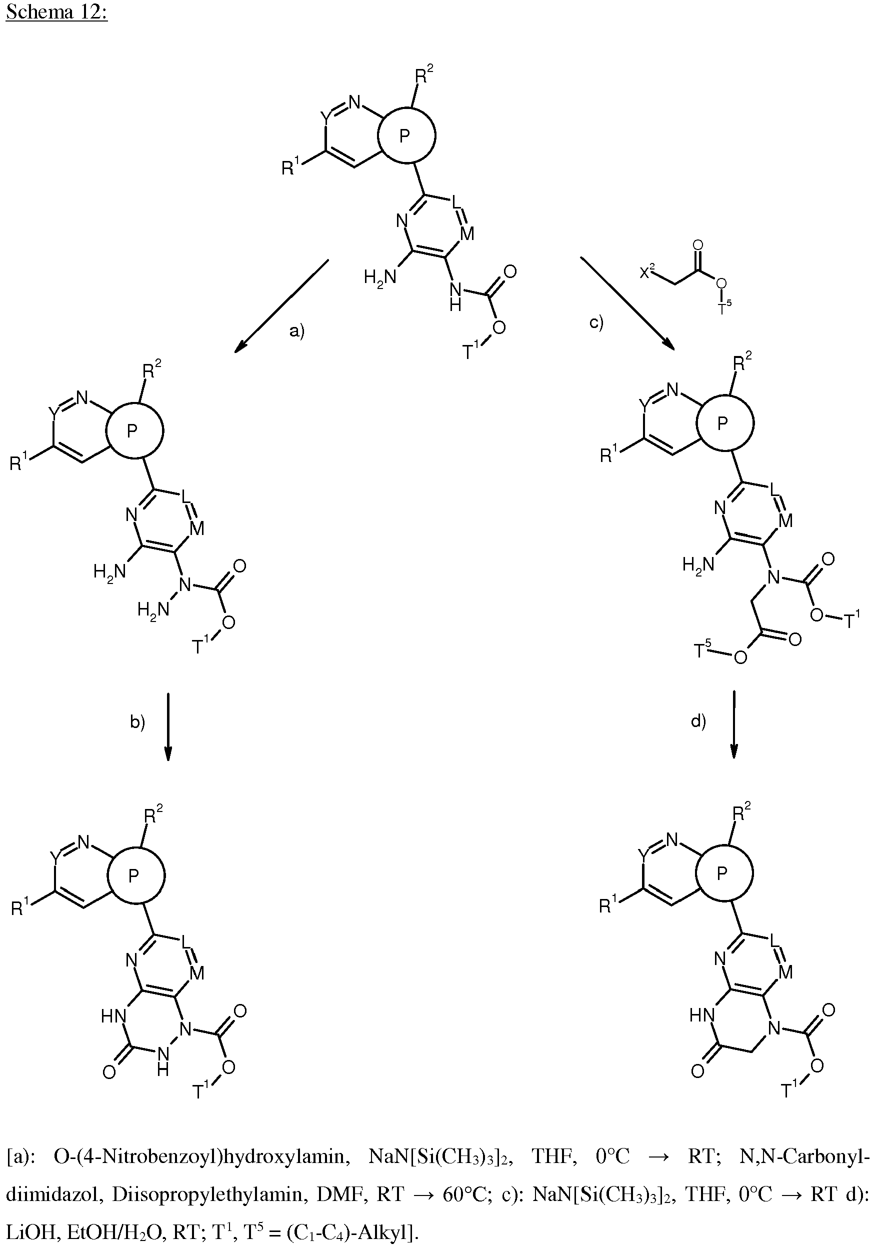

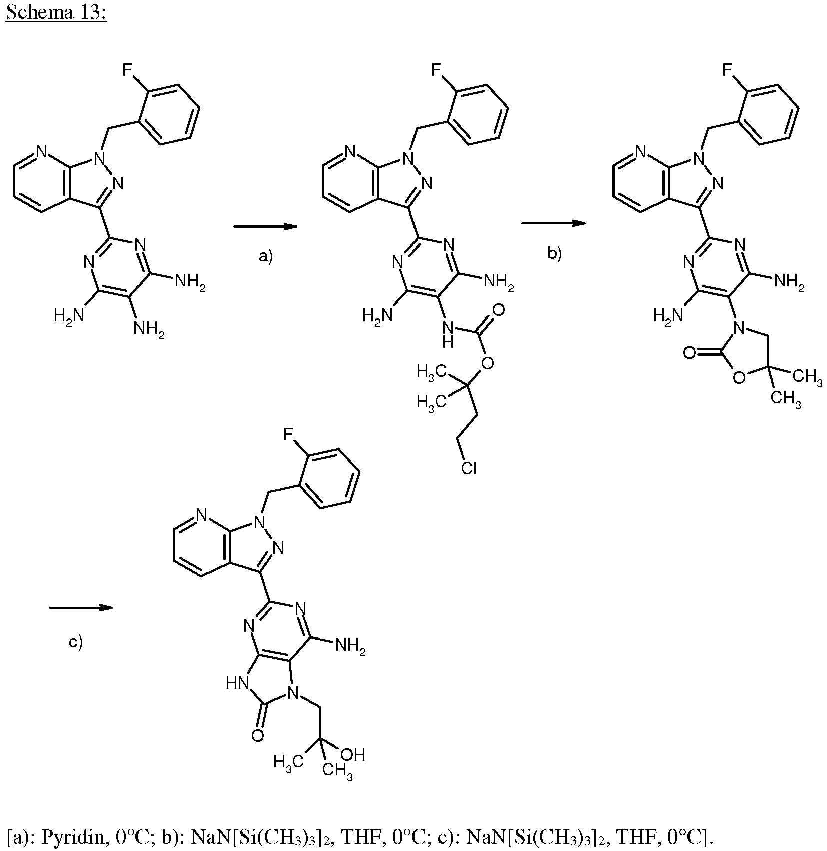

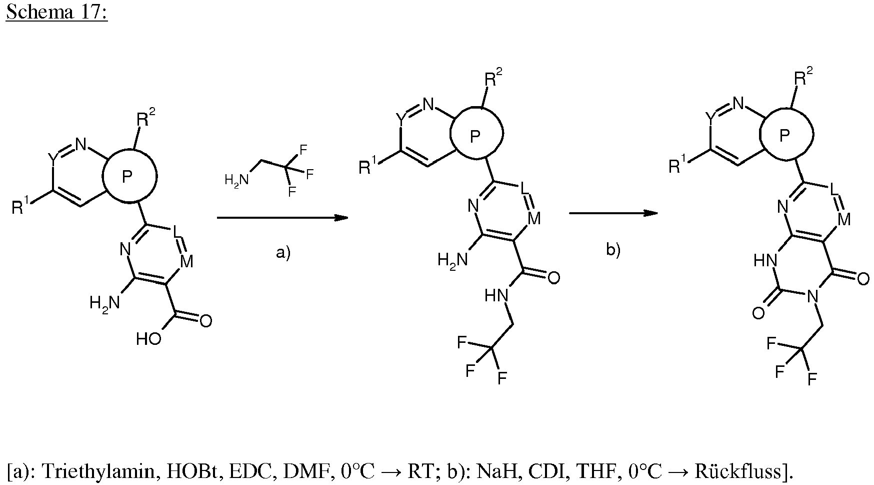

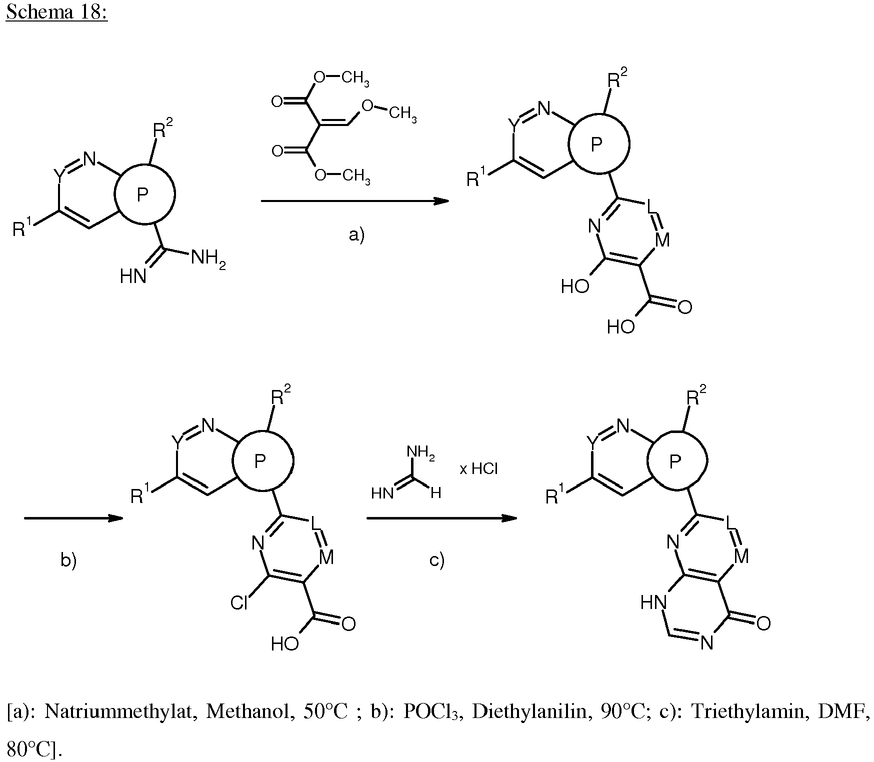

Die Verbindungen der Formeln (III), (V), (VIII), (X), (XI), (XIII), (XV-1), (XV-2) und (XVI) sind kommerziell erhältlich, literaturbekannt oder können in Analogie zu literaturbekannten Verfahren hergestellt werden.

Die Herstellung der Verbindungen (II-1), (II-2), (IV), (VII) und (XIV) erfolgt wie in WO 03/095451 bzw. WO 2008/031513 beschrieben, oder wie in den nachfolgenden Syntheseschemata (Schema 14 und 15) beispielhaft gezeigt:

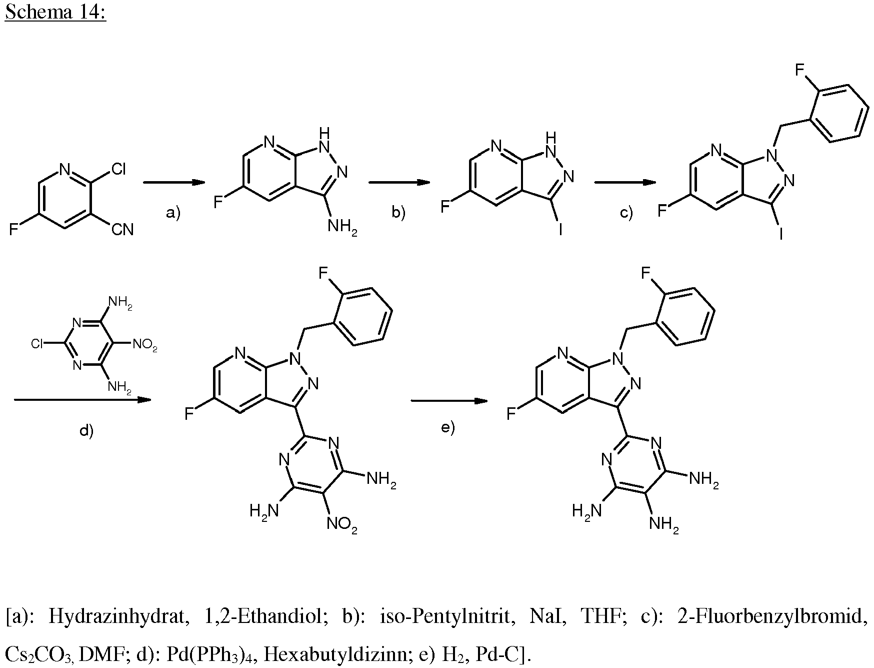

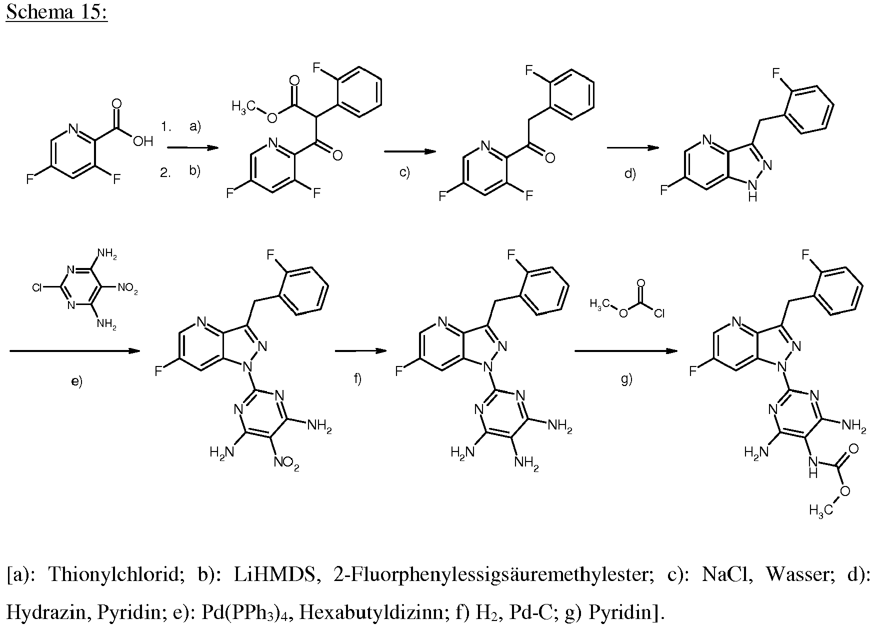

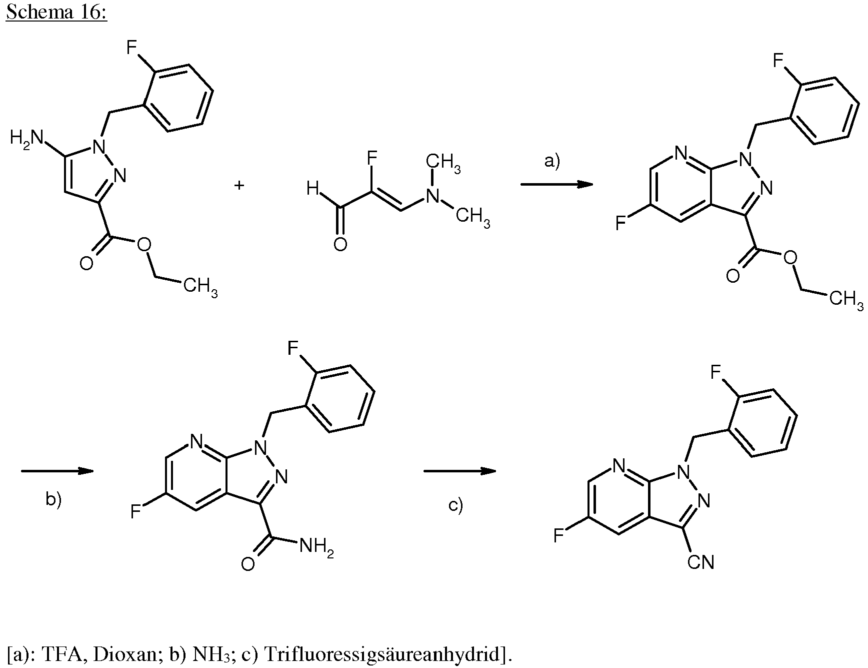

Weitere Edukte können wie im vorliegenden experimentellen Teil unter anderem für Beispiel 4A, 37A, 39A, 42A, 45A. 50A, 51A, 61A und 65A beschrieben hergestellt werden.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden wie im vorliegenden experimentellen Teil beschrieben, nach üblichen, dem Fachmann bekannten Methoden durchgeführt, und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die erfindungsgemäßen Verbindungen wirken als sehr potente Stimulatoren der löslichen Guanylatcyclase, besitzen wertvolle pharmakologische Eigenschaften, und eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittelinduzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer offenbarter Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- BEMP: 2-(tert.-Butylimino)-N,N-diethyl-1,3-dimethyl-1,3,2lambda⁵-diazaphosphinan-2-amin
- ber.: berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd/C: Palladium auf Aktivkohle (10%-ig)
- Pd₂dba₃: Tris-(dibenzylidenaceton)-dipalladium
- Ph: Phenyl
- RT: Raumtemperatur
- Rₜ: Retentionszeit (bei HPLC)
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-triisopropylbiphenyl-2-yl)-phosphin

### LC/MS-Methoden:

### Methode 1 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 2 (LC-MS):

Instrument: Waters ACQUITY SQD UPLC System; Säule: Waters Acquity UPLC HSS T3 1,8µ 50 x 1mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 3 (LC-MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 4 (LC-MS):

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

Methode 5 (LC-MS): MCW-SQ3-HSST3-2-30mm

Instrument: Waters ACQUITY SQD UPLC System; Säule: Waters Acquity UPLC HSS T3 1.8µ 30 x 2 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Allgemeine Arbeitsvorschriften:

### Allgemeine Arbeitsvorschrift 1:

In einem Mikrowellengefäß mit Rührmagnet wurde 1.0 eq Beispiel 3A zusammen mit 1.0 eq des entsprechenden Aminonitrils und 1.0 eq Kalium-tert.-butylat in Dimethylformamid (1.5 ml) gelöst, verschlossen und 1h bei 160° C unter Mikrowellenbestrahlung erhitzt. Danach erfolgte Reaktionskontrolle. Bei unvollständigem Umsatz wurden weitere 0.5 eq Kalium-tert.-butylat zugegeben und es wurde erneut bei 160° C unter Mikrowellenbestrahlung bis zum vollständigen Umsatz erhitzt. Die Reaktionsmischung wurde mittels präparativer HPLC gereinigt (Eluent: Acetonitril/Wasser mit 0.05% Ameisensäure, Gradient).

### Ausgangsverbindungen und Intermediate:

### Beispiel 1A

### 2-[1-(2-Fluorbenzyl)-1H-pyrazolo[3,4-b]pyridin-3-yl]pyrimidin-4,5,6-triamin

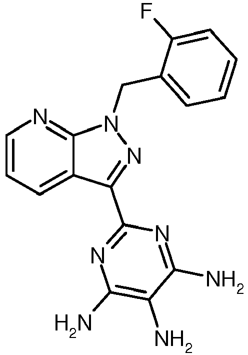

Die Synthese dieser Verbindung ist beschrieben in WO 03/095451, Beispiel 8A.

### Beispiel 2A

### 2-[3-(2-Fluorbenzyl)-1H-pyrazolo[4,3-b]pyridin-1-yl]pyrimidin-4,5,6-triamin

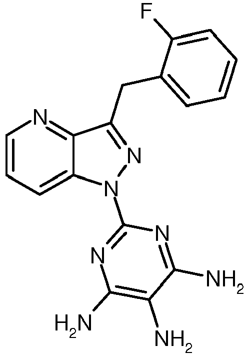

Die Synthese dieser Verbindung ist beschrieben in WO 2008/031513, Beispiel 8.

### Beispiel 3A

### 1-(2-Fluorbenzyl)-1H-pyrazolo[3,4-b]pyridin-3-carbonitril

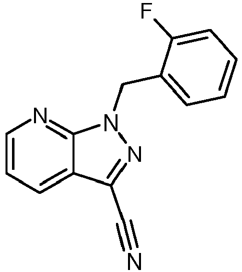

Die Synthese dieser Verbindung ist beschrieben in WO 03/095451, Beispiel 4A.

### Beispiel 4A

### 4-Amino-2-[1-(2-fluorbenzyl)-1H-pyrazolo[3,4-b]pyridin-3-yl]pyrimidin-5-ol

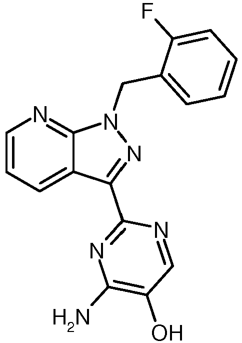

Die Synthese dieser Verbindung ist beschrieben in ChemMedChem, 4(5), 853-865; 2009.

### Beispiel 5A

### 1-(2-Fluorbenzyl)-1H-pyrazolo[3,4-b]pyridin-3-carboximidamid-Hydrochlorid

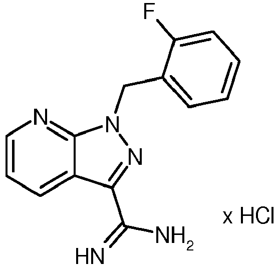

Die Synthese dieser Verbindung ist beschrieben in WO 03/095451, Beispiel 6A.

### Beispiel 6A

### 2-Chlorethyl-{4,6-diamino-2-[1-(2-fluorbenzyl)-1H-pyrazolo[3,4-b]pyridin-3-yl]pyrimidin-5-yl}carbamat

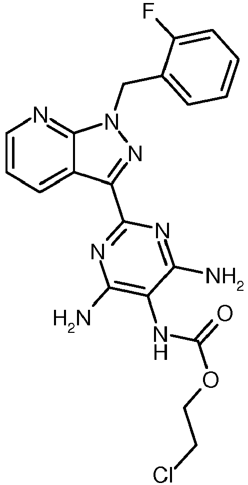

1.50 g (4.281 mmol) der Verbindung aus Beispiel 1A wurden in 15 ml Dichlormethan und 15 ml Pyridin gelöst. Danach wurde unter Rühren 612 mg (4.281 mmol) Chlorameisensäure-2-chlorethylester zugegeben. Anschliessend wurde auf RT erwärmt und 12h bei dieser Temperatur gerührt. Der Ansatz wurde danach auf Eiswasser gegossen und zweimal mit Dichlormethan extrahiert. Die vereinigten organischen Phasen wurden mit Natriumsulfat getrocknet und im Vakuum eingeengt. Man erhielt 1.031 g (52 % d. Th.) der Titelverbindung, die ohne weitere Reinigung weiter umgesetzt wurde.

LC-MS (Methode 2): Rₜ = 0.78 min; MS (EIpos): m/z = 457 [M+H]⁺.

### Beispiel 7A

### 1-Chlor-2-methylpropan-2-yl-{4,6-diamino-2-[1-(2-fluorbenzyl)-1H-pyrazolo[3,4-b]pyridin-3-yl]pyrimidin-5-yl}carbamat

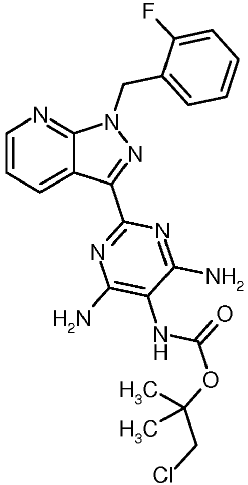

175 µl (1.713 mmol) 1-Chlor-2-methyl-2-propanol wurden in 6 ml Dichlormethan vorgelegt und mit 169 mg (0.571 mmol) Bis(trichlormethyl)carbonat versetzt und auf 0°C abgekühlt. Danach wurden 110 µl (1.37 mmol) Pyridin zugetropft und es wurde 30 min bei 0°C gerührt. Anschliessend wurden 400 mg (1.142 mmol) der Verbindung aus Beispiel 1A zugegeben und danach 2.93 ml (36.26 mmol) Pyridin. Es wurde weitere 30 min bei 0°C gerührt. Anschliessend wurden in einem separaten Kolben 87 µl (0.856 mmol) 1-Chlor-2-methyl-2-propanol in 3 ml Dichlormethan vorgelegt und mit 85 mg (0.285 mmol) Bis(trichlormethyl)carbonat versetzt. Nach Zugabe von 55 µl (0.685 mmol) Pyridin wurde 30 min bei 0°C gerührt und die so hergestellte Lösung zum weiter oben beschriebenen Ansatz zugegeben. Nach weiteren 30 min Rühren bei 0°C wurde der Ansatz durch Zugabe von 10 ml gesättigter wässriger Natriumhydrogencarbonat-Lösung abgebrochen und dreimal mit Dichlormethan extrahiert. Die vereinigten organischen Phasen wurden mit Natriumsulfat getrocknet und im Vakuum eingeengt. Man erhielt 500 mg (84 % d. Th.) der Titelverbindung, die ohne weitere Reinigung in den folgenden Versuchen verwendet wurde.

LC-MS (Methode 2): Rₜ = 0.86 min; MS (EIpos): m/z = 485 [M+H]⁺.

### Beispiel 8A

### 3-{4,6-Diamino-2-[1-(2-fluorbenzyl)-1H-pyrazolo[3,4-b]pyridin-3-yl]pyrimidin-5-yl}-5,5-dimethyl-1,3-oxazolidin-2-on

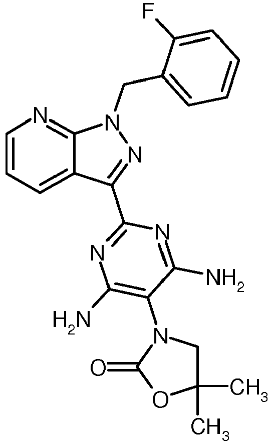

500 mg (1.033 mmol) der unter Beispiel 7A hergestellten Verbindung wurden in Tetrahydrofuran (10 ml) gelöst und bei 0°C mit 1.033 ml einer 1M Lösung von Bis-(trimethylsilyl)natriumamid-Lösung in Tetrahydrofuran versetzt. Nach 5 min Rühren bei 0°C wurde der Ansatz durch Zugabe von gesättigter wässriger Natriumhydrogencarbonat-Lösung abgebrochen und zweimal mit Essigsäureethylester extrahiert. Die vereinigten organischen Phasen wurden mit Natriumsulfat getrocknet und im Vakuum eingeengt. Der Rückstand wurde mittels präparativer HPLC gereinigt (Acetonitril:Wasser (+0.05 % Ameisensäure) - Gradient). Es wurden 329 mg der Titelverbindung erhalten (71 % d. Th.).

LC-MS (Methode 2): Rₜ = 0.83 min; MS (EIpos): m/z = 449 [M+H]⁺.

¹H-NMR (400 MHz, DMSO-d₆): δ [ppm] = 1.55 (s, 6H), 3.43 (s, 2H), 5.81 (s, 2H), 6.44 (s br, 4H) 7.10-7.14 (m, 2H), 7.20-7.25 (m, 1H), 7.32-7.37 (m, 2H), 8.61 (dd, 1H), 9.08 (dd, 1H).

### Beispiel 9A

### 3-Brom-1,1,1-trifluorpropan-2-yl-{4,6-diamino-2-[1-(2-fluorbenzyl)-1H-pyrazolo[3,4-b]pyridin-3-yl]pyrimidin-5-yl}carbamat

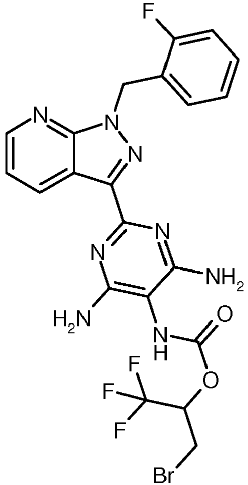

0.888 ml (8.563 mmol) 3-Brom-1,1,1-trifluor-2-propanol wurden in 22 ml Dichlormethan vorgelegt und mit 952 mg (3.211 mmol) Bis(trichlormethyl)carbonat versetzt und auf 0°C abgekühlt. Danach wurden 0.519 ml (6.422 mmol) Pyridin zugetropft und es wurde 1h bei 0°C gerührt. Anschliessend wurden 1.5 g (4.281 mmol) der Verbindung aus Beispiel 1A gelöst in Pyridin (11 ml) zugegeben und es wurde weitere 30 min bei 0°C gerührt. Nach weiteren 12 h bei RT wurde der Ansatz durch Zugabe von 30 ml gesättigter wässriger Natriumhydrogencarbonat-Lösung abgebrochen und dreimal mit Dichlormethan extrahiert. Die vereinigten organischen Phasen wurden mit Natriumsulfat getrocknet und im Vakuum eingeengt. Der Rückstand wurde durch Chromatographie an Kieselgel (Dichlormethan/Methanol-Gradient) gereinigt. Man erhielt 532 mg (21 % d. Th.) der Titelverbindung.

LC-MS (Methode 2): Rₜ = 0.92 min; MS (EIpos): m/z = 569/571 [M+H, Br-Muster]⁺.

### Beispiel 10A

### 3-{4,6-Diamino-2-[1-(2-fluorbenzyl)-1H-pyrazolo[3,4-b]pyridin-3-yl]pyrimidin-5-yl}-5-(trifluormethyl)-1,3-oxazolidin-2-on

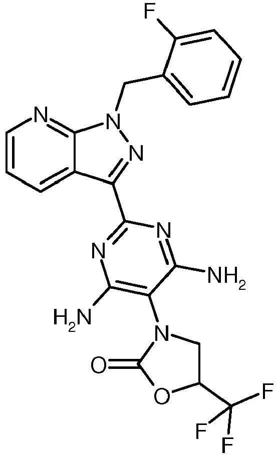

Beispiel 10A wurde aus Beispiel 9A in Analogie zur Herstellung von Beispiel 8A hergestellt.

LC-MS (Methode 2): Rₜ = 0.89 min; MS (EIpos): m/z = 489 [M+H]⁺.

### Beispiel 11A

### Methyl-14,6-diamino-2-[1-(2-fluorbenzyl)-1H-pyrazolo[3,4-b]pyridin-3-yl]pyrimidin-5-yl}carbamat

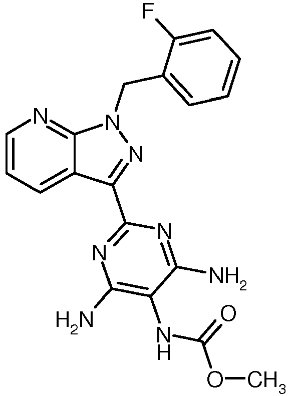

Die Synthese dieser Verbindung ist beschrieben in WO 03/095451, Beispiel 5.

### Beispiel 12A

### Methyl-{4,6-diamino-2-[1-(2-fluorbenzyl)-1H-pyrazolo[3,4-b]pyridin-3-yl]pyrimidin-5-yl}(2,2-difluorethyl)carbamat

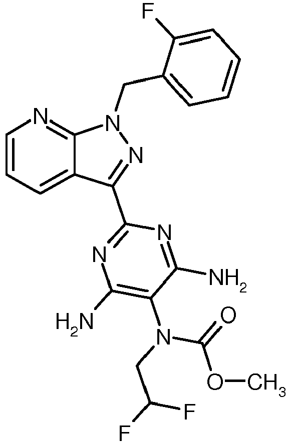

400 mg (0.979 mmol) der Verbindung aus Beispiel 11A wurden in 10 ml Tetrahydrofuran vorgelegt und auf 0°C abgekühlt. Danach wurden 43 mg (1.077 mmol) Natriumhydrid (60%-ig in Mineralöl) zugegeben und es wurde unter Rühren 30min bei 0°C gerührt. Anschliessend wurden 230 mg (1.077 mmol) 2,2-Difluorethyltrifluormethansulfonat zugegeben und es wurde 1h bei RT gerührt. Danach wurde noch mal die gleiche Menge Natriumhydrid und 2,2-Difluorethyltrifluormethansulfonat zugegeben und man rührte bei RT über Nacht. Im Anschluss daran wurde der Ansatz mit 0.5 ml Wasser abgebrochen. Die Reaktionsmischung wurde mittels präparativer HPLC gereinigt (Acetonitril/Wasser (+0.05 % Ameisensäure) - Gradient). Es wurden 198 mg der Titelverbindung erhalten (42 % d. Th.).

LC-MS (Methode 2): Rₜ = 0.86 min; MS (EIpos): m/z = 473 [M+H]⁺.

¹H-NMR (400 MHz, DMSO-d₆): δ [ppm] = 3.61 (s, 3H), 3.69-3.75 (m, 2H), 5.81 (s, 2H), 6.15-6.47 (m, 1H), 6.55 (s br, 4H) 7.07-7.14 (m, 2H), 7.20-7.26 (m, 1H), 7.31-7.40 (m, 2H), 8.61 (dd, 1H), 9.04 (dd, 1H).

### Beispiel 13A

### Methyl-{4,6-diamino-2-[1-(2-fluorbenzyl)-1H-pyrazolo[3,4-b]pyridin-3-yl]pyrimidin-5-yl}methylcarbamat

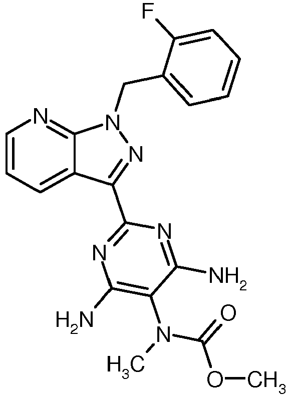

Die Synthese dieser Verbindung ist beschrieben in WO 03/095451, Beispiel 1.

### Beispiel 14A

### Methyl-{4,6-diamino-2-[1-(2-fluorbenzyl)-1H-pyrazolo[3,4-b]pyridin-3-yl]pyrimidin-5-yl}ethylcarbamat

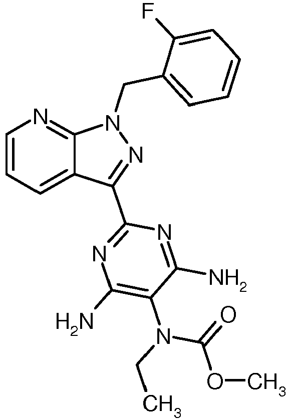

Es wurden 200 mg (0.490 mmol) der Verbindung aus Beispiel 11A in 2 ml Tetrahydrofuran gelöst, bei 0°C mit 22 mg (0.539 mmol) Natriumhydrid (60%-ig in Mineralöl) versetzt und 90 min bei 0°C gerührt. Es wurden 84 mg (0.539 mmol) Ethyliodid addiert und das Gemisch für 48 h bei RT gerührt. Es wurde Wasser addiert und das Reaktionsgemisch im Vakuum eingeengt und der Rückstand mittels präparativer HPLC gereinigt (Eluent: Methanol/Wasser, Gradient 30:70 → 90:10). Es wurden 59 mg der Titelverbindung erhalten (28 % d. Th.).

LC-MS (Methode 2): Rₜ = 0.82 min; MS (EIpos): m/z = 437 [M+H]⁺.

¹H-NMR (400 MHz, DMSO-d₆): δ [ppm] = 1.06 (t, 3H), 3.50 (q, 2H), 3.54 (s, 3H) 5.80 (s, 2H), 6.31 (s br, 4H) 7.07-7.14 (m, 2H), 7.20-7.25 (m, 1H), 7.32-7.37 (m, 2H), 8.60 (dd, 1H), 9.07 (dd, 1H).

### Beispiel 15A

### Methyl-{4,6-diamino-2-[1-(2-fluorbenzyl)-1H-pyrazolo[3,4-b]pyridin-3-yl]pyrimidin-5-yl}(2,2,2-trifluorethyl)carbamat

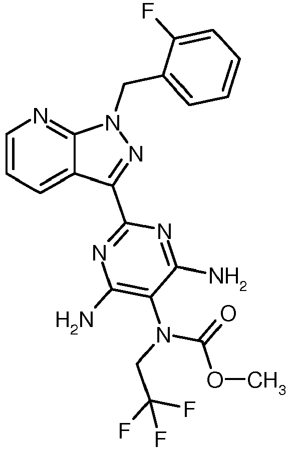

Es wurden 5.000 g (12.243 mmol) der Verbindung aus Beispiel 11A in 50 ml Tetrahydrofuran suspendiert, bei 0°C mit 539 mg (13.467 mmol) Natriumhydrid (60%ig in Mineralöl) versetzt und 90 min bei 0°C gerührt, wobei sich eine Lösung gebildet hatte. Es wurden 3.791 g (13.467 mmol) 2,2,2-Trifluorethyltrichlormethansulfonat addiert und das Gemisch für 48 h bei RT gerührt. Es wurde Wasser und 1N Salzsäure addiert und mit Essigsäureethylester extrahiert. Die vereinigten organischen Phasen wurden über Natriumsulfat getrocknet und am Rotationsverdampfer eingeengt. Der Rückstand wurde mittels präparativer HPLC gereinigt (Eluent: Methanol/Wasser, Gradient 20:80 → 90:10). Es wurden 2.900 g der Titelverbindung erhalten (48 % d. Th.).

LC-MS (Methode 2): Rₜ = 0.89 min; MS (EIpos): m/z = 491 [M+H]⁺.

¹H-NMR (400 MHz, DMSO-d₆): δ [ppm] = 3.63 (s, 3H), 4.06-4.15 (m, 2H), 5.80 (s, 2H), 6.40 (s br, 4H) 7.08-7.14 (m, 2H), 7.20-7.25 (m, 1H), 7.32-7.38 (m, 2H), 8.61 (dd, 1H), 9.07 (dd, 1H).

### Beispiel 16A

### Methyl-{4,6-diamino-2-[1-(2-fluorbenzyl)-1H-pyrazolo[3,4-b]pyridin-3-yl]pyrimidin-5-yl}(²H₃)methylcarbamat

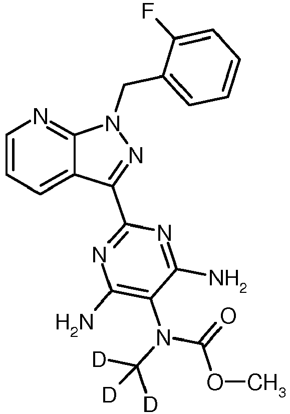

Es wurden 200 mg (0.490 mmol) der Verbindung aus Beispiel 11A in 2 ml Tetrahydrofuran gelöst, bei 0°C mit 43 mg (1.077 mmol) Natriumhydrid (60%ig in Mineralöl) versetzt und 90 min bei 0°C gerührt. Es wurden 78 mg (0.539 mmol) Iodmethan-d₃ addiert und das Gemisch für 48 h bei RT gerührt. Es wurde Wasser addiert und das Reaktionsgemisch im Vakuum eingeengt und der Rückstand mittels präparativer HPLC gereinigt (Eluent: Methanol/Wasser, Gradient 30:70 → 90:10). Es wurden 49 mg der Titelverbindung erhalten (24 % d. Th.).

LC-MS (Methode 2): Rₜ = 0.78 min; MS (EIpos): m/z = 426 [M+H]⁺.

¹H-NMR (400 MHz, DMSO-d₆): δ [ppm] = 3.52 (s, 1.9H), 3.65 (s, 1.1H), 5.80 (s, 2H), 6.34-6.38 (m, 4H) 7.06-7.14 (m, 2H), 7.20-7.25 (m, 1H), 7.31-7.37 (m, 2H), 8.59 (dd, 1H), 9.06 (dd, 1H).

### Beispiel 17A

### 2-[1-(2-Fluorbenzyl)-1H-pyrazolo[3,4-b]pyridin-3-yl]-N5-(2,2,2-trifluorethyl)pyrimidin-4,5,6-triamin

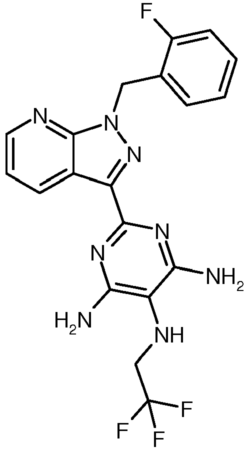

Es wurden 500 mg (1.427 mmol) der Verbindung aus Beispiel 1A mit 3,5 ml Dimethylformamid und 1205 mg (4.281 mmol) 2,2,2-Trifluorethyltrichlormethansulfonat versetzt und das Gemisch für 1 h für bei 150°C in der Mikrowelle erhitzt. Das Reaktionsgemisch wurde im Vakuum eingeengt und der Rückstand mittels präparativer HPLC gereinigt (Eluent: Acetonitril/Wasser mit 0.1% Ameisensäure, Gradient 50:50 → 70:30). Es wurden 236 mg der Titelverbindung erhalten (29 % d. Th.).

LC-MS (Methode 2): Rₜ = 0.87 min; MS (EIpos): m/z = 433 [M+H]⁺.

¹H-NMR (400 MHz, DMSO-d₆): δ [ppm] = 3.43-3.53 (m, 2H), 4.05 (t, 1H), 5.78 (s, 2H), 6.13 (s br, 4H) 7.10-7.15 (m, 2H), 7.20-7.25 (m, 1H), 7.32-7.38 (m, 2H), 8.60 (dd, 1H), 9.04 (dd, 1H).

### Beispiel 18A

### 3-{4,6-Diamino-2-[1-(2-fluorbenzyl)-1H-pyrazolo[3,4-b]pyridin-3-yl]pyrimidin-5-yl}-1,3-oxazolidin-2-on

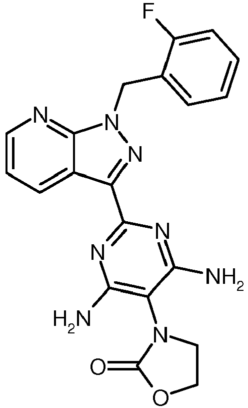

Die Verbindung wurde ausgehend von 1.109 mg (2.427 mmol) Beispiel 6A in Analogie zu Beispiel 8A hergestellt. Es wurden 362 mg der Titelverbindung erhalten (35 % d. Th.).

LC-MS (Methode 2): Rₜ = 0.73 min; MS (EIpos): m/z = 421 [M+H]⁺.

¹H-NMR (400 MHz, DMSO-d₆): δ [ppm] = 3.63-3.67 (m, 2H), 4.40-4.44 (m, 2H), 5.80 (s, 2H), 6.60 (s br, 4H) 7.12-7.14 (m, 2H), 7.21-7.25 (m, 1H), 7.32-7.37 (m, 2H), 8.61 (dd, 1H), 9.04 (dd, 1H).

### Beispiel 19A

### 2,6-Dichlor-5-fluornicotinamid

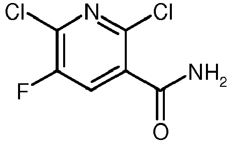

Eine Suspension aus 25 g (130.90 mmol) 2,6-Dichlor-5-fluor-3-cyanopyridin in konz. Schwefelsäure (125 ml) wurde 1 h bei 60-65°C gerührt. Nach Abkühlen auf RT wurde der Kolbeninhalt auf Eiswasser gegossen und dreimal mit Essigsäureethylester (je 100 ml) extrahiert. Die vereinigten organischen Phasen wurden mit Wasser (100 ml) und anschliessend mit gesättigter wässriger Natriumhydrogencarbonat-Lösung (100 ml) gewaschen, getrocknet und am Rotationsverdampfer eingeengt. Das erhaltene Material wurde am Hochvakuum getrocknet.

Ausbeute: 24.5 g (90 % d. Theorie)
¹H-NMR (400 MHz, DMSO-d₆): δ= 7.95 (br s, 1H), 8.11 (br s, 1H), 8.24 (d, 1H).

### Beispiel 20A

### 2-Chlor-5-fluornicotinamid

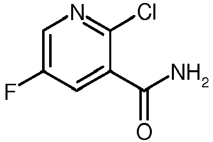

Einer Suspension von 21.9 g (335.35 mmol) Zink in Methanol (207 ml) wurden bei RT 44 g (210.58 mmol) 2,6-Dichlor-5-fluornicotinamid zugegeben. Danach wurde mit Essigsäure (18.5 ml) versetzt und unter Rühren für 24 h zum Rückfluss erhitzt. Danach wurde der Kolbeninhalt vom Zink dekantiert und Essigsäureethylester (414 ml) sowie gesättigte wässrige Natriumhydrogencarbonat-Lösung (414 ml) zugegeben und intensiv ausgerührt. Anschliessend wurde über Kieselgur abgesaugt und dreimal mit Essigsäureethylester (je 517 ml) nachgewaschen. Die organische Phase wurde abgetrennt und die wässrige Phase mit Essigsäureethylester (258 ml) gewaschen. Die vereinigten organischen Phasen wurden einmal mit gesättigter wässriger Natriumhydrogencarbonat-Lösung (414 ml) gewaschen, getrocknet und im Vakuum eingeengt. Die so erhaltenen Kristalle wurden mit Dichlormethan (388 ml) versetzt und 20 min. ausgerührt. Es wurde erneut abgesaugt und mit Diethylether nachgewaschen und trockengesaugt.

Ausbeute: 20.2 g (53 % d. Theorie)

¹H-NMR (400 MHz, DMSO-d₆): δ = 7.87 (br s, 1H), 7.99 (dd, 1H), 8.10 (br s, 1H), 8.52 (d, 1H).

### Beispiel 21A

### 2-Chlor-5-fluornicotinonitril

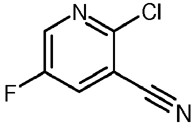

Eine Suspension von 46.2 g (264.66 mmol) 2-Chlor-5-fluornicotinamid in Dichlormethan (783 ml) wurde mit 81.2 ml (582.25 mmol) Triethylamin versetzt und auf 0°C gekühlt. Unter Rühren wurden dann 41.12 ml (291.13 mmol) Trifluoressigsäureanhydrid langsam zugetropft und 1.5 h bei 0°C nachgerührt. Die Reaktionslösung wurde danach zweimal mit gesättigter wässriger Natriumhydrogencarbonat-Lösung (je 391 ml) gewaschen, getrocknet und im Vakuum eingeengt.

Ausbeute: 42.1 g (90 % d. Theorie).

¹H-NMR (400 MHz, DMSO-d₆): δ= 8.66 (dd, 1H), 8.82 (d, 1H).

### Beispiel 22A

### 5-Fluor-1H-pyrazolo[3,4-b]pyridin-3-amin

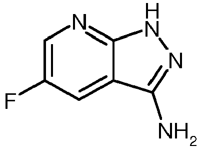

Eine Suspension aus 38.5 g (245.93 mmol) 2-Chlor-5-fluornicotinonitril wurde in 1,2-Ethandiol (380 ml) vorgelegt und danach mit Hydrazinhydrat (119.6 ml, 2.459 mol) versetzt. Es wurde unter Rühren für 4 h zum Rückfluss erhitzt. Beim Abkühlen fiel das Produkt aus. Die gelben Kristalle wurden mit Wasser (380 ml) versetzt und 10 min. bei RT ausgerührt. Anschliessend wurde die Suspension über eine Fritte abgesaugt, mit Wasser (200 ml) und mit -10°C-kaltem THF (200 ml) nachgewaschen. Der Rückstand wurde am Hochvakuum über Phosphorpentoxid getrocknet.

Ausbeute: 22.8 g (61 % d. Theorie)

¹H-NMR (400 MHz, DMSO-d₆): δ = 5.54 (s, 2H), 7.96 (dd, 1H), 8.38 (m, 1H), 12.07(m, 1H).

### Beispiel 23A

### 5-Fluor-3-iod-1H-pyrazolo[3,4-b]pyridin

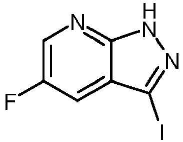

In THF (329 ml) wurden 10 g (65.75 mmol) 5-Fluor-1H-pyrazolo[3,4-b]pyridin-3-amin vorgelegt und auf 0°C abgekühlt. Anschliessend wurden 16.65 ml (131.46 mmol) Bortrifluorid-Diethylether-Komplex langsam zugesetzt. Die Reaktionsmischung wurde weiter auf -10°C abgekühlt. Danach wurde eine Lösung von 10.01 g (85.45 mmol) Isopentylnitrit in THF (24.39 ml) langsam zugefügt und weitere 30 min nachgerührt. Die Mischung wurde mit kaltem Diethylether (329 ml) verdünnt und der entstandene Feststoff abfiltriert. Das so hergestellte Diazoniumsalz wurde portionsweise in eine 0°C kalte Lösung von 12.81 g (85.45 mmol) Natriumiodid in Aceton (329 mal) gegeben und die Mischung 30 min bei RT nachgerührt. Die Reaktionsmischung wurde auf Eiswasser (1.8 1) gegeben und zweimal mit Essigsäureethylester (je 487 mal) extrahiert. Die gesammelten organischen Phasen wurden mit gesättigter wässriger Natriumchorid-Lösung (244 mal) gewaschen, getrocknet, filtriert und eingeengt. Man erhielt 12.1 g (86%-ige Reinheit, 60 % d. Th.) der Titelverbindung als braunen Feststoff. Das Rohprodukt wurde ohne weitere Reinigung umgesetzt.

### LC-MS (Methode 1): Rₜ = 1.68 min; MS (ESIpos): m/z = 264 (M+H)⁺

### Beispiel 24A

### 5-Fluor-1-(2-fluorbenzyl)-3-iod-1H-pyrazolo[3,4-b]pyridin

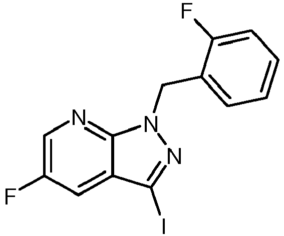

In DMF (2538 ml) wurden 141 g (462.11 mmol) der Verbindung aus Beispiel 23A vorgelegt und anschließend 96.09 g (508.32 mmol) 2-Fluorbenzylbromid sowie 165.62 g (508.32 mmol) Cäsiumcarbonat zugefügt. Die Mischung wurde zwei Stunden bei RT gerührt. Anschließend wurde die Reaktionsmischung auf gesättigte wässrige Natriumchlorid-Lösung (13670 ml) gegeben und zweimal mit Essigester (5858 ml) extrahiert. Die gesammelten organischen Phasen wurden mit gesättigter wässriger Natriumchlorid-Lösung (3905 ml) gewaschen, getrocknet, filtriert und eingeengt. Der Rückstand wurde an Kieselgel (Laufmittel: Petrolether/Essigsäureethylester 97:3) chromatographiert und die Produktfraktionen eingeengt. Der erhaltene Feststoff wurde in Dichlormethan gelöst und einmal mit gesättigter wässriger Natriumthiosulfatlösung (500 ml) und anschliessend mit gesättigter wässriger Natriumchlorid-Lösung (500 ml) gewaschen. Es wurde zur Trockene eingeengt und der Rückstand mit Diethylether aufgeschlämmt, abgesaugt und am Hochvakuum getrocknet. Man erhielt 106.6 g (62 % d. Th.) der Titelverbindung.

LC-MS (Methode 1): Rₜ = 2.57 min

MS (ESIpos): m/z = 372 (M+H)⁺

¹H-NMR (400 MHz, DMSO-d₆): δ = 5.73 (s, 2H), 7.13 - 7.26 (m, 3H), 7.33 - 7.41 (m, 1H), 7.94 (dd, 1H), 8.69 - 8.73 (m, 1H).

### Beispiel 25A

### Ethyl-5-fluor-1-(2-fluorbenzyl)-1H-pyrazolo[3,4-b]pyridin-3-carboxylat

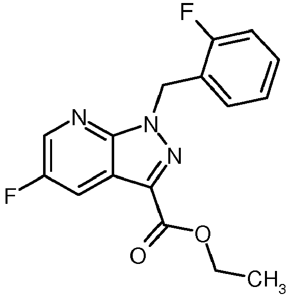

13.487 g (51.228 mmol) Ethyl-5-amino-1-(2-fluorbenzyl)-1H-pyrazol-3-carboxylat (Darstellung beschrieben für Beispiel 20A in WO 00/06569) wurden in 300 ml Dioxan vorgelegt und bei RT mit 6 g (51.228 mmol) 3-(Dimethylamino)-2-fluoracrylaldehyd (Darstellung beschrieben in Justus Liebigs Annalen der Chemie 1970; 99 - 107) versetzt. Anschliessend wurden 4.736 ml (61.473 mmol) Trifluoressigsäure zugegeben und der Ansatz wurde für 3 Tage unter Rühren zum Rückfluss erhitzt. Nach Abkühlen wurde im Vakuum eingeengt und der Rückstand mit Wasser und Essigsäureethylester versetzt. Die Phasen wurden getrennt und die organische Phase wurde zweimal mit Wasser gewaschen. Die vereinigten wässrigen Phasen wurden anschliessend zweimal mit Essigsäureethylester extrahiert. Die vereinigten organischen Phasen wurden über Natriumsulfat getrocknet, filtriert und im Vakuum eingeengt. Der Rückstand (22 g) wurde anschliessend durch Chromatographie an Kieselgel (Laufmittel: Dichlormethan) gereinigt. Man erhielt 5.67 g (35 % d. Th.) der Titelverbindung.

LC-MS (Methode 2): Rₜ = 1.17 min

MS (ESIpos): m/z = 318 (M+H)⁺

¹H-NMR (400 MHz, DMSO-d₆): δ = 1.37 (t, 3H), 4.40 (q, 2H), 5.86 (s, 2H), 7.15 - 7.27 (m, 3H), 7.36 - 7.41 (m, 1H), 8.25 (d, 1H), 8.78 (s br., 1H).

### Beispiel 26A

### 5-Fluor-1-(2-fluorbenzyl)-1H-pyrazolo[3,4-b]pyridin-3-carboxamid

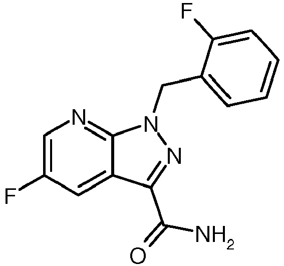

1.00 g (3.152 mmol) der unter Beispiel 25A erhaltenen Verbindung wurden in 10 ml einer 7N Lösung von Ammoniak in Methanol bei RT für drei Tage gerührt. Anschliessend wurde im Vakuum eingeengt. Man erhielt 908 mg (99 % d. Th.) der Titelverbindung.

LC-MS (Methode 2): Rₜ = 0.85 min

MS (ESIpos): m/z = 289 (M+H)⁺

¹H-NMR (400 MHz, DMSO-d₆): δ = 5.87 (s, 2H), 7.12 - 7.26 (m, 3H), 7.34 - 7.40 (m, 1H), 7.60 (s br., 1H), 7.87 (s br., 1H), 8.28 (dd, 1H), 8.72 (dd, 1H).

### Beispiel 27A

### 5-Fluor-1-(2-fluorbenzyl)-1H-pyrazolo[3,4-b]pyridin-3-carbonitril

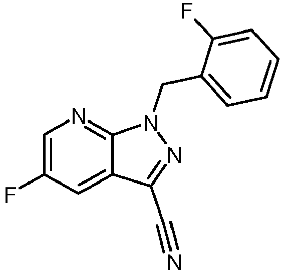

### Variante A:

Eine Suspension aus 16.03 g (43.19 mmol) 5-Fluor-1-(2-fluorbenzyl)-3-iod-1H-pyrazolo[3,4-b]pyridin (Beispiel 24A) und 4.25 g (47.51 mmol) Kupfercyanid wurden in DMSO (120 ml) vorgelegt und 2 h bei 150°C gerührt. Nach Abkühlen wurde der Kolbeninhalt auf ca. 40°C abgekühlt, auf eine Lösung aus konz. Ammoniakwasser (90 ml) und Wasser (500 ml) gegossen, mit Essigsäureethylester (200 ml) versetzt und kurz ausgerührt. Die wässrige Phase wurde abgetrennt und noch zweimal mit Essigsäureethylester (je 200 ml) extrahiert. Die vereinigten organischen Phasen wurden zweimal mit 10%-iger wässriger Natriumchlorid-Lösung (je 100 ml) gewaschen, getrocknet und im Vakuum eingeengt. Das Rohprodukt wurde ohne weitere Reinigung umgesetzt.

Ausbeute: 11.1 g (91 % d. Theorie)

### Variante B:

900 mg (3.122 mmol) der unter Beispiel 26A erhaltenen Verbindung wurden in THF (14 ml) gelöst und mit 0.646 ml (7.993 mmol) Pyridin versetzt. Danach wurden unter Rühren 1.129 ml (7.993 mmol) Trifluoressigsäureanhydrid langsam zugetropft und anschliessend über Nacht bei RT gerührt. Danach wurde das Reaktionsgemisch auf Wasser gegossen und dreimal mit Essigsäureethylester extrahiert. Die vereinigten organischen Phasen wurden mit gesättigter wässriger Natriumhydrogencarbonat-Lösung und 1N Salzsäure extrahiert und danach mit gesättigter wässriger Natriumchlorid-Lösung gewaschen. Die organische Phase wurde über Natriumsulfat getrocknet, filtriert und eingeengt. Man erhielt 850 mg (99 % d. Th.) der Titelverbindung.

LC-MS (Methode 2): Rₜ = 1.06 min

MS (ESIpos): m/z = 271 (M+H)⁺

¹H-NMR (400 MHz, DMSO-d₆): δ = 5.87 (s, 2H), 7.17 - 7.42 (m, 4H), 8.52 (dd, 1H), 8.87 (dd, 1H).

### Beispiel 28A

### 5-Fluor-1-(2-fluorbenzyl)-1H-pyrazolo[3,4-b]pyridin-3-carboximidamid-Acetat

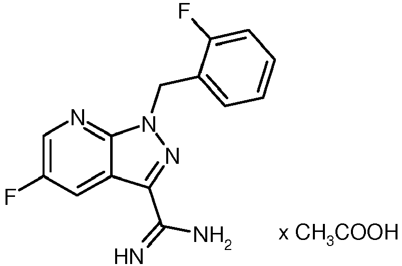

Zu 2.22 g (41.07 mmol) Natriummethanolat in Methanol (270 ml) wurden 11.1 g (41.07 mmol) 5-Fluor-1-(2-fluorbenzyl)-1H-pyrazolo[3,4-b]pyridin-3-carbonitril (Beispiel 27A) gegeben und 2 h bei RT gerührt. Danach wurden 2.64 g (49.29 mmol) Ammoniumchlorid und Essigsäure (9.17 ml) zugegeben und über Nacht zum Rückfluss erhitzt. Danach wurde bis zur Trockene eingeengt und der Rückstand in Wasser (100 ml) und Essigsäureethylester (100 ml) aufgenommen und mit 2N Ntronlauge auf pH 10 gestellt. Es wurde für ca. 1 h bei RT intensiv gerührt. Die erhaltene Suspension wurde abgesaugt und mit Essigsäureethylester (100 ml), Wasser (100 ml) und nochmals Essigsäureethylester (100 ml) nachgewaschen. Der Rückstand wurde über Phosphorpentoxid am Hochvakuum getrocknet.

Ausbeute: 9.6 g (78 % d. Th.)

MS (ESIpos): m/z = 288 (M+H)⁺

¹H-NMR (400 MHz, DMSO-d₆): δ = 1.85 (s, 3H), 5.80 (s, 2H), 7.14 - 7.25 (m, 3H), 7.36 (m, 1H), 8.42 (dd, 1H), 8.72 (dd, 1H).

### Beispiel 29A

### 2-[5-Fluor-1-(2-fluorbenzyl)-1H-pyrazolo[3,4-b]pyridin-3-yl]-5-[(E)-phenyldiazenyl]pyrimidin-4,6-diamin

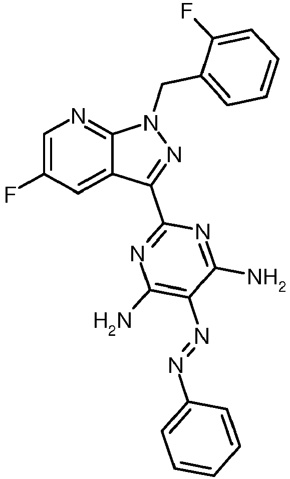

Zu Wasser (40 ml) und konz. Salzsäure (7.07 ml) wurden unter Rühren 3.85 g (41.34 mmol) Anilin gegeben und auf 0°C gekühlt. Anschliessend wurde eine Lösung von 2.85 g (41.34 mmol) Natriumnitrit in Wasser (21 ml) zwischen 0°C und 5°C zugetropft und 15 min bei 0°C nachgerührt. Danach wurde bei 0°C eine Lösung aus 4.28 g (52.25 mmol) Natriumacetat in Wasser (19 ml) zügig zugetropft und dann unter gutem Rühren eine Lösung aus 2.73 g (41.34 mmol) Malonsäuredinitril in Ethanol (10 ml) zugetropft. Nach 2 h bei 0°C wurde der entstandene Niederschlag abgesaugt und dreimal mit Wasser (je 50 ml) und mit Petrolether (50 ml) gewaschen. Der noch feuchte Rückstand wurde in DMF (46 ml) gelöst und in eine Lösung aus 9.5 g (33.07 mmol) 5-Fluor-1-(2-fluorbenzyl)-1H-pyrazolo[3,4-b]pyridin-3-carboximidamid-Acetat (Beispiel 28A) in DMF (46 ml) und Triethylamin (5.76 ml) bei genau 85°C zugetropft. Anschließend wurde 4 h bei 100°C nachgerührt und über Nacht auf RT abkühlen lassen. Die Mischung wurde auf Wasser (480 ml) gegossen und 1 h bei RT ausgerührt. Nach Absaugen des Niederschlags wurde dieser zweimal mit Wasser (je 100 ml) und zweimal mit Methanol (je 50 ml) nachgewaschen, und anschliessend am Hochvakuum getrocknet.

Ausbeute: 9.6 g (59 % d. Theorie)

LC-MS (Methode 2): Rₜ = 1.21 min

MS (ESIpos): m/z = 458 (M+H)⁺

### Beispiel 30A

### 2-[5-Fluor-1-(2-fluorbenzyl)-1H-pyrazolo[3,4-b]pyridin-3-yl]pyrimidin-4,5,6-triamin

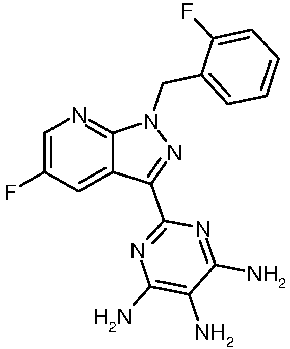

In DMF (800 ml) wurden 39.23 g (85.75 mmol) der Verbindung aus Beispiel 29A vorgelegt und anschliessend 4 g Palladium (10%ig auf Kohle) zugefügt. Es wurde unter Rühren über Nacht bei Wasserstoff-Normaldruck hydriert. Der Ansatz wurde über Kieselgur filtriert und mit wenig DMF und danach mit wenig Methanol nachgewaschen und zur Trockene eingeengt. Der Rückstand wurde mit Essigsäureethylester versetzt und kräftig gerührt, der Niederschlag abgesaugt, mit Essigsäureethylester und Diisopropylether nachgewaschen und über Sicapent am Hochvakuum getrocknet.

Ausbeute: 31.7 g (100 % d. Theorie)

LC-MS (Methode 2): Rₜ = 0.81 min

MS (ESIpos): m/z = 369 (M+H)⁺

### Beispiel 31A

### Methyl-{4,6-diamino-2-[5-fluor-1-(2-fluorbenzyl)-1H-pyrazolo[3,4-b]pyridin-3-yl] pyrimidin-5-yl}carbamat

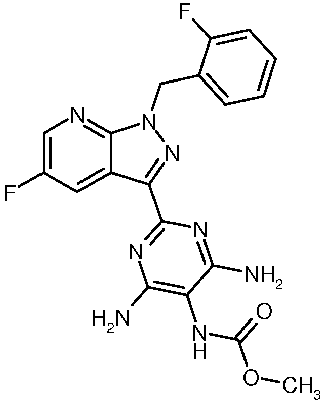

In Pyridin (600 ml) wurden 31.75 g (86.20 mmol) der Verbindung aus Beispiel 30A unter Argon vorgelegt und auf 0°C abgekühlt. Anschließend wurde eine Lösung von 6.66 ml (86.20 mmol) Chlorameisensäuremethylester in Dichlormethan (10 ml) zugetropft und die Mischung 1h bei 0°C gerührt. Danach wurde die Reaktionsmischung auf RT gebracht, im Vakuum eingeengt und mehrfach mit Toluol kodestilliert. Der Rückstand wurde mit Wasser/Ethanol verrührt und danach über eine Fritte abgesaugt und nachfolgend mit Ethanol und Essigsäureethylester gewaschen. Anschliessend wurde der Rückstand abermals mit Diethylether verrührt, abgesaugt und anschliessend am Hochvakuum getrocknet.

Ausbeute: 24.24 g (65 % d. Theorie)

LC-MS (Methode 2): Rₜ = 0.79 min

MS (ESIpos): m/z = 427 (M+H)⁺

¹H-NMR (400 MHz, DMSO-d₆): δ = 3.62 (br. s, 3H), 5.79 (s, 2H), 6.22 (br. s, 4H), 7.10 - 7.19 (m, 2H), 7.19 - 7.26 (m, 1H), 7.32 - 7.40 (m, 1H), 7.67 (br. s, 0.2H), 7.99 (br. s, 0.8H), 8.66 (m, 1H), 8.89 (d, 1H).

### Beispiel 32A

### Methyl- {4,6-diamino-2-[5-fluor-1-(2-fluorbenzyl)-1H-pyrazolo[3,4-b]pyridin-3-yl] pyrimidin-5-yl}(2,2,2-trifluorethyl)carbamat

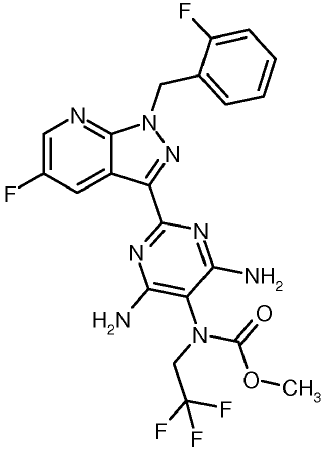

Es wurden 3.470 g (8.138 mmol) der Verbindung aus Beispiel 31A in 35 ml THF suspendiert, bei 0°C mit 358 mg (8.952 mmol) Natriumhydrid (60%-ige Suspension in Mineralöl) versetzt und 90 Min. bei 0°C gerührt, wobei sich eine Lösung bildete. Es wurden 2.519 g (8.952 mmol) 2,2,2-Trifluorethyltrichlormethansulfonat addiert und das Gemisch für 48 h bei RT gerührt. Anschliessend wurde mit Wasser verrührt und am Rotationsverdampfer eingeengt. Der Rückstand wurde in Essigsäureethylester aufgenommen, die organische Phase zweimal mit Wasser gewaschen und über Natriumsulfat getrocknet. Es wurden 5.005 g der Zielverbindung erhalten (79 % d. Th., Reinheit nach HPLC 65%). 250 mg des Rückstands wurden mittels präparativer HPLC gereinigt (Eluent: Methanol/Wasser, Gradient 30:70 → 90:10).

LC-MS (Methode 2): Rₜ = 0.97 min; MS (EIpos): m/z = 509 [M+H]⁺.

¹H-NMR (400 MHz, DMSO-d₆): δ [ppm] = 3.63 (s, 3H), 4.06-4.15 (m, 2H), 5.80 (s, 2H), 6.46 (s br, 4H) 7.11-7.15 (m, 2H), 7.20-7.25 (m, 1H), 7.33-7.38 (m, 1H), 8.66 (dd, 1H), 8.91 (dd, 1H).

### Beispiel 33A

### 1-(2-Fluorbenzyl)-3-iod-1H-pyrazolo[3,4-b]pyridin

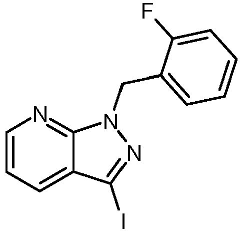

In Analogie zu Beispiel 24A wurden 25.00 g (102.031 mmol) 3-Iod-1H-pyrazolo[3,4-b]pyridin (Synthese beschrieben in WO 2006/130673, Schema D) mit 21.21 g (112.234 mmol) 2-Fluorbenzylbromid umgesetzt. Es wurden 34.49 g der Titelverbindung erhalten (95 % d. Th.).

LC-MS (Methode 2): Rₜ = 1.16 min; MS (ESIpos): m/z = 354 (M+H)⁺

¹H-NMR (400 MHz, DMSO-d₆): δ = 5.74 (s, 2H), 7.13-7.24 (m, 3H), 7.32 - 7.37 (m, 2H), 7.97 (dd, 1H), 8.65 (dd, 1H).

### Beispiel 34A

### N⁵-(1-Cyclopropylpiperidin-4-yl)-2-[1-(2-fluorbenzyl)-1H-pyrazolo[3,4-b]pyridin-3-yl]pyrimidin-4,5-diamin

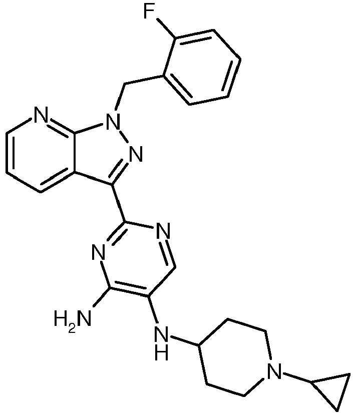

200 mg (0.596 mmol) 2-[1-(2-Fluorbenzyl)-1H-pyrazolo[3,4-b]pyridin-3-yl]pyrimidin-4,5-diamin (Synthese beschrieben in US2004/67937; Example V) wurden in Methanol (16 ml) vorgelegt, mit 75 µl (1.312 mmol) Essigsäure versetzt und anschliessend wurde 182 mg (1.312 mmol) 1-Cyclopropyl-4-piperidinon zugegeben. Nach 15 min Rühren bei RT wurde 104 mg (1.67 mmol) Natriumcyanoborhydrid zugegeben und 2.5 h bei RT gerührt. Im folgenden wurde innerhalb von 2 Tagen dreimal die oben angegebenen Mengen an Reagenzien (1-Cyclopropyl-4-piperidinon, Essigsäure, Natriumcyanoborhydrid) zugesetzt, um einen weitestgehend vollständigen Umsatz zu erzielen. Danach wurde mit gesättigter wässriger Natriumhydrogencarbonat-Lösung (5 ml) versetzt und 10 min kräftig gerührt. Anschliessend wurde mit Wasser und Essigsäureethylester extrahiert. Die Phasen wurden getrennt und die wässrige Phase wurde zweimal mit Essigsäureethylester extrahiert. Die vereinigten organischen Phasen wurden mit gesättigter wässriger Natriumchlorid-Lösung gewaschen, über Natriumsulfat getrocknet, filtriert, eingeengt und danach mittels präparativer HPLC gereinigt (Acetonitril:Wasser (+0.05 % Ameisensäure) - Gradient). Es wurden 165 mg der Titelverbindung erhalten (60 % d. Th.).

LC-MS (Methode 2): Rₜ = 0.60 min; MS (EIpos): m/z = 459 [M+H]⁺.

### Beispiel 35A

### 3,5-Difluorpyridin-2-carbonylchlorid

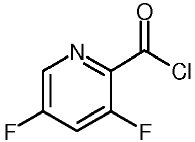

Eine Suspension von 5.00 g (31.4 mmol) 3,5-Difluorpyridin-2-carbonsäure in Thionylchlorid (21 mL) wurde 5 h zum Rückfluss erhitzt. Die Lösung wurde eingeengt, der Rückstand zweimal in wenig Toluol aufgenommen und wieder eingeengt. Man erhielt 3.80 g eines Feststoffs., der ohne weitere Reinigung direkt weiter umgesetzt wurde.

### Beispiel 36A

### Methyl-3-(3,5-difluorpyridin-2-yl)-2-(2-fluorphenyl)-3-oxopropanoat

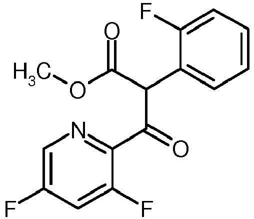

In THF (30 mL) wurden unter Argon 21.4 mL (21.4 mmol) Lithiumhexamethyldisilazid (1.0 M in THF) vorgelegt und bei -78°C eine Lösung von 3.00 g (17.8 mmol) 2-Fluorphenylessigsäuremethylester in THF (15 mL) zugetropft. Die Reaktionsmischung wurde 1 h bei -78°C gerührt und anschließend eine Lösung von 3.80 g (21.4 mmol) der Verbindung aus Beispiel 1A in THF (15 mL) zugetropft. Die Lösung wurde 1h bei -78°C gerührt, danach auf RT gebracht und portionsweise mit gesättigter wässriger Ammoniumchlorid-Lösung versetzt. Die Mischung wurde mit Wasser verdünnt und zweimal mit Essigsäureethylester extrahiert. Die vereinigten organischen Phasen wurden über Natriumsulfat getrocknet, filtriert und eingeengt. Der Rückstand wurde mit tert.-Butylmethylether verrührt, der Feststoff abfiltriert und das Filtrat eingeengt. Kieselgelchromatographie (Laufmittel: Cyclohexan-Essigsäureethylester 30:1, 20:1) des Rückstands lieferte 3.66 g (87%-ige Reinheit, 57 % d. Th.) der Titelverbindung. Das Rohprodukt wurde ohne weitere Reinigung umgesetzt.

LC-MS (Methode 2): Rₜ = 1.05 min; MS (ESIpos): m/z = 310 (M+H)⁺

¹H-NMR (400 MHz, DMSO-d₆): δ = 3.66 (s, 3H), 6.25 (s, 1H), 7.20 - 7.28 (m, 4H), 7.31 - 7.38 (m, 1H), 8.15 - 8.23 (m, 1H), 8.68 - 8.71 (m, 1H).

### Beispiel 37A

### 1-(3,5-Difluorpyridin-2-yl)-2-(2-fluorphenyl)ethanon

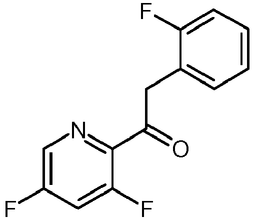

In DMSO (37 mL) wurden 11.65 g (37.67 mmol) der Verbindung aus Beispiel 36A vorgelegt. Anschließend wurden 2.42 g (41.44 mmol) Natriumchlorid sowie Wasser (7 mL) zugefügt und die Mischung 30 min bei 150°C in der Mikrowelle gerührt. Die Reaktionsmischung wurde mit Essigsäureethylester verdünnt, die organische Phase dreimal mit Wasser sowie einmal mit gesättigter wässriger Natriumchlorid-Lösung gewaschen, über Natriumsulfat getrocknet, filtriert und eingeengt. Man erhielt 9.07 g (89%-ige Reinheit, 85 % d. Th.) der Titelverbindung als Feststoff, der ohne weitere Reinigung umgesetzt wurde.

LC-MS (Methode 2): Rt = 1.05 min; MS (ESIpos): m/z = 252 (M+H)⁺

¹H-NMR (400 MHz, DMSO-d₆): δ = 4.53 (s, 2H), 7.15 - 7.22 (m, 2H), 7.30 - 7.37 (m, 2H), 8.11 - 8.18 (m, 1H), 8.70 - 8.72 (m, 1H).

### Beispiel 38A

### 6-Fluor-3-(2-fluorbenzyl)-1H-pyrazolo[4,3-b]pyridin

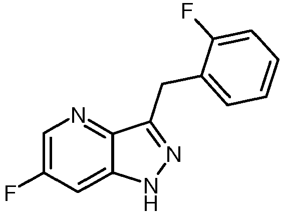

In Pyridin (84 mL) wurden 9.07 g (32.4 mmol) der Verbindung aus Beispiel 37A vorgelegt. Anschließend wurden 8.10 g (162 mmol) Hydrazinhydrat sowie 19.8 mg (0.162 mmol) 4-Dimethylaminopyridin zugefügt und die Mischung 30 min zum Rückfluss erhitzt. Die Reaktionsmischung wurde bei RT mit Essigsäureethylester verdünnt und viermal mit 10%iger wässriger Zitronensäure-Lösung gewaschen. Die organische Phase wurde anschließend mit gesättigter wässriger Natriumchlorid-Lösung gewaschen, über Natriumsulfat getrocknet, filtriert und eingeengt. Der Rückstand wurde mit tert.-Butylmethylether versetzt und der Feststoff abfiltriert. Dieser wurde am Hochvakuum getrocknet und lieferte 1.79 g (79%-ige Reinheit, 18% d. Th.) der Titelverbindung. Das Filtrat wurde eingeengt und lieferte weitere 4.86 g (61%-ige Reinheit, 37% d. Th.) der Titelverbindung. Die beiden Fraktionen wurden vereinigt und ohne weitere Reinigung umgesetzt.

LC-MS (Methode 4): Rₜ = 1.87 min

MS (ESIpos): m/z = 246 (M+H)⁺

¹H-NMR (400 MHz, DMSO-d₆): δ = 4.33 (s, 2H), 7.06 - 7.12 (m, 1H), 7.12 - 7.19 (m, 1H), 7.22 - 7.29 (m, 1H), 7.29 - 7.35 (m, 1H), 7.87 (dd, 1H), 7.84 - 7.89 (m, 1H), 8.48 - 8.51 (br. s, 1H).

### Beispiel 39A

### 6-[6-Fluor-3-(2-fluorbenzyl)-1H-pyrazolo[4,3-b]pyridin-1-yl]-3-nitropyridin-2-amin

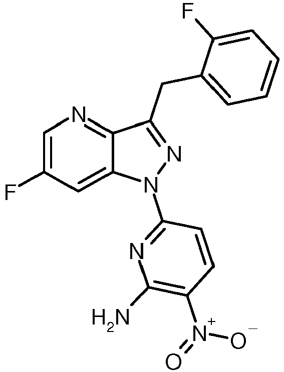

In Dimethylformamid (4 mL) wurden 192 mg (ca. 0.517 mmol) der Verbindung aus Beispiel 38A gelöst, anschließend 34.5 mg (0.862 mmol) Natriumhydrid (60%-ig in Mineralöl) zugefügt und die Mischung 30 min bei RT gerührt. Danach wurden 129 mg (0.744 mmol) 6-Chlor-3-nitropyridin-2-amin zugefügt und die Reaktionsmischung 1 h bei RT gerührt. Die Mischung wurde auf Wasser gegeben, der Feststoff abfiltriert, mehrmals mit Wasser gewaschen und über Nacht am Hochvakuum getrocknet. Man erhielt 348 mg (85%-ige Reinheit, 84 % d. Th.) der Titelverbindung als Feststoff.

LC-MS (Methode 2): Rₜ = 1.29 min

MS (ESIpos): m/z = 383 (M+H)⁺

¹H-NMR (400 MHz, DMSO-d₆): δ = 4.46 (s, 2H), 7.11 - 7.17 (m, 1H), 7.19 - 7.24 (m, 2H), 7.27 - 7.34 (m, 1H), 7.39 - 7.45 (m, 1H), 8.22 - 8.30 (m, 1H), 8.55 (d, 1H), 8.74 - 8.76 (m, 2H), 9.30 - 9.35 (m, 1H).

### Beispiel 40A

### 6-[6-Fluor-3-(2-fluorbenzyl)-1H-pyrazolo[4,3-b]pyridin-1-yl]pyridin-2,3-diamin

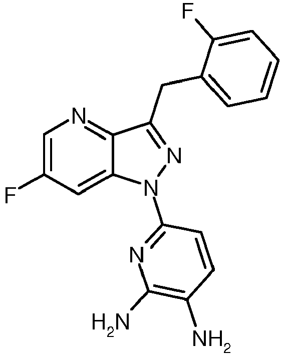

In Pyridin (35 mL) wurden 300 mg (0.785 mmol) der Verbindung aus Beispiel 39A vorgelegt, anschließend 119 mg Palladium (10%-ig auf Kohle) zugefügt und die Mischung über Nacht bei Wasserstoff-Normaldruck hydriert. Die Reaktionsmischung wurde über Kieselgur filtriert, mit Ethanol nachgewaschen und das Filtrat eingeengt. Man erhielt 219 mg (92%-ige Reinheit, 73 % d. Th.) der Titelverbindung als Feststoff.

LC-MS (Methode 2): Rₜ = 1.05 min

MS (ESIpos): m/z = 353 (M+H)⁺

1H-NMR (400 MHz, DMSO-d₆): δ = 4.40 (s, 2H), 4.72 (s, 2H), 5.98 (s, 2H), 6.88 (m, 2H), 7.08 - 7.14 (m, 1H), 7.15 - 7.22 (m, 1H), 7.24 - 7.31 (m, 1H), 7.33 - 7.41 (m, 1H), 8.56 - 8.60 (m, 1H), 8.97 (dd, 1H).

### Beispiel 41A

### tert.-Butyl-({4-amino-2-[1-(2-fluorbenzyl)-1H-pyrazolo[3,4-b]pyridin-3-yl]-7,8-dihydropteridin-5(6H)-yl} sulfonyl)carbamat

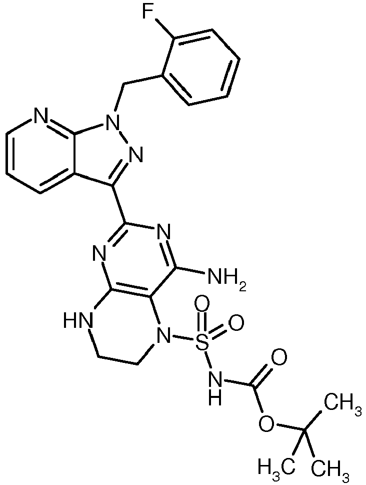

In Dimethylformamid (5 mL) wurden 300 mg (0.727 mmol) der Verbindung aus Beispiel 81 vorgelegt, anschließend 0.25 mL (1.45 mmol) N,N-Diisopropylethylamin zugetropft und danach 188 mg (0.872 mmol) tert.-Butyl-(chlorsulfonyl)carbamat (hergestellt gemäß US 6,313,312, Preparation 83) zugefügt. Die Reaktionslösung wurde über Nacht bei RT gerührt, direkt mittels präparativer HPLC getrennt (Laufmittel: Acetonitril/Wasser mit 0.1 % Ameisensäure Gradient) und die Produktfraktionen eingeengt. Man erhielt 97 mg (92%ig, 22 % d. Th.) der Titelverbindung als Feststoff.

LC-MS (Methode 2): Rt = 0.93 min

MS (ESIpos): m/z = 556 (M+H)⁺

¹H-NMR (400 MHz, DMSO-d₆): δ = 1.38 (s, 9H), 3.17 - 3.28 (m, 2H), 3.47 - 3.56 (m, 2H), 5.80 (s, 2H), 6.79 - 6.92 (m, 2H), 7.08 - 7.15 (m, 2H), 7.19 - 7.26 (m, 1H), 7.30 - 7.39 (m, 2H), 8.58 - 8.62 (m, 1H), 8.99 - 9.04 (m, 1H).

### Beispiel 42A

### 6-[1-(2-Fluorbenzyl)-1H-pyrazolo[3,4-b]pyridin-3-yl]-3-nitropyridin-2-amin

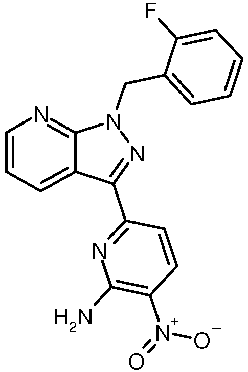

In Dimethylformamid (5.3 ml) wurden 200 mg (0.57 mmol) der Verbindung aus Beispiel 33A unter Argon vorgelegt, anschließend 0.43 mL (0.85 mmol) Hexabutyldizinn sowie 160 mg (0.92 mmol) 6-Chlor-3-nitropyridin-2-amin zugefügt und die Reaktionsmischung 10 min mit Argon gespült. Anschließend wurden 231 mg (0.28 mmol) [1,1'-Bis-(diphenylphosphino)-ferrocen]-dichlorpalladium(II)-Dichlormethan-Komplex (1:1) zugegeben und die Reaktionsmischung über Nacht bei 110°C gerührt. Danach wurde auf RT abgekühlt, mit gesättigter wässriger Natriumhydrogencarbonat-Lösung versetzt und dreimal mit Essigsäureethylester extrahiert. Die vereinigten organischen Phasen wurden über Magnesiumsulfat getrocknet, filtriert und eingeengt. Der Rückstand wurde in Dichlormethan-Methanol (1:1) ausgerührt, der Feststoff abfiltriert und am Hochvakuum getrocknet. Man erhielt 95 mg (54%-ige Reinheit, 15% d. Th.) der Titelverbindung. Das Filtrat wurde an Kieselgel chromatographiert (Laufmittel: Cyclohexan-Essigsäureethylester, 20:1) und die Produktfraktionen eingeengt. Der Rückstand wurde in Methanol suspendiert, der Feststoff abfiltriert und man erhielt nach Trocknung am Hochvakuum weitere 30 mg (15% d. Th.) der Titelverbindung als Feststoff. Die Fraktionen wurden vereinigt und ohne weitere Reinigung umgesetzt (Reinheit ca. 60%).

LC-MS (Methode 2): Rₜ = 1.21 min

MS (ESIpos): m/z = 365 (M+H)⁺

¹H-NMR (400 MHz, DMSO-d₆): δ = 4.46 (s, 2H), 7.11 - 7.17 (m, 1H), 7.19 - 7.24 (m, 2H), 7.27 - 7.34 (m, 1H), 7.39 - 7.45 (m, 2H), 8.22 - 8.30 (m, 2H), 8.55 (d, 1H), 8.74 - 8.76 (m, 1H), 9.30 - 9.35 (m, 1H).

### Beispiel 43A

### 6-[1-(2-Fluorbenzyl)-1H-pyrazolo[3,4-b]pyridin-3-yl]pyridin-2,3-diamin

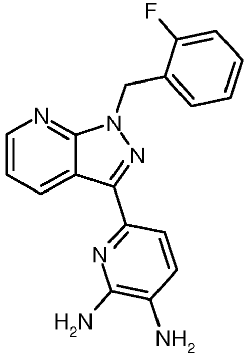

In Pyridin (149 ml) wurden 1.22 g (ca. 3.34 mmol, Reinheit ca. 60%) der Verbindung aus Beispiel 42A vorgelegt und anschließend 505 mg (0.47 mmol) Palladium (10%ig auf Kohle) zugefügt. Die Mischung wurde über Nacht bei RT unter Wasserstoff-Normaldruck hydriert. Anschließend wurde die Reaktionsmischung über Kieselgur filtriert und der Filterkuchen mit Methanol nachgewaschen. Das Filtrat wurde eingeengt, mit Methanol versetzt und der Feststoff abfiltriert. Das Filtrat wurde einrotiert, der Rückstand an Kieselgel chromatographiert (Laufmittel: Cyclohexan-Essigsäureethylester 5:1, 3:1) und die Produktfraktionen eingeengt. Man erhielt 491 mg (44% d. Th.) der Titelverbindung.

LC-MS (Methode 2): Rₜ = 0.74 min

MS (ESIpos): m/z = 335 (M+H)⁺

¹H-NMR (400 MHz, DMSO-d₆): δ = 4.95 (m, 2H), 5.64 (m, 2H), 5.73 (s, 2H), 6.78 (d, 1H), 7.09 - 7.14 (m, 2H), 7.16 - 7.28 (m, 3H), 7.30 - 7.37 (m, 1H), 8.55 (dd, 1H), 9.06 (dd, 1H).

### Beispiel 44A

### Methyl-2-[1-(2-fluorbenzyl)-1H-pyrazolo[3,4-b]pyridin-3-yl]-4-methylpyrimidin-5-carboxylat

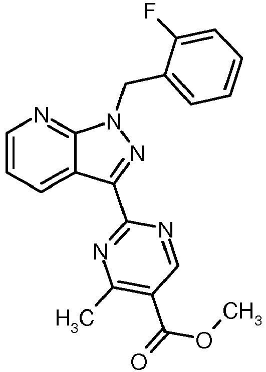

In Methanol (10 mL) wurden 2.00 g (6.54 mmol) der Verbindung aus Beispiel 5A vorgelegt und eine Lösung von 6.5 mL 1M Natronlauge in Methanol (10 mL) zugefügt. Anschließend wurden 1.03 g (6.54 mmol) Methyl-(2E)-2-(methoxymethylen)-3-oxobutanoat (Russ. J. Org. Chem. 2003, 39, 273) zugetropft und die Reaktionsmischung über Nacht bei RT gerührt. Anschließend wurden weitere 6.5 mL 1M Natronlauge in Methanol sowie 2.06 g (13.08 mmol) Methyl-(2E)-2-(methoxymethylen)-3-oxobutanoat zugefügt und die Mischung erneut über Nacht bei RT gerührt. Danach wurde die Reaktionslösung auf die Hälfte des Volumens eingeengt und mit tert.-Butylmethylether versetzt, wobei ein Feststoff ausfiel. Dieser wurde abfiltriert, mit Wasser sowie tert.-Butylmethylether gewaschen und am Hochvakuum getrocknet. Man erhielt 1.68 g (68 % d. Th.) der Titelverbindung als Feststoff.

LC-MS (Methode 2): Rₜ = 1.17 min

MS (ESIpos): m/z = 378 (M+H)⁺

¹H-NMR (400 MHz, DMSO-d₆): δ = 2.86 (s, 3H), 3.92 (s, 3H), 5.91 (s, 2H), 7.13 - 7.19 (m, 1H), 7.24 (m, 2H), 7.34 - 7.41 (m, 1H), 7.46 - 7.51 (m, 1H), 8.69 - 8.74 (m, 1H), 8.93 - 8.99 (m, 1H), 9.22 (s, 1H).

### Beispiel 45A

### 2-[1-(2-Fluorbenzyl)-1H-pyrazolo[3,4-b]pyridin-3-yl]-4-methylpyrimidin-5-carbonsäure

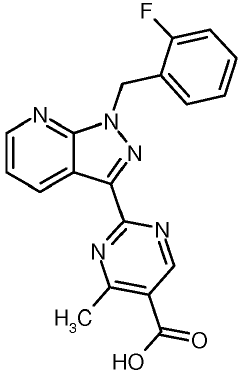

In Tetrahydrofuran (10 mL) wurde 830 mg (2.20 mmol) der Verbindung aus Beispiel 44A vorgelegt, mit 4.40 mL (4.40 mmol) wässriger Lithiumhydroxid-Lösung (1.0 M) versetzt und die Suspension 90 min bei RT gerührt. Anschließend wurde die nun klare Lösung eingeengt und der Rückstand zwischen Essigsäureethylester und Wasser verteilt. Die wässrige Phase wurde mit 1M Salzsäure auf pH < 7 gestellt, wobei ein Feststoff ausfiel. Dieser wurde abfiltriert, mit Wasser sowie tert.-Butylmethylether gewaschen und am Hochvakuum getrocknet. Man erhielt 710 mg (89 % d. Th.) der Titelverbindung als Feststoff.

LC-MS (Methode 2): Rₜ = 0.99 min

MS (ESIpos): m/z = 364 (M+H)⁺

¹H-NMR (400 MHz, DMSO-d₆): δ = 2.85 (s, 3H), 5.90 (s, 2H), 7.13 - 7.19 (m, 1H), 7.20 - 7.27 (m, 2H), 7.33 - 7.41 (m, 1H), 7.45 - 7.50 (m, 1H), 8.68 - 8.72 (m, 1H), 8.93 - 8.98 (m, 1H), 9.17 (s, 1H).

### Beispiel 46A

### 2-[1-(2-Fluorbenzyl)-1H-pyrazolo[3,4-b]pyridin-3-yl]-4-methylpyrimidin-5-carboxamid

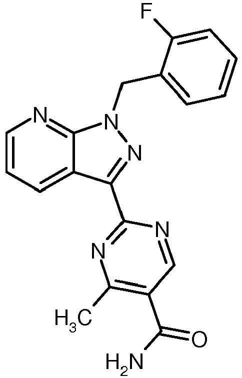

Unter Argon wurden 250 mg (0.688 mmol) der Verbindung aus Beispiel 45A in Dimethylformamid (3.8 mL) vorgelegt. Anschließend wurden 145 mg (0.757 mmol) EDC sowie 116 mg (0.757 mmol) HOBt zugefügt und schließlich 77 µL (1.03 mmol) wässrige AmmoniakLösung (25%ig) zugegeben. Die Reaktionsmischung wurde über Nacht bei RT gerührt und danach mit Essigsäureethylester verdünnt. Die organische Phase wurde je einmal mit Wasser sowie gesättigter wässriger Natriumchlorid-Lösung gewaschen, über Magnesiumsulfat getrocknet, filtriert und einrotiert. Der Rückstand wurde mit tert.-Butylmethylether versetzt, der Feststoff abfiltriert und am Hochvakuum getrocknet. Man erhielt 171 mg (66 % d. Th.) der Titelverbindung als Feststoff.

LC-MS (Methode 2): Rₜ = 0.88 min

MS (ESIpos): m/z = 363 (M+H)⁺

¹H-NMR (400 MHz, DMSO-d₆): δ = 2.70 (s, 3H), 5.89 (s, 2H), 7.13 - 7.18 (m, 1H), 7.19 - 7.27 (m, 2H), 7.34 - 7.41 (m, 1H), 7.46 (dd, 2H), 7.78 (br. s, 1H), 8.13 (br. s, 1H), 8.67 - 8.71 (m, 1H), 8.89 (s, 1H), 8.91 - 8.95 (m, 1H).

### Beispiel 47A

### N-(5-Cyanpyrimidin-4-yl)-1-(2-fluorbenzyl)-1H-pyrazolo[3,4-b]pyridin-3-carboxamid

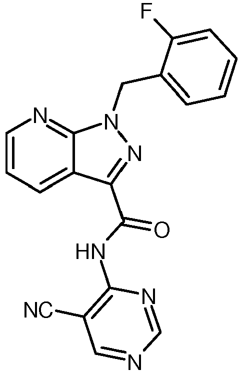

Bei RT wurden 39.4 g (145 mmol) 1-(2-Fluorbenzyl)-1H-pyrazolo[3,4-b]pyridin-3-carbonsäure (Herstellung beschrieben in US2010/004235, Seite 27) vorgelegt und anschließend Thionylchlorid (370 mL) zugefügt. Die Suspension wurde 2 h zum Rückfluss erhitzt und die nun klare Lösung im Vakuum eingeengt. Vom so hergestellten Säurechlorid wurden 100 mg (0.345 mmol) in Pyridin (1.0 mL) vorgelegt und anschließend 41.5 mg (0.345 mmol) 4-Amino-5-cyanopyrimidin portionsweise zugefügt. Die Mischung wurde 7 h bei RT gerührt und über Nacht stehen gelassen. Anschließend wurden flüchtige Bestandteile am Hochvakuum entfernt, der Rückstand mittels präparativer HPLC getrennt (Laufmittel: Acetonitril/Wasser mit 0.1 % Ameisensäure Gradient) und die Produktfraktionen eingeengt. Man erhielt 30 mg (23 % d. Th.) der Titelverbindung als Feststoff.

LC-MS (Methode 1): Rₜ = 2.06 min

MS (ESIpos): m/z = 374 (M+H)⁺

¹H-NMR (400 MHz, DMSO-d₆): δ = 5.91 (s, 2H), 7.14 - 7.20 (m, 1H), 7.22 - 7.33 (m, 2H), 7.35 - 7.42 (m, 1H), 7.49 - 7.54 (m, 1H), 8.57 - 8.62 (m, 1H), 8.73 - 8.77 (m, 1H), 9.28 (d, 2H), 11.64 (s, 1H).

### Beispiel 48A

### N-(5-Carbamoylpyrimidin-4-yl)-1-(2-fluorbenzyl)-1H-pyrazolo[3,4-b]pyridin-3-carboxamid

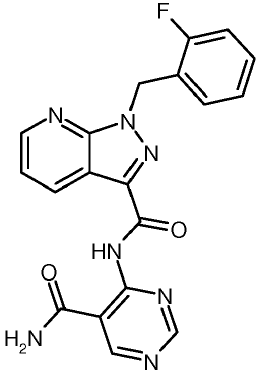

In konz. Schwefelsäure (2.0 mL) wurden 581 mg (1.56 mmol) der Verbindung aus Beispiel 47A bei 0°C portionsweise eingetragen und die Mischung 30 min nachgerührt. Anschließend wurde die Lösung auf Eis gegeben und zweimal mit Essigsäureethylester extrahiert. Die vereinigten organischen Phasen wurden über Natriumsulfat getrocknet, filtriert und eingeengt. Der Rückstand wurde mit Essigsäureethylester verrührt, der Feststoff abfiltriert und am Hochvakuum getrocknet. Man erhielt 246 mg (40 % d. Th.) der Titelverbindung als Feststoff.

LC-MS (Methode 3): Rₜ = 0.92 min

MS (ESIpos): m/z = 392 (M+H)⁺.

### Beispiel 49A

### Prop-1-en-2-yl-{4,6-diamino-2-[3-(2-fluorbenzyl)-1H-pyrazolo[4,3-b]pyridin-1-yl]pyrimidin-5-yl}carbamat

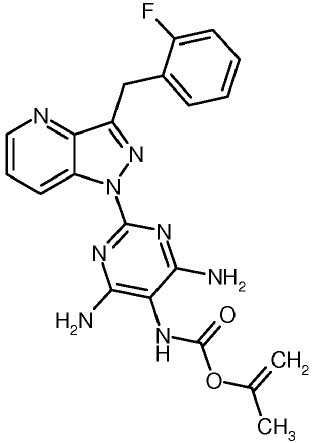

In Pyridin (5.4 mL) wurden 540 mg (1.54 mmol) 2-[3-(2-Fluorbenzyl)-1H-pyrazolo[4,3-b]pyridin-1-yl]pyrimidin-4,5,6-triamin (Beispiel 2A) vorgelegt und die Mischung auf 0°C abgekühlt. Anschließend wurden 223 mg (1.85 mmol) Chlorameisensäurepropenylester zugetropft und die Mischung weitere 5 min bei 0°C sowie 1 h bei RT gerührt. Anschließend wurde die Reaktionsmischung mit Wasser verdünnt und dreimal mit Essigsäureethylester extrahiert. Die gesammelten organischen Phasen wurden über Magnesiumsulfat getrocknet, filtriert und einrotiert. Der Rückstand wurde mit tert.-Butylmethylether ausgerührt, filtriert und 2 h bei 40°C am Hochvakuum getrocknet. Man erhielt 335 mg (79%-ige Reinheit, 39 % d. Th.) der Titelverbindung als Feststoff. Das Rohprodukt wurde ohne weitere Reinigung umgesetzt.

LC-MS (Methode 2): Rₜ = 0.87 min

MS (ESIpos): m/z = 435 (M+H)⁺.

### Beispiel 50A

### Methyl-{4,6-diamino-2-[3-(2-fluorbenzyl)-1H-pyrazolo[4,3-b]pyridin-1-yl]pyrimidin-5-yl}(4-fluorbenzyl)carbamat

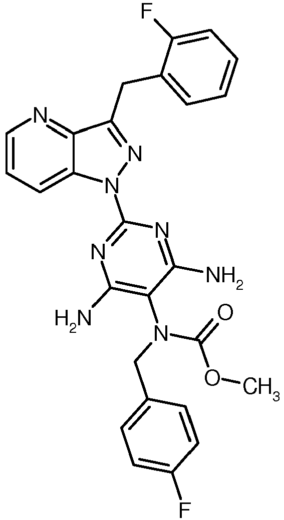

In Tetrahydrofuran (10.0 mL) wurden unter Argon 100 mg (0.245 mmol) Methyl-{4,6-diamino-2-[3-(2-fluorbenzyl)-1H-pyrazolo[4,3-b]pyridin-1-yl]pyrimidin-5-yl}carbamat (Herstellung beschrieben in WO 2008/031513, Beispiel 9) vorgelegt und die Suspension auf 0°C abgekühlt. Anschließend wurden 9.8 mg (0.245 mmol) Natriumhydrid (60%-ig in Mineralöl) zugegeben und die Mischung weitere 30 min bei 0°C gerührt. Danach wurden 30.5 µL (0.245 mmol) 4-Fluorbenzylbromid zugetropft und die Reaktionsmischung bei RT über Nacht gerührt. Anschließend wurde die Reaktionsmischung mit Essigsäureethylester verdünnt, die organische Phase zweimal mit gesättigter wässriger Natriumhydrogencarbonat-Lösung gewaschen, über Magnesiumsulfat getrocknet, filtriert und einrotiert. Der Rückstand wurde mittels präparativer HPLC (Laufmittel: Acetonitril/Wasser mit 0.1% Ameisensäure Gradient) getrennt und die Produktfraktionen eingeengt. Man erhielt 91.8 mg (72 % d. Th.) der Titelverbindung als Feststoff.

LC-MS (Methode 2): Rₜ = 1.03 min

MS (ESIpos): m/z = 517 (M+H)⁺

¹H-NMR (400 MHz, DMSO-d₆): δ = 3.60 (s, 2H), 3.75 (s, 1H), 4.39 (s, 2H), 4.59 (m, 2H), 6.34 (br. s, 4H), 7.01 - 7.13 (m, 3H), 7.13 - 7.21 (m, 1H), 7.22 - 7.35 (m, 2H), 7.36 - 7.44 (m, 2H), 7.44 - 7.50 (m, 1H), 8.56 - 8.61 (m, 1H), 9.11 (d, 1H).

### Beispiel 51A

### 2-Chlorethyl-{4,6-diamino-2-[3-(2-fluorbenzyl)-1H-pyrazolo[4,3-b]pyridin-1-yl]pyrimidin-5-yl}carbamat

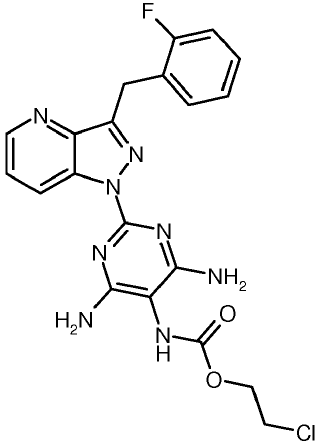

Die Verbindung wurde aus 100 mg (0.285 mmol) 2-[3-(2-Fluorbenzyl)-1H-pyrazolo[4,3-b]pyridin-1-yl]pyrimidin-4,5,6-triamin (Beispiel 2A) und 38 µL (0.371 mmol) 2-Chlorethanol gemäß der Vorschrift für Beispiel 6A hergestellt. Man erhielt 140 mg (91%-ige Reinheit, 98 % d. Th.) der Titelverbindung als Feststoff.

LC-MS (Methode 4): Rₜ = 1.75 min

MS (ESIpos): m/z = 457 (M+H)⁺.

### Beispiel 52A

### 5-[1-(2-Fluorbenzyl)-1H-pyrazolo[3,4-b]pyridin-3-yl]pyrazin-2,3-diamin

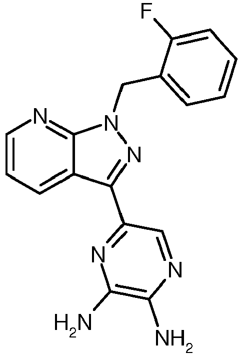

In 1,4-Dioxan (11 mL) wurden 425 mg (1.20 mmol) der Verbindung aus Beispiel 33A unter Argon vorgelegt und die Reaktionsmischung 10 min mit Argon gespült. Danach wurden 0.91 mL (1.80 mmol) Hexabutyldizinn sowie 250 mg (1.32 mmol) 2,3-Diamino-5-brompyrazin zugefügt. Anschließend wurden 422 mg (0.60 mmol) Bis(triphenylphosphin)palladium(II)chlorid zugegeben und die Reaktionsmischung über Nacht zum Rückfluss erhitzt. Danach wurde auf RT abgekühlt, die Mischung durch Celite filtriert und das Filtrat eingeengt. Der Rückstand wurde mit Methanol versetzt, der Feststoff abfiltriert und verworfen. Das Filtrat wurde in Methanol-Dichlormethan aufgenommen, an Diatomeenerde absorbiert und an Kieselgel (Laufmittel: Cyclohexan-Essigsäureethylester 2:1, 1:1) gereinigt. Man erhielt 151 mg (31%-ige Reinheit, 11% d. Th.) der Titelverbindung. Das Rohprodukt wurde ohne weitere Reinigung weiter umgesetzt.

LC-MS (Methode 3): Rₜ = 0.91 min

MS (ESIpos): m/z = 336 (M+H)⁺

### Beispiel 53A

### 2-[1-(2-Fluorbenzyl)-1H-pyrazolo[3,4-b]pyridin-3-yl]-6-methyl-5-nitropyrimidin-4-amin

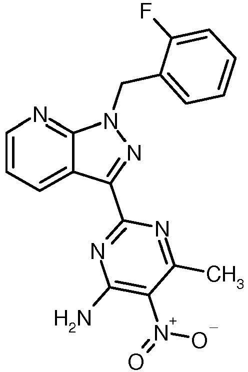

In 1,4-Dioxan (37.5 mL) wurden 1.50 g (4.25 mmol) der Verbindung aus Beispiel 33A unter Argon vorgelegt und die Reaktionsmischung 10 min mit Argon gespült. Danach wurden 3.22 mL (6.37 mmol) Hexabutyldizinn sowie 881 mg (4.67 mmol) 2-Chlor-6-methyl-5-nitropyrimidin-4-amin zugefügt. Anschließend wurden 1.49 g (2.12 mmol) Bis(triphenylphosphin)palladium(II)-chlorid zugegeben und die Reaktionsmischung über Nacht zum Rückfluss erhitzt. Danach wurde auf RT abgekühlt, die Mischung durch Celite filtriert und mit Methanol nachgewaschen. Der gebildete Feststoff wurde abfiltriert und verworfen. Das verbliebene Filtrat wurde eingeengt, mit Essigsäureethylester versetzt, der entstandene Feststoff abfiltriert und am Hochvakuum getrocknet. Man erhielt 640 mg (69%-ige Reinheit, 27% d. Th.) der Titelverbindung. Das Rohprodukt wurde ohne weitere Reinigung weiter umgesetzt.

LC-MS (Methode 2): Rₜ = 1.09 min

MS (ESIpos): m/z = 380 (M+H)⁺

### Beispiel 54A

### 2-[1-(2-Fluorbenzyl)-1H-pyrazolo[3,4-b]pyridin-3-yl]-6-methylpyrimidin-4,5-diamin

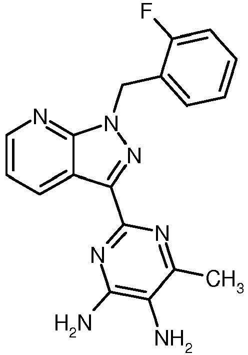

In Pyridin (25 mL) wurden 592 mg (1.56 mmol) der Verbindung aus Beispiel 53A vorgelegt und anschließend 133 mg (0.125 mmol) Palladium (10%-ig auf Kohle) zugefügt. Die Mischung wurde über Nacht bei Wasserstoff-Normaldruck gerührt. Anschließend wurde die Suspension durch Celite filtriert und der Filterkuchen mit Methanol nachgewaschen. Das Filtrat wurde eingeengt, mit Methanol versetzt, filtriert und erneut eingeengt. Der Rückstand wurde am Hochvakuum getrocknet. Man erhielt 396 mg der Titelverbindung (80%-ige Reinheit, 45% d. Th.), die ohne weitere Reinigung umgesetzt wurden.

LC-MS (Methode 3): Rₜ = 0.86 min

MS (ESIpos): m/z = 350 (M+H)⁺

### Beispiel 55A

### Methyl-N-{4,6-diamino-2-[1-(2-fluorbenzyl)-1H-pyrazolo[3,4-b]pyridin-3-yl]pyrimidin-5-yl}-N-(methoxycarbonyl)glycinat

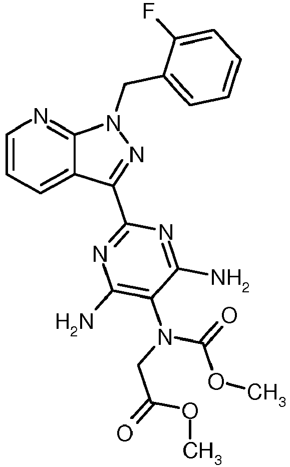

In Tetrahydrofuran (50 ml) wurden 3.00 g (7.346 mmol) der Verbindung aus Beispiel 11A auf 0°C abgekühlt und mit 7.346 ml (7.346 mmol) Bis-(trimethylsilyl)natriumamid-Lösung (1.0 M in Tetrahydrofuran) versetzt. Anschliessend wurde unter Rühren 0.695 ml (7.346 mmol) Bromessigsäuremethylester zugetropft und für weitere 20 min bei 0°C belassen. Danach wurde über Nacht bei RT gerührt. Dann wurde der Ansatz mit gesättigter wässriger Ammoniumchlorid-Lösung (20 ml) abgebrochen und die Phasen wurden getrennt. Die organische Phase wurde noch zweimal mit gesättigter wässriger Ammoniumchlorid-Lösung gewaschen, mit Natriumsulfat getrocknet, filtriert und bis zur Trockene eingeengt. Man erhielt 3.68 g (100% d. Th.) der Titelverbindung. Das Rohprodukt wurde ohne weitere Reinigung weiter umgesetzt.

LC-MS (Methode 2): Rₜ = 0.83 min

MS (ESIpos): m/z = 481 (M+H)⁺

### Beispiel 56A

### Methyl-1-{4,6-diamino-2-[1-(2-fluorbenzyl)-1H-pyrazolo[3,4-b]pyridin-3-yl]pyrimidin-5-yl}hydrazincarboxylat

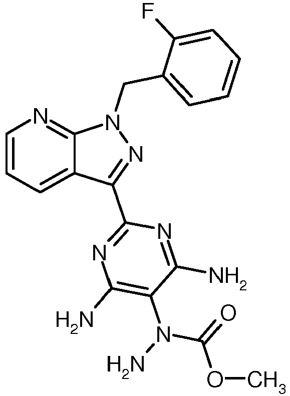

500 mg (1.224 mmol) der unter Beispiel 11A erhaltenen Verbindung wurden in 12.5 ml Tetrahydrofuran bei 0°C vorgelegt. Dann wurden 1.224 ml einer 1M Lösung von Bis-(trimethylsilyl)natriumamid in Tetrahydrofuran zugetropft und es wurde für weitere 20 min bei 0°C gerührt. Dann wurden 334 mg (1.836 mmol) 0-(4-Nitrobenzoyl)hydroxylamin zugegeben und es wurde 2 h bei RT gerührt. Anschliessend wurde das Reaktionsgemisch bis zur Trockene eingeengt. Der Rückstand wurde mittels präparativer HPLC gereinigt (Acetonitril/Wasser (+0.05 % Ameisensäure) - Gradient). Die vereinigten, eingeengten produkt-haltigen Fraktionen wurden nochmals mittels Chromatographie an Kieselgel (Dichlormethan-Methanol 100:1) gereinigt. Abschliessend wurden die vereinigten, eingeengten produkt-haltigen Fraktionen der Reinigung an Kieselgel abermals mittels präparativer HPLC gereinigt (Acetonitril:Wasser (+0.05 % Ameisensäure) - Gradient). Es wurden 79 mg der Titelverbindung erhalten (14 % d. Th.).

LC-MS (Methode 2): Rₜ = 0.71 min; MS (ESIpos): m/z = 424 (M+H)⁺

¹H-NMR (400 MHz, DMSO-d₆): δ [ppm] = 3.55 (s, 2.3H), 3.68 (s, 0.7H), 5.02 (s br, 2H), 5.80 (s, 2H), 6.37 (s br, 4H) 7.09-7.15 (m, 2H), 7.20-7.25 (m, 2H), 7.32-7.38 (m, 2H), 8.60 (dd, 1H), 9.04 (dd, 1H).

### Beispiel 57A

### 5-{[tert.-Butyl(dimethyl)silyl]oxy}-2-[1-(2-fluorbenzyl)-1H-pyrazolo[3,4-b]pyridin-3-yl]pyrimidin-4,6-diamin

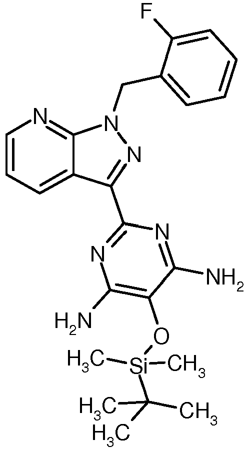

1.245 g (4.075 mmol) der unter Beispiel 5A erhaltenen Verbindung wurden in 10 ml tert.-Butanol vorgelegt und mit 548 mg (4.890 mmol) Kalium-tert.-butylat versetzt. Dann wurden 800 mg (4.075 mmol) [tert.-Butyl(dimethyl)silyl]oxy}malononitril (Journal of Organic Chemistry; 55, 1990; 4515 - 4516) in 6 ml tert.-Butanol zugegeben und es wurde über Nacht zum Rückfluss erhitzt. Nach Abkühlen wurde das Reaktionsgemisch mit Essigsäureethylester und Wasser versetzt und die Phasen getrennt. Die wässrige Phase wurde zweimal mit Essigsäureethylester extrahiert und die vereinigten organischen Phasen wurden einmal mit Wasser und einmal mit gesättigter wässriger Natriumchlorid-Lösung gewaschen, mit Natriumsulfat getrocknet, filtriert und bis zur Trockene eingeengt. Der Rückstand wurde mittels Chromatographie an Kieselgel (Cyclohexan-Essigsäureethylester) gereinigt. Es wurden 450 mg der Titelverbindung erhalten (Reinheit 73%, 23 % d. Th.).

LC-MS (Methode 2): Rₜ = 1.11 min; MS (ESIpos): m/z = 466 (M+H)⁺

### Beispiel 58A

### 4,6-Diamino-2-[1-(2-fluorbenzyl)-1H-pyrazolo[3,4-b]pyridin-3-yl]pyrimidin-5-ol

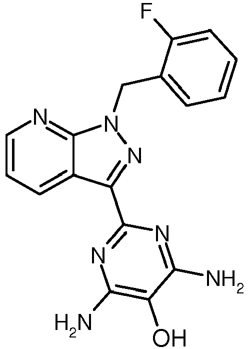

447 mg (0.96 mmol) der unter Beispiel 57A erhaltenen Verbindung wurden in 10 ml Tetrahydrofuran vorgelegt und mit 6 ml 3M Salzsäure versetzt. Am nächsten Tag wurde bis zur Trockene eingeengt und der Rückstand mit Acetonitril, Wasser und Dimethylformamid verrührt. Der Niederschlag wurde abfiltriert und mit Acetonitril nachgewaschen. Es wurden 159 mg der Titelverbindung erhalten (Reinheit 92 %, 43 % d. Th.).

LC-MS (Methode 2): Rₜ = 0.71 min; MS (ESIpos): m/z = 352 (M+H)⁺

### Beispiel 59A

### 6-Amino-7-(2-{[tert.-butyl(dimethyl)silyl]oxy}ethyl)-2-[1-(2-fluorbenzyl)-1H-pyrazolo[3,4-b]pyridin-3-yl]-7,9-dihydro-8H-purin-8-on

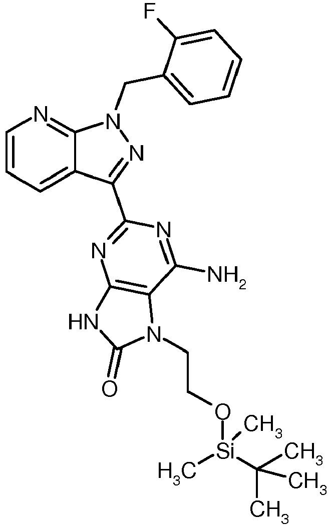

186 mg (0.442 mmol) der unter Beispiel 1 erhaltenen Verbindung wurden in 3 ml Dimethylformamid mit 47 mg (0.686 mmol) Imidazol und 100 mg (0.664 mmol) tert.-Butyldimethlysilylchlorid versetzt. Danach wurde 2 h bei RT gerührt. Anschliessend wurde der Ansatz mit Essigsäureethylester versetzt und mit 0.1 M Salzsäure extrahiert. Die Phasen wurden getrennt und die wässrige Phase wurde mit Essigsäureethylester extrahiert. Die vereinigten organischen Phasen wurden mit Wasser, gesättigter wässriger Natriumhydrogencarbonat-Lösung und dann mit gesättigter wässriger Natriumchlorid-Lösung gewaschen, über Natriumsulfat getrocknet, filtriert und bis zur Trockene eingeengt.. Es wurden 243 mg der Titelverbindung erhalten (94%-ige Reinheit, 96 % d. Th.), welche ohne weitere Reinigung in der nächsten Stufe eingesetzt wurdem.

LC-MS (Methode 2): Rₜ = 1.20 min; MS (ESIpos): m/z = 535 (M+H)⁺

¹H-NMR (400 MHz, DMSO-d₆): δ [ppm] = 0.00 (s, 6H), 0.86 (s, 9H), 3.91 (t, 2H), 4.19 (t, 2H), 5.92 (s, 2H), 6.76 (s, 2H), 7.25-7.37 (m, 3H), 7.46-7.51 (m, 2H), 8.75 (dd, 1H), 9.15 (dd, 1H), 11.76 (s br, 1H).

### Beispiel 60A

### 7-(2-{[tert.-Butyl(dimethyl)silyl]oxy}ethyl)-2-[1-(2-fluorbenzyl)-1H-pyrazolo[3,4-b]pyridin-3-yl]-6-iod-7,9-dihydro-8H-purin-8-on

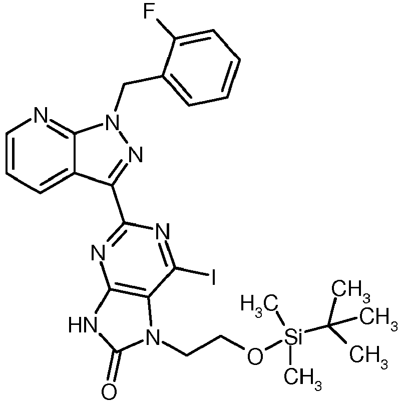

241 mg (0.423 mmol) der unter Beispiel 59A erhaltenen Verbindung wurden in Analogie zur Vorschrift beschrieben unter Beispiel 101 umgesetzt. Das Reaktionsgemisch wurde per Chromatographie an Kieselgel (Dichlormethan/ Methanol) gereinigt. Es wurden 232 mg der Titelverbindung erhalten (46 %-ige Reinheit, 80 % d. Th.), welche ohne weitere Reinigung in der nächsten Stufe eingesetzt worden sind.

LC-MS (Methode 2): Rₜ = 1.45 min; MS (ESIpos): m/z = 646 (M+H)⁺

### Beispiel 61A

### 2-[1-(2-Fluorbenzyl)-1H-pyrazolo[3,4-b]pyridin-3-yl]-N⁵-isopropylpyrimidin-4,5,6-triamin

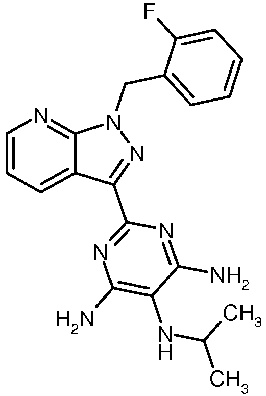

Zu einer Lösung von Methanol (20.0 mL) sowie 180 µL Essigsäure wurden 500 mg (1.427 mmol) der Verbindung aus Beispiel 1A addiert, anschließend 231 µL Aceton zugefügt und die Mischung 15 min gerührt. Danach wurden 251 mg (4.00 mmol) Natriumcyanoborhydrid zugefügt und die Reaktionsmischung über Nacht weiter bei RT gerührt. Anschließend wurde gesättigte wässrige Natriumhydrogencarbonat-Lösung zugefügt und weitere 30 min gerührt. Danach wurde die Mischung eingeengt und der Rückstand in Essigsäureethylester aufgenommen. Die organische Phase wurde zweimal mit Wasser sowie einmal mit gesättigter wässriger Natriumchlorid-Lösung gewaschen, über Natriumsulfat getrocknet, filtriert und eingeengt. Man erhielt 517 mg (92 % d. Th.) der Titelverbindung als Feststoff.

LC-MS (Methode 2): Rₜ = 0.84 min

MS (EIpos): m/z = 393 (M+H)⁺.

¹H-NMR (400MHz, DMSO-d₆): δ [ppm]= 1.05 (d, 6H), 3.11 - 3.19 (m, 2H), 5.77 (s, 2H), 5.99 (s, 4H), 7.09 - 7.16 (m, 2H), 7.19 - 7.26 (m, 1H), 7.29 - 7.38 (m, 2H), 8.57 - 8.60 (m, 1H), 9.03 - 9.08 (m, 1H).

### Beispiel 62A

### Ethyl-6-amino-2-[1-(2-fluorbenzyl)-1H-pyrazolo[3,4-b]pyridin-3-yl]-5-nitropyrimidin-4-carboxylat

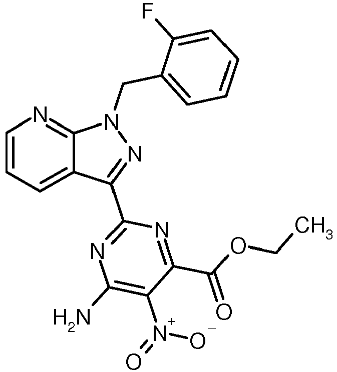

In Dioxan (100 ml) wurden 3.85 g (10.91 mmol) der Verbindung aus Beispiel 24A unter Argon vorgelegt und die Reaktionsmischung mit Argon gespült. Anschließend wurden 8.27 mL (16.36 mmol) Hexabutyldizinn sowie 2.96 g (12.00 mmol) Ethyl-6-amino-2-chlor-5-nitropyrimidin-4-carboxylat (hergestellt gemäß J. Chem. Res. 1989, 2086-2097) zugefügt. Danach wurden 3.83 g (5.45 mmol) Bis(triphenylphosphin)palladium(II)chlorid zugegeben und die Reaktionsmischung über Nacht bei 100°C gerührt. Danach wurde die Mischung auf RT abgekühlt, über Celite filtriert und der Rückstand mit Methanol nachgewaschen. Das Filtrat wurde eingeengt, der Rückstand mit Essigsäureethylester ausgerührt und abfiltriert. Das Filtrat wurde eingeengt und der Rückstand mittels präp HPLC (Laufmittel: Acetonitril/Wasser mit 0.1% Ameisensäure Gradient) gereinigt. Man erhielt 1.36 g (71%-ige Reinheit, 14% d. Th.) Rohprodukt, das ohne weitere Reinigung umgesetzt wurde.

LC-MS (Methode 2): Rₜ = 1.13 min; MS (ESIpos): m/z = 438 [M+H]⁺.

### Beispiel 63A

### Ethyl-5,6-diamino-2-[1-(2-fluorbenzyl)-1H-pyrazolo[3,4-b]pyridin-3-yl]pyrimidin-4-carboxylat

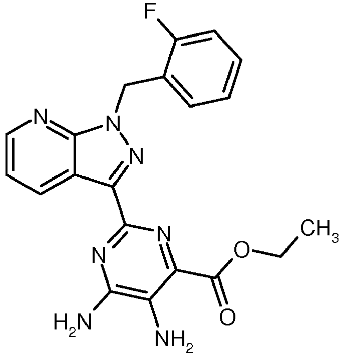

In Pyridin (9 ml) wurden 130 mg (0.30 mmol) der Verbindung aus Beispiel 62A vorgelegt und anschließend 25 mg Palladium (10%ig auf Kohle) zugefügt. Die Mischung wurde über Nacht bei RT unter Wasserstoff-Normaldruck hydriert. Anschließend wurde die Reaktionsmischung durch Celite filtriert und der Filterkuchen mit Methanol nachgewaschen. Das Filtrat wurde eingeengt, der Rückstand mit Methanol versetzt und der Feststoff abfiltriert. Das Filtrat wurde einrotiert und der Rückstand am Hochvakuum getrocknet. Man erhielt 51 mg (83%-ige Reinheit, 36% d. Th.) der Titelverbindung, die ohne weitere Reinigung umgesetzt wurden.

LC-MS (Methode 2): Rₜ = 0.98 min

MS (ESIpos): m/z = 408 (M+H)⁺

¹H-NMR (400MHz, DMSO-d₆): δ [ppm]= 1.40 (t, 3H), 4.36 (q, 2H), 5.79 (s, 2H), 6.61 - 6.66 (m, 2H), 7.08 - 7.16 (m, 2H), 7.20 - 7.28 (m, 3H), 7.31 - 7.39 (m, 2H), 8.58 - 8.62 (m, 1H), 9.02 - 9.06 (m, 1H).

### Beispiel 64A

### N⁵-(2,2-Dimethylpropyl)-2-[1-(2-fluorbenzyl)-1H-pyrazolo[3,4-b]pyridin-3-yl]pyrimidin-4,5,6-triamin

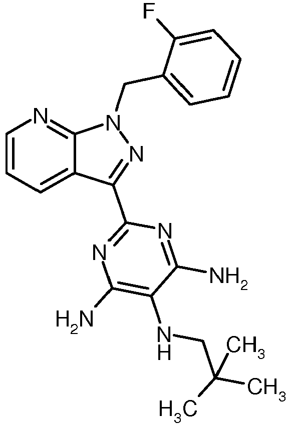

Zu einer Lösung von Methanol (20.0 mL) sowie 180 µL Essigsäure wurden 500 mg (1.43 mmol) der Verbindung aus Beispiel 1A addiert, anschließend 349 µL (3.14 mmol) Pivalaldehyd zugefügt und die Mischung 15 min bei RT gerührt. Danach wurden 251 mg (4.00 mmol) Natriumcyanoborhydrid zugefügt und die Reaktionsmischung über Nacht weiter bei RT gerührt. Der Reaktionsmischung wurden dann weitere 349 µL Pivalaldehyd, 180 µL Essigsäure sowie 251 mg Natriumcyanoborhydrid hinzugefügt und weiter über Nacht bei RT gerührt. Danach wurden erneut 349 µL Pivalaldehyd, 180 µL Essigsäure sowie 251 mg Natriumcyanoborhydrid zugefügt und weiter über Nacht bei RT nachgerührt. Anschließend wurde gesättigte wässrige Natriumhydrogencarbonatlösung zugefügt und weitere 30 min gerührt. Danach wurde die Mischung eingeengt und der Rückstand in Essigsäureethylester aufgenommen. Die organische Phase wurde zweimal mit Wasser sowie einmal mit gesättigter wässriger Natriumchloridlösung gewaschen, über Natriumsulfat getrocknet, filtriert und eingeengt. Der Rückstand wurde mit tert.-Butylmethylether verrührt, filtriert, mit tert.-Butylmethylether gewaschen und anschließend am Hochvakuum getrocknet. Man erhielt 447 mg (88%-ige Reinheit, 66% d. Th.) der Titelverbindung, die ohne weitere Reinigung umgesetzt wurden.

LC-MS (Methode 2): Rₜ = 0.96 min

MS (EIpos): m/z = 421 (M+H)⁺.

¹H-NMR (400MHz, DMSO-d₆): δ [ppm]= 0.96 (s, 9H), 3.30 (s, 2H), 5.78 (s, 2H), 5.97 (s, 4H), 7.10 - 7.15 (m, 2H), 7.19 - 7.26 (m, 1H), 7.30 - 7.39 (m, 2H), 8.57 - 8.61 (m, 1H), 9.02 - 9.07 (m, 1H).

### Beispiel 65A

### N⁵-Cyclobutyl-2-[1-(2-fluorbenzyl)-1H-pyrazolo[3,4-b]pyridin-3-yl]pyrimidin-4,5,6-triamin

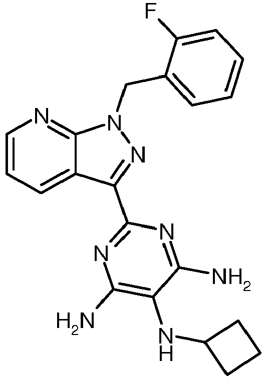

Zu einer Lösung von Methanol (20.0 mL) sowie 180 µL Essigsäure wurden 500 mg (1.43 mmol) der Verbindung aus Beispiel 1A addiert, anschließend 237 µL (3.14 mmol) Cyclobutanon zugefügt und die Mischung 15 min bei RT gerührt. Anschließend wurden 251 mg (4.00 mmol) Natriumcyanoborhydrid zugefügt und die Reaktionsmischung über Nacht bei RT gerührt. Danach wurde gesättigte wässrige Natriumhydrogencarbonatlösung zugefügt und weitere 30 min gerührt. Anschließend wurde die Mischung eingeengt und der Rückstand in Essigsäureethylester aufgenommen. Die organische Phase wurde zweimal mit Wasser sowie einmal mit gesättigter wässriger Natriumchloridlösung gewaschen, über Natriumsulfat getrocknet, filtriert und eingeengt. Der Rückstand wurde mit Cyclohexan/Essigsäureethylester verrührt, der Feststoff abfiltriert, mit Essigsäureethylester gewaschen und anschließend am Hochvakuum getrocknet. Man erhielt 134 mg (22% d. Th.) der Titelverbindung als gelben Feststoff. Einengen der Mutterlauge lieferte weitere 285 mg (76%-ige Reinheit, 38% d. Th.) der Titelverbindung.

LC-MS (Methode 2): Rₜ = 0.88 min

MS (EIpos): m/z = 405 (M+H)⁺.

¹H-NMR (400MHz, DMSO-d₆): δ [ppm]= 1.38 - 1.62 (m, 2H), 1.84 - 1.96 (m, 2H), 2.01 - 2.11 (m, 2H), 3.45 - 3.56 (m, 1H), 3.68 (d, 1H), 5.77 (s, 2H), 6.00 (s, 4H), 7.09 - 7.15 (m, 2H), 7.20 - 7.26 (m, 1H), 7.29 - 7.38 (m, 2H), 8.57 - 8.61 (m, 1H), 9.02 - 9.07 (m, 1H).

### Beispiel 66A

### N⁵-(2-Fluorbenzyl)-2-[1-(2-fluorbenzyl)-1H-pyrazolo[3,4-b]pyridin-3-yl]pyrimidin-4,5,6-triamin

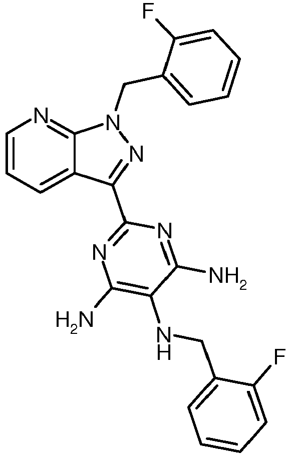

Zu einer Lösung von Methanol (20.0 mL) sowie 180 µL Essigsäure wurden 500 mg (1.43 mmol) der Verbindung aus Beispiel 1A addiert, anschließend 333 µL (3.14 mmol) 2-Fluorbenzaldehyd zugefügt und die Mischung 15 min bei RT gerührt. Anschließend wurden 251 mg (4.00 mmol) Natriumcyanoborhydrid zugefügt und die Reaktionsmischung über Nacht bei RT gerührt. Danach wurde gesättigte wässrige Natriumhydrogencarbonat-Lösung zugefügt und weitere 30 min gerührt. Anschließend wurde die Mischung eingeengt und der Rückstand in Essigsäureethylester aufgenommen. Die organische Phase wurde zweimal mit Wasser sowie einmal mit gesättigter wässriger Natriumchlorid-Lösung gewaschen, über Natriumsulfat getrocknet, filtriert und eingeengt. Der Rückstand wurde mit Cyclohexan/Essigsäureethylester verrührt, der Feststoff abfiltriert, mit Essigsäureethylester gewaschen und anschließend am Hochvakuum getrocknet. Man erhielt 267 mg (82%-ige Reinheit, 34% d. Th.) der Titelverbindung als Feststoff. Einengen der Mutterlauge lieferte weitere 490 mg (53%-ige Reinheit, 40% d. Th.) der Titelverbindung. Das Rohprodukt (82%-ige Reinheit) wurde ohne weitere Reinigung umgesetzt.

LC-MS (Methode 2): Rₜ = 0.90 min

MS (EIpos): m/z = 459 (M+H)⁺.

### Beispiel 67A

### 1-[1-(2-Fluorbenzyl)-1H-pyrazolo[3,4-b]pyridin-3-yl]ethanon

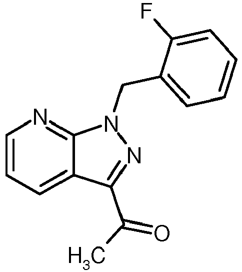

Unter Argonatmosphäre wurden 20 g (79.285 mmol) der Verbindung aus Beispiel 3A in 250 ml Ether gelöst und unter heftigem Rühren mit 40 ml (120.000 mmol) einer 3 N Lösung von Methylmagnesiumiodid in Diethylether versetzt. Es wurde für 1 h bei RT gerührt und erneut 20 ml (60.000 mmol) einer 3 N Lösung von Methylmagnesiumiodid in Diethylether addiert. Das Reagtionsgemisch wurde für 20 h unter Rückfluß gekocht und anschließend auf eine Mischung aus 400 ml tert.-Butylmethylether, 400 ml Eiswasser und 400 ml 1 N Salzsäure gegeben. Die organische Phase wurde abgetrennt und die wässrige Phase einmal mit tert.-Butylmethylether extrahiert. Die vereinigten organischen Phasen wurden über Natriumsulfat getrocknet und am Rotationsverdampfer eingeengt. Man erhielt 20.95 g (Reinheit 94%, 92% d. Th.) der Titelverbindung. Das Rohprodukt wurde ohne weitere Reinigung weiter umgesetzt.

LC-MS (Methode 2): Rₜ = 1.08 min; MS (EIpos): m/z = 270 [M+H]⁺.

### Beispiel 68A

### 1-[1-(2-Fluorbenzyl)-1H-pyrazolo[3,4-b]pyridin-3-yl]-2,2-dihydroxyethanon

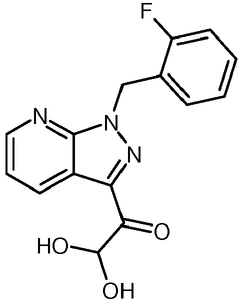

Es wurden 9.7 g (36.022 mmol) Beispiel 67A in 68 ml DMSO gelöst mit 12.15 ml (108.066 mmol) einer 48%-igen wässrigen Bromwasserstoff-Lösung versetzt und für 5 h bei 55°C gerührt. Das Reaktionsgemisch wurde eingeengt und der Rückstand mit Methanol verrührt. Man erhielt 9.94 g (75%-ige Reinheit, 68% d. Th.) der Titelverbindung. Das Rohprodukt wurde ohne weitere Reinigung weiter umgesetzt.

LC-MS (Methode 2): Rₜ = 0.81 min; MS (EIpos): m/z = 302 [M+H]⁺.

### Beispiel 69A

### Methyl-{4-amino-2-[1-(2-fluorbenzyl)-1H-pyrazolo[3,4-b]pyridin-3-yl]-6-methylpyrimidin-5-yl}carbamat-Formiat

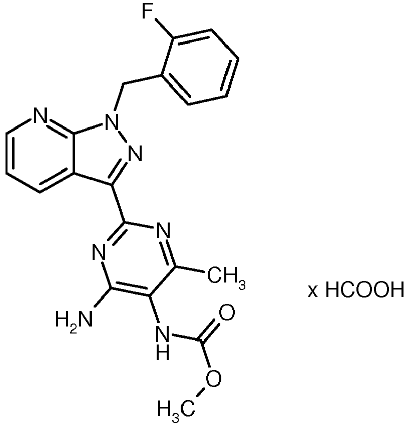

In Pyridin (3 mL) wurden 150 mg (0.343 mmol) der Verbindung aus Beispiel 54A unter Argon vorgelegt und auf 0°C abgekühlt. Anschließend wurde eine Lösung von 27 µL (0.34 mmol) Chlorameisensäuremethylester in Dichlormethan (1 mL) zugetropft, die Mischung auf RT gebracht und über Nacht gerührt. Danach wurde erneut auf 0°C abgekühlt, anschließend 5 µL Chlorameisensäuremethylester (gelöst in 0.5 mL Dichlormethan) zugegeben und die Mischung weitere 30 min bei RT gerührt. Anschließend wurden erneut 5 µL Chlorameisensäuremethylester (gelöst in 0.5 mL Dichlormethan) bei 0°C zugegeben und die Mischung über Nacht bei RT gerührt. Die Reaktionsmischung wurde auf RT gebracht und einrotiert. Der Rückstand wurde mittels präparativer HPLC (Laufmittel: Acetonitril-Wasser mit 0.1% Ameisensäure Gradient) aufgetrennt und die Produktfraktionen eingeengt. Man erhielt 113 mg (71% d. Th.) der Titelverbindung als Feststoff.

LC-MS (Methode 3): Rₜ = 0.86 min

MS (ESIpos): m/z = 408 (M+H)⁺

¹H-NMR (400 MHz, DMSO-d₆): δ = 2.26 (s, 3H), 3.65 (m, 3H), 5.83 (s, 2H), 6.88 (br. s, 2H), 7.10 - 7.16 (m, 2H), 7.20 - 7.27 (m, 1H), 7.32 - 7.41 (m, 2H), 8.15 (s, 1H), 8.56 (br. s, 1H), 8.61 - 8.65 (m, 1H), 8.97 - 9.01 (m, 1H).

### Beispiel 70A

### 2-[5-Fluor-1-(2-fluorbenzyl)-1H-pyrazolo[3,4-b]pyridin-3-yl]-5-nitropyrimidin-4-ol

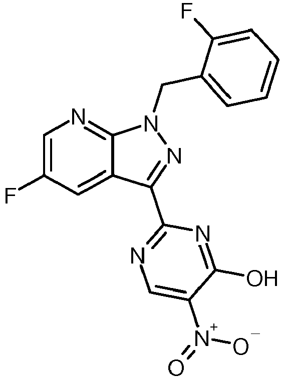

25 g (71.978 mmol) von Beispiel 28A in p-Xylol (250 ml) wurden mit 20.318 g (107.967 mmol) Ethyl-3-(dimethylamino)-2-nitroacrylat (Synthese beschrieben in Chemische Berichte 101; 8; 1968; 2925 - 2930) versetzt und 5 h zum Rückfluss erhitzt. Nach Abkühlen wurde der gebildete Niederschlag abgesaugt, mit Diethylether gewaschen und anschliessend getrocknet. Man erhielt 24.7 g (89 % d. Th.) der Titelverbindung.

LC-MS (Methode 2): Rₜ = 0.97 min; MS (ESIpos): m/z = 385 (M+H)⁺

¹H-NMR (400 MHz, DMSO-d₆): δ [ppm] = 5.82 (s, 2H), 7.16 (t, 1H), 7.21-7.25 (m, 2H), 7.34-7.40 (m, 1H), 8.40 (s br, 1H), 8.57 (dd, 1H), 8.69 (dd, 1H), 8.76 (s, 1H).

### Beispiel 71A

### 3-(4-Chlor-5-nitropyrimidin-2-yl)-5-fluor-1-(2-fluorbenzyl)-1H-pyrazolo[3,4-b]pyridin

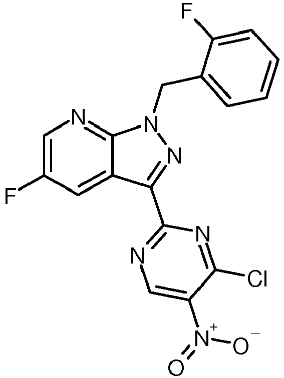

24.5 g (63.752 mmol) von Beispiel 70A wurden in Sulfolan (125 ml) vorgelegt und anschliessend mit 11.885 ml (127.503 mmol) Phosphorylchlorid versetzt und 1h auf 120°C erhitzt. Nach Abkühlen wurde vorsichtig auf Wasser (700 ml) gegeben und 15 min gerührt. Der entstandene Niederschlag wurde erst mit Wasser, dann mit Isopropanol und abschliessend mit Diethylether gewaschen und anschliessend getrocknet. Man erhielt 21.5 g (80 % d. Th.) der Titelverbindung.

LC-MS (Methode 3): Rₜ = 1.51 min; MS (ESIpos): m/z = 403 (M+H)⁺

¹H-NMR (400 MHz, DMSO-d₆): δ [ppm] = 5.94 (s, 2H), 7.16-7.26 (m, 2H), 7.33-7.42 (m, 2H), 8.52 (dd, 1H), 8.83 (dd, 1H), 9.61 (s, 1H).

### Beispiel 72A

### 2-[5-Fluor-1-(2-fluorbenzyl)-1H-pyrazolo[3,4-b]pyridin-3-yl]-5-nitropyrimidin-4-amin

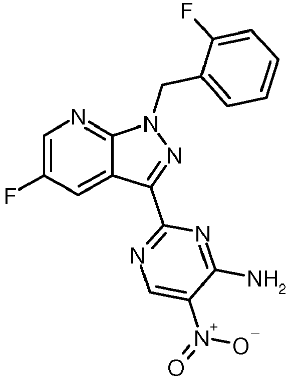

11.00 g (27.312 mmol) Beispiel 71A wurden in Isopropanol (185 ml) und Dimethylformamid (123 ml) vorgelegt und anschliessend mit 54.62 ml einer 2M Lösung von Ammoniak in Isopropanol versetzt und 1.5h auf 60°C erhitzt. Nach Abkühlen wurde auf Wasser (350 ml) gegeben und 10 min gerührt. Der entstandene Niederschlag wurde erst mit Wasser, dann mit Isopropanol und abschliessend mit Diethylether gewaschen und anschliessend getrocknet. Man erhielt 10.3 g (92 % d. Th.) der Titelverbindung.

LC-MS (Methode 2): Rₜ = 1.05 min; MS (ESIpos): m/z = 384 (M+H)⁺

¹H-NMR (400 MHz, DMSO-d₆): δ [ppm] = 5.87 (s, 2H), 7.15-7.30 (m, 3H), 7.35-7.41 (m, 1H), 8.46 (s br, 1H), 8.75 (m, 1H), 8.93 (dd, 1H), 9.11 (s br, 1H), 9.22 (s, 1H).

### Beispiel 73A

### 2-[5-Fluor-1-(2-fluorbenzyl)-1H-pyrazolo[3,4-b]pyridin-3-yl]pyrimidin-4,5-diamin

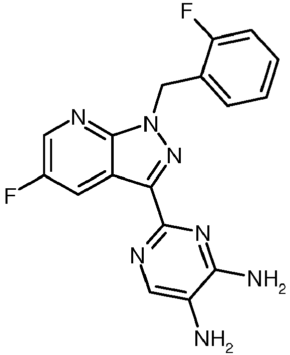

10.00 g (26.088 mmol) Beispiel 72A wurden in Ethanol (600 ml) mit 2 g Palladium auf Kohle (10%) versetzt und über Nacht bei RT und unter Wasserstoff-Normaldruck hydriert. Dann wurde mit Dichlormethan (500 ml) versetzt und 30 min gerührt. Anschliessend wurde über Kieselgur filtriert und das Filtrat wurde etwas eingeengt. Danach wurde mit Diethylether versetzt und der entstandene Niederschlag wurde abgesaugt, mit Diethylether gewaschen und anschliessend getrocknet. Man erhielt 6.7 g (71 % d. Th.) der Titelverbindung.

LC-MS (Methode 2): Rₜ = 0.76 min; MS (ESIpos): m/z = 354 (M+H)⁺

¹H-NMR (400 MHz, DMSO-d₆): δ [ppm] = 4.97-5.01 (m, 2H), 5.76 (s, 2H), 6.60 (s br, 2H), 7.12-7.25 (m, 3H), 7.33-7.39 (m, 1H), 7.71 (s, 1H), 8.65 (m, 1H), 8.70 (dd, 1H).

### Beispiel 74A

### Methyl-{4-amino-2-[5-fluor-1-(2-fluorbenzyl)-1H-pyrazolo[3,4-b]pyridin-3-yl]pyrimidin-5-yl}carbamat

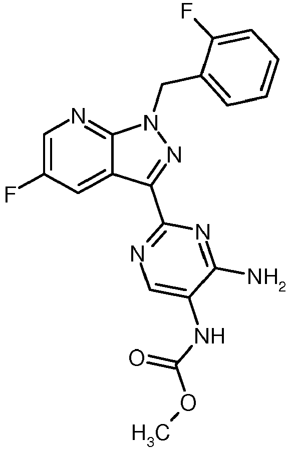

Die Verbindung wurde in Analogie zu Beispiel 31A hergestellt. Man erhielt 140 mg (12 % d. Th.) der Titelverbindung ausgehend von 1.00 g (2.830 mmol) Beispiel 73A.

LC-MS (Methode 2): Rₜ = 0.86 min; MS (ESIpos): m/z = 412 (M+H)⁺

¹H-NMR (400 MHz, DMSO-d₆): δ [ppm] = 3.68 (s, 3H), 5.81 (s, 2H), 6.95-7.25 (m, 5H), 7.34-7.40 (m, 1H), 8.40 (s br, 1H), 8.69 (dd, 1H), 8.75 (dd, 1H), 8.88 (s br, 1H).

### Beispiel 75A

### Methyl-{4-amino-2-[5-fluor-1-(2-fluorbenzyl)-1H-pyrazolo[3,4-b]pyridin-3-yl]pyrimidin-5-yl}methylcarbamat

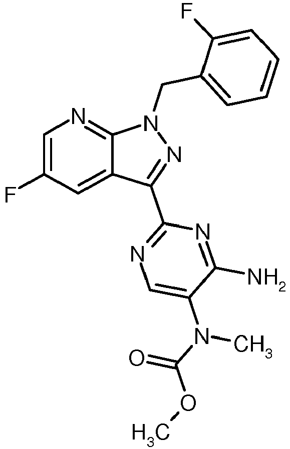

700 mg (1.702 mmol) der unter Beispiel 74A erhaltenen Verbindung wurden in Tetrahydrofuran (15 ml) vorgelegt und bei 0°C mit 2.552 ml (2.552 mmol) Lithiumhexamethyldisilazid (1.0 M in Tetrahydrofuran) versetzt. Nach 20 min bei 0°C wurden 158 µl (2.552 mmol) Iodmethan zugegeben und anschliessend wurde über Nacht bei RT gerührt. Danach wurde mit Wasser versetzt (1 ml), 10 min verrührt und anschliessend bis zur Trockene eingeengt. Von der Rohmasse (ca. 1 g) wurden 300 mg entnommen und mittels präparativer HPLC gereinigt (Acetonitril:Wasser (+0.05 % Ameisensäure) - Gradient). Es wurden 31 mg der Titelverbindung erhalten (4 % d. Th.).

Die restliche Menge wurde über Nacht am Hochvakuum getrocknet. Man erhielt 417 mg der Titelverbindung in ca. 70% Reinheit, die ohne weitere Reinigung in Beispiel 95 eingesetzt worden sind.

LC-MS (Methode 2): Rₜ = 0.89 min; MS (ESIpos): m/z = 426 (M+H)⁺

¹H-NMR (400 MHz, DMSO-d₆): δ [ppm] = 3.09 (s, 3H), 3.55 and 3.69 (s br, zusammen 3H), 5.82 (s, 2H), 7.13-7.26 (m, 3H), 7.33-7.40 (m, 1H), 8.18 (s, 1H), 8.70 (dd, 1H), 8.74 (dd, 1H).

### Beispiel 76A

### 3-Brom-1,1,1-trifluorpropan-2-yl-{4-amino-2-[1-(2-fluorbenzyl)-1H-pyrazolo[3,4-b]pyridin-3-yl]pyrimidin-5-yl}carbamat

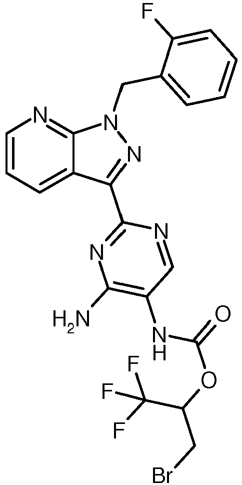

Die Titelverbindung wurde in Analogie zu Beispiel 9A ausgehend von 1.30 g (3.877 mmol) 2-[1-(2-Fluorbenzyl)-1H-pyrazolo[3,4-b]pyridin-3-yl]pyrimidin-4,5-diamin (Synthese beschrieben in WO 02/042302, Ausgangsverbindung V) hergestellt.. Man erhielt 281 mg (13 % d. Th.) der Titelverbindung.

LC-MS (Methode 2): Rₜ = 1.01 min; MS (EIpos): m/z = 554/556 [M+H, Br-Muster]⁺.

### Beispiel 77A

### 3-{4-Amino-2-[1-(2-fluorbenzyl)-1H-pyrazolo[3,4-b]pyridin-3-yl]pyrimidin-5-yl}-5-(trifluormethyl)-1,3-oxazolidin-2-on

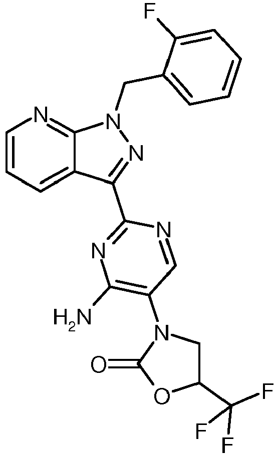

Ausgehend von 768 mg (1.386 mmol) Beispiel 76A wurden in Analogie zu Beispiel 8A 658 mg (100 % d. Th.) der Titelverbindung erhalten.

LC-MS (Methode 2): Rₜ = 0.98 min; MS (EIpos): m/z = 474 [M+H]⁺.

### Beispiel 78A

### 2-[1-(2-Fluorbenzyl)-1H-pyrazolo[3,4-b]pyridin-3-yl]-5-nitropyrimidin-4-ol

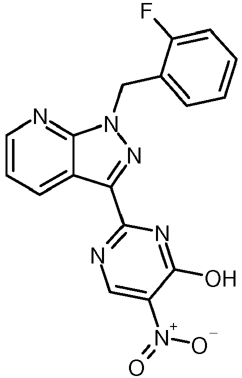

100.00 g (327.07 mmol) der Verbindung aus Beispiel 5A wurden in p-Xylol (1 l) suspendiert und mit 92.32 g (490.60 mmol) Ethyl-3-(dimethylamino)-2-nitroacrylat (Synthese beschrieben in Chemische Berichte 101; 8; 1968; 2925 - 2930) versetzt und 6 h zum Rückfluss erhitzt. Nach Abkühlen wurde der gebildete Niederschlag abgesaugt, mit Diethylether gewaschen und anschliessend getrocknet. Man erhielt 109.00 g (76 % d. Th.) der Titelverbindung.

LC-MS (Methode 2): Rₜ = 0.91 min; MS (ESIpos): m/z = 367 (M+H)⁺

¹H-NMR (400 MHz, DMSO-d₆): δ [ppm] = 5.92 (s, 2H), 7.17 (t, 1H), 7.22-7.27 (m, 1H), 7.34-7.41 (m, 1H), 7.52-7.55 (m, 1H), 8.67 (s br, 1H), 8.75-8.77 (m, 2H), 9.01 (s br, 1H), 13.91 (s br, 1H).

### Beispiel 79A

### 3-(4-Chlor-5-nitropyrimidin-2-yl)-1-(2-fluorbenzyl)-1H-pyrazolo[3,4-b]pyridin

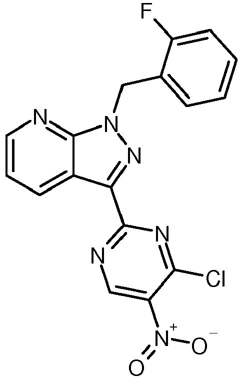

109.00 g (249.95 mmol) der Verbindung aus Beispiel 78A wurden in Sulfolan (490 ml) vorgelegt und anschliessend mit 55.92 ml (599.88 mmol) Phosphorylchlorid versetzt und 1h auf 120°C erhitzt. Nach Abkühlen wurde vorsichtig auf Wasser (3 l) gegeben und 15 min gerührt. Es wurde portionsweise Natriumhydrogencarbonat addiert bis der pH-Wert bei 6 lag. Der entstandene Niederschlag wurde abgesaugt und mit Wasser gewaschen. Der Rückstand wurde 30 min mit 200 ml Dichlormethan/Methanol (v/v = 4:1) verrührt, abgesugt mit Dichlormethan, Aceton und Petrolether gewaschen und am Hochvakuum getrocknet. Man erhielt 63.00 g (62 % d. Th.) der Titelverbindung.

LC-MS (Methode 2): Rₜ = 1.18 min; MS (ESIpos): m/z = 385 (M+H)⁺

### Beispiel 80A

### N-(2,4-Dimethoxybenzyl)-2-[1-(2-fluorbenzyl)-1H-pyrazolo[3,4-b]pyridin-3-yl]-5-nitropyrimidin-4-amin

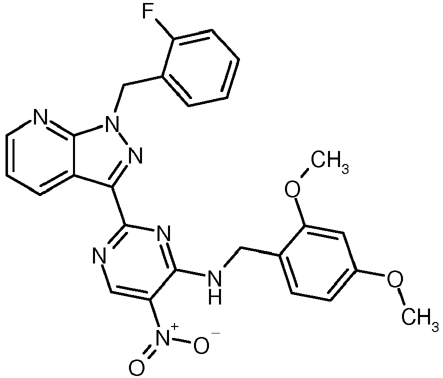

15.00 g (38.99 mmol) der Verbindung aus Beispiel 79A wurden in 200 ml Dioxan gelöst, mit 6.98 g (40.94 mmol) 2,4-Dimethoxybenzylamin sowie 5.54 g (42.88 mmol) N,N-Diisopropylamin versetzt und für 1h bei 85°C gerührt. Nach dem Abkühlen wurde filtriert und das Filtrat am Rotationsverdampfer eingeengt und der Rückstand am Hochvakuum getrocknet. Der Rückstand wurde gemörsert, mit 250 ml Wasser verrührt, abgesaugt und am Hochvakuum getrocknet. Es wurden 19.96 g (89% d. Th.) der Titelverbindung erhalten.

LC-MS (Methode 2): Rₜ = 1.29 min; MS (ESIpos): m/z = 516 (M+H)⁺

¹H-NMR (400 MHz, DMSO-d₆): δ [ppm] = 3.71 (s, 3H), 3.91 (s, 3H), 4.83 (d, 2H), 5.87 (s, 2H), 6.39 (dd, 1H), 6.66 (d, 1H), 7.13-7.25 (m, 4H), 7.29-7.40 (m, 2H), 8.35 (dd, 1H), 8.65 (dd, 1H), 9.25 (s, 1H), 9.29 (t, 1H).

### Beispiel 81A

### N⁴-(2,4-Dimethoxybenzyl)-2-[1-(2-fluorbenzyl)-1H-pyrazolo[3,4-b]pyridin-3-yl]pyrimidin-4,5-diamin

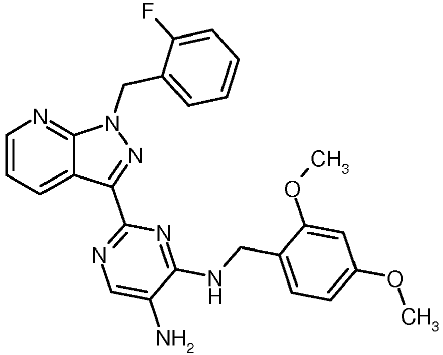

19.64 g (38.10 mmol) der Verbindung aus Beispiel 80A wurden in 300 ml Pyridin gelöst, mit 2.00 g Palladium auf Kohle (10%) versetzt und 2h bei RT und Wasserstoff-Normaldruck hydriert. Es wurde über Celite filtriert, mit wenig Pyridin nachgewaschen und das Filtrat am Rotationsverdampfer einrotiert. Es wurden 19.02 g (78% d. Th.) der Titelverbindung erhalten.

LC-MS (Methode 2): Rₜ = 0.89 min; MS (ESIpos): m/z = 486 (M+H)⁺

### Beispiel 82A

### 9-(2,4-Dimethoxybenzyl)-2-[1-(2-fluorbenzyl)-1H-pyrazolo[3,4-b]pyridin-3-yl]-7,9-dihydro-8H-purin-8-on

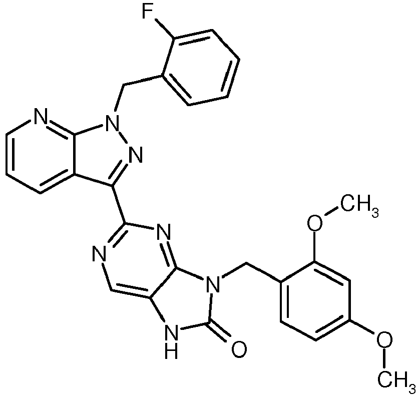

14.00 g (21.91 mmol) der Verbindung aus Beispiel 81A wurden in 800 ml Acetonitril gelöst, mit 4.62 g (28.49 mmol) N,N'-Carbonyldiimidazol versetzt und 4h unter Rückfluß gekocht. Nach dem Abkühlen wurde der Niederschlag abfiltriert und am Hochvakuum getrocknet. Es wurden 10.16 g (79% d. Th.) der Titelverbindung erhalten.

LC-MS (Methode 2): Rₜ = 1.08 min; MS (ESIpos): m/z = 512 (M+H)⁺

¹H-NMR (400 MHz, DMSO-d₆): δ [ppm] = 3.71 (s, 3H), 3.81 (s, 3H), 5.03 (s, 2H), 5.83 (s, 2H), 6.44 (dd, 1H), 6.60 (d, 1H), 7.02 (d, 1H), 7.10-7.17 (m, 2H), 7.20-7.25 (m, 1H), 7.32-7.36 (m, 1H), 7.39 (dd, 1H), 8.39 (s, 1H), 8.64 (dd, 1H), 8.77 (dd, 1H), 11.59 (s, 1H).

### Beispiel 83A

### 9-(2,4-Dimethoxybenzyl)-2-[1-(2-fluorbenzyl)-1H-pyrazolo[3,4-b]pyridin-3-yl]-7-(2-methoxyethyl)-7,9-dihydro-8H-purin-8-on

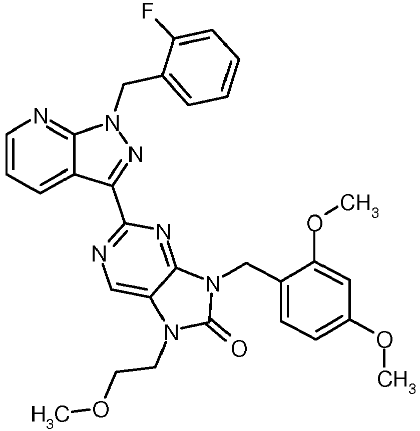

500 mg (0.977 mmol) der Verbindung aus Beispiel 82A wurden in 10 ml DMF gelöst, mit 414 mg (1.271 mmol) Cäsiumcarbonat sowie 149 mg (1.075 mmol) 2-Bromethylmethylether versetzt und 18h bei RT gertührt. Es wurde Wasser addiert und mit Essigsäureethylester extrahiert. Die vereinigten organischen Phasen wurden über Natriumsulfat getrocknet und am Rotationsverdampfer eingeengt. Der Rückstand wurde am Hochvakuum getrocknet. Es wurden 453 mg (74% d. Th.) der Titelverbindung erhalten.

LC-MS (Methode 2): Rₜ = 1.19 min; MS (ESIpos): m/z = 570 (M+H)⁺

¹H-NMR (400 MHz, DMSO-d₆): δ [ppm] = 3.26 (s, 3H), 3.67 (t, 2H), 3.71 (s, 3H), 3.80 (s, 3H), 4.13 (t, 2H), 5.08 (s, 2H), 5.84 (s, 2H), 6.45 (dd, 1H), 6.59 (d, 1H), 7.04 (d, 1H), 7.10-7.18 (m, 2H), 7.21-7.25 (m, 1H), 7.32-7.38 (m, 1H), 7.40 (dd, 1H), 8.61 (s, 1H), 8.65 (dd, 1H), 8.78 (dd, 1H).

### Beispiel 84A

### 7-(Cyclopropylmethyl)-9-(2,4-dimethoxybenzyl)-2-[1-(2-fluorbenzyl)-1H-pyrazolo[3,4-b]pyridin-3-yl]-7,9-dihydro-8H-purin-8-on

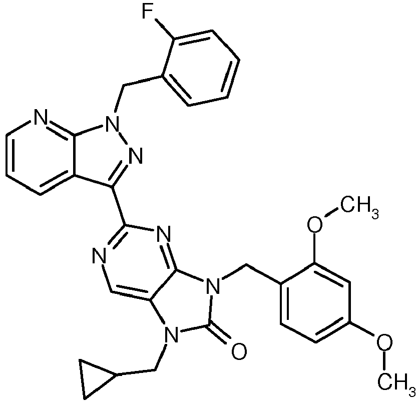

500 mg (0.980 mmol) der Verbindung aus Beispiel 82A wurden in 10 ml DMF gelöst, mit 414 mg (1.270 mmol) Cäsiumcarbonat sowie 150 mg (1.08 mmol) 2-Brommethylcyclopropan versetzt und 18h bei RT gerührt. Es wurde Wasser addiert und mit Essigsäureethylester extrahiert. Die vereinigten organischen Phasen wurden über Natriumsulfat getrocknet und am Rotationsverdampfer eingeengt. Der Rückstand wurde am Hochvakuum getrocknet. Es wurden 482 mg (72% d. Th., Reinheit 82%) der Titelverbindung erhalten.

LC-MS (Methode 2): Rₜ = 1.25 min; MS (ESIpos): m/z = 566 (M+H)⁺

### Beispiel 85A

### 9-(2,4-Dimethoxybenzyl)-2-[1-(2-fluorbenzyl)-1H-pyrazolo[3,4-b]pyridin-3-yl]-7-(2,2,3,3,3-pentafluorpropyl)-7,9-dihydro-8H-purin-8-on

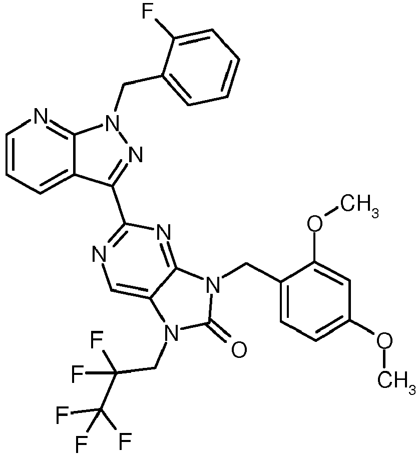

500 mg (0.980 mmol) der Verbindung aus Beispiel 82A wurden in 10 ml DMF gelöst, mit 414 mg (1.270 mmol) Cäsiumcarbonat sowie 313 mg (1.08 mmol) 2,3,3,3,3-Pentafluoropropyltrifluormethansulfonat versetzt und 18h bei RT gerührt. Es wurde Wasser addiert und mit Essigsäureethylester extrahiert. Die vereinigten organischen Phasen wurden über Natriumsulfat getrocknet und am Rotationsverdampfer eingeengt. Der Rückstand wurde am Hochvakuum getrocknet. Es wurden 471 mg (61% d. Th., Reinheit 82%) der Titelverbindung erhalten.

LC-MS (Methode 2): Rₜ = 1.31 min; MS (ESIpos): m/z = 644 (M+H)⁺

### Beispiel 86A

### 9-(2,4-Dimethoxybenzyl)-2-[1-(2-fluorbenzyl)-1H-pyrazolo[3,4-b]pyridin-3-yl]-7-(oxetan-2-ylmethyl)-7,9-dihydro-8H-purin-8-on

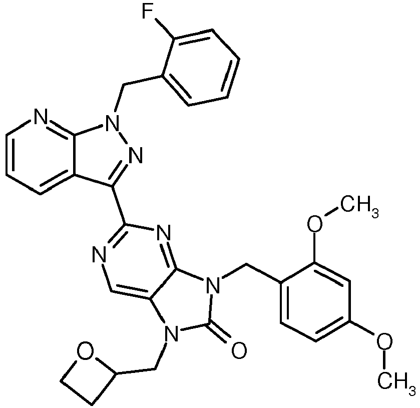

500 mg (0.980 mmol) der Verbindung aus Beispiel 82A wurden in 10 ml DMF gelöst, mit 414 mg (1.270 mmol) Cäsiumcarbonat sowie 150 mg (1.08 mmol) 2-(Brommethyl)oxetan versetzt und 18h bei RT, 18h bei 40°C und 4h bei 100°C gerührt. Nach dem Abkühlen wurde Wasser addiert und mit Essigsäureethylester extrahiert. Die vereinigten organischen Phasen wurden über Natriumsulfat getrocknet und am Rotationsverdampfer eingeengt. Der Rückstand wurde am Hochvakuum getrocknet. Es wurden 581 mg (53% d. Th., Reinheit 63%) der Titelverbindung erhalten.

LC-MS (Methode 2): Rₜ = 1.19 min; MS (ESIpos): m/z = 582 (M+H)⁺

### Beispiel 87A

### 9-(2,4-Dimethoxybenzyl)-2-[1-(2-fluorbenzyl)-1H-pyrazolo[3,4-b]pyridin-3-yl]-7-[2-(morpholin-4-yl)ethyl]-7,9-dihydro-8H-purin-8-on

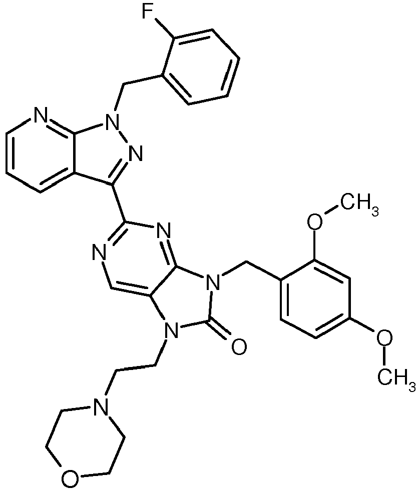

500 mg (0.980 mmol) der Verbindung aus Beispiel 82A wurden in 10 ml DMF gelöst, mit 414 mg (1.270 mmol) Cäsiumcarbonat sowie 259 mg (1.08 mmol) 4-(2-Iodethyl)morpholin versetzt und 18h bei RT, 18h bei 40°C und 8h bei 100°C gerührt. Nach dem Abkühlen wurde Wasser addiert und mit Essigsäureethylester extrahiert. Die vereinigten organischen Phasen wurden über Natriumsulfat getrocknet und am Rotationsverdampfer eingeengt. Die Reinigung erfolgte mittels präparativer HPLC (Eluent: Methanol/Wasser, Gradient 30:70 → 90:10). Es wurden 102 mg (17% d. Th.) der Titelverbindung erhalten.

LC-MS (Methode2): Rₜ = 0.86 min; MS (ESIpos): m/z = 625 (M+H)⁺

### Beispiel 88A

### 2-[1-(2-Fluorbenzyl)-1H-pyrazolo[3,4-b]pyridin-3-yl]-N⁵-[1-(2,2,2-trifluorethyl)piperidin-4-yl]pyrimidin-4,5-diamin

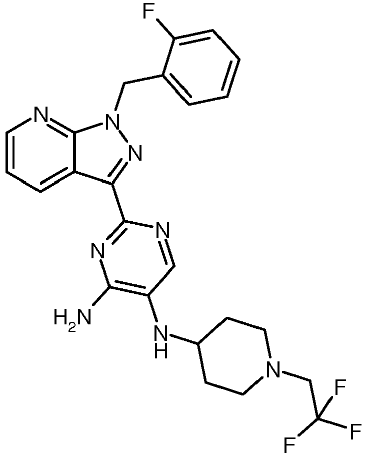

200 mg (0.596 mmol) 2-[1-(2-Fluorbenzyl)-1H-pyrazolo[3,4-b]pyridin-3-yl]pyrimidin-4,5-diamin (Synthese beschrieben in US2004/67937; Example V) wurden in Methanol (16 ml) vorgelegt, mit 75 µl (1.312 mmol) Essigsäure versetzt und anschliessend wurde 237 mg (1.312 mmol) 1-(2,2,2-Trifluorethyl)piperidin-4-on zugegeben. Nach 15 min Rühren bei RT wurde 104 mg (1.67 mmol) Natriumcyanoborhydrid zugegeben und 2.5 h bei RT gerührt. Im folgenden wurde innerhalb von 2 Tagen dreimal die oben angegebenen Mengen an Reagenzien (1-(2,2,2-Trifluorethyl)piperidin-4-on, Essigsäure, Natriumcyanoborhydrid) zugesetzt, um einen weitestgehend vollständigen Umsatz zu erzielen. Danach wurde das Reaktionsgemisch mit gesättigter wässriger Natriumhydrogencarbonat-Lösung (5 ml) versetzt und 10 min kräftig gerührt. Anschliessend wurde das Reaktionsgemisch mit Wasser und Essigsäureethylester extrahiert. Die Phasen wurden getrennt und die wässrige Phase wurde zweimal mit Essigsäureethylester extrahiert. Die vereinigten organischen Phasen wurden mit gesättigter wässriger Natriumchlorid-Lösung gewaschen, über Natriumsulfat getrocknet, filtriert, eingeengt und danach mittels präparativer HPLC gereinigt (Acetonitril:Wasser (+0.05 % Ameisensäure) - Gradient). Es wurden 269 mg der Titelverbindung erhalten (90 % d. Th.).

LC-MS (Methode 5): Rt = 0.80 min; MS (EIpos): m/z = 501 [M+H]⁺.

### Beispiel 89A

### 2-[1-(2-Fluorbenzyl)-1H-pyrazolo[3,4-b]pyridin-3-yl]-N5-[1-(2,2,2-trifluorethyl)pyrrolidin-3-yl]pyrimidin-4,5-diamin

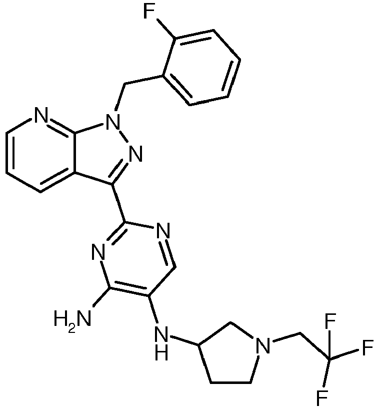

400 mg (1.193 mmol) 2-[1-(2-Fluorbenzyl)-1H-pyrazolo[3,4-b]pyridin-3-yl]pyrimidin-4,5-diamin (Synthese beschrieben in US2004/67937; Example V) wurden in Analogie zur Vorschrift unter Beispiel 88A mit 1-(2,2,2-Trifluorethyl)pyrrolidin-on umgesetzt. Man erhielt nach Reinigung mittels präparativer HPLC (Acetonitril:Wasser (+0.05 % Ameisensäure) - Gradient) 482 mg der Titelverbindung erhalten (83 % d. Th.).

LC-MS (Methode 2): Rₜ = 0.86 min; MS (EIpos): m/z = 487 [M+H]⁺.

### Beispiel 90A

### N5-[1-(2,2-Difluorethyl)piperidin-4-yl]-2-[1-(2-fluorbenzyl)-1H-pyrazolo[3,4-b]pyridin-3-yl]pyrimidin-4,5-diamin

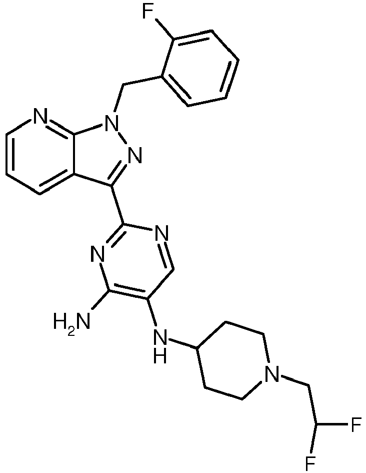

200 mg (0.596 mmol) 2-[1-(2-Fluorbenzyl)-1H-pyrazolo[3,4-b]pyridin-3-yl]pyrimidin-4,5-diamin (Synthese beschrieben in US2004/67937; Example V) wurden in Analogie zur Vorschrift unter Beispiel 88A mit 1-(2,2-Difluorethyl)piperidin-4-on umgesetzt. Man erhielt nach Reinigung mittels präparativer HPLC (Acetonitril:Wasser (+0.05 % Ameisensäure) - Gradient) 219 mg der Titelverbindung (76 % d. Th.).

LC-MS (Methode 2): Rₜ = 0.60 min; MS (EIpos): m/z = 483 [M+H]⁺.

### Beispiel 91A

### N-(2,4-Dimethoxybenzyl)-2-[5-fluor-1-(2-fluorbenzyl)-1H-pyrazolo[3,4-b]pyridin-3-yl]-5-nitropyrimidin-4-amin

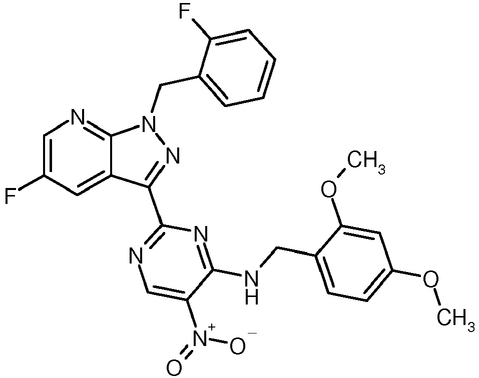

10.070 g (25.003 mmol) der Verbindung aus Beispiel 71A wurden in Analogie zur Vorschrift unter Beispiel 80A umgesetzt. Es wurden 13.31 g (99% d. Th.) der Titelverbindung erhalten.

LC-MS (Methode 2): Rₜ = 1.38 min; MS (ESIpos): m/z = 534 (M+H)⁺

### Beispiel 92A

### N4-(2,4-Dimethoxybenzyl)-2-[5-fluor-1-(2-fluorbenzyl)-1H-pyrazolo[3,4-b]pyridin-3-yl]pyrimidin-4,5-diamin

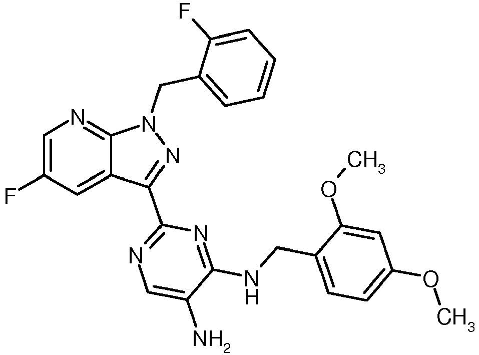

13.310 g (24.949 mmol) der Verbindung aus Beispiel 91A wurden in Analogie zur Vorschrift unter Beispiel 81A umgesetzt. Es wurden 14.93 g (ca. 100 % d. Th., 84 %ige Reinheit) der Titelverbindung erhalten.

LC-MS (Methode 2): Rₜ = 1.02 min; MS (ESIpos): m/z = 504 (M+H)⁺

### Beispiel 93A

### 9-(2,4-Dimethoxybenzyl)-2-[5-fluor-1-(2-fluorbenzyl)-1H-pyrazolo[3,4-b]pyridin-3-yl]-7,9-dihydro-8H-purin-8-on

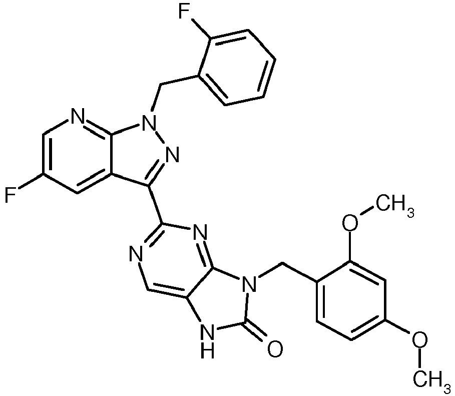

6.281 g (10.479 mmol, 84 %ige Reinheit) der Verbindung aus Beispiel 92A wurden in Analogie zur Vorschrift unter Beispiel 82A umgesetzt. Es wurden 5.61 g (ca. 100 % d. Th.) der Titelverbindung erhalten.

LC-MS (Methode 2): Rₜ = 1.11 min; MS (ESIpos): m/z = 530 (M+H)⁺

### Beispiel 94A

### 9-(2,4-Dimethoxybenzyl)-2-[5-fluor-1-(2-fluorbenzyl)-1H-pyrazolo[3,4-b]pyridin-3-yl]-7-[2-(methylsulfonyl)ethyl]-7,9-dihydro-8H-purin-8-on

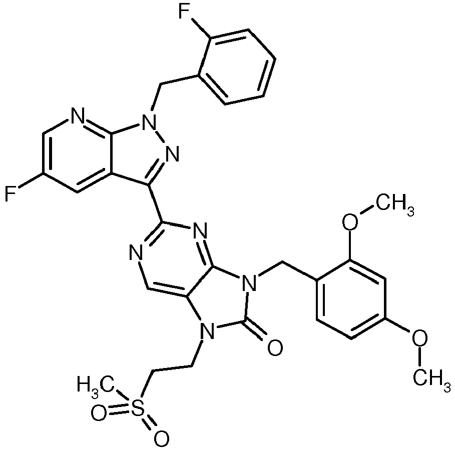

300 mg (0.567 mmol) der Verbindung aus Beispiel 93A wurden in Analogie zur Vorschrift unter Beispiel 83A mit 2-Bromethylmethylsulfon umgesetzt. Es wurden 490 mg (100 % d. Th., 73 %ige Reinheit) der Titelverbindung erhalten.

LC-MS (Methode 2): Rₜ = 1.16 min; MS (ESIpos): m/z = 636 (M+H)⁺

### Beispiel 95A

### tert.-Butyl-3-({4,6-diamino-2-[1-(2-fluorbenzyl)-1H-pyrazolo[3,4-b]pyridin-3-yl]pyrimidin-5-yl}amino)azetidin-1-carboxylat

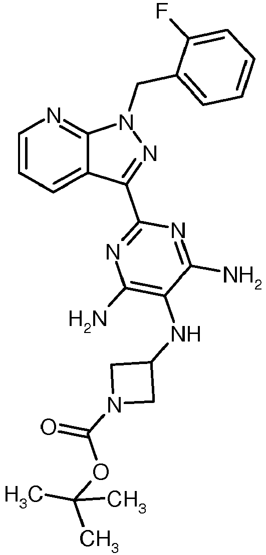

7.70 g (21.977 mmol) der Verbindung aus Beispiel 1A wurden in Analogie zur Vorschrift unter Beispiel 34A mit tert.-Butyl-3-oxoazetidin-1-carboxylat umgesetzt. Es wurden 5.26 g (47 % d. Th.) der Titelverbindung erhalten.

LC-MS (Methode 5): Rₜ = 0.81 min; MS (ESIpos): m/z = 506 (M+H)⁺

### Beispiel 96A

### tert.-Butyl-3-{6-amino-2-[1-(2-fluorbenzyl)-1H-pyrazolo[3,4-b]pyridin-3-yl]-8-oxo-8,9-dihydro-7H-purin-7-yl }azetidin-1-carboxylat

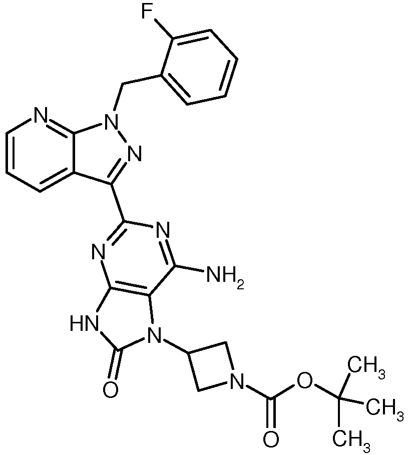

2.61 g (5.163 mmol) der Verbindung aus Beispiel 95A wurden in Analogie zur Vorschrift unter Beispiel 82A umgesetzt. Es wurden 2.24 g (81 % d. Th.) der Titelverbindung erhalten.

LC-MS (Methode 2): Rₜ = 1.02 min; MS (ESIpos): m/z = 532 (M+H)⁺

### Beispiel 97A

### tert.-Butyl-3-{2-[1-(2-fluorbenzyl)-1H-pyrazolo[3,4-b]pyridin-3-yl]-6-iod-8-oxo-8,9-dihydro-7H-purin-7-yl}azetidin-1-carboxylat

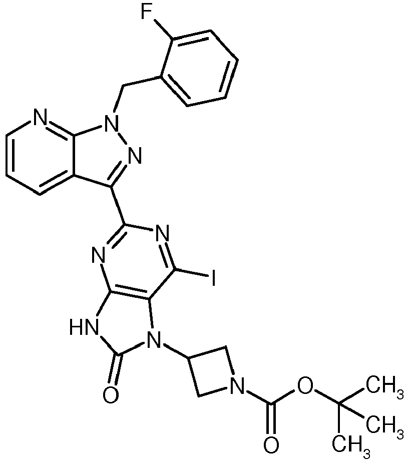

1.788 g (3.364 mmol) der Verbindung aus Beispiel 96A wurden in 20 ml 1,2-Dimethoxyethan vorgelegt und anschliessend bei RT mit 873 mg (3.364 mmol) Cäsiumiodid, 426 mg (1.682 mmol) Iod und 192 mg (1.009 mmol) Kupfer-(I)-iodid versetzt. Nach Zugabe von 2.961 ml Isopentylnitrit wurde über Nacht auf 60°C erhitzt. Nach Abkühlen wurde von einem Niederschlag filtriert und mit Ethylacetat nachgewaschen. Anschliessend wurde der Niederschlag mit Wasser versetzt, verrührt und danach erneut abgesaugt und mit wenig Wasser und Acetonitril nachgewaschen. Es wurden 0.373 g (17 % d. Th.) der Titelverbindung nach Trocknung im Hochvakuum erhalten.

LC-MS (Methode 2): Rₜ = 1.23 min; MS (ESIpos): m/z = 643 (M+H)⁺

### Beispiel 98A

### tert.-Butyl-3-{2-[1-(2-fluorbenzyl)-1H-pyrazolo[3,4-b]pyridin-3-yl]-8-oxo-8,9-dihydro-7H-purin-7-yl}azetidin-1-carboxylat

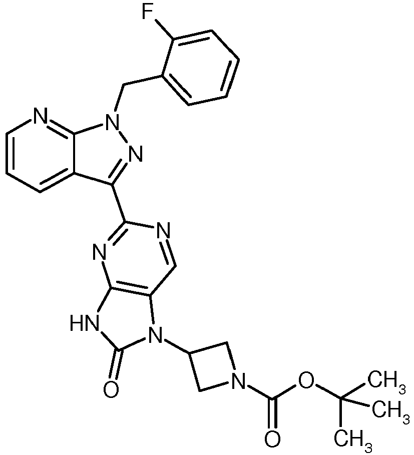

570 mg (0.887 mmol) der Verbindung aus Beispiel 97A wurden Analogie zur Vorschrift unter Beispiel 102 hydriert. Es wurden 0.669 g (90 % d. Th., Reinheit 79 %) der Titelverbindung nach Trocknung im Hochvakuum erhalten.

LC-MS (Methode 5): Rₜ = 1.08 min; MS (ESIpos): m/z = 517 (M+H)⁺

### Beispiel 99A

### 3-(2,4-Dimethoxybenzyl)-5-[5-fluor-1-(2-fluorbenzyl)-1H-pyrazolo[3,4-b]pyridin-3-yl]-1,3-dihydro[1,2,5]thiadiazolo[3,4-d]pyrimidin-2,2-dioxid

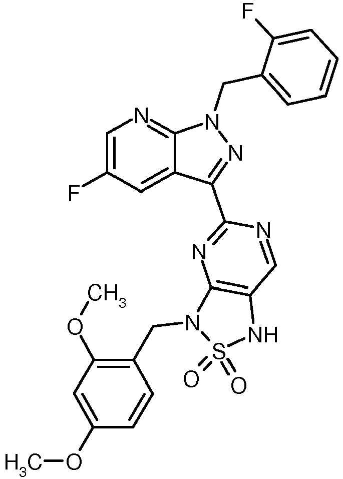

550 mg (1.093 mmol) der Verbindung aus Beispiel 92A wurden auf 11 Mikrowellengefäße verteilt. Jeweils 50 mg der Substanz wurden mit 48 mg (0.496 mmol) Sulfamid und 2.5 ml Pyridin versetzt und anschliessend 30 min bei 160°C unter Mikrowellenstrahlung erhitzt. Anschliessend wurden alle Ansätze vereinigt und im Vakuum vom Lösungsmittel befreit. Der Rückstand wurde in Dichlormethan aufgenommen und dreimal mit Wasser extrahiert. Die Phasen wurden separiert und die organische Phase wurde mit Natriumsulfat getrocknet, filtriert und am Vakuum eingeengt. Der Rückstand wurde anschliessend mittels präparativer HPLC (Acetonitril:Wasser (+0.05 % Ameisensäure) - Gradient) gereinigt. Man erhielt 0.130 g (12 % d. Th., Reinheit 50 %) der Titelverbindung.

LC-MS (Methode 5): Rₜ = 1.12 min; MS (ESIpos): m/z = 566 (M+H)⁺

### Beispiel 100A

### 3-(2,4-Dimethoxybenzyl)-5-[5-fluor-1-(2-fluorbenzyl)-1H-pyrazolo[3,4-b]pyridin-3-yl]-1-methyl-1,3-dihydro[1,2,5]thiadiazolo[3,4-d]pyrimidin-2,2-dioxid

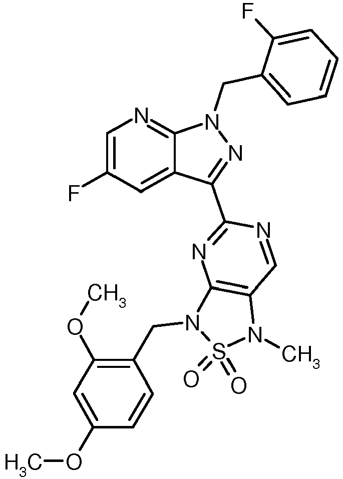

130 mg (ca. 0.115 mmol, 50 %ige Reinheit) der Verbindung aus Beispiel 99A wurden in 5 ml DMF vorgelegt, mit 44 mg (0.138 mmol) Cäsiumcarbonat und 8.5 µl (0.138 mmol) Iodmethan versetzt und über Nacht bei RT gerührt. Anschliessend wurden nochmals 14.3 µl (0.230 mmol) Iodmethan zugegeben und der Ansatz wurde über Nacht auf 50°C erhitzt. Nach Abkühlen wurde der Ansatz mittels präparativer HPLC (Acetonitril:Wasser (+0.05 % Ameisensäure) - Gradient) gereinigt. Man erhielt 30.9 mg (46 % d. Th.) der Titelverbindung.

LC-MS (Methode 2): Rₜ = 1.30 min; MS (ESIpos): m/z = 580 (M+H)⁺

### Beispiel 101A

### Ethyl-4-amino-2-[1-(2-fluorbenzyl)-1H-pyrazolo[3,4-b]pyridin-3-yl]pyrimidin-5-carboxylat

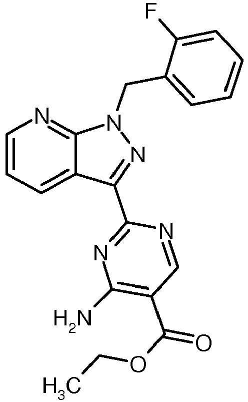

Die Synthese dieser Verbindung ist beschrieben in A. Straub et al., Bioorg. Med. Chem., 10, 1711-1717; 2002.

### Beispiel 102A

### 4-Amino-2-[1-(2-fluorobenzyl)-1H-pyrazolo[3,4-b]pyridin-3-yl]pyrimidin-5-carbonsäure

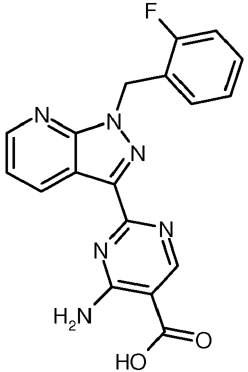

1000 mg (2.55 mmol) Ethyl-4-amino-2-[1-(2-fluorbenzyl)-1H-pyrazolo[3,4-b]pyridin-3-yl]pyrimidin-5-carboxylat (Beispiel 101A) wurden in 80 ml Dioxan suspendiert und vorsichtig mit 50.1 ml (948 mmol) wässriger Natriumhydroxid-Lösung (50 Gew-%) versetzt. Es wurde in der Folge 4h bei 60°C gerührt und dann auf 200 ml Eiswasser gegeben. Es wurde mit konzentrierter Salzsäure vorsichtig auf pH 2 eingestellt und der beobachtete Niederschlag mittels Filtration abgetrennt. Es wurde mit etwas Wasser gewaschen und abschließend am Hochvakuum getrocknet. So wurden 993 mg (ca. 105% d. Th., der Batch enthielt vermutlich noch geringe Mengen anorganischer Salze) der Zielverbindung erhalten.

LC-MS (Methode 2): Rₜ = 0.77 min; MS (ESIpos): m/z = 360 (M+H)⁺

¹H-NMR (400 MHz, DMSO-d₆): δ [ppm] = 5.91 (s, 2H), 7.16 (t, 1H), 7.20-7.30 (m, 2H), 7.38 (dd, 1H), 7.51 (dd, 1H), 8.48 (s br, 1H), 8.72 (s, 1H), 8.74 (d, 1H), 9.07 (d, 1H), 9.14 (s br, 1H), COOH nicht zugeordnet.

### Beispiel 103A

### 4-Amino-2-[1-(2-fluorbenzyl)-1H-pyrazolo[3,4-b]pyridin-3-yl]-N-(2,2,2-trifluorethyl)pyrimidin-5-carboxamid

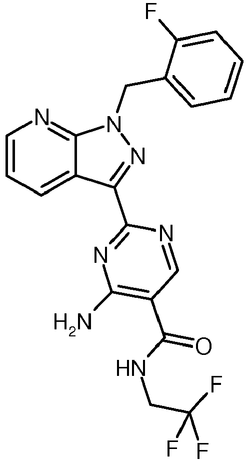

300 mg (0.82 mmol) 4-Amino-2-[1-(2-fluorobenzyl)-1H-pyrazolo[3,4-b]pyridin-3-yl]pyrimidin-5-carbonsäure (Beispiel 102A), 0.230 ml (1.65 mmol) Triethylamin und 139 mg (0.91 mmol) 1-Hydroxy-1H-benzotriazol wurden in 2.2 ml DMF gelöst und mit 174 mg (0.91 mmol) 1-(3-Dimethylaminopropyl)-3-ethylcarbodiimid-Hydrochlorid versetzt. Unter Eiskühlung wurden 70 µl (90 mg, 0.91 mmol) 2,2,2-Trifluorethylamin zugegeben und über Nacht bei Raumtemperatur umgesetzt. Der Ansatz wurde mit 4 ml Acetonitril/Wasser in Lösung gebracht und mittels präparativer HPLC aufgereinigt [Säule: Reprosil C18, 10 µm, 250*40mm; Eluent: Acetonitril/0.05 %-ige Ameisensäure; Gradient: 15% Acetonitril→ 95% Acetonitril]. So wurden 110 mg (28% d. Th.) der Zielverbindung erhalten.

LC-MS (Methode 5): Rₜ = 0.99 min; MS (ESIpos): m/z = 446 (M+H)⁺

¹H-NMR (400 MHz, DMSO-d₆): δ [ppm] = 4.11 (m, 2H), 5.86 (s, 2H), 7.12-7.28 (m, 3H), 7.37 (m, 1H), 7.43 (dd, 1H), 8.07 (s br, 2H), 8.67 (d, 1H), 8.86 (s, 1H), 9.02 (d, 1H), 9.23 (t, 1H).

### Beispiel 104A

### 4-Amino-2-[1-(2-fluorbenzyl)-1H-pyrazolo[3,4-b]pyridin-3-yl]pyrimidin-5-carboxamid

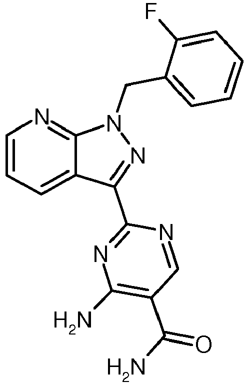

300 mg (0.82 mmol) 4-Amino-2-[1-(2-fluorobenzyl)-1H-pyrazolo[3,4-b]pyridin-3-yl]pyrimidin-5-carbonsäure (Beispiel 102A) und 139 mg (0.91 mmol) 1-Hydroxy-1H-benzotriazol wurden in 2.2 ml DMF gelöst und mit 174 mg (0.91 mmol) 1-(3-Dimethylaminopropyl)-3-ethylcarbodiimid-Hydrochlorid versetzt. Unter Eiskühlung wurden 140 µl (0.99 mmol) Ammoniumhydroxid (28%-ig) zugegeben und über Nacht bei Raumtemperatur umgesetzt. Es wurden 4 ml Acetonitril/Wasser (1/1) addiert und der entstandene Niederschlag abfiltriert. Nach dem Trocknen am Hochvakuum wurden 145 mg (48% d. Th.) der Zielverbindung erhalten.

LC-MS (Methode 2): Rₜ = 0.78 min; MS (ESIpos): m/z = 364 (M+H)⁺

¹H-NMR (400 MHz, DMSO-d₆): δ [ppm] = 5.85 (s, 2H), 7.10-7.29 (m, 3H), 7.36 (t, 1H), 7.42 (dd, 1H), 7.52 (s br, 1H), 7.95 (s, 1H), 8.15 (s br, 1H), 8.66 (d, 1H), 8.83 (s, 1H), 9.00 (d, 1H), 1xNH nicht zugeordnet.

### Beispiel 105A

### Methyl-2-[1-(2-fluorbenzyl)-1H-pyrazolo[3,4-b]pyridin-3-yl]-4-hydroxypyrimidin-5-carboxylat

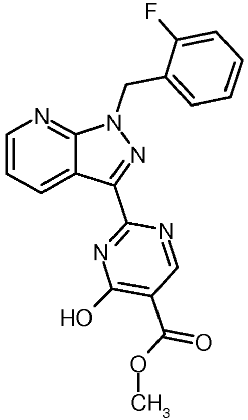

10.0 g (32.7 mmol) 1-(2-Fluorbenzyl)-1H-pyrazolo[3,4-b]pyridin-3-carboximidamid-Hydrochlorid (Beispiel 5A) und 8.54 g (49.1 mmol) Dimethyl-(methoxymethylen)malonat wurden in 400 ml Methanol vorgelegt und vorsichtig mit 1.77 g (32.7 mmol) Natriummethylat versetzt. Es wurde über Nacht bei 50°C umgesetzt. Der entstandene Niederschlag wurde abfiltriert, mit etwas Methanol gewaschen und am Hochvakuum getrocknet. So wurden 6.54 g (52 % d. Th.) der Zielverbindung erhalten.

LC-MS (Methode 2): Rₜ = 0.88 min; MS (ESIpos): m/z = 380 (M+H)⁺

¹H-NMR (400 MHz, DMSO-d₆): δ [ppm] = 3.73 (s, 3H), 5.84 (s, 2H), 7.15 (t, 1H), 7.20-7.30 (m, 2H), 7.36 (m, 1H), 7.42 (dd, 1H), 8.55 (s, 1H), 8.67 (d, 1H), 8.84 (dd, 1H), OH nicht zugeordnet.

### Beispiel 106A

### Methyl-4-chlor-2-[1-(2-fluorbenzyl)-1H-pyrazolo[3,4-b]pyridin-3-yl]pyrimidin-5-carboxylat

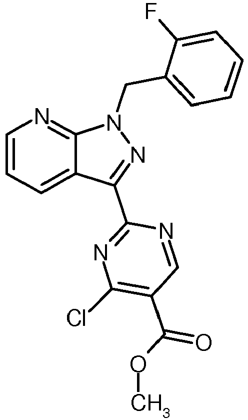

6.54 g (17.2 mmol) Methyl-2-[1-(2-fluorbenzyl)-1H-pyrazolo[3,4-b]pyridin-3-yl]-4-hydroxy-pyrimidin-5-carboxylat (Beispiel 105A) wurden in 26.5 ml (284 mmol) Phosphorylchlorid aufgenommen. Dann wurden 5.95 g (34.5 mmol) Diethylanilin addiert und 60 min bei 90 °C umgesetzt. Nach dem Abkühlen wurde der entstandene Niederschlag abfiltriert und mit Wasser gewaschen. Dann wurde am Hochvakuum getrocknet. So wurden 6.11 g (78 % d. Th.) der Zielverbindung erhalten.

LC-MS (Methode 5): Rₜ = 1.22 min; MS (ESIpos): m/z = 398 (M+H)⁺

¹H-NMR (400 MHz, DMSO-d₆): δ [ppm] = 3.94 (s, 3H), 5.92 (s, 2H), 7.17 (dt, 1H), 7.21-7.31 (m, 2H), 7.38 (m, 1H), 7.53 (dd, 1H), 8.74 (dd, 1H), 8.85 (dd, 1H), 9.32 (s, 1H).

### Ausführungsbeispiele:

### Beispiel 1

### 6-Amino-2-[1-(2-fluorbenzyl)-1H-pyrazolo[3,4-b]pyridin-3-yl]-7-(2-hydroxyethyl)-7,9-dihydro-8H-purin-8-on

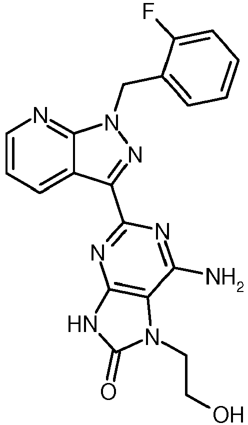

350 mg (0.833 mmol) der unter Beispiel 6A erhaltenen Verbindung wurden in 12 ml Tetrahydrofuran bei 0°C vorgelegt. Dann wurden 1.665 ml einer 1M Lösung von Bis-(trimethylsilyl)natriumamid in Tetrahydrofuran zugetropft und es wurde für weitere 10 min bei 0°C gerührt. Danach wurde über Nacht bei RT gerührt. Anschliessend wurde der Ansatz mit 2 ml Wasser versetzt und bis zur Trockene eingeengt. Der Rückstand wurde mittels präparativer HPLC gereinigt (Acetonitril/Wasser (+0.05 % Ameisensäure) - Gradient). Es wurden 188 mg der Titelverbindung erhalten (53 % d. Th.).

LC-MS (Methode 2): Rt = 0.80 min; MS (ESIpos): m/z = 421 (M+H)⁺

¹H-NMR (400 MHz, DMSO-d₆): δ [ppm] = 3.63 (q, 2H), 3.97 (t, 2H), 5.35 (t, 1H), 5.80 (s, 2H), 6.74 (s, 2H) 7.13-7.25 (m, 3H), 7.33-7.39 (m, 2H), 8.63 (dd, 1H), 9.03 (dd, 1H), 11.64 (s, 1H).

### Beispiel 2

### 6-Amino-2-[1-(2-fluorbenzyl)-1H-pyrazolo[3,4-b]pyridin-3-yl]-7-(2-hydroxy-2-methylpropyl)-7,9-dihydro-8H-purin-8-on

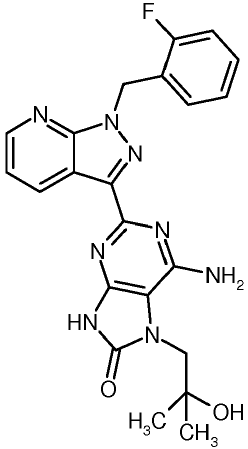

In Analogie zur Vorschrift unter Beispiel 1 wurden ausgehend von 150 mg (0.334 mmol) Beispiel 8A und 0.368 ml einer 1M Lösung von Bis-(trimethylsilyl)natriumamid in Tetrahydrofuran 87.8 mg (58.5% d. Th.) der Titelverbindung erhalten.

LC-MS (Methode 2): Rt = 0.89 min; MS (EIpos): m/z = 449 [M+H]⁺.

¹H-NMR (400 MHz, DMSO-d₆): δ [ppm] = 1.20 (s, 6H), 3.78 (s, 2H), 5.62 (s, 1H), 5.80 (s, 2H), 6.95 (s br, 2H) 7.14 (m, 1H), 7.20 (m, 1H), 7.23 (m, 1H), 7.33-7.39 (m, 2H), 8.62 (dd, 1H), 9.02 (dd, 1H), 11.64 (s, 1H).

### Beispiel 3

### 6-Amino-2-[1-(2-fluorbenzyl)-1H-pyrazolo[3,4-b]pyridin-3-yl]-7-(3,3,3-trifluor-2-hydroxypropyl)-7,9-dihydro-8H-purin-8-on

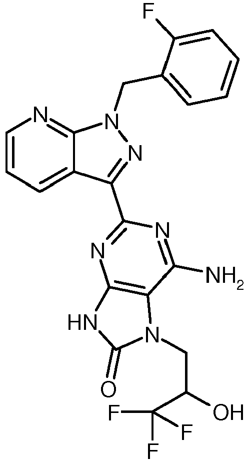

In Analogie zur Vorschrift unter Beispiel 1 wurden ausgehend von 28.5 mg (0.058 mmol) Beispiel 10A und 0.117 ml einer 1M Lösung von Bis-(trimethylsilyl)natriumamid in Tetrahydrofuran 10.4 mg (36.5% d. Th.) der Titelverbindung erhalten.

LC-MS (Methode 2): Rₜ = 0.89 min; MS (EIpos): m/z = 489 [M+H]⁺.

¹H-NMR (400 MHz, DMSO-d₆): δ [ppm] = 4.01-4.07 (m, 1H), 4.21-4.26 (m, 2H), 5.81 (s, 2H), 6.66 (br s, 2H), 7.10-7.24 (m, 4H), 7.33-7.40 (m, 2H), 8.63 (dd, 1H), 9.02 (dd, 1H), 11.78 (s, 1H).

### Beispiel 4

### 6-Amino-7-(2,2-difluorethyl)-2-[1-(2-fluorbenzyl)-1H-pyrazolo[3,4-b]pyridin-3-yl]-7,9-dihydro-8H-purin-8-on

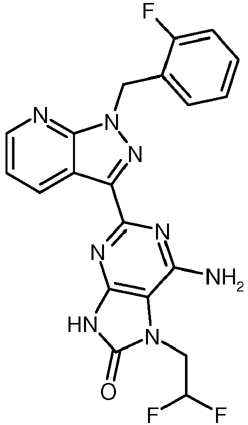

132 mg (0.279 mmol) der unter Beispiel 12A hergestellten Verbindung wurden in Tetrahydrofuran (8 ml) gelöst und bei 0°C mit 0.307 ml einer 1M Lösung von Bis-(trimethylsilyl)natriumamid in Tetrahydrofuran versetzt. Es wurde über Nacht bei RT weitergerührt. Anschliessend wurde eingeengt und der Rückstand mittels präparativer HPLC gereinigt (Eluent: Acetonitril/Wasser mit 0.05% Ameisensäure, Gradient). Es wurden 81 mg der Titelverbindung erhalten (62 % d. Th.).

LC-MS (Methode 2): Rt = 0.90 min; MS (EIpos): m/z = 441 [M+H]⁺.

¹H-NMR (400 MHz, DMSO-d₆): δ [ppm] = 4.48 (m, 2H), 5.81 (s, 2H), 6.06-6.36 (m, 1H), 6.81 (br s, 2H), 7.12-7.39 (m, 5H), 7.33-7.40 (m, 2H), 8.63 (dd, 1H), 9.03 (dd, 1H), 11.82 (s, 1H).

### Nicht erfindungsgemäßes Beispiel 5

### 2-[1-(2-Fluorbenzyl)-1H-pyrazolo[3,4-b]pyridin-3-yl]chinazolin-4-amin

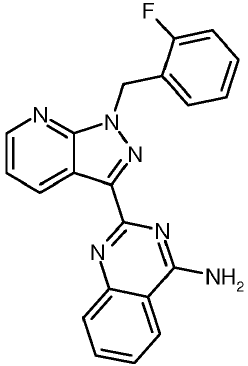

Nach der Allgemeinen Arbeitsvorschrift 1 wurden 95 mg (0.377 mmol) von Beispiel 3A zusammen mit 1.0 eq 2-Aminobenzonitril und 0.1 eq Kalium-tert.-butylat umgesetzt. Nach Reaktionskontrolle wurden weitere 0.5 eq Kalium-tert.-butylat zugegeben und es wurde erneut bei 160° C unter Mikrowellenbestrahlung bis zum vollständigen Umsatz erhitzt. Die Reaktionsmischung wurde mittels präparativer HPLC gereinigt (Eluent: Acetonitril/Wasser mit 0.05% Ameisensäure, Gradient).

Ausbeute: 27 mg (19 % d. Th.)

LC-MS (Methode 2): Rt = 0.82 min; MS (EIpos): m/z = 371 [M+H]⁺.

¹H-NMR (400 MHz, DMSO-d₆): δ [ppm] = 5.86 (s, 2H), 7.12-7.37 (m, 3H), 7.32-7.43 (m, 2H), 7.50 (m, 1H), 7.78-7.84 (m, 2H), 8.02 (br s, 2H), 8.25 (d, 1H), 8.65 (dd, 1H), 9.19 (dd, 1H).

### Nicht erfindungsgemäßes Beispiel 6

### 6-Chlor-2-[1-(2-fluorbenzyl)-1H-pyrazolo[3,4-b]pyridin-3-yl]chinazolin-4-amin

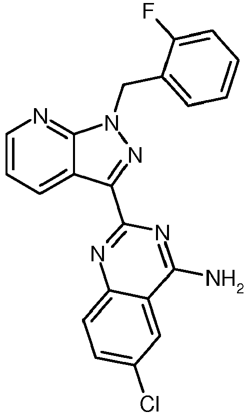

Die Substanz wurde nach der allgemeinen Arbeitsvorschrift 1 hergestellt. Es wurden 200 mg (0.793 mmol) Beispiel 3A und 121 mg (0.793 mmol) 2-Amino-5-chlorbenzonitril, sowie von Anfang an 1.0 eq Kalium-tert.-butylat (89 mg, 0.793 mmol) verwendet. Reinigung erfolgte durch Ausfällen der Substanz aus einem Acetonitril-Wasser Gemisch.

Ausbeute: 218 mg (65 % d. Th.)

LC-MS (Methode 2): Rt = 0.97 min; MS (EIpos): m/z = 405 [M+H]⁺.

¹H-NMR (400 MHz, DMSO-d₆): δ [ppm] = 5.86 (s, 2H), 7.11-7.27 (m, 3H), 7.33-7.44 (m, 2H), 7.81-7.87 (m, 2H), 8.11 (br s, 2H), 8.41 (d, 1H), 8.66 (dd, 1H), 9.15 (dd, 1H).

### Nicht erfindungsgemäßes Beispiel 7

### 5-Chlor-2-[1-(2-fluorbenzyl)-1H-pyrazolo[3,4-b]pyridin-3-yl]chinazolin-4-amin

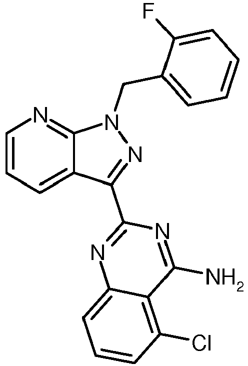

Die Substanz wurde nach der allgemeinen Arbeitsvorschrift 1 hergestellt. Es wurden 200 mg (0.793 mmol) Beispiel 3A und 121 mg (0.793 mmol) 2-Amino-6-chlorbenzonitril, sowie von Anfang an 1.0 eq Kalium-tert.-butylat (89 mg, 0.793 mmol) verwendet. Reinigung erfolgte durch Ausfällen der Substanz aus einem Acetonitril-Wasser Gemisch.

Ausbeute: 160 mg (47 % d. Th.)

LC-MS (Methode 2): Rₜ = 1.01 min; MS (EIpos): m/z = 405 [M+H]⁺.

¹H-NMR (400 MHz, DMSO-d₆): δ [ppm] = 5.87 (s, 2H), 7.14-7.16 (m, 2H), 7.24 (t, 1H), 7.33-7.44 (m, 2H), 7.55 (d, 1H), 7.74 (t, 1H), 7.81 (d, 1H), 8.66 (d, 1H), 9.21 (dd, 1H).

### Nicht erfindungsgemäßes Beispiel 8

### 7-Chlor-2-[1-(2-fluorbenzyl)-1H-pyrazolo[3,4-b]pyridin-3-yl]chinazolin-4-amin

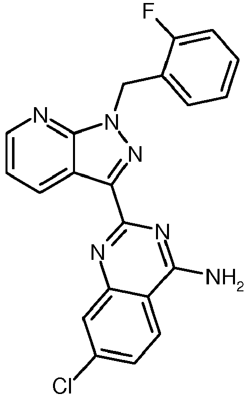

Die Substanz wurde nach der allgemeinen Arbeitsvorschrift 1 hergestellt. Es wurden 200 mg (0.793 mmol) Beispiel 3A und 121 mg (0.793 mmol) 2-Amino-4-chlorbenzonitril, sowie von Anfang an 1.0 eq Kalium-tert.-butylat (89 mg, 0.793 mmol) verwendet.

Ausbeute: 40 mg (12 % d. Th.)

LC-MS (Methode 2): Rₜ = 1.00 min; MS (EIpos): m/z = 405 [M+H]⁺.

¹H-NMR (400 MHz, DMSO-d₆): δ [ppm] = 5.86 (s, 2H), 7.12-7.27 (m, 3H), 7.33-7.43 (m, 2H), 7.54 (dd, 1H), 7.90 (d, 1H), 8.15 (br s, 2H), 8.28 (d, 1H), 8.65 (dd, 1H), 9.18 (dd, 1H).

### Nicht erfindungsgemäßes Beispiel 9

### 2-[1-(2-Fluorbenzyl)-1H-pyrazolo[3,4-b]pyridin-3-yl]-6-(trifluormethyl)chinazolin-4-amin

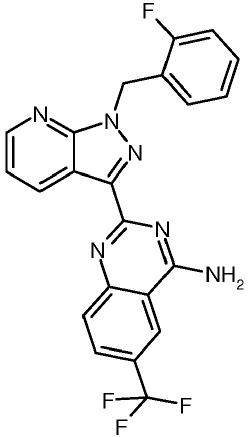

Die Substanz wurde nach der allgemeinen Arbeitsvorschrift 1 hergestellt. Es wurden 200 mg (0.793 mmol) Beispiel 3A und 147 mg (0.793 mmol) 2-Amino-5-(trifluormethyl)benzonitril, sowie von Anfang an 1.0 eq Kalium-tert.-butylat (89 mg, 0.793 mmol) verwendet. Reinigung erfolgte durch Ausfällen der Substanz aus einem Acetonitril-Wasser Gemisch.

Ausbeute: 144 mg (39 % d. Th.)

LC-MS (Methode 2): Rₜ = 1.06 min; MS (EIpos): m/z = 439 [M+H]⁺.

¹H-NMR (400 MHz, DMSO-d₆): δ [ppm] = 5.88 (s, 2H), 7.13-7.27 (m, 3H), 7.36 (m, 1H), 7.44 (dd, 1H), 7.99 (d, 1H), 8.05 (dd, 1H), 8.36 (br s, 2H), 8.67 (dd, 1H), 8.77 (m, 1H), 9.18 (dd, 1H).

### Nicht erfindungsgemäßes Beispiel 10

### 6-Fluor-2-[1-(2-fluorbenzyl)-1H-pyrazolo[3,4-b]pyridin-3-yl]chinazolin-4-amin

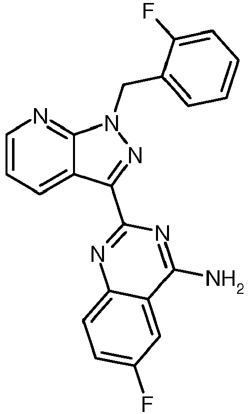

Die Substanz wurde nach der allgemeinen Arbeitsvorschrift 1 hergestellt. Es wurden 200 mg (0.793 mmol) Beispiel 3A und 108 mg (0.793 mmol) 2-Amino-5-fluorbenzonitril, sowie von Anfang an 1.0 eq Kalium-tert.-butylat (89 mg, 0.793 mmol) verwendet. Reinigung erfolgte durch Ausfällen der Substanz aus einem Acetonitril-Wasser Gemisch.

Ausbeute: 145 mg (45 % d. Th.)

LC-MS (Methode 2): Rₜ = 0.89 min; MS (EIpos): m/z = 389 [M+H]⁺.

¹H-NMR (400 MHz, DMSO-d₆): δ [ppm] = 5.86 (s, 2H), 7.12-7.20 (m, 2H), 7.22-7.27 (m, 1H), 7.33-7.37 (m, 1H), 7.41 (dd, 1H), 7.72 (dt, 1H), 7.91 (dd, 1H), 8.02 (br s, 2H), 8.10 (dd, 1H), 8.65 (dd, 1H), 9.16(dd, 1H).

### Nicht erfindungsgemäßes Beispiel 11

### 6,8-Dichlor-2-[1-(2-fluorbenzyl)-1H-pyrazolo[3,4-b]pyridin-3-yl]chinazolin-4-amin

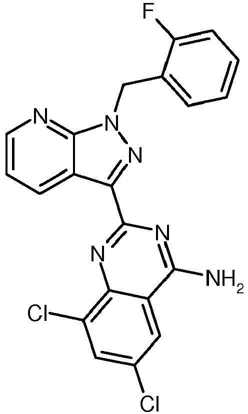

Die Substanz wurde nach der allgemeinen Arbeitsvorschrift 1 hergestellt. Es wurden 200 mg (0.793 mmol) Beispiel 3A und 148 mg (0.793 mmol) 2-Amino-3,5-dichlorbenzonitril, sowie von Anfang an 1.0 eq Kalium-tert.-butylat (89 mg, 0.793 mmol) verwendet.

Ausbeute: 11 mg (3 % d. Th.)

LC-MS (Methode 2): Rt = 1.30 min; MS (EIpos): m/z = 440 [M+H]⁺.

¹H-NMR (400 MHz, DMSO-d₆): δ [ppm] = 5.87 (s, 2H), 7.12-7.20 (m, 2H), 7.22-7.27 (m, 1H), 7.32-7.40 (m, 1H), 7.47 (dd, 1H), 8.14 (d, 1H), 8.40 (d, 1H), 8.67 (dd, 1H), 9.22 (dd, 1H).

### Nicht erfindungsgemäßes Beispiel 12

### 2-[1-(2-Fluorbenzyl)-1H-pyrazolo[3,4-b]pyridin-3-yl]thieno[2,3-d]pyrimidin-4-amin

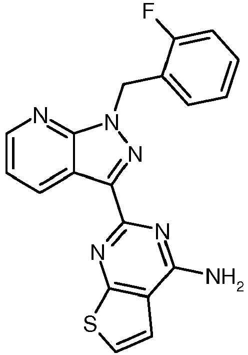

Die Substanz wurde nach der allgemeinen Arbeitsvorschrift 1 hergestellt. Es wurden 200 mg (0.793 mmol) Beispiel 3A und 98 mg (0.793 mmol) 2-Aminothiophen-3-carbonitril, sowie von Anfang an 1.0 eq Kalium-tert.-butylat (89 mg, 0.793 mmol) verwendet.

Ausbeute: 111 mg (36 % d. Th.)

LC-MS (Methode 2): Rt = 1.03 min; MS (EIpos): m/z = 377 [M+H]⁺.

¹H-NMR (400 MHz, DMSO-d₆): δ [ppm] = 5.84 (s, 2H), 7.13-7.26 (m, 3H), 7.33-7.42 (m, 2H), 7.57-7.61 (m, 2H), 7.72 (br s, 2H), 8.64 (dd, 1H), 9.05 (dd, 1H).

### Nicht erfindungsgemäßes Beispiel 13

### 6-[1-(2-Fluorbenzyl)-1H-pyrazolo[3,4-b]pyridin-3-yl]-3-methyl[1,2]thiazolo[5,4-d]pyrimidin-4-amin

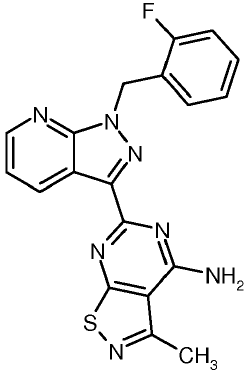

Die Substanz wurde nach der allgemeinen Arbeitsvorschrift 1 hergestellt. Es wurden 200 mg (0.793 mmol) Beispiel 3A und 110 mg (0.793 mmol) 5-Amino-3-methyl-isothiazol-4-carbonitril, sowie von Anfang an 1.0 eq Kalium-tert.-butylat (89 mg, 0.793 mmol) verwendet. Reinigung erfolgte durch Ausfällen der Substanz aus einem Acetonitril-Wasser Gemisch.

Ausbeute: 234 mg (75 % d. Th.)

LC-MS (Methode 2): Rₜ = 1.05 min; MS (EIpos): m/z = 392 [M+H]⁺.

¹H-NMR (400 MHz, DMSO-d₆): δ [ppm] = 2.80 (s, 3H), 5.87 (s, 2H), 7.13-7.26 (m, 3H), 7.34-7.39 (m, 1H), 7.42-7.45 (m, 1H), 8.67 (dd, 1H), 9.12 (dd, 1H).

### Nicht erfindungsgemäßes Beispiel 14

### 6-[1-(2-Fluorbenzyl)-1H-pyrazolo[3,4-b]pyridin-3-yl]-3-methyl[1,2]oxazolo[5,4-d]pyrimidin-4-amin

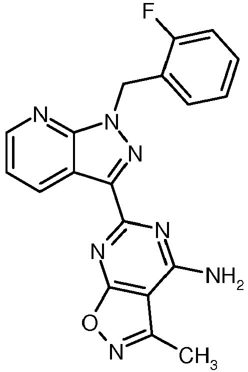

Die Substanz wurde nach der allgemeinen Arbeitsvorschrift 1 hergestellt. Es wurden 300 mg (1.189 mmol) Beispiel 3A und 146 mg (1.189 mmol) 5-Amino-3-methyl-4-isoxazol-4-carbonitril, sowie von Anfang an 1.0 eq Kalium-tert.-butylat (133 mg, 1.189 mmol) verwendet.

Ausbeute: 43 mg (10 % d. Th.)

LC-MS (Methode 2): Rt = 0.99 min; MS (EIpos): m/z = 376 [M+H]⁺.

¹H-NMR (400 MHz, DMSO-d₆): δ [ppm] = 2.60 (s, 3H), 5.87 (s, 2H), 7.13-7.27 (m, 3H), 7.34-7.39 (m, 1H), 7.43-7.46 (m, 1H), 8.67 (dd, 1H), 9.06 (dd, 1H).

### Nicht erfindungsgemäßes Beispiel 15

### 6-[1-(2-Fluorbenzyl)-1H-pyrazolo[3,4-b]pyridin-3-yl]-3-(trifluormethyl)[1,2]oxazolo[5,4-d]pyrimidin-4-amin

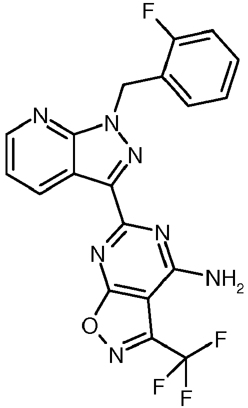

Die Substanz wurde nach der allgemeinen Arbeitsvorschrift 1 hergestellt. Es wurden 200 mg (0.793 mmol) Beispiel 3A und 140 mg (0.793 mmol) 5-Amino-3-(trifluormethyl)-isoxazol-4-carbonitril, sowie von Anfang an 1.0 eq Kalium-tert.-butylat (89 mg, 0.793 mmol) verwendet. Die Reaktionsmischung wurde dreimal für jeweils 2h in der Mikrowelle bestrahlt.

Ausbeute: 19 mg (5 % d. Th.)

LC-MS (Methode 3): Rt = 1.30 min; MS (EIpos): m/z = 430 [M+H]⁺.

¹H-NMR (400 MHz, DMSO-d₆): δ [ppm] = 5.89 (s, 2H), 7.14-7.18 (m, 1H), 7.21-7.27 (m, 2H), 7.35-7.40 (m, 1H), 7.46-7.49 (m, 1H), 8.70 (dd, 1H), 9.15 (dd, 1H).

### Beispiel 16

### 6-[1-(2-Fluorbenzyl)-1H-pyrazolo[3,4-b]pyridin-3-yl]-1H-pyrazolo[3,4-d]pyrimidin-4-amin

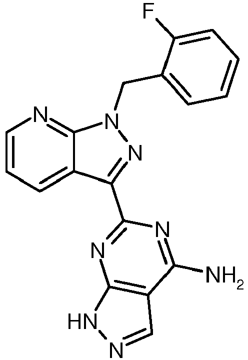

Die Substanz wurde nach der allgemeinen Arbeitsvorschrift 1 hergestellt. Es wurden 200 mg (0.793 mmol) Beispiel 3A und 140 mg (0.793 mmol) 3-Amino-1H-pyrazol-4-carbonitril, sowie 0.1 eq Kalium-tert.-butylat (8.9 mg, 0.079 mmol) verwendet. Nach 1h bei 160°C in der Mikrowelle wurden nochmals 0.5 eq Kalium-tert.-butylat (44 mg, 0.396 mmol) zugegeben und weitere 2h bei 160°C in der Mikrowelle belassen. Danach wurden nochmals 0.25 eq Kalium-tert.-butylat (22 mg, 0.198 mmol) zugegeben und weitere 2h bei 160°C in der Mikrowelle belassen und danach nochmals 0.25 eq Kalium-tert.-butylat (22 mg, 0.198 mmol) zugegeben und weitere 2h bei 160°C in der Mikrowelle reagieren lassen. Reinigung erfolgte per HPLC.

Ausbeute: 115 mg (37 % d. Th.)

LC-MS (Methode 2): Rₜ = 0.79 min; MS (EIpos): m/z = 361 [M+H]⁺.

¹H-NMR (400 MHz, DMSO-d₆): δ [ppm] = 5.89 (s, 2H), 7.13-7.26 (m, 3H), 7.33-7.42 (m, 2H), 7.81 (s br, 2H), 8.11 (s, 1H), 8.64 (dd, 1H), 9.06 (dd, 1H), 13.41 (s br, 1H).

### Beispiel 17

### 6-[1-(2-Fluorbenzyl)-1H-pyrazolo[3,4-b]pyridin-3-yl]-1-methyl-1H-pyrazolo[3,4-d]pyrimidin-4-amin

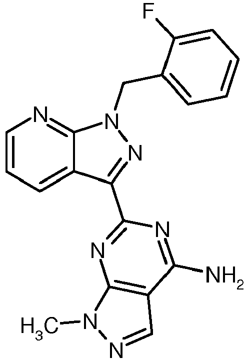

Die Substanz wurde nach der allgemeinen Arbeitsvorschrift 1 hergestellt. Es wurden 200 mg (0.793 mmol) Beispiel 3A und 96 mg (0.793 mmol) 5-Amino-1-methyl-1H-pyrazol-4-carbonitril, sowie von Anfang an 1.0 eq Kalium-tert.-butylat (89 mg, 0.793 mmol) verwendet. Reinigung erfolgte durch Ausfällen der Substanz aus einem Acetonitril-Wasser Gemisch.

Ausbeute: 155 mg (52 % d. Th.)

LC-MS (Methode 2): Rₜ = 0.89 min; MS (EIpos): m/z = 375 [M+H]⁺.

¹H-NMR (400 MHz, DMSO-d₆): δ [ppm] = 4.00 (s, 3H), 5.86 (s, 2H), 7.13-7.15 (m, 2H), 7.22-7.27 (m, 1H), 7.33-7.37 (m, 1H), 7.40-7.43 (m, 1H), 7.90 (s br, 2H), 8.10 (s, 1H), 8.65 (dd, 1H), 9.13 (dd, 1H).

### Beispiel 18

### 6-[1-(2-Fluorbenzyl)-1H-pyrazolo[3,4-b]pyridin-3-yl]-3-methoxy-1H-pyrazolo[3,4-d]pyrimidin-4-amin

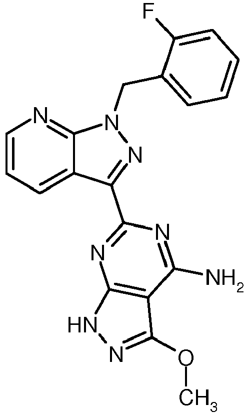

Die Substanz wurde nach der allgemeinen Arbeitsvorschrift 1 hergestellt. Es wurden 1000 mg (3.964 mmol) Beispiel 3A und 547 mg (3.964 mmol) 5-Amino-3-methoxy-1H-pyrazol-4-carbonitril, sowie von Anfang an 1.0 eq Kalium-tert.-butylat (444 mg, 3.964 mmol) verwendet. Reinigung erfolgte durch Ausfällen der Substanz aus einem Acetonitril-Wasser Gemisch und anschliessende präparative HPLC-Reinigung (Eluent: Acetonitril/Wasser mit 0.05% Ameisensäure, Gradient).

Ausbeute: 39 mg (3 % d. Th.)

LC-MS (Methode 2): Rₜ = 0.87 min; MS (EIpos): m/z = 391 [M+H]⁺.

¹H-NMR (400 MHz, DMSO-d₆): δ [ppm] = 3.98 (s, 3H), 5.83 (s, 2H), 7.13-7.26 (m, 3H), 7.33-7.41 (m, 2H), 8.64 (dd, 1H), 9.08 (dd, 1H), 12.43 (s, 1H).

### Beispiel 19

### 6-[1-(2-Fluorbenzyl)-1H-pyrazolo[3,4-b]pyridin-3-yl]-3-methyl-1H-pyrazolo[3,4-d]pyrimidin-4-amin

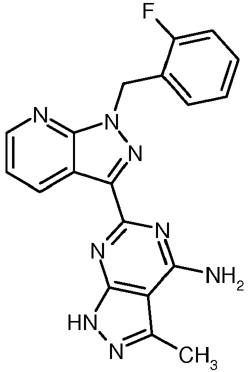

Die Substanz wurde nach der allgemeinen Arbeitsvorschrift 1 hergestellt. Es wurden 200 mg (0.793 mmol) Beispiel 3A und 96 mg (0.793 mmol) 3-Amino-4-cyano-5-methylpyrazol, sowie von Anfang an 1.0 eq Kalium-tert.-butylat (89 mg, 0.793 mmol) verwendet. Reinigung erfolgte durch Ausfällen der Substanz aus einem Acetonitril-Wasser Gemisch.

Ausbeute: 115 mg (38 % d. Th.)

LC-MS (Methode 2): Rₜ = 0.81 min; MS (EIpos): m/z = 375 [M+H]⁺.

¹H-NMR (400 MHz, DMSO-d₆): δ [ppm] = 2.56 (s, 3H), 5.83 (s, 2H), 7.13-7.17 (m, 1H), 7.19-7.26 (m, 2H), 7.34-7.41 (m, 2H), 8.64 (dd, 1H), 9.10 (dd, 1H), 12.92 (s br, 1H).

### Beispiel 20

### 3-({4-Amino-6-[1-(2-fluorbenzyl)-1H-pyrazolo[3,4-b]pyridin-3-yl]-1H-pyrazolo[3,4-d]pyrimidin-3-yl}oxy)propan-1-ol

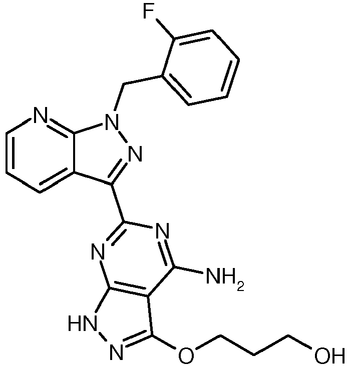

Die Substanz wurde nach der allgemeinen Arbeitsvorschrift 1 hergestellt. Es wurden 300 mg (1.189 mmol) Beispiel 3A und 216 mg (1.189 mmol) 5-Amino-3-(3-hydroxypropoxy)-1H-pyrazol-4-carbonitril, sowie von Anfang an 1.0 eq Kalium-tert.-butylat (133 mg, 1.189 mmol) verwendet.

Ausbeute: 128 mg (22 % d. Th.)

LC-MS (Methode 2): Rₜ = 0.83 min; MS (EIpos): m/z = 435 [M+H]⁺.

¹H-NMR (400 MHz, DMSO-d₆): δ [ppm] = 1.92-1.99 (m, 2H), 3.61 (q, 2H), 4.36 (t, 2H), 4.55 (t, 1H), 5.83 (s, 2H), 7.13-7.26 (m, 3H), 7.34-7.41 (m, 2H), 8.64 (dd, 1H), 9.08 (dd, 1H), 12.39 (s, 1H).

### Beispiel 21

### 2-({4-Amino-6-[1-(2-fluorbenzyl)-1H-pyrazolo[3,4-b]pyridin-3-yl]-1H-pyrazolo[3,4-d]pyrimidin-3-yl}oxy)ethanol

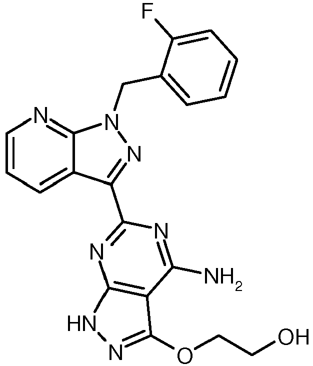

Die Substanz wurde nach der allgemeinen Arbeitsvorschrift 1 hergestellt. Es wurden 300 mg (1.189 mmol) Beispiel 3A und 199 mg (1.189 mmol) 5-Amino-3-(2-hydroxyethoxy)-1H-pyrazol-4-carbonitril, sowie von Anfang an 1.0 eq Kalium-tert.-butylat (133 mg, 1.189 mmol) verwendet.

Ausbeute: 223 mg (42 % d. Th.)

LC-MS (Methode 2): Rₜ = 0.80 min; MS (EIpos): m/z = 421 [M+H]⁺.

¹H-NMR (400 MHz, DMSO-d₆): δ [ppm] = 3.76 (q, 2H), 4.25 (t, 2H), 5.07 (t, 1H), 5.83 (s, 2H), 7.13-7.26 (m, 3H), 7.34-7.42 (m, 2H), 8.64 (dd, 1H), 9.07 (dd, 1H), 12.40 (s, 1H).

### Beispiel 22

### N³,N³-Diethyl-6-[1-(2-fluorbenzyl)-1H-pyrazolo[3,4-b]pyridin-3-yl]-1H-pyrazolo[3,4-d]pyrimidin-3,4-diamin

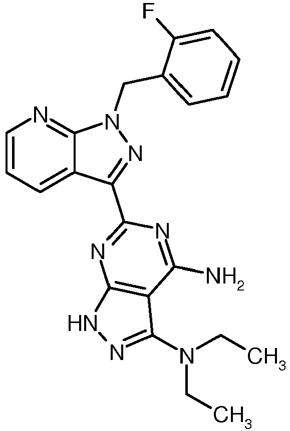

Die Substanz wurde nach der allgemeinen Arbeitsvorschrift 1 hergestellt. Es wurden 250 mg (0.991 mmol) Beispiel 3A und 177 mg (0.991 mmol) 5-Amino-3-(3-hydroxypropoxy)-1H-pyrazol-4-carbonitril, sowie von Anfang an 1.0 eq Kalium-tert.-butylat (111 mg, 0.991 mmol) verwendet.

Ausbeute: 131 mg (30 % d. Th.)

LC-MS (Methode 2): Rₜ = 0.98 min; MS (EIpos): m/z = 432 [M+H]⁺.

¹H-NMR (400 MHz, DMSO-d₆): δ [ppm] = 1.04 (t, 6H), 3.21 (q, 4H), 5.83 (s, 2H), 7.13-7.26 (m, 3H), 7.33-7.41 (m, 2H), 8.64 (dd, 1H), 9.09 (dd, 1H), 12.61 (s, 1H).

### Nicht erfindungsgemäßes Beispiel 23

### 2-[1-(2-Fluorbenzyl)-1H-pyrazolo[3,4-b]pyridin-3-yl]-6-methyl-8,9-dihydro-7H-pyrimido[4,5-b][1,4]diazepin-4-amin

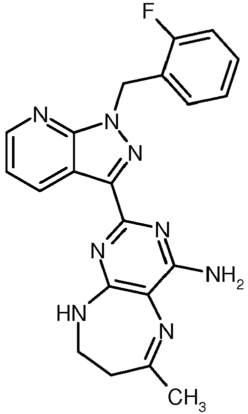

300 mg (0.856 mmol) der Verbindung aus Beispiel 1A wurden in 3 ml Ethanol gelöst und mit 129 mg (0.856 mmol) 4-(Dimethylamino)butan-2-on-Hydrochlorid versetzt. Es wurde für 1 h in der Mikrowelle bei 150°C gerührt. Nach dem Abkühlen wurde am Rotationsverdampfer eingengt, der Rückstand in Dichlormethan aufgenommen und über Kieselgel filtriert. Der Nutschkuchen wurde mit Dichlormethan nachgewaschen und das Filtrat am Rotationsverdampfer eingeengt. Der Rückstand wurde mittels präparativer HPLC gereinigt (Eluent: Acetonitril/Wasser mit 0.1% Trifluoressigsäure, Gradient 10:90 → 90:10). Der Rückstand wurde zwischen Essigsäureethylester und gesättigter wässriger Natriumhydrogencarbonat-Lösung verteilt, und die organische Phase eingeengt. Es wurden 18 mg (5 % d. Th.) der Titelverbindung erhalten.

LC-MS (Methode 2): Rₜ = 0.82 min; MS (EIpos): m/z = 403 [M+H]⁺.

¹H-NMR (400 MHz, DMSO-d₆): δ [ppm] = 2.17 (s, 3H), 2.64-2.71 (m, 2H), 3.35-3.38(m, 2H), 5.79 (s, 2H), 6.56 (s br, 2H), 7.11-7.16 (m, 2H), 7.20-7.25 (m, 1H), 7.32-7.38 (m, 3H), 8.61 (dd, 1H), 9.08 (dd, 1H).

### Nicht erfindungsgemäßes Beispiel 24

### 6-(4-Chlorphenyl)-2-[1-(2-fluorbenzyl)-1H-pyrazolo[3,4-b]pyridin-3-yl]-8,9-dihydro-7H-pyrimido[4,5-b][1,4]diazepin-4-amin

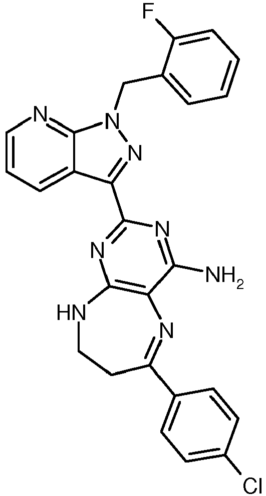

Die Darstellung erfolgte analog zu Beispiel 23 aus 300 mg (0.856 mmol) der Verbindung aus Beispiel 1A und 212 mg (0.856 mmol) 1-(4-Chlorphenyl)-3-(dimethylamino)propan-1-on-Hydrochlorid. Es wurden 104 mg (23% d. Th.) der Titelverbindung erhalten.

LC-MS (Methode 2): Rₜ = 1.42 min; MS (EIpos): m/z = 499 [M+H]⁺.

¹H-NMR (400 MHz, DMSO-d₆): δ [ppm] = 3.14-3.18 (m, 2H), 3.46-3.51 (m, 2H), 5.81 (s, 2H), 6.78 (s br, 2H), 7.11-7.18 (m, 2H), 7.21-7.26 (m, 1H), 7.33-7.39 (m, 2H), 7.46 (d, 2H), 7.63 (t, 1H) 8.04 (d, 2H), 8.62 (dd, 1H), 9.13 (dd, 1H).

### Nicht erfindungsgemäßes Beispiel 25

### 6-(2-Chlorphenyl)-2-[1-(2-fluorbenzyl)-1H-pyrazolo[3,4-b]pyridin-3-yl]-8,9-dihydro-7H-pyrimido[4,5-b][1,4]diazepin-4-amin

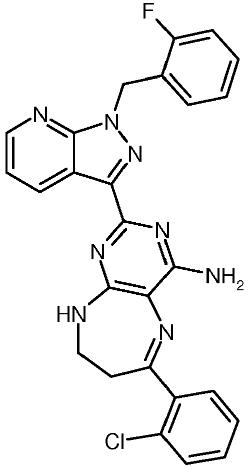

Die Darstellung erfolgte analog zu Beispiel 23 aus 300 mg (0.856 mmol) der Verbindung aus Beispiel 1A und 212 mg (0.856 mmol) 1-(2-Chlorphenyl)-3-(dimethylamino)propan-1-on-Hydrochlorid. Es wurden 77 mg (17% d. Th.) der Titelverbindung erhalten.

LC-MS (Methode 2): Rₜ = 1.02 min; MS (EIpos): m/z = 499 [M+H]⁺.

¹H-NMR (400 MHz, DMSO-d₆): δ [ppm] = 3.05-3.08 (m, 2H), 3.52-3.56 (m, 2H), 5.81 (s, 2H), 6.40 (s br, 1H), 6.83 (s br, 1H), 7.12-7.19 (m, 2H), 7.20-7.26 (m, 1H), 7.33-7.43 (m, 4H), 7.47-7.52 (m, 1H), 7.56-7.59 (m, 1H), 7.74 (t, 1H), 8.62 (dd, 1H), 9.12 (dd, 1H).

### Nicht erfindungsgemäßes Beispiel 26

### 6-(3-Chlorphenyl)-2-[1-(2-fluorbenzyl)-1H-pyrazolo[3,4-b]pyridin-3-yl]-8,9-dihydro-7H-pyrimido[4,5-b][1,4]diazepin-4-amin

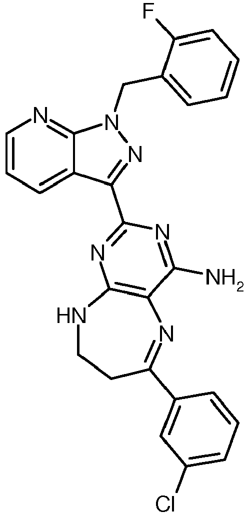

Die Darstellung erfolgte analog zu Beispiel 23 aus 300 mg (0.856 mmol) der Verbindung aus Beispiel 1A und 212 mg (0.856 mmol) 1-(3-Chlorphenyl)-3-(dimethylamino)propan-1-on-Hydrochlorid. Es wurden 104 mg (23% d. Th.) der Titelverbindung erhalten.

LC-MS (Methode 2): Rₜ = 1.04 min; MS (EIpos): m/z = 499 [M+H]⁺.

¹H-NMR (400 MHz, DMSO-d₆): δ [ppm] = 3.15-3.17 (m, 2H), 3.47-3.51 (m, 2H), 5.81 (s, 2H), 6.80 (s br, 2H), 7.12-7.18 (m, 2H), 7.21-7.26 (m, 1H), 7.33-7.39 (m, 2H), 7.43-7.49 (m, 2H), 7.64 (t, 1H), 7.92-7.98 (m, 1H), 8.04 (s, 1H), 8.62 (dd, 1H), 9.13 (dd, 1H).

### Nicht erfindungsgemäßes Beispiel 27

### 2-[1-(2-Fluorbenzyl)-1H-pyrazolo[3,4-b]pyridin-3-yl]-6-phenyl-8,9-dihydro-7H-pyrimido[4,5-b][1,4]diazepin-4-amin

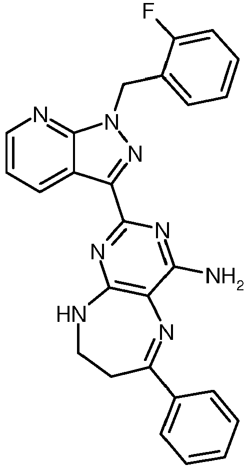

Die Darstellung erfolgte analog zu Beispiel 23 aus 300 mg (0.856 mmol) der Verbindung aus Beispiel 1A und 183 mg (0.856 mmol) 1-Phenyl-3-(dimethylamino)propan-1-on-Hydrochlorid. Es wurden 35 mg (9% d. Th.) der Titelverbindung erhalten.

LC-MS (Methode 2): Rₜ = 0.97 min; MS (EIpos): m/z = 465 [M+H]⁺.

¹H-NMR (400 MHz, DMSO-d₆): δ [ppm] = 3.16-3.19 (m, 2H), 3.48-3.52 (m, 2H), 5.81 (s, 2H), 6.74 (s br, 2H), 7.12-7.18 (m, 2H), 7.21-7.26 (m, 1H), 7.33-7.39 (m, 2H), 7.41-7.46 (m, 3H), 7.60 (t, 1H), 7.99-8.02 (m, 2H), 8.62 (dd, 1H), 9.13 (dd, 1H).

### Nicht erfindungsgemäßes Beispiel 28

### 6-Amino-2-[1-(2-fluorbenzyl)-1H-pyrazolo[3,4-b]pyridin-3-yl]-7,9-dihydro-8H-purin-8-thion

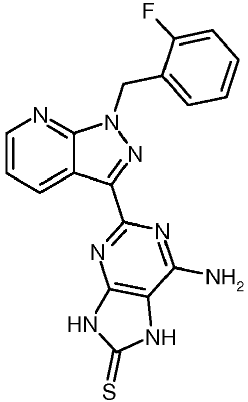

Es wurden 500 mg (1.427 mmol) der Verbindung aus Beispiel 1A mit 3 ml Ethanol und 423 mg (2.640 mmol) Kaliumethylxantogenat versetzt und für 1 h in der Mikrowelle bei 150°C gerührt. Die Reaktionsmischung wurde mit 100 ml Wasser aufgekocht. Es wurde filtriert und die wässrige Phase mit Essigsäure angesäuert wobei ein Niederschlag anfällt der abfiltriert wurde. Der Rückstand wurde mit gesättigter wässriger Natriumhydrogencarbonat-Lösung verrührt, abfiltriert und am Hochvakuum getrocknet. Es wurden 269 mg der Titelverbindung (48% d. Th.) erhalten, die ohne weitere Reinigung umgesetzt wurde.

LC-MS (Methode 2): Rₜ = 0.85 min; MS (EIpos): m/z = 493 [M+H]⁺.

¹H-NMR (400 MHz, DMSO-d₆): δ [ppm] = 5.82 (s, 2H), 6.97 (s br, 2H), 7.12-7.26 (m, 3H), 7.33-7.41 (m, 2H), 8.64 (dd, 1H), 9.00 (dd, 1H), 12.16 (s br, 1H), 13.12 (s br, 1H).

### Nicht erfindungsgemäßes Beispiel 29

### 2-[1-(2-Fluorbenzyl)-1H-pyrazolo[3,4-b]pyridin-3-yl]-8-(methylsulfanyl)-7H-purin-6-amin

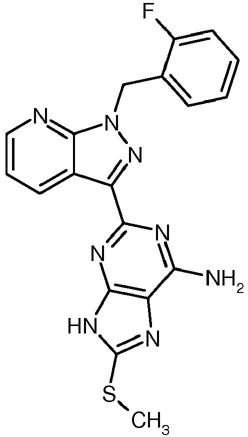

Es wurden 100 mg (0.255 mmol) der Verbindung aus Beispiel 28 in 5 ml Dimethylformamid gelöst und mit 49 mg (0.357 mmol) Kaliumcarbonat versetzt. Das Gemisch wurde für 10 Min. auf 70° C erwärmt und nach dem Abkühlen mit 18 µl (0.291 mmol) Iodmethan versetzt und 48 h bei RT gerührt. Das Gemisch wurde eingeengt und der Rückstand in Wasser aufgenommen und mit Essigsäure angesäuert. Es fällt ein Niederschlag aus, der abfiltriert und mit Wasser gewaschen wurde. Die Reinigung erfolgte mittels präparativer HPLC (Eluent: Methanol/Wasser, Gradient 30:70 → 90:10). Es wurden 18 mg der Titelverbindung erhalten (17% d. Th.).

LC-MS (Methode 2): Rₜ = 0.88 min; MS (EIpos): m/z = 407 [M+H]⁺.

¹H-NMR (400 MHz, DMSO-d₆): δ [ppm] = 2.70 (s, 2H), 2.75 (s, 1H), 5.81 (s, 2H), 7.07-7.26 (m, 5H), 7.33-7.39 (m, 2H), 8.63 (dd, 1H), 9.06-9.09 (m, 1H), 13.06 (s, 1H).

### Nicht erfindungsgemäßes Beispiel 30

### 2-[1-(2-Fluorbenzyl)-1H-pyrazolo[3,4-b]pyridin-3-yl]-9-methyl-8-(methylsulfanyl)-9H-purin-6-amin

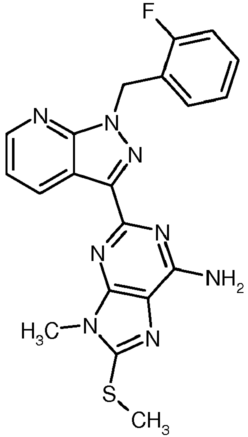

Es wurden 100 mg (0.255 mmol) der Verbindung aus Beispiel 29 in 5 ml Dimethylformamid gelöst und mit 49 mg (0.357 mmol) Kaliumcarbonat versetzt. Das Gemisch wurde wurde für 10 Min. auf 70° C erwärmt und nach dem Abkühlen mit 18 µl (0.291 mmol) Iodmethan versetzt und 48 h bei RT gerührt. Das Gemisch wurde eingeengt und der Rückstand in Wasser aufgenommen und mit Essigsäure angesäuert. Es fällt ein Niederschlag aus, der abfiltriert und mit Wasser gewaschen wurde. Die Reinigung erfolgte mittels präparativer HPLC (Eluent: Methanol/Wasser, Gradient 30:70 → 90:10). Es wurden 26 mg der Titelverbindung erhalten (24% d. Th.).

LC-MS (Methode 2): Rₜ = 1.01 min; MS (EIpos): m/z = 421 [M+H]⁺.

¹H-NMR (400 MHz, DMSO-d₆): δ [ppm] = 2.75 (s, 3H), 3.69 (s, 3H), 5.84 (s, 2H), 7.10-7.16 (m, 2H), 7.22-7.30 (m, 3H), 7.33-7.41 (m, 2H), 8.63 (d, 1H), 9.14 (dd, 1H).

### Nicht erfindungsgemäßes Beispiel 31

### 8-(Ethylsulfanyl)-2-[1-(2-fluorbenzyl)-1H-pyrazolo[3,4-b]pyridin-3-yl]-7H-purin-6-amin

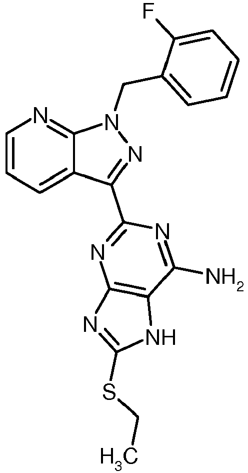

Es wurden 150 mg (0.382 mmol) der Verbindung aus Beispiel 28 in 2 ml Dimethylformamid gelöst und mit 58 mg (0.420 mmol) Kaliumcarbonat versetzt. Das Gemisch wurde wurde für 10 Min. auf 70° C erwärmt und nach dem Abkühlen mit 31 µl (0.382 mmol) Iodethan versetzt und 16 h bei RT gerührt. Das Gemisch wurde eingeengt, der Rückstand an Kieselgel adsorbiert und mittels Chromatographie an Kieselgel gereinigt (Eluent: Dichlormethan/Methanol = 20:1). Es wurden 91 mg der Titelverbindung erhalten (51% d. Th.).

LC-MS (Methode 3): Rₜ = 1.02 min; MS (EIpos): m/z = 421 [M+H]⁺.

¹H-NMR (400 MHz, DMSO-d₆): δ [ppm] = 1.36 (t, 3H), 3.27 (q, 2H), 5.81 (s, 2H), 7.12-7.26 (m, 4H), 7.33-7.39 (m, 2H), 8.63 (dd, 1H), 9.07 (d, 1H), 13.06 (s, 1H).

### Nicht erfindungsgemäßes Beispiel 32

### 2-[1-(2-Fluorbenzyl)-1H-pyrazolo[3,4-b]pyridin-3-yl]-8-[(2,2,2-trifluorethyl)sulfanyl]-7H-purin-6-amin

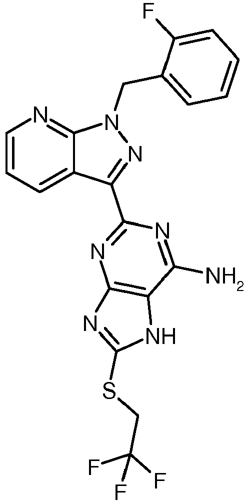

Es wurden 150 mg (0.382 mmol) der Verbindung aus Beispiel 28 in 2 ml Dimethylformamid gelöst und mit 58 mg (0.420 mmol) Kaliumcarbonat versetzt. Das Gemisch wurde wurde für 10 Min. auf 70° C erwärmt und nach dem Abkühlen mit 38 µl (0.382 mmol) Trifluorethyliodid versetzt und 16 h bei RT gerührt. Das Gemisch wurde eingeengt, der Rückstand an Kieselgel adsorbiert und mittels Chromatographie an Kieselgel gereinigt (Eluent: Dichlormethan/Methanol = 20:1). Es wurden 49 mg der Titelverbindung erhalten (25% d. Th.).

LC-MS (Methode 3): Rₜ = 1.11 min; MS (EIpos): m/z = 475 [M+H]⁺.

¹H-NMR (400 MHz, DMSO-d₆): δ [ppm] = 4.35 (q, 2H), 5.82 (s, 2H), 7.12-7.26 (m, 3H), 7.33-7.41 (m, 4H), 8.63 (d, 1H), 9.06 (d, 1H), 13.35 (s, 1H).

### Beispiel 33

### 6-Amino-2-[1-(2-fluorbenzyl)-1H-pyrazolo[3,4-b]pyridin-3-yl]-7,9-dihydro-8H-purin-8-on

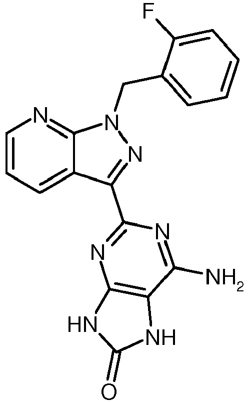

Es wurden 5.000 g (14.271 mmol) der Verbindung aus Beispiel 1A in 50 ml Dimethylformamid gelöst und mit 11.570 g (71.355 mmol) Carbonyldiimidazol sowie 18.051 g (178.387 mmmol) Triethylamin versetzt. Das Gemisch wurde 16 h bei 100°C gerührt. Das Reaktionsgemisch wurde zwischen Wasser und Essigsäureethylester verteilt, und die wässrige Phase mit Essigsäureethylester extrahiert. Die vereinigten organischen Phasen wurden im Vakuum eingeengt und der Rückstand mit tert-Butylmethylether verrührt. Der entstandene Feststoff wurde abfiltriert und mittels präparativer HPLC gereinigt (Eluent: Methanol/Wasser, Gradient 20:80 → 90:10). Es wurden 2.120 g der Titelverbindung erhalten (38% d. Th.).

LC-MS (Methode 2): Rₜ = 0.80 min; MS (EIpos): m/z = 377 [M+H]⁺.

¹H-NMR (400 MHz, DMSO-d₆): δ [ppm] = 5.81 (s, 2H), 6.63 (s br, 2H) 7.12-7.25 (m, 3H), 7.33-7.38 (m, 2H), 8.63 (dd, 1H), 8.96 (dd, 1H), 10.08 (s, 1H), 11.45 (s, 1H).

### Beispiel 34

### 6-Amino-2-[1-(2-fluorbenzyl)-1H-pyrazolo[3,4-b]pyridin-3-yl]-9-(²H₃)methyl-7,9-dihydro-8H-purin-8-on

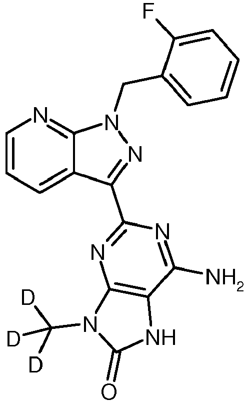

Es wurden 200 mg (0.531 mmol) der Verbindung aus Beispiel 33 sowie 146 mg (0.531 mmol) BEMP in 10 ml Dimethylformamid vorgelegt und bei 0°C innerhalb von 10 min mit einer Lösung aus 77 mg (0.531 mmol) Iodmethan-d₃ in 2 ml Dimethylformamid tropfenweise versetzt. Es wurde 1 h bei 0°C gerührt. Anschließend wurde die Reaktionslösung im Vakuum eingeengt und mit Acetonitril/Wasser versetzt. Der entstandene Niederschlag wurde abfiltriert und mittels präparativer HPLC gereinigt (Eluent: Methanol/Wasser mit 0.2% Trifluoressigsäure = 60:40). Es wurden 88 mg der Titelverbindung erhalten (42% d. Th.).

LC-MS (Methode 2): Rₜ = 0.86 min; MS (EIpos): m/z = 494 [M+H]⁺.

¹H-NMR (400 MHz, DMSO-d₆): δ [ppm] = 5.83 (s, 2H), 6.63 (s br, 2H) 7.09-7.15 (m, 2H), 7.21-7.26 (m, 1H), 7.32-7.40 (m, 2H), 8.63 (dd, 1H), 9.04 (dd, 1H), 10.27 (s, 1H).

### Beispiel 35

### 6-Amino-9-ethyl-2-[1-(2-fluorbenzyl)-1H-pyrazolo[3,4-b]pyridin-3-yl]-7,9-dihydro-8H-purin-8-on

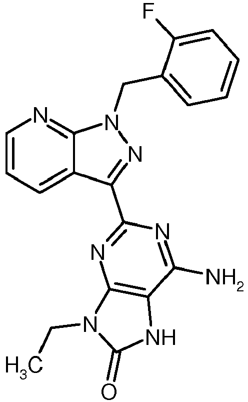

Es wurden 200 mg (0.531 mmol) der Verbindung aus Beispiel 33 sowie 146 mg (0.531 mmol) BEMP in 10 ml Dimethylformamid vorgelegt und bei 0°C innerhalb von 10 min mit einer Lösung aus 82 mg (0.531 mmol) Ethyliodid in 2 ml Dimethylformamid tropfenweise versetzt. Es wurde 20 min bei 0°C gerührt. Anschließend wurde die Reaktionslösung im Vakuum eingeengt und der Rückstand mittels präparativer HPLC gereinigt (Eluent: Acetonitril/Wasser, Gradient 10:90 → 90:10). Es wurden 67 mg der Titelverbindung erhalten (31% d. Th.).

LC-MS (Methode 4): Rₜ = 0.86 min; MS (EIpos): m/z = 405 [M+H]⁺.

¹H-NMR (400 MHz, DMSO-d₆): δ [ppm] = 1.31 (t, 3H), 3.90 (q, 2H), 5.84 (s, 2H), 6.62 (s br, 2H) 7.09-7.15 (m, 2H), 7.22-7.26 (m, 1H), 7.33-7.42 (m, 2H), 8.63 (dd, 1H), 9.00 (dd, 1H), 10.27 (s, 1H).

### Beispiel 36

### 6-Amino-2-[1-(2-fluorbenzyl)-1H-pyrazolo[3,4-b]pyridin-3-yl]-9-(2,2,2-trifluorethyl)-7,9-dihydro-8H-purin-8-on

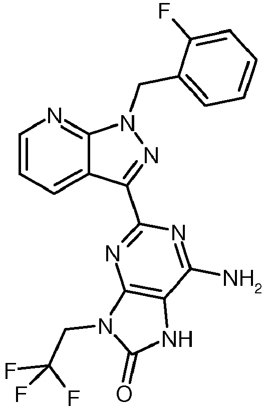

Es wurden 100 mg (0.266 mmol) der Verbindung aus Beispiel 33 sowie 73 mg (0.266 mmol) BEMP in 9 ml Dimethylformamid vorgelegt und bei 0°C innerhalb von 10 min mit einer Lösung aus 75 mg (0.266 mmol) 2,2,2-Trifluorethyltrichlormethansulfonat in 1 ml Dimethylformamid tropfenweise versetzt. Es wurde 20 min bei 0°C gerührt. Anschließend wurde die Reaktionslösung im Vakuum eingeengt und der Rückstand mittels präparativer HPLC gereinigt (Eluent: Methanol/Wasser, Gradient 30:70 → 90:10). Es wurden 60 mg der Titelverbindung erhalten (47% d. Th.).

LC-MS (Methode 2): Rₜ = 1.00 min; MS (EIpos): m/z = 459 [M+H]⁺.

¹H-NMR (400 MHz, DMSO-d₆): δ [ppm] = 4.71 (q, 2H), 5.83 (s, 2H), 6.87 (s br, 2H) 7.08-7.15 (m, 2H), 7.21-7.26 (m, 1H), 7.32-7.42 (m, 2H), 8.62 (dd, 1H), 9.01 (dd, 1H).

### Beispiel 37

### 6-Amino-9-(cyclopropylmethyl)-2-[1-(2-fluorbenzyl)-1H-pyrazolo[3,4-b]pyridin-3-yl]-7,9-dihydro-8H-purin-8-on

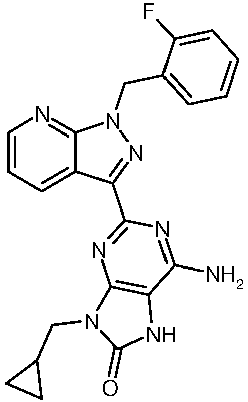

Es wurden 200 mg (0.531 mmol) der Verbindung aus Beispiel 33 sowie 145 mg (0.531 mmol) BEMP in 10 ml Dimethylformamid vorgelegt und bei 0°C innerhalb von 10 min mit einer Lösung aus 71 mg (0.531 mmol) (Brommethyl)cyclopropan in 2 ml Dimethylformamid tropfenweise versetzt. Es wurde 20 min bei 0°C gerührt. Anschließend wurde die Reaktionslösung im Vakuum eingeengt und der Rückstand mittels präparativer HPLC gereinigt (Eluent: Acetonitril/Wasser, Gradient 10:90 → 90:10). Es wurden 27 mg der Titelverbindung erhalten (11% d. Th.).

LC-MS (Methode 3): Rₜ = 1.11 min; MS (EIpos): m/z = 431 [M+H]⁺.

### Beispiel 38

### 6-Amino-2-[1-(2-fluorbenzyl)-1H-pyrazolo[3,4-b]pyridin-3-yl]-9-(2-methylprop-2-en-1-yl)-7,9-dihydro-8H-purin-8-on

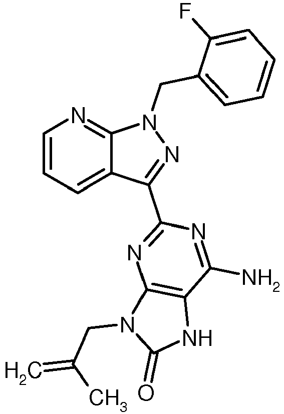

Es wurden 200 mg (0.531 mmol) der Verbindung aus Beispiel 33 sowie 145 mg (0.531 mmol) BEMP in 10 ml Dimethylformamid vorgelegt und bei 0°C innerhalb von 10 min mit einer Lösung aus 72 mg (0.531 mmol) 3-Brom-2-methylprop-1-en 2 ml Dimethylformamid tropfenweise versetzt. Es wurde 1 h bei 0°C gerührt. Anschließend wurde die Reaktionslösung im Vakuum eingeengt und der Rückstand mittels präparativer HPLC gereinigt (Eluent: Acetonitril/Wasser, Gradient 10:90 → 90:10). Es wurden 88 mg der Titelverbindung erhalten (37% d. Th.).

LC-MS (Methode 2): Rₜ = 1.00 min; MS (EIpos): m/z = 431 [M+H]⁺.

¹H-NMR (400 MHz, DMSO-d₆): δ [ppm] = 1.76 (s, 3H), 4.40 (s, 2H), 4.71 (s, 1H, 4.87 (s, 1H), 5.82 (s, 2H), 6.66 (s br, 2H) 7.08-7.14 (m, 2H), 7.20-7.25 (m, 1H), 7.32-7.39 (m, 2H), 8.62 (dd, 1H), 8.96 (dd, 1H), 10.33 (s, 1H).

### Beispiel 39

### 6-Amino-2-[1-(2-fluorbenzyl)-1H-pyrazolo[3,4-b]pyridin-3-yl]-9-isopropyl-7,9-dihydro-8H-purin-8-on

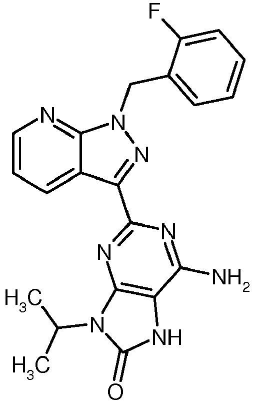

Es wurden 200 mg (0.531 mmol) der Verbindung aus Beispiel 33 sowie 145 mg (0.531 mmol) BEMP in 10 ml Dimethylformamid vorgelegt und bei 0°C innerhalb von 10 min mit einer Lösung aus 90 mg (0.531 mmol) 2-Iodpropan ml Dimethylformamid tropfenweise versetzt. Es wurde 20 min bei 0°C gerührt. Anschließend wurde die Reaktionslösung im Vakuum eingeengt und der Rückstand mittels präparativer HPLC gereinigt (Eluent: Methanol/Wasser, Gradient 30:70 → 90:10). Es wurden 117 mg der Titelverbindung erhalten (50% d. Th.).

LC-MS (Methode 4): Rₜ = 2.02 min; MS (EIpos): m/z = 419 [M+H]⁺.

¹H-NMR (400 MHz, DMSO-d₆): δ [ppm] = 1.56 (d, 6H), 4.67 (hept, 1H), 5.83 (s, 2H), 6.65 (s br, 2H) 7.10-7.15 (m, 2H), 7.21-7.26 (m, 1H), 7.33-7.43 (m, 2H), 8.63 (dd, 1H), 8.96 (dd, 1H), 10.43 (s br, 1H).

### Beispiel 40

### 6-Amino-2-[1-(2-fluorbenzyl)-1H-pyrazolo[3,4-b]pyridin-3-yl]-7-methyl-9-(2,2,2-trifluorethyl)-7,9-dihydro-8H-purin-8-on

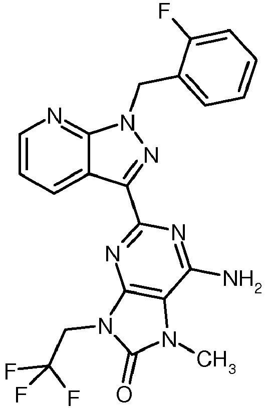

Es wurden 80 mg (0.175 mmol) der Verbindung aus Beispiel 36 sowie 48 mg (0.175 mmol) BEMP in 10 ml Dimethylformamid vorgelegt und bei 0°C innerhalb von 10 min mit einer Lösung aus 25 mg (0.175 mmol) Iodmethan in 2 ml Dimethylformamid tropfenweise versetzt. Es wurde 20 min bei 0°C gerührt. Anschließend wurde die Reaktionslösung im Vakuum eingeengt und der Rückstand mittels präparativer HPLC gereinigt (Eluent: Methanol/Wasser, Gradient 30:70 → 90:10). Es wurden 30 mg der Titelverbindung erhalten (35% d. Th.).

LC-MS (Methode 2): Rₜ = 1.02 min; MS (EIpos): m/z = 473 [M+H]⁺.

¹H-NMR (400 MHz, DMSO-d₆): → [ppm] = 3.56 (s, 3H), 4.74 (q, 2H), 5.84 (s, 2H), 6.98 (s br, 2H) 7.08-7.15 (m, 2H), 7.21-7.26 (m, 1H), 7.32-7.42 (m, 2H), 8.63 (dd, 1H), 9.03 (dd, 1H).

### Beispiel 41

### 6-Amino-2-[1-(2-fluorbenzyl)-1H-pyrazolo[3,4-b]pyridin-3-yl]-7-methyl-7,9-dihydro-8H-purin-8-on

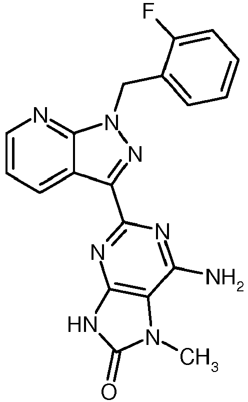

Es wurden 119 mg (0.282 mmol) der Verbindung aus Beispiel 13A in 5 ml Tetrahydrofuran gelöst, auf 0°C gekühlt und mit 310 µl (0.3100 mmol) einer 1N Lösung von Bis-(trimethylsilyl)natriumamid in Tetrahydrofuran tropfenweise versetzt. Es wurde für 3 h bei 0°C und für 16 h bei RT gerührt. Es wurde Wasser addiert, der anfallende Niederschlag abfiltriert, mit Tetrahydrofuran gewaschen und am Hochvakuum getrocknet. Es wurden 44 mg der Titelverbindung erhalten (39% d. Th.).

LC-MS (Methode 2): Rₜ = 0.83 min; MS (EIpos): m/z = 391 [M+H]⁺.

¹H-NMR (400 MHz, DMSO-d₆): δ [ppm] = 3.39 (s, 3H), 5.54 (s br, 2H), 5.77 (s, 2H) 7.09-7.14 (m, 2H), 7.20-7.25 (m, 1H), 7.28-7.37 (m, 2H), 8.55 (dd, 1H), 9.06 (dd, 1H).

### Beispiel 42

### 6-Amino-7-ethyl-2-[1-(2-fluorbenzyl)-1H-pyrazolo[3,4-b]pyridin-3-yl]-7,9-dihydro-8H-purin-8-on

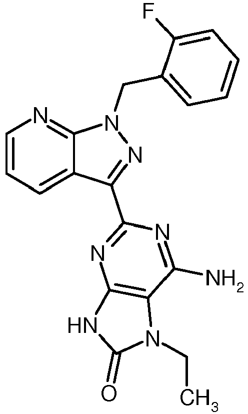

Es wurden 59 mg (0.135 mmol) der Verbindung aus Beispiel 14A in 7.5 ml Tetrahydrofuran gelöst, auf 0°C gekühlt und mit 150 µl (0.150 mmol) einer 1N Lösung von Bis-(trimethylsilyl)natriumamid in Tetrahydrofuran tropfenweise versetzt. Es wurde für 3 h bei 0°C und für 16 h bei RT gerührt. Es wurden erneut 150 µl (0.150 mmol) einer 1N Lösung von Bis-(trimethylsilyl)natriumamid in Tetrahydrofuran tropfenweise zugegeben und 2 Tage bei RT gerührt. Es wurde Wasser addiert und das Reaktionsgemisch im Vakuum eingeengt und der Rückstand mittels präparativer HPLC gereinigt (Eluent: Methanol/Wasser, Gradient 30:70 → 90:10). Es wurden 29 mg der Titelverbindung erhalten (53 % d. Th.).

LC-MS (Methode 2): Rₜ = 0.86 min; MS (EIpos): m/z = 405 [M+H]⁺.

¹H-NMR (400 MHz, DMSO-d₆): δ [ppm] = 1.18 (t, 3H), 3.99 (q, 2H), 5.80 (s, 2H), 6.75 (s br, 2H), 7.12-7.25 (m, 3H), 7.33-7.39 (m, 2H), 8.63 (dd, 1H), 9.05 (dd, 1H), 11.62 (s, 1H).

### Beispiel 43

### 6-Amino-2-[1-(2-fluorbenzyl)-1H-pyrazolo[3,4-b]pyridin-3-yl]-7-isopropyl-7,9-dihydro-8H-purin-8-on

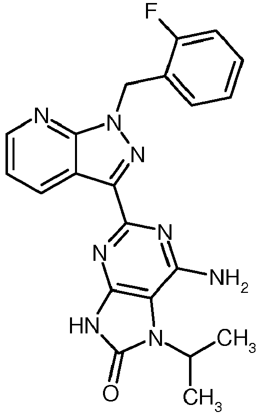

Es wurden 2000 mg (4.897 mmol) der Verbindung aus Beispiel 11A in 20 ml Tetrahydrofuran gelöst, auf 0°C gekühlt und mit 294 mg (5.387 mmol) Natriumhydrid versetzt. Es wurde für 1.5 h bei 0°C gerührt und 916 mg (5.387 mmol) 2-Iodpropan addiert. Es wurde für 48 h bei RT gerührt und anschließend Wasser addiert. Das Reaktionsgemisch wurde im Vakuum eingeengt und der Rückstand mittels präparativer HPLC zwei mal gereinigt (Eluent: Methanol/Wasser, Gradient 30:70 → 90:10). Es wurden 19 mg der Titelverbindung erhalten (1 % d. Th.).

LC-MS (Methode 1): Rₜ = 1.91 min; MS (EIpos): m/z = 419 [M+H]⁺.

¹H-NMR (400 MHz, DMSO-d₆): δ [ppm] = 1.47 (d, 6H), 4.60 (hept, 1H), 5.80 (s, 2H), 6.50 (s br, 2H), 7.12-7.25 (m, 3H), 7.33-7.38 (m, 2H), 8.62 (dd, 1H), 9.07 (dd, 1H).

### Beispiel 44

### 6-Amino-2-[1-(2-fluorbenzyl)-1H-pyrazolo[3,4-b]pyridin-3-yl]-7-(2,2,2-trifluorethyl)-7,9-dihydro-8H-purin-8-on

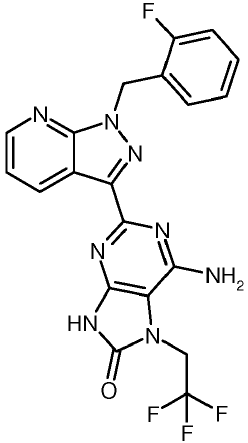

Unter einer Argon-Atmosphäre wurden 2.100 g (4.282 mmol) der Verbindung aus Beispiel 15A in 235 ml Tetrahydrofuran gelöst, auf 0°C gekühlt und mit 10.705 ml (10.705 mmol) einer 1N Lösung von Bis-(trimethylsilyl)natriumamid in Tetrahydrofuran tropfenweise versetzt. Es wurde für 2 h bei 0°C und für 16 h bei RT gerührt. Das Tetrahydrofuran wurde im Argonstrom teilweise eingeengt und der Niederschlag abfiltriert. Es wurden 1.290 g der Titelverbindung erhalten (66 % d. Th.).

LC-MS (Methode 3): Rₜ = 1.03 min; MS (EIpos): m/z = 459 [M+H]⁺.

¹H-NMR (400 MHz, DMSO-d₆): δ [ppm] = 4.91 (q, 2H), 5.81 (s, 2H), 6.95 (s br, 2H), 7.12-7.26 (m, 3H), 7.33-7.40 (m, 2H), 8.64 (dd, 1H), 9.04 (dd, 1H), 11.97 (s, 1H).

### Beispiel 45

### 2-[1-(2-Fluorbenzyl)-1H-pyrazolo[3,4-b]pyridin-3-yl]-7-(2,2,2-trifluorethyl)-7,9-dihydro-8H-purin-8-on

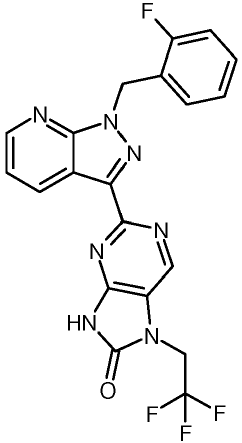

Es wurden 447 mg (4.337 mmol) *tert*.-Butylnitrit in 20 ml trockenem Dimethylformamid gelöst und bei 65°C 994 mg (2.169 mmol) der Verbindung aus Beispiel 44 gelöst in 15 ml Dimethylformamid mittels einer Spritzenpumpe innerhalb 1 h addiert. Nach einer weiteren Stunde Rühren bei 65°C wurden 40 ml Wasser addiert, wobei sich ein Niederschlag bildete. Der Niederschlag wurde abfiltriert und mittels präparativer HPLC gereinigt (Eluent: Wasser/Acetonitril/Wasser mit 1% Trifluoressigsäure, Gradient 68:15:17 → 0:100:0). Es wurden 99 mg der Titelverbindung erhalten (10 % d. Th.).

LC-MS (Methode 2): Rₜ = 1.03 min; MS (EIpos): m/z = 444 [M+H]⁺.

¹H-NMR (400 MHz, DMSO-d₆): δ [ppm] = 4.87 (q, 2H), 5.85 (s, 2H), 7.14-7.17 (m, 1H), 7.21-7.27 (m, 2H), 7.34-7.40 (m, 1H), 7.43 (dd, 1H), 8.64 (s, 1H), 8.67 (dd, 1H), 8.88 (dd, 1H), 12.53 (s, 1H).

### Beispiel 46

### 6-Amino-2-[1-(2-fluorbenzyl)-1H-pyrazolo[3,4-b]pyridin-3-yl]-7,9-dimethyl-7,9-dihydro-8H-purin-8-on

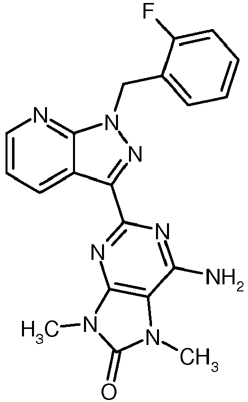

Es wurden 300 mg (0.768 mmol) der Verbindung aus Beispiel 41 sowie 211 mg (0.768 mmol) BEMP in 10 ml Dimethylformamid vorgelegt und bei 0°C innerhalb von 10 min mit einer Lösung aus 109 mg (0.768 mmol) Iodmethan in 2 ml Dimethylformamid tropfenweise versetzt. Es wurde 3 h bei 0°C gerührt. Es wurde Wasser addiert wobei ein Niederschlag anfällt. Der Niederschlag wurde abfiltriert und mittels präparativer HPLC gereinigt (Eluent: Methanol/Wasser, Gradient 30:70 → 90:10). Es wurden 203 mg der Titelverbindung erhalten (65% d. Th.).

LC-MS (Methode 4): Rₜ = 1.83 min; MS (EIpos): m/z = 405 [M+H]⁺.

¹H-NMR (400 MHz, DMSO-d₆): δ [ppm] = 3.37 (s, 3H), 3.53 (s, 3H), 5.84 (s, 2H), 6.80 (s br, 2H) 7.09-7.15 (m, 2H), 7.21-7.26 (m, 1H), 7.32-7.40 (m, 2H), 8.63 (d, 1H), 9.09 (d, 1H).

### Beispiel 47

### 6-Amino-2-[1-(2-fluorbenzyl)-1H-pyrazolo[3,4-b]pyridin-3-yl]-7-(²H₃)methyl-7,9-dihydro-8H-purin-8-on

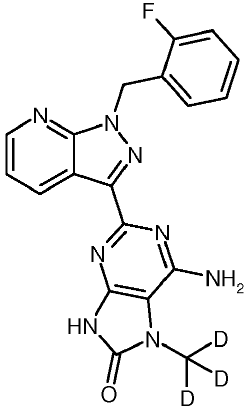

Es wurden 47 mg (0.110 mmol) der Verbindung aus Beispiel 16A in 6 ml Tetrahydrofuran gelöst, auf 0°C gekühlt und mit 122 µl (0.112 mmol) einer 1N Lösung von Bis-(trimethylsilyl)natriumamid in Tetrahydrofuran tropfenweise versetzt. Es wurde für 3 h bei 0°C und für 16 h bei RT gerührt. Es wurden erneut 122 µl (0.112 mmol) einer 1N Lösung von Bis-(trimethylsilyl)natriumamid in Tetrahydrofuran tropfenweise zugegeben und 2 Tage bei RT gerührt. Es wurde Wasser addiert und das Reaktionsgemisch im Vakuum eingeengt und der Rückstand mittels präparativer HPLC zwei mal gereinigt (Eluent: Methanol/Wasser, Gradient 30:70 → 90:10 und Acetonitril/Wasser mit 0.1% Trifluoressigsäure = 45:55). Es wurden 3 mg der Titelverbindung erhalten (7 % d. Th.).

LC-MS (Methode 2): Rₜ = 0.82 min; MS (EIpos): m/z = 394 [M+H]⁺.

**Nicht erfindungsgemäßes Beispiel 48**

### 4-Amino-2-[1-(2-fluorbenzyl)-1H-pyrazolo[3,4-b]pyridin-3-yl]pteridin-7(8H)-on

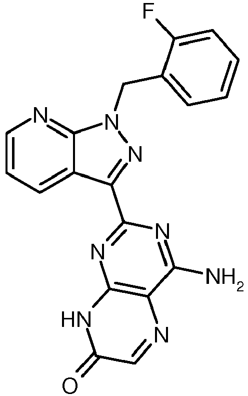

Es wurden 300 mg (0.856 mmol) der Verbindung aus Beispiel 1A in 20 ml Ethanol gelöst und mit 187 µl (0.942 mmol) einer 50%igen Lösung von Etylglyoxalat in Toluol versetzt. Es wurde für 1 h zum Rückfluss erhitzt, mit 2 Tropfen konzentrierter Schwefelsäure versetzt und weitere 16 h zum Rückfluss erhitzt. Der Niederschlag wurde abfiltriert und am Hochvakuum getrocknet. Es wurden 83 mg der Titelverbindung erhalten (23 % d. Th.).

LC-MS (Methode 3): Rₜ = 0.93 min; MS (EIpos): m/z = 389 [M+H]⁺.

¹H-NMR (400 MHz, DMSO-d₆): δ [ppm] = 5.85 (s, 2H), 7.13-7.26 (m, 3H), 7.34-7.39 (m, 1H), 7.43 (dd, 1H), 7.74-8.01 (m, 3H), 8.66 (dd, 1H), 9.17 (dd, 1H), 12.86 (s, 1H).

### Nicht erfindungsgemäßes Beispiel 49

### 4-Amino-2-[1-(2-fluorbenzyl)-1H-pyrazolo[3,4-b]pyridin-3-yl]-6-methylpteridin-7(8H)-on

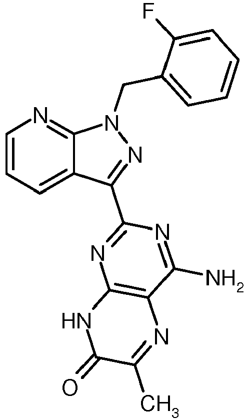

Es wurden 300 mg (0.856 mmol) der Verbindung aus Beispiel 1A in 20 ml Ethanol gelöst und mit 105 µl (0.942 mmol) Ethyl-2-oxopropanoat versetzt. Es wurde für 16 h zum Rückfluss erhitzt. Der Niederschlag wurde heiß abfiltriert, in Trifluoressigsäure aufgenommen und mit Wasser gefällt. Der Niederschlag wurde abfiltriert und am Hochvakuum getrocknet. Es wurden 74 mg der Titelverbindung erhalten (19 % d. Th.).

LC-MS (Methode 3): Rₜ = 0.98 min; MS (EIpos): m/z = 403 [M+H]⁺.

¹H-NMR (400 MHz, Trifluoressigsäure-d₁): δ [ppm] = 2.88 (s, 3H), 6.24 (s, 2H), 7.25 (t, 1H), 7.44 (t, 1H), 7.59-7.69 (m, 2H), 8.24 (dd, 1H), 9.25 (d, 1H), 9.29 (d, 1H).

### Nicht ßerfindungsgemäßes Beispiel 50

### 4-Amino-2-[1-(2-fluorbenzyl)-1H-pyrazolo[3,4-b]pyridin-3-yl]-6-(trifluormethyl)pteridin-7(8H)-on

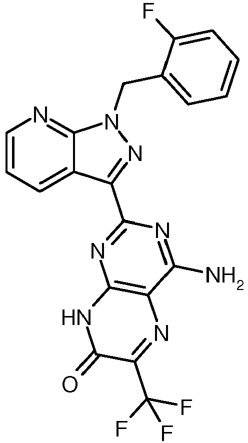

Es wurden 300 mg (0.856 mmol) der Verbindung aus Beispiel 1A in 25 ml Ethanol gelöst und mit 118 µl (0.942 mmol) Ethyl-3,3,3-trifluor-2-oxopropanoat versetzt. Es wurde für 16 h zum Rückfluss erhitzt. Der Niederschlag wurde abfiltriert und mit Acetonitril verrührt. Der Niederschlag wurde abfiltriert und am Hochvakuum getrocknet. Es wurden 99 mg der Titelverbindung erhalten (24 % d. Th.).

LC-MS (Methode 2): Rₜ = 1.01 min; MS (EIpos): m/z = 457 [M+H]⁺.

¹H-NMR (400 MHz, DMSO-d₆): δ [ppm] = 5.87 (s, 2H), 7.12-7.26 (m, 3H), 7.35-7.39 (m, 1H), 7.45 (dd, 1H), 7.90 (s br, 1H), 8.36 (s br, 1H), 8.68 (dd, 1H), 9.18 (dd, 1H), 13.41 (s br, 1H).

### Nicht erfindungsgemäßes Beispiel 51

### 4-Amino-2-[1-(2-fluorbenzyl)-1H-pyrazolo[3,4-b]pyridin-3-yl]-5,8-dihydropteridin-6,7-dion

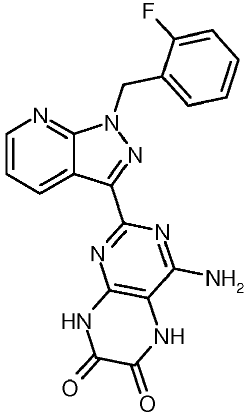

Es wurden 300 mg (0.856 mmol) der Verbindung aus Beispiel 1A in 3 ml Ethanol gelöst und mit 116 µl (0.856 mmol) Oxalsäurediethylester sowie 79 mg (1.156 mmol) Natriummethylat versetzt. Es wurde für 10 h zum Rückfluss erhitzt. Die Reaktionsmischung wurde mit 10 ml Ethanol verrührt. Der Niederschlag wurde abfiltriert, mit 10 ml Wasser und anschließend mit 10 ml Acetonitril verrührt. Der Niederschlag wurde abfiltriert und am Hochvakuum getrocknet. Es wurden 112 mg der Titelverbindung erhalten (30 % d. Th.).

LC-MS (Methode 2): Rₜ = 0.80 min; MS (EIpos): m/z = 405 [M+H]⁺.

¹H-NMR (400 MHz, DMSO-d₆): δ [ppm] = 5.82 (s, 2H), 7.12-7.25 (m, 4H), 7.33-7.40 (m, 2H), 8.64 (dd, 1H), 9.14 (dd, 1H), 12.44 (s, 1H).

### Nicht erfindungsgemäßes Beispiel 52

### 2-[1-(2-Fluorbenzyl)-1H-pyrazolo[3,4-b]pyridin-3-yl]pteridin-4-amin

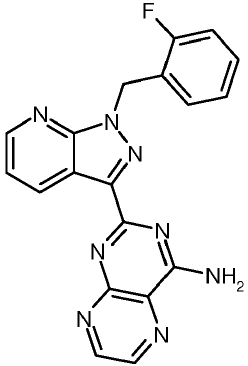

Es wurden 400 mg (1.142 mmol) der Verbindung aus Beispiel 1A in 20 ml Ethanol gelöst und mit 274 mg (2.283 mmol) 2,3-Dihydroxy-1,4-dioxan versetzt. Es wurde für 16 h bei RT gerührt. Der Niederschlag wurde abfiltriert und mit wenig Ethanol gewaschen. Der Rückstand wurde mittels präparativer HPLC gereinigt (Eluent: Acetonitril/Wasser mit 0.05% Trifluoressigsäure, Gradient 20:80 → 50:50). Es wurden 96 mg der Titelverbindung erhalten (23 % d. Th.).

LC-MS (Methode 2): Rₜ = 0.87 min; MS (EIpos): m/z = 373 [M+H]⁺.

¹H-NMR (400 MHz, DMSO-d₆): δ [ppm] = 5.89 (s, 2H), 7.16 (t, 1H), 7.21-7.27 (m, 2H), 7.35-7.40 (m, 1H), 7.47 (dd, 1H), 8.50 (s br, 1H), 8.64 (s br, 1H), 8.69 (dd, 1H), 8.82 (d, 1H), 9.11 (d, 1H), 9.14 (dd, 1H).

### Beispiel 53

### 5-[1-(2-Fluorbenzyl)-1H-pyrazolo[3,4-b]pyridin-3-yl]-3H-[1,2,3]triazolo[4,5-d]pyrimidin-7-amin

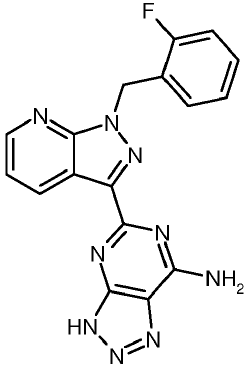

Es wurden 1500 mg (4.281 mmol) der Verbindung aus Beispiel 1A in 7.3 ml Eisessig gelöst und mit 54 ml Wasser versetzt wobei sich eine Suspension bildet. Unter Rühren wurden 295 mg (4.281 mmol) Natriumnitrit gelöst in 4 ml Wasser zugetropft. Es wurde für 20 min zum Rückfluss erhitzt. Der Niederschlag wurde abfiltriert, mit Wasser gewaschen und am Hochvakuum getrocknet. Es wurden 1345 mg der Titelverbindung erhalten (87 % d. Th.).

LC-MS (Methode 4): Rₜ = 1.75 min; MS (EIpos): m/z = 362 [M+H]⁺.

¹H-NMR (400 MHz, DMSO-d₆): δ [ppm] = 5.84 (s, 2H), 7.13-7.26 (m, 3H), 7.33-7.39 (m, 1H), 7.42 (dd, 1H), 8.20 (s br, 2H), 8.66 (dd, 1H), 9.07 (dd, 1H), 16.00 (s br, 1H).

### Beispiel 54

### 5-[1-(2-Fluorbenzyl)-1H-pyrazolo[3,4-b]pyridin-3-yl]-3-methyl-3H-[1,2,3]triazolo[4,5-d]pyrimidin-7-amin

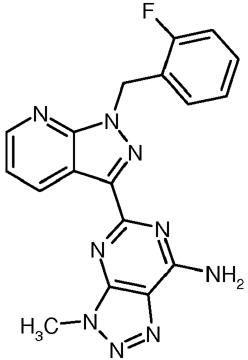

Es wurden 250 mg (0.692 mmol) der Verbindung aus Beispiel 53 sowie 190 mg (0.692 mmol) BEMP in 12 ml Dimethylformamid vorgelegt und bei 0°C innerhalb von 10 min mit einer Lösung aus 98 mg (0.692 mmol) Iodmethan in 3 ml Dimethylformamid tropfenweise versetzt. Es wurde 20 min bei 0°C gerührt. Anschließend wurde die Reaktionslösung im Vakuum eingeengt und der Rückstand mittels präparativer HPLC gereinigt (Eluent: Methanol/wässrige 0.1%-ige AmmoniakLösung = 65:35). Der Rückstand wurde mit Acetonitril verrührt, abgesaugt und am Hochvakuum getrocknet. Es wurden 78 mg der Titelverbindung (30% d. Th.) sowie 50 mg von Beispiel 55 erhalten (19% d. Th.).

LC-MS (Methode 2): Rₜ = 0.93 min; MS (EIpos): m/z = 376 [M+H]⁺.

¹H-NMR (400 MHz, DMSO-d₆): δ [ppm] = 4.23 (s, 3H) 5.88 (s, 2H), 7.12-7.17 (m, 2H), 7.22-7.27 (m, 1H), 7.33-7.39 (m, 1H), 7.44 (dd, 1H), 8.36 (s br, 1H), 8.48 (s br, 1H), 8.67 (dd, 1H), 9.16 (dd, 1H).

### Beispiel 55

### 5-[1-(2-Fluorbenzyl)-1H-pyrazolo[3,4-b]pyridin-3-yl]-2-methyl-2H-[1,2,3]triazolo[4,5-d]pyrimidin-7-amin

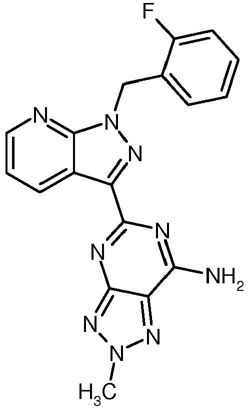

Es wurden 250 mg (0.692 mmol) der Verbindung aus Beispiel 53 sowie 190 mg (0.692 mmol) BEMP in 12 ml Dimethylformamid vorgelegt und bei 0°C innerhalb von 10 min mit einer Lösung aus 98 mg (0.692 mmol) Iodmethan in 3 ml Dimethylformamid tropfenweise versetzt. Es wurde 20 min bei 0°C gerührt. Anschließend wurde die Reaktionslösung im Vakuum eingeengt und der Rückstand mittels präparativer HPLC gereinigt (Eluent: Methanol/wässrige 0.1%-ige AmmoniakLösung = 65:35). Der Rückstand wurde mit Acetonitril verrührt, abgesaugt und am Hochvakuum getrocknet. Es wurden 50 mg (19% d. Th.) der Titelverbindung sowie 78 mg (30% d. Th.) von Beispiel 54 erhalten.

LC-MS (Methode 2): Rₜ = 0.89 min; MS (EIpos): m/z = 376 [M+H]⁺.

¹H-NMR (400 MHz, DMSO-d₆): δ [ppm] = 4.48 (s, 3H) 5.88 (s, 2H), 7.13-7.28 (m, 3H), 7.34-7.40 (m, 1H), 7.45 (dd, 1H), 8.60 (s br, 1H), 8.68 (dd, 1H), 8.80 (s br, 1H), 9.04 (dd, 1H).

### Beispiel 56

### 5-[1-(2-Fluorbenzyl)-1H-pyrazolo[3,4-b]pyridin-3-yl]-3-(2,2,2-trifluorethyl)-3H-[1,2,3]triazolo[4,5-d]pyrimidin-7-amin

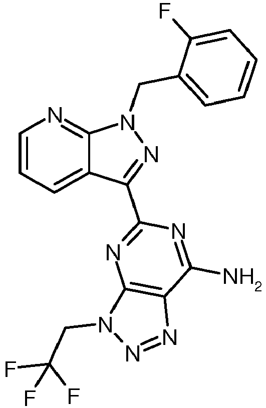

Es wurden 150 mg (0.415 mmol) der Verbindung aus Beispiel 53 sowie 113 mg (0.0415 mmol) BEMP in 7 ml Dimethylformamid vorgelegt und bei 0°C innerhalb von 10 min mit einer Lösung aus 117 mg (0.415 mmol) 2,2,2-Trifluorethyltrichlormethansulfonat in 2.5 ml Dimethylformamid tropfenweise versetzt. Es wurde für 16 h bei RT gerührt. Anschließend wurde die Reaktionslösung im Vakuum eingeengt und der Rückstand mittels präparativer HPLC gereinigt (Eluent: Acetonitril/Wasser mit 0.05% Trifluoressigsäure, Gradient 10:90 →90:10). Es wurden 22 mg der Titelverbindung erhalten (11% d. Th.).

LC-MS (Methode 2): Rₜ = 1.04 min; MS (EIpos): m/z = 444 [M+H]⁺.

¹H-NMR (400 MHz, DMSO-d₆): δ [ppm] = 5.73 (q, 2H), 5.88 (s, 2H), 7.11-7.16 (m, 2H), 7.22-7.26 (m, 1H), 7.33-7.39 (m, 1H), 7.45 (dd, 1H), 8.54 (s br, 1H), 8.67 (dd, 1H), 9.17 (dd, 1H).

### Nicht erfindungsgemäßes Beispiel 57

### 5-[1-(2-Fluorbenzyl)-1H-pyrazolo[3,4-b]pyridin-3-yl][1,2,5]thiadiazolo[3,4-d]pyrimidin-7-amin

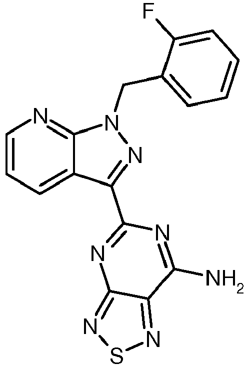

Es wurden 250 mg (0.714 mmol) der Verbindung aus Beispiel 1A in 4 ml Thionylchlorid gelöst. Es wurde für 5 h zum Rückfluss erhitzt. Das Reaktionsgemisch wurde am Rotationsverdampfer eingeengt. Dreimal mit Dichlormethan versetzt und eingeengt. Der Rückstand wurde in Methanol gelöst, mit Wasser gefällt, abgesaugt und am Hochvakuum getrocknet. Es wurden 192 mg der Titelverbindung erhalten (71% d. Th.).

LC-MS (Methode 4): Rₜ = 1.90 min; MS (EIpos): m/z = 379 [M+H]⁺.

¹H-NMR (400 MHz, DMSO-d₆): δ [ppm] = 5.80 (s, 2H), 7.13-7.27 (m, 3H), 7.34-7.40 (m, 1H), 7.46 (dd, 1H), 8.68 (s br, 1H), 8.78 (s br, 1H), 9.10 (d, 1H).

### Nicht erfindungsgemäßes Beispiel 58

### 2-[1-(2-Fluorbenzyl)-1H-pyrazolo[3,4-b]pyridin-3-yl]-9H-purin-6-amin

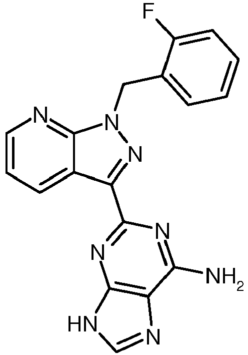

Es wurden 300 mg (0.856 mmol) der Verbindung aus Beispiel 1A mit 6 ml Ameisensäure und 600 mg (7.314 mmol) wasserfreiem Natriumacetat versetzt. Es wurde für 16 h zum Rückfluss erhitzt. Das Reaktionsgemisch wurde am Rotationsverdampfer eingeengt. Der Rückstand wurde in 10 ml Acetonitril/Wasser aufgenommen, mit 1 ml Trifluoressigsäure versetzt und erwärmt. Es bildete sich ein Niederschlag. Nach dem Abkühlen wurde der Niederschlag abgesaugt, mit 8 ml Acetonitril gewaschen und am Hochvakuum getrocknet. Es wurden 244 mg der Titelverbindung erhalten (76% d. Th.).

LC-MS (Methode 4): Rₜ = 1.60 min; MS (EIpos): m/z = 361 [M+H]⁺.

¹H-NMR (400 MHz, DMSO-d₆): δ [ppm] = 5.87 (s, 2H), 7.15 (t, 1H), 7.19-7.26 (m, 2H), 7.34-7.39 (m, 1H), 7.46 (dd, 1H), 7.98 (s br, 2H), 8.37 (s, 1H), 8.69 (dd, 1H), 9.00 (d, 1H).

### Nicht erfindungsgemäßes Beispiel 59

### 2-[1-(2-Fluorbenzyl)-1H-pyrazolo[3,4-b]pyridin-3-yl]-9-(2,2,2-trifluorethyl)-9H-purin-6-amin

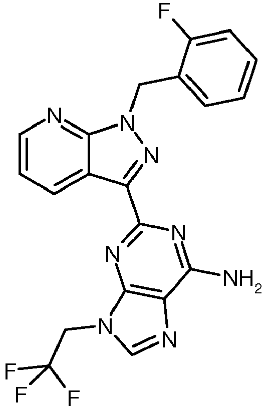

Es wurden 100 mg (0.278 mmol) der Verbindung aus Beispiel 58 sowie 76 mg (0.278 mmol) BEMP in 3 ml Dimethylformamid vorgelegt, bei 0°C innerhalb von 10 min mit einer Lösung aus 78 mg (0.266 mmol) 2,2,2-Trifluorethyltrichlormethansulfonat in 2 ml Dimethylformamid tropfenweise versetzt und 20 min bei RT gerührt. Das Produkt wurde durch Zugabe von Wasser gefällt, abgesaugt und mittels präparativer HPLC gereinigt (Eluent: Acetonitril/Wasser mit 0.05% Trifluoressigsäure, Gradient 20:80 → 50:50). Es wurden 53 mg der Titelverbindung erhalten (43% d. Th.).

LC-MS (Methode 3): Rₜ = 1.11 min; MS (EIpos): m/z = 443 [M+H]⁺.

¹H-NMR (400 MHz, DMSO-d₆): δ [ppm] = 5.31 (q, 2H), 5.87 (s, 2H), 7.11-7.15 (m, 2H), 7.22-7.26 (m, 1H), 7.33-7.39 (m, 1H), 7.43 (dd, 1H), 7.80 (s br, 2H), 8.31 (s, 1H), 8.66 (dd, 1H), 9.12 (dd, 1H).

### Nicht erfindungsgemäßes Beispiel 60

### 2-[1-(2-Fluorbenzyl)-1H-pyrazolo[3,4-b]pyridin-3-yl]-7-(2,2,2-trifluorethyl)-7H-purin-6-amin

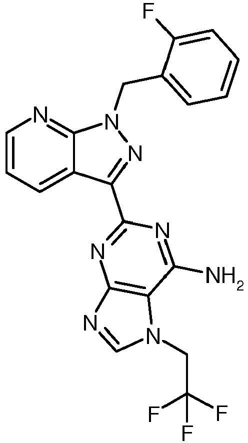

Es wurden 68 mg (0.157 mmol) der Verbindung aus Beispiel 17A mit 1.1 ml Ameisensäure und 110 mg (1.343 mmol) wasserfreiem Natriumacetat versetzt. Es wurde für 3 Tage zum Rückfluss erhitzt. Das Reaktionsgemisch wurde am Rotationsverdampfer eingeengt. Der Rückstand wurde mit 4 ml Acetonitril/Wasser (v/v = 1:1) verrührt, abgesaugt und am Hochvakuum getrocknet. Es wurden 30 mg der Titelverbindung erhalten (41% d. Th.).

LC-MS (Methode 2): Rₜ = 1.60 min; MS (EIpos): m/z = 361 [M+H]⁺.

¹H-NMR (400 MHz, DMSO-d₆): δ [ppm] = 5.50 (q, 2H), 5.83 (s, 2H), 7.11-7.26 (m, 3H), 7.33-7.41 (m, 4H), 8.43 (s, 1H), 8.64 (dd, 1H), 9.09 (dd, 1H).

### Nicht erfindungsgemäßes Beispiel 61

### 2-[1-(2-Fluorbenzyl)-1H-pyrazolo[3,4-b]pyridin-3-yl]-7-(2,2,2-trifluorethyl)-8-(trifluormethyl)-7H-purin-6-amin

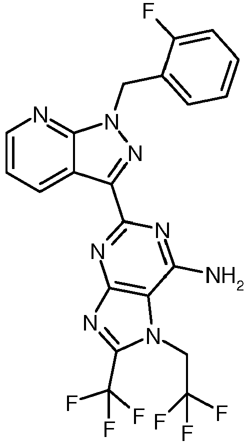

Es wurden 194 mg (0.449 mmol) der Verbindung aus Beispiel 17A in 5 ml Tetrahydrofuran gelöst und mit 141 mg (0.673 mmol) Trifluoressigsäureanhydrid versetzt. Es wurde für 16 h bei 40°C gerührt. Anschließend wurden das Reaktionsgemisch am Rotationsverdampfer eingeengt und mit 20 ml Pyridin und 521 mg (3.773 mmol) Kaliumcarbonat versetzt. Es wurde für 16 h bei RT gerührt und das Reaktionsgemisch im Vakuum eingeengt. Der Rückstand wurde mittels präparativer HPLC gereinigt (Eluent: Methanol/Wasser, Gradient 30:70 → 90:10). Es wurden 45 mg der Titelverbindung erhalten (20% d. Th.).

LC-MS (Methode 2): Rₜ = 1.09 min; MS (EIpos): m/z = 511 [M+H]⁺.

¹H-NMR (400 MHz, DMSO-d₆): δ [ppm] = 5.62 (q, 2H), 5.85 (s, 2H), 7.12-7.26 (m, 3H), 7.34-7.39 (m, 1H), 7.42 (dd, 1H), 7.84 (s br, 2H), 8.66 (dd, 1H), 9.08 (dd, 1H).

### Beispiel 62

### 6-Amino-2-[5-fluor-1-(2-fluorbenzyl)-1H-pyrazolo[3,4-b]pyridin-3-yl]-7-(2,2,2-trifluorethyl)-7,9-dihydro-8H-purin-8-on

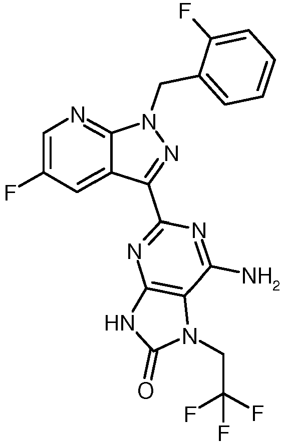

Unter einer Argon-Atmosphäre wurden 5.005 g (6.458 mmol) der Verbindung aus Beispiel 32A in 355 ml Tetrahydrofuran gelöst, auf 0°C gekühlt und mit 16.145 ml (16.145 mmol) einer 1N Lösung von Bis-(trimethylsilyl)natriumamid in Tetrahydrofuran tropfenweise versetzt. Es wurde für 2 h bei 0°C und für 16 h bei RT gerührt. Es wurde 16.145 ml (16.145 mmol) 1N Salzsäure addiert und das Gemisch am Rotationsverdampfer eingeengt. Der Rückstand wurde in Essigsäureethylester aufgenommen und die organische Phase zwei mal mit Wasser gewaschen, über Natriumsulfat getrocknet und am Rotationsverdampfer eingeengt. Es wurden 6.13 g der Titelverbindung erhalten (Reinheit nach HPLC 61%). 500 mg des Rückstands wurden mittels präparativer HPLC gereinigt (Eluent: Methanol/Wasser, Gradient 30:70 → 90:10). Es wurden 93 mg der Titelverbindung erhalten (36% d. Th.).

LC-MS (Methode 2): Rₜ = 1.01 min; MS (EIpos): m/z = 477 [M+H]⁺.

¹H-NMR (400 MHz, DMSO-d₆): δ [ppm] = 4.91 (q, 2H), 5.80 (s, 2H), 7.01 (s br, 2H), 7.13-7.18 (m, 1H), 7.21-7.26 (m, 2H), 7.34-7.40 (m, 1H), 8.70 (dd, 1H), 8.87 (dd, 1H), 11.96 (s, 1H).

### Beispiel 63

### Methyl-6-[1-(2-fluorbenzyl)-1H-pyrazolo[3,4-b]pyridin-3-yl]-3,4-dihydropyrido[2,3-b]pyrazin-1(2H)-carboxylat

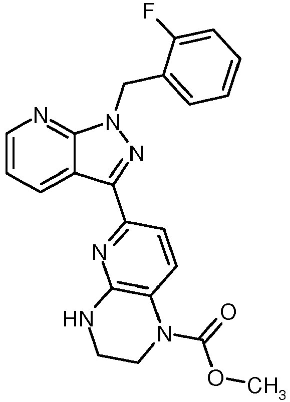

In Tetrahydrofuran (3 mL) wurden unter Argon 78 mg (0.22 mmol) der Verbindung aus Beispiel 142 vorgelegt und auf 0°C abgekühlt. Anschließend wurden 17 mg (0.43 mmol) Natriumhydrid (60%ig in Mineralöl) zugefügt und die Mischung weitere 30 min bei 0°C gerührt. Danach wurde eine Lösung von 20 µL (0.24 mmol) Chlorameisensäuremethylester in Dichlormethan (1 mL) zugetropft und die Reaktionsmischung über Nacht bei RT gerührt. Die Reaktionsmischung wurde mit gesättigter wässriger Natriumhydrogencarbonat-Lösung versetzt und zweimal mit Essigsäureethylester extrahiert. Die gesammelten organischen Phasen wurden über Natriumsulfat getrocknet, filtriert und eingeengt. Der Rückstand wurde mittels präparativer HPLC (Laufmittel: Acetonitril-Wasser mit 0.1% Ameisensäure Gradient) getrennt und die Produktfraktionen eingeengt. Man erhielt 30 mg (32 % d. Th.) der Titelverbindung als Feststoff.

LC-MS (Methode 2): Rₜ = 1.07 min

MS (ESIpos): m/z = 419 (M+H)⁺

¹H-NMR (400 MHz, DMSO-d₆): δ = 3.41 - 3.46 (m, 2H), 3.74 - 3.75 (m, 5H), 5.79 (s, 2H), 7.11 - 7.19 (m, 2H), 7.20 - 7.25 (m, 2H), 7.28 (d, 1H), 7.31 - 7.36 (m, 2H), 7.81 - 7.86 (m, 1H), 8.61 (dd, 1H), 9.04 (dd, 1H).

### Nicht erfindungsgemäßes Beispiel 64

### 5-[6-Fluor-3-(2-fluorbenzyl)-1H-pyrazolo[4,3-b]pyridin-1-yl]-1,3-dihydro-2H-imidazo[4,5-b]pyridin-2-on

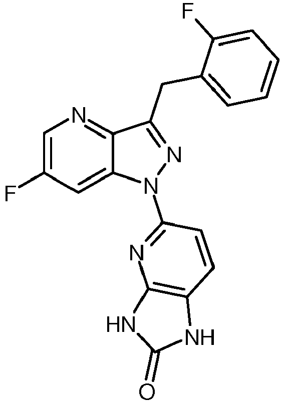

In Dimethylformamid (5 mL) wurden 70 mg (0.20 mmol) der Verbindung aus Beispiel 40A vorgelegt, anschließend 161 mg (0.993 mmol) N,N-Carbonyldiimidazol sowie 0.33 mL (2.38 mmol) Triethylamin zugefügt und die Mischung über Nacht bei 100°C gerührt. Die Reaktionsmischung wurde anschließend mit Wasser sowie gesättigter wässriger Natriumhydrogencarbonat-Lösung verdünnt und zweimal mit Essigsäureethylester extrahiert. Die gesammelten organischen Phasen wurden über Natriumsulfat getrocknet, filtriert und einrotiert. Präparative HPLC (Laufmittel: Acetonitril-Wasser mit 0.1% Ameisensäure Gradient) des Rückstands lieferte 38 mg (48% d. Th.) der gewünschten Titelverbindung als Feststoff.

LC-MS (Methode 2): Rₜ = 1.00 min

MS (ESIpos): m/z = 379 (M+H)⁺

¹H-NMR (400 MHz, DMSO-d₆): δ = 4.45 (s, 2H), 7.11 - 7.16 (m, 1H), 7.16 - 7.23 (m, 1H), 7.26 - 7.33 (m, 1H), 7.39 - 7.50 (m, 3H), 8.67 - 8.71 (m, 2H), 10.98 (s, 1H), 11.51 (s, 1H).

### Nicht erfindungsgemäßes Beispiel 65

### 4-Amino-2-[1-(2-fluorbenzyl)-1H-pyrazolo[3,4-b]pyridin-3-yl]-7,8-dihydropteridin-5(6H)-sulfonamid

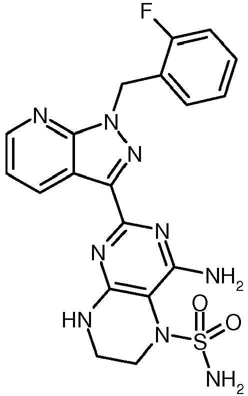

In Dichlormethan (5 mL) wurden 96 mg (0.17 mmol) der Verbindung aus Beispiel 41A vorgelegt, anschließend 0.13 mL (1.73 mmol) Trifluoressigsäure zugefügt und die Reaktionsmischung über Nacht bei RT gerührt. Die Mischung wurde eingeengt, der Rückstand mittels präparativer HPLC (Laufmittel: Acetonitril-Wasser mit 0.1% Ameisensäure Gradient) aufgetrennt und die Produktfraktionen eingeengt. Man erhielt 52 mg (66 % d. Th.) der Titelverbindung als Feststoff.

LC-MS (Methode 2): Rₜ = 0.75 min

MS (ESIpos): m/z = 456 (M+H)⁺

¹H-NMR (400 MHz, DMSO-d₆): δ = 3.35 - 3.49 (m, 2H), 3.54 - 3.63 (m, 2H), 5.81 (s, 2H), 6.34 - 6.50 (m, 2H), 7.09 - 7.16 (m, 2H), 7.19 - 7.26 (m, 1H), 7.30 - 7.40 (m, 4H), 7.51 - 7.64 (m, 1H), 8.60 - 8.64 (m, 1H), 8.99 - 9.06 (m, 1H).

### Nicht erfindungsgemäßes Beispiel 66

### 6-[1-(2-Fluorbenzyl)-1H-pyrazolo[3,4-b]pyridin-3-yl]-2-methylpyrido[2,3-b]pyrazin-3(4H)-on

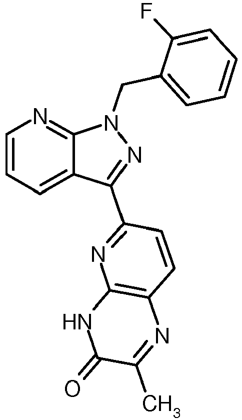

In Ethanol (8 mL) wurden 146 mg (0.435 mmol) der Verbindung aus Beispiel 43A vorgelegt, anschließend 56 mg (0.48 mmol) Ethyl-2-oxopropanoat zugefügt und die Reaktionsmischung über Nacht zum Rückfluss erhitzt. Danach wurde eine katalytische Menge konz. Schwefelsäure addiert und die Mischung erneut über Nacht zum Rückfluss erhitzt. Anschließend wurde auf RT abgekühlt, der entstandene Feststoff abfiltriert, mit Ethanol gewaschen und am Hochvakuum getrocknet. Das Rohprodukt wurde in DMSO gelöst, mittels präparativer HPLC (Laufmittel: Acetonitril-Wasser mit 0.1% Ameisensäure Gradient) getrennt und die Produktfraktionen eingeengt. Man erhielt 30 mg (18 % d. Th.) der Titelverbindung als Feststoff.

LC-MS (Methode 2): Rₜ = 1.07 min

MS (ESIpos): m/z = 387 (M+H)⁺

¹H-NMR (400 MHz, DMSO-d₆): δ = 2.45 (s, 3H), 5.86 (s, 2H), 7.11 - 7.18 (m, 1H), 7.20 - 7.29 (m, 2H), 7.33 - 7.41 (m, 1H), 7.42 - 7.48 (m, 1H), 7.98 - 8.04 (m, 1H), 8.14 - 8.21 (m, 1H), 8.65 - 8.71 (m, 1H), 9.24 - 9.31 (m, 1H), 12.86 - 13.04 (m, 1H).

### Nicht erfindungsgemäßes Beispiel 67

### 6-[1-(2-Fluorbenzyl)-1H-pyrazolo[3,4-b]pyridin-3-yl]-2-isopropylpyrido[2,3-b]pyrazin-3(4H)-on

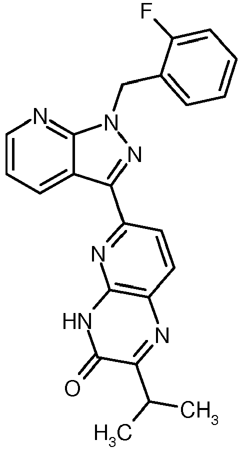

In Ethanol (8 mL) wurden 146 mg (0.435 mmol) der Verbindung aus Beispiel 43A vorgelegt, anschließend 69 mg (0.48 mmol) Ethyl-3-methyl-2-oxobutanoat zugefügt und die Reaktionsmischung über Nacht zum Rückfluss erhitzt. Danach wurde eine katalytische Menge konz. Schwefelsäure addiert und erneut über Nacht zum Rückfluss erhitzt. Anschließend wurde auf RT abgekühlt, der entstandene Feststoff abfiltriert, mit Methanol gewaschen und am Hochvakuum getrocknet. Man erhielt 26 mg (14 % d. Th.) der Titelverbindung als Feststoff.

LC-MS (Methode 2): Rₜ = 1.28 min

MS (ESIpos): m/z = 415 (M+H)⁺

¹H-NMR (400 MHz, DMSO-d₆): δ = 1.25 (d, 6H), 3.46 - 3.54 (m, 1H), 5.86 (s, 2H), 7.12 - 7.19 (m, 1H), 7.21 - 7.30 (m, 2H), 7.34 - 7.41 (m, 1H), 7.42 - 7.48 (m, 1H), 8.02 (d, 1H), 8.22 (d, 1H), 8.67 - 8.71 (m, 1H), 9.25 - 9.30 (m, 1H), 12.96 (s, 1H).

### Nicht erfindungsgemäßes Beispiel 68

### 6-[1-(2-Fluorbenzyl)-1H-pyrazolo[3,4-b]pyridin-3-yl]pyrido[2,3-b]pyrazin

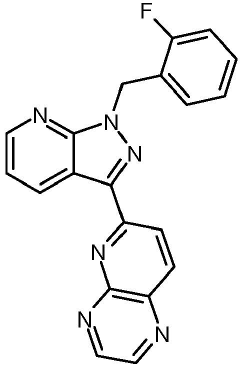

In Ethanol (10 mL) wurden 200 mg (0.598 mmol) der Verbindung aus Beispiel 43A vorgelegt, anschließend 144 mg (1.20 mmol) 2,3-Dihydroxy-1,4-dioxan zugefügt und die Reaktionsmischung 5 h bei RT gerührt. Der entstandene Feststoff wurde abfiltriert, mit Ethanol gewaschen und am Hochvakuum getrocknet. Man erhielt 90 mg (41 % d. Th.) der Titelverbindung als Feststoff.

LC-MS (Methode 2): Rₜ = 1.09 min

MS (ESIpos): m/z = 357 (M+H)⁺

1H-NMR (400 MHz, DMSO-d6): δ = 5.92 (s, 2H), 7.15 - 7.20 (m, 1H), 7.23 - 7.34 (m, 2H), 7.35 - 7.42 (m, 1H), 7.52 - 7.56 (m, 1H), 8.59 - 8.66 (m, 2H), 8.73 - 8.76 (m, 1H), 9.05 - 9.08 (m, 1H), 9.14 - 9.20 (m, 2H).

### Beispiel 69

### Cyclopentyl-4-amino-2-[1-(2-fluorbenzyl)-1H-pyrazolo[3,4-b]pyridin-3-yl]-7,8-dihydropteridin-5(6H)-carboxylat

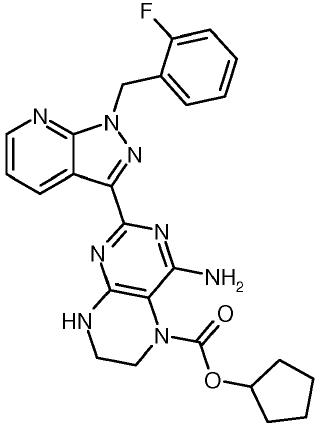

In Tetrahydrofuran (6 mL) wurden 200 mg (0.484 mmol) der Verbindung aus Beispiel Beispiel 81 vorgelegt und auf 0°C abgekühlt. Anschließend wurden 38.8 mg (0.484 mmol) Natriumhydrid (60%ige Suspension in Mineralöl) zugefügt und die Mischung weitere 30 min bei 0°C gerührt. Danach wurde eine Lösung von 72.0 mg (0.484 mmol) Chlorameisensäurecyclopentylester in Dichlormethan (1 mL) zugetropft und die Reaktionsmischung weitere 3 h bei RT gerührt. Anschließend wurden weitere 72.0 mg (0.484 mmol) Chlorameisensäurecyclopentylester in Dichlormethan (1 mL) zugetropft und über Nacht bei RT gerührt. Die Reaktionsmischung wurde mit Wasser versetzt und am Rotationsverdampfer aufkonzentriert. Der Rückstand wurde in Dimethylformamid aufgenommen, mittels präparativer HPLC (Laufmittel: Acetonitril-Wasser mit 0.1 % Ameisensäure Gradient) getrennt und die Produktfraktionen eingeengt. Das Rohprodukt wurde mit gesättigter wässriger Natriumhydrogencarbonat-Lösung versetzt und zweimal mit Essigsäureethylester extrahiert. Die gesammelten organischen Phasen wurden über Magnesiumsulfat getrocknet, filtriert und eingeengt. Der Rückstand wurde mit tert.-Butylmethylether verrührt und der Feststoff abfiltriert. Lyophilisation und weitere Trocknung am Hochvakuum lieferte 42 mg (17 % d. Th.) der Titelverbindung als Feststoff.

LC-MS (Methode 2): Rₜ = 0.94 min

MS (ESIpos): m/z = 489 (M+H)⁺

¹H-NMR (400 MHz, DMSO-d₆): δ = 1.42 - 1.85 (m, 8H), 3.27 - 3.40 (m., 4H), 5.08 (m, 1H), 5.79 (s, 2H), 6.15 - 6.29 (m, 2H), 7.13 (m, 2H), 7.19 - 7.27 (m, 1H), 7.35 (m, 3H), 8.57 - 8.63 (m, 1H), 9.00 - 9.08 (m, 1H).

### Nicht erfindungsgemäßes Beispiel 70

### 2-[1-(2-Fluorbenzyl)-1H-pyrazolo[3,4-b]pyridin-3-yl]pyrido[4,3-d]pyrimidin-5(6H)-on

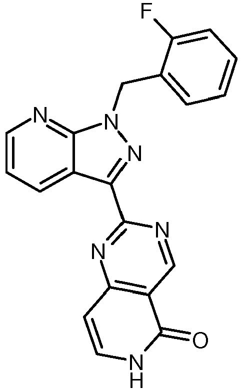

Unter Argon wurden 21 mg (0.522 mmol) Natriumhydrid (60%ige Suspension in Mineralöl) vorgelegt und mehrmals mit Hexan gewaschen. Anschließend wurde Dimethylformamid (0.18 mL) zugefügt und zur Suspension eine Lösung von 158 mg (0.435 mmol) der Verbindung aus Beispiel 46A in Dimethylformamid (6.0 mL) addiert. Die Reaktionsmischung wurde zwei Tage bei 150°C gerührt, anschließend bei RT mit Acetonitril verdünnt, danach filtriert und das Filtrat eingeengt. Der Rückstand wurde mittels präparativer HPLC (Laufmittel: Acetonitril-Wasser mit 0.1% Ameisensäure Gradient) getrennt und die Produktfraktionen eingeengt. Man erhielt 31 mg (18 % d. Th.) der Titelverbindung als Feststoff.

LC-MS (Methode 3): Rₜ = 1.02 min

MS (ESIpos): m/z = 373 (M+H)⁺

¹H-NMR (400 MHz, DMSO-d₆): δ = 5.91 (s, 2H), 6.72 (d, 1H), 7.12 - 7.20 (m, 1H), 7.21 - 7.29 (m, 2H), 7.34 - 7.42 (m, 1H), 7.49 (dd, 1H), 7.73 - 7.79 (m, 1H), 8.69 - 8.74 (m, 1H), 9.00 - 9.06 (m, 1H), 9.53 (s, 1H), 11.90 (br. s, 1H).

### Beispiel 71

### 5-[1-(2-Fluorbenzyl)-1H-pyrazolo[3,4-b]pyridin-3-yl]-1,3-dihydro-2H-imidazo[4,5-b]pyridin-2-on

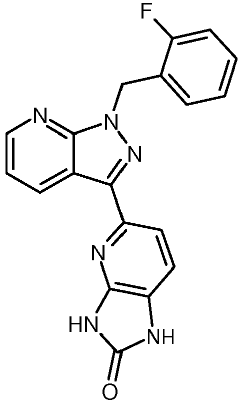

23 mg (0.069 mmol) Beispiel 43A und 12 mg (0.076 mmol) N-N'-Carbonylimidazol wurden in Acetonitril (4 ml) zusammengegeben und über Nacht zum Rückfluss erhitzt. Anschliessend wurde eingeengt und der Rückstand mittels präparativer HPLC gereinigt (Eluent: Acetonitril/Wasser mit 0.05% Ameisensäure, Gradient). Es wurden 13 mg der Titelverbindung erhalten (52 % d. Th.).

LC-MS (Methode 2): Rₜ = 0.90 min

MS (ESIpos): m/z = 361 (M+H)⁺

¹H-NMR (400 MHz, DMSO-d₆): δ = 5.79 (s, 2H), 7.12 - 7.25 (m, 3H), 7.33 - 7.39 (m, 3H), 7.73 (d, 1H), 8.63 (dd, 1H), 8.87 (dd, 1H), 11.00 (s br, 1H), 11.46 (s br, 1H).

### Nicht erfindungsgemäßes Beispiel 72

### 2-[1-(2-Fluorbenzyl)-1H-pyrazolo[3,4-b]pyridin-3-yl]pyrido[3,4-d]pyrimidin-4-amin

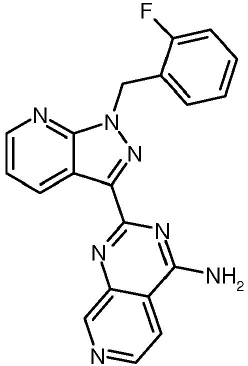

In Dimethylformamid (5.1 mL) wurden 341 mg (1.35 mmol) der Verbindung aus Beispiel 3A vorgelegt, danach 177 mg (1.49 mmol) 3-Amino-4-cyanopyridin (hergestellt gemäß J. Org. Chem. Vol. 46, No.21, 1981, 4179-4182) sowie 152 mg (1.35 mmol) Kalium-tert.-butylat addiert und die Reaktionsmischung 30 min bei 200°C in der Mikrowelle verrührt. Die Reaktionslösung wurde auf ges. Natriumhydrogencarbonatlösung gegeben und zweimal mit Essigsäureethylester extrahiert. Die gesammelten organischen Phasen wurden mit gesättigter wässriger Natriumchlorid-Lösung gewaschen, über Magnesiumsulfat getrocknet, filtriert und eingeengt. Der Rückstand wurde mittels präparativer HPLC (Laufmittel: Acetonitril-Wasser mit 0.1% Ameisensäure Gradient) aufgetrennt und die Produktfraktionen eingeengt. Das Rohprodukt wurde mit Methanol versetzt, der Feststoff abfiltriert und 2 h bei 50°C am Hochvakuum getrocknet. Man erhielt 194 mg (39 % d. Th.) der Titelverbindung als Feststoff.

LC-MS (Methode 2): Rₜ = 0.88 min

MS (ESIpos): m/z = 372 (M+H)⁺

1H-NMR (400 MHz, DMSO-d6): δ = 5.88 (s, 2H), 7.12 - 7.28 (m, 3H), 7.33 - 7.41 (m, 1H), 7.42-7.47 (m, 1H), 8.10 - 8.14 (m, 1H), 8.30 - 8.47 (m, 2H), 8.57 - 8.62 (m, 1H), 8.65 - 8.70 (m, 1H), 9.16 - 9.21 (m, 1H), 9.21 - 9.23 (m, 1H).

### Nicht erfindungsgemäßes Beispiel 73

### 2-[1-(2-Fluorbenzyl)-1H-pyrazolo[3,4-b]pyridin-3-yl]pyrimido[4,5-d]pyrimidin-4-ol

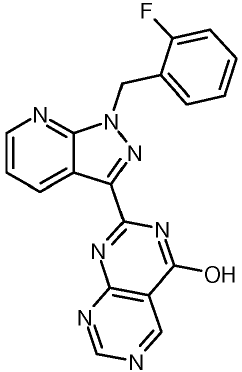

In N,N-Dimethylacetamid (22 mL) wurden 246 mg (0.629 mmol) der Verbindung aus Beispiel 48A vorgelegt und 30 min bei 220°C in der Mikrowelle gerührt. Die Reaktionsmischung wurde auf Eiswasser gegossen und filtriert. Der Filterrückstand wurde mit Wasser gewaschen und über Nacht bei 50°C am Hochvakuum getrocknet. Man erhielt 142 mg (57 % d. Th.) der Titelverbindung als Feststoff.

LC-MS (Methode 2): Rₜ = 0.89 min

MS (ESIpos): m/z = 374 (M+H)⁺

¹H-NMR (400 MHz, DMSO-d₆): δ = 5.92 (s, 2H), 7.15 - 7.20 (m, 1H), 7.22 - 7.28 (m, 1H), 7.34 - 7.45 (m, 2H), 7.54 - 7.60 (m, 1H), 8.76 - 8.79 (m, 1H), 8.88 - 8.93 (m, 1H), 9.42 (s, 1H), 9.46 (s, 1H), 13.16 (br. s, 1H).

### Nicht erfindungsgemäßes Beispiel 74

### 2-[1-(2-Fluorbenzyl)-1H-pyrazolo[3,4-b]pyridin-3-yl]chinazolin

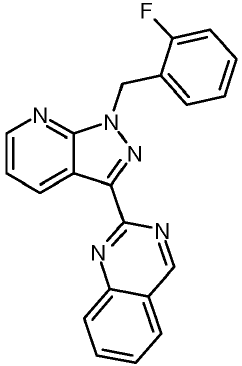

In Tetrahydrofuran (2.0 mL) wurden 200 mg (0.540 mmol) der Verbindung aus Beispiel 5 suspendiert, danach 145 µL (1.08 mmol) Isopentylnitrit zugefügt und die Reaktionsmischung 30 min zum Rückfluss erhitzt. Anschließend wurden Dimethylformamid (1.0 mL) sowie 145 µL Isopentylnitrit zugefügt und weitere 5 h bei 95°C gerührt. Die Reaktionsmischung wurde anschließend direkt mittels präparativer HPLC (Laufmittel: Acetonitril-Wasser mit 0.1% Ameisensäure Gradient) aufgetrennt und die produkthaltigen Mischfraktionen eingeengt. Das Rohprodukt wurde anschließend mittels HPLC (Laufmittel: Wasser/Methanol/1%-ige Trifluoressigsäure 28:65:7) nachgereinigt. Man erhielt 30 mg (15 % d. Th.) der Titelverbindung als Feststoff.

LC-MS (Methode 3): Rₜ = 1.28 min

MS (ESIpos): m/z = 356 (M+H)⁺

¹H-NMR (400 MHz, DMSO-d₆): δ = 5.92 (s, 2H), 7.14 - 7.20 (m, 1H), 7.21 - 7.30 (m, 2H), 7.34 - 7.41 (m, 1H), 7.47 - 7.52 (m, 1H), 7.76 - 7.81 (m, 1H), 8.05 - 8.11 (m, 1H), 8.15 - 8.20 (m, 1H), 8.20 - 8.25 (m, 1H), 8.70 - 8.73 (m, 1H), 9.10 - 9.16 (m, 1H), 9.75 (s, 1H).

### Beispiel 75

### 2-Methoxyethyl-4-amino-2-[1-(2-fluorbenzyl)-1H-pyrazolo[3,4-b]pyridin-3-yl]-7,8-dihydro-pteridin-5 (6H)-c arboxylat-Formiat

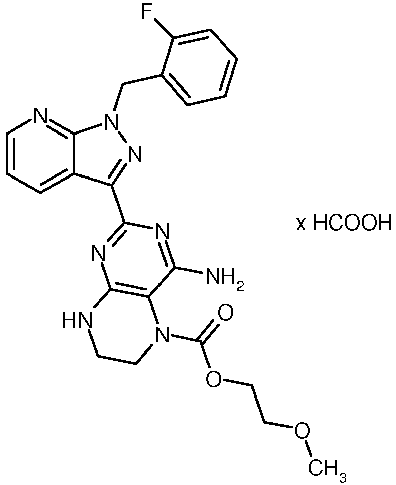

In Tetrahydrofuran (3 mL) wurden 100 mg (0.242 mmol) der Verbindung aus Beispiel 81 vorgelegt und auf 0°C abgekühlt. Anschließend wurden 19.4 mg (0.484 mmol) Natriumhydrid (60%ig in Mineralöl) zugefügt und die Mischung weitere 30 min bei 0°C gerührt. Danach wurden 34 mg (0.24 mmol) 2-Methoxyethylchlorocarbonat zugetropft und die Mischung 2 h bei RT gerührt. Anschließend wurde mit gesättigter wässriger Natriumhydrogencarbonat-Lösung verdünnt und zweimal mit Essigsäureethylester extrahiert. Die gesammelten organischen Phasen wurden über Natriumsulfat getrocknet, filtriert und einrotiert. Der Rückstand wurde mittels präparativer HPLC (Laufmittel: Acetonitril-Wasser mit 0.1% Ameisensäure Gradient) getrennt und die Produktfraktionen eingeengt. Man erhielt 12 mg (9 % d. Th.) der Titelverbindung.

LC-MS (Methode 2): Rₜ = 0.81 min

MS (ESIpos): m/z = 479 (M+H)⁺

¹H-NMR (400 MHz, DMSO-d₆): δ = 3.27 - 3.41 (m, 7H), 3.52 - 3.58 (m, 2H), 4.23 (br. s, 2H), 5.79 (s, 2H), 6.24 (br. s, 2H), 7.09 - 7.16 (m, 2H), 7.19 - 7.26 (m, 1H), 7.31 - 7.42 (m, 3H), 8.23 (s, 0.5H), 8.58 - 8.63 (m, 1H), 9.02 - 9.07 (m, 1H).

### Beispiel 76

### Ethyl-4-amino-2-[1-(2-fluorbenzyl)-1H-pyrazolo[3,4-b]pyridin-3-yl]-7,8-dihydropteridin-5(6H)-carboxylat-Formiat

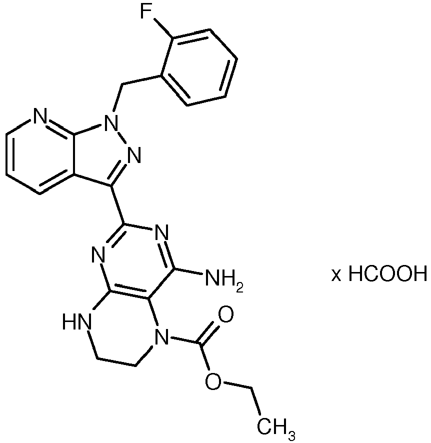

In Tetrahydrofuran (6 mL) wurden 200 mg (0.484 mmol) der Verbindung aus Beispiel Beispiel 81 vorgelegt und auf 0°C abgekühlt. Anschließend wurden 38.8 mg (0.969 mmol) Natriumhydrid zugefügt und die Mischung weitere 30 min bei 0°C gerührt. Danach wurde eine Lösung von 51.0 µL (0.533 mmol) Chlorameisensäureethylester in Dichlormethan (1.0 mL) zugetropft und die Reaktionsmischung über Nacht bei RT gerührt. Die Reaktionsmischung wurde mit gesättigter wässriger Natriumhydrogencarbonat-Lösung versetzt und zweimal mit Essigsäureethylester extrahiert. Die gesammelten organischen Phasen wurden über Natriumsulfat getrocknet, filtriert und einrotiert. Der Rückstand wurde mittels präparativer HPLC (Laufmittel: Acetonitril-Wasser mit 0.1% Ameisensäure Gradient) getrennt und die Produktfraktionen eingeengt. Man erhielt 110 mg (45 % d. Th.) der Titelverbindung.

LC-MS (Methode 2): Rₜ = 0.82 min

MS (ESIpos): m/z = 449 (M+H)⁺

¹H-NMR (400 MHz, DMSO-d₆): δ = 1.20 (t, 2H), 3.27 - 3.41 (m, 7H), 4.13 (m, 2H), 4.23 (br. s, 2H), 6.24 (br. s, 2H), 7.09 - 7.16 (m, 2H), 7.19 - 7.26 (m, 1H), 7.31 - 7.42 (m, 3H), 8.23 (s, 0.5H), 8.58 - 8.63 (m, 1H), 9.02 - 9.07 (m, 1H), 12.72 (br. s, 0.5H).

### Beispiel 77

### Isopropyl-4-amino-2-[1-(2-fluorbenzyl)-1H-pyrazolo[3,4-b]pyridin-3-yl]-7,8-dihydropteridin-5(6H)-carboxylat

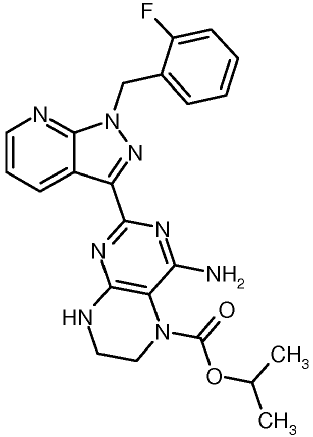

In Tetrahydrofuran (3 mL) wurden 100 mg (0.242 mmol) der Verbindung aus Beispiel 81 vorgelegt und auf 0°C abgekühlt. Anschließend wurden 19.4 mg (0.484 mmol) Natriumhydrid (60%ig in Mineralöl) zugefügt und die Mischung weitere 30 min bei 0°C gerührt. Danach wurden 242 µL (0.242 mmol) Chlorameisensäure-iso-propylester (1.0 M in Toluol) zugetropft und die Reaktionsmischung über Nacht bei RT gerührt. Die Reaktionsmischung wurde mit gesättigter wässriger Natriumhydrogencarbonat-Lösung versetzt und zweimal mit Essigsäureethylester extrahiert. Die gesammelten organischen Phasen wurden über Natriumsulfat getrocknet, filtriert und einrotiert. Der Rückstand wurde mittels präparativer HPLC (Laufmittel: Acetonitril-Wasser mit 0.1% Ameisensäure Gradient) getrennt und die Produktfraktionen eingeengt. Das Rohprodukt wurde in Essigsäureethylester gelöst und je einmal mit gesättigter wässriger Natriumhydrogencarbonat-Lösung und Wasser gewaschen, über Natriumsulfat getrocknet, filtriert und eingeengt. Weitere Trocknung am Hochvakuum lieferte 29 mg (26 % d. Th.) der Titelverbindung als Feststoff.

LC-MS (Methode 2): Rₜ = 0.89 min

MS (ESIpos): m/z = 463 (M+H)⁺

¹H-NMR (400 MHz, DMSO-d₆): δ = 1.16 - 1.26 (m, 6H), 3.32 (s, 4H), 4.83 - 4.92 (m, 1H), 5.79 (s, 2H), 6.22 (br. s, 2H), 7.09 - 7.16 (m, 2H), 7.19 - 7.26 (m, 1H), 7.31 - 7.40 (m, 3H), 8.58 - 8.63 (m, 1H), 9.01 - 9.07 (m, 1H).

### Nicht erfindungsgemäßes Beispiel 78

### 4-Amino-2-[1-(2-fluorbenzyl)-1H-pyrazolo[3,4-b]pyridin-3-yl]-6-(4-methylphenyl)pteridin-7(8H)-on

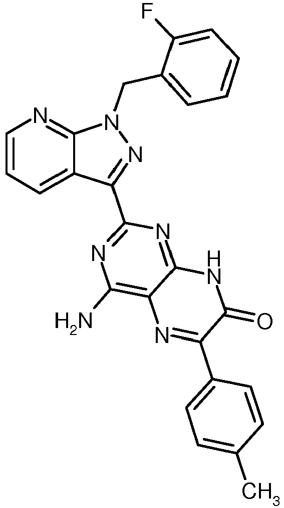

In Ethanol (3.0 mL) wurden 200 mg (0.571 mmol) der Verbindung aus Beispiel 1A vorgelegt, danach wurden 121 mg (0.628 mmol) Ethyl-4-methylphenyl-2-oxo-acetat zugefügt und die Reaktionsmischung 21 h zum Rückfluss erhitzt. Die Reaktionsmischung wurde auf RT gebracht und filtriert. Der Feststoff wurde mit Ethanol gewaschen, anschließend in Dimethylformamid suspendiert und filtriert. Das Filtrat wurde mittels präparativer HPLC (Laufmittel: Acetonitril-Wasser mit 0.1 % Ameisensäure Gradient) getrennt und die Produktfraktionen eingeengt. Das Rohprodukt wurde mit Dichlormethan/Methanol verrührt, der Feststoff abfiltriert und am Hochvakuum getrocknet. Man erhielt 17 mg (6 % d. Th.) der Titelverbindung als Feststoff.

LC-MS (Methode 2): Rₜ = 1.09 min

MS (ESIpos): m/z = 479 (M+H)⁺

¹H-NMR (400 MHz, DMSO-d₆): δ = 2.39 - 2.61 (s, 3H), 5.76 - 5.93 (m, 2H), 7.06 - 7.30 (m, 3H), 7.31 - 7.50 (m, 4H), 7.57 - 7.80 (m, 2H), 8.32 - 8.46 (m, 2H), 8.61 - 8.71 (m, 1H), 9.02 - 9.14 (m, 1H), 12.25 - 12.37 (m, 1H).

### Nicht erfindungsgemäßes Beispiel 79

### 4-Amino-6-cyclopentyl-2-[1-(2-fluorbenzyl)-1H-pyrazolo[3,4-b]pyridin-3-yl]pteridin-7(8H)-on

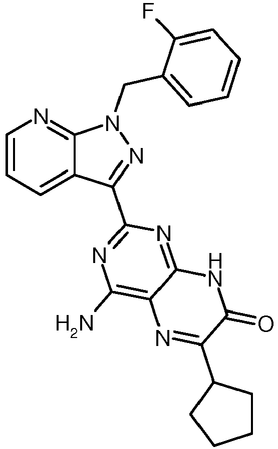

In Ethanol (3.0 mL) wurden 200 mg (0.571 mmol) der Verbindung aus Beispiel 1A vorgelegt, danach wurden 107 mg (0.628 mmol) Ethylcyclopentyl(oxo)acetat zugefügt und die Reaktionsmischung 30 h zum Rückfluss erhitzt. Die Reaktionsmischung wurde auf RT gebracht und filtriert. Der Feststoff wurde mit Ethanol gewaschen, anschließend in Trifluoressigsäure gelöst und mit gesättigter wässriger Natriumhydrogencarbonat-Lösung basisch gestellt. Die erhaltene Suspension wurde filtriert und der Filterrückstand mit Dimethylformamid gewaschen. Das Filtrat wurde mittels präparativer HPLC (Laufmittel: Acetonitril-Wasser mit 0.1% Ameisensäure Gradient) getrennt und die Produktfraktionen eingeengt. Man erhielt 12 mg (90%-ige Reinheit, 4 % d. Th.) der Titelverbindung.

LC-MS (Methode 2): Rₜ = 1.07 min

MS (ESIpos): m/z = 457 (M+H)⁺

¹H-NMR (400 MHz, DMSO-d₆): δ = 1.60 - 1.83 (m, 4H), 1.83 - 2.06 (m, 4H), 3.59 - 3.69 (m, 1H), 5.83 (s, 2H), 7.12 - 7.28 (m, 3H), 7.32 - 7.45 (m, 2H), 7.49 - 7.69 (m, 2H), 8.62 - 8.69 (m, 1H), 9.01 - 9.08 (m, 1H), 12.70 (m, 1H).

### Beispiel 80

### Methyl-4-amino-2-[1-(2-fluorbenzyl)-1H-pyrazolo[3,4-b]pyridin-3-yl]-7,8-dihydropteridin-5(6H)-carboxylat

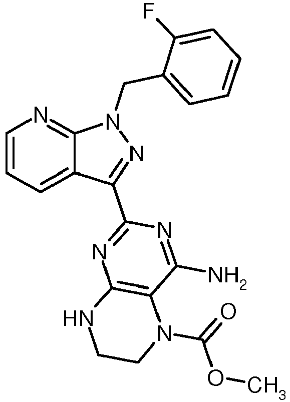

In Tetrahydrofuran (7 mL) wurden 80.0 mg (0.194 mmol) der Verbindung aus Beispiel 81 vorgelegt und auf 0°C abgekühlt. Anschließend wurden 15.5 mg (0.388 mmol) Natriumhydrid (60%ig in Mineralöl) zugefügt und die Mischung weitere 30 min bei 0°C gerührt. Danach wurde eine Lösung von 745 µL (0.194 mmol) Chlorameisensäuremethylester in Dichlormethan (5.0 mL) zugetropft und die Reaktionsmischung weitere 2 h bei RT gerührt. Die Reaktionsmischung wurde mit gesättigter wässriger Natriumhydrogencarbonat-Lösung versetzt und zweimal mit Essigsäureethylester extrahiert. Die gesammelten organischen Phasen wurden über Natriumsulfat getrocknet, filtriert und einrotiert. Der Rückstand wurde mittels präparativer HPLC (Laufmittel: Acetonitril-Wasser mit 0.1% Ameisensäure Gradient) getrennt und die Produktfraktionen eingeengt. Das Rohprodukt wurde in Essigsäureethylester gelöst und einmal mit gesättigter wässriger Natriumhydrogencarbonat-Lösung sowie zweimal mit Wasser gewaschen, über Natriumsulfat getrocknet, filtriert und eingeengt. Weitere Trocknung am Hochvakuum lieferte 39.8 mg (47 % d. Th.) der Titelverbindung als Feststoff.

LC-MS (Methode 2): Rₜ = 0.78 min

MS (ESIpos): m/z = 435 (M+H)⁺

¹H-NMR (400 MHz, DMSO-d₆): δ = 3.32 (m, 4H), 3.67 (s, 3H), 5.79 (s, 2H), 6.32 (br. s, 2H), 7.09 - 7.16 (m, 2H), 7.19 - 7.26 (m, 1H), 7.31 - 7.38 (m, 3H), 8.58 - 8.62 (m, 1H), 9.02 - 9.06 (m, 1H).

### Nicht erfindungsgemäßes Beispiel 81

### 2-[1-(2-Fluorbenzyl)-1H-pyrazolo[3,4-b]pyridin-3-yl]-5,6,7,8-tetrahydropteridin-4-amin-Hydrochlorid

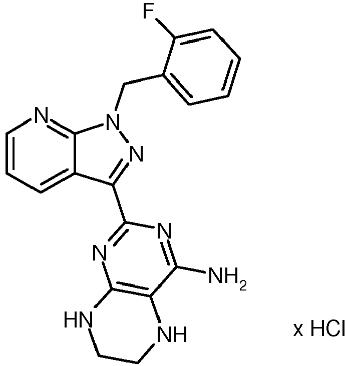

In Methanol (20.0 mL) wurden 437 mg (1.17 mmol) der Verbindung aus Beispiel 52 vorgelegt und die Suspension mit Argon überschichtet. Anschließend wurden 64 mg (0.24 mmol) Platin(IV)oxid (83%ig w/w) zugefügt und die Reaktionsmischung über Nacht bei Wasserstoff-Normaldruck hydriert. Die Reaktionsmischung wurde durch Celite filtriert und der Filterkuchen mit Methanol nachgewaschen. Das Filtrat wurde mit konz. Salzsäure versetzt und einrotiert. Der Rückstand wurde mit Methanol/tert.-Butylmethylether ausgerührt und abfiltriert. Der Feststoff wurde mit Methanol/tert.-Butylmethylether gewaschen und am Hochvakuum getrocknet. Man erhielt 451 mg (93 % d. Th.) der Titelverbindung als Feststoff.

LC-MS (Methode 3): Rₜ = 0.87 min

MS (ESIpos): m/z = 377 (M+H)⁺

¹H-NMR (400 MHz, DMSO-d₆): δ = 3.23 - 3.30 (m, 2H), 3.46 - 3.53 (m, 2H), 4.20 - 4.63 (br. s, 1H), 5.90 (s, 2H), 7.11 - 7.20 (m, 2H), 7.21 - 7.30 (m, 2H), 7.31-7.41 (m, 1H), 7.47 - 7.54 (m, 1H), 8.32 - 8.47 (br. s, 1H), 8.72 - 8.76 (m, 1H), 8.86 - 8.92 (m, 1H).

### Beispiel 82

### 2-{4-Amino-6-[1-(2-fluorbenzyl)-1H-pyrazolo[3,4-b]pyridin-3-yl]-1H-pyrazolo[3,4-d]pyrimidin-1-yl}ethanol

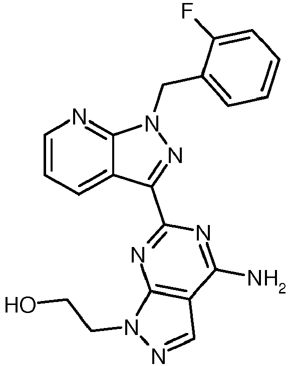

In Dimethylformamid (3.0 mL) wurden 200 mg (0.793 mmol) der Verbindung aus Beispiel 3A vorgelegt, danach 133 mg (0.872 mmol) 5-Amino-1-(2-hydroxyethyl)-1H-pyrazol-4-carbonitril sowie 89.0 mg (0.793 mmol) Kalium-tert.-butylat addiert und die Reaktionsmischung 30 min bei 180°C in der Mikrowelle verrührt. Die Reaktionsmischung wurde mittels präparativer HPLC (Laufmittel: Acetonitril-Wasser mit 0.1% Ameisensäure Gradient) aufgetrennt und die Produktfraktionen gesammelt. Man erhielt 126 mg (94%ig, 37 % d. Th.) der Titelverbindung als Feststoff.

LC-MS (Methode 2): Rₜ = 0.81 min

MS (ESIpos): m/z = 405 (M+H)⁺

¹H-NMR (400 MHz, DMSO-d₆): δ = 3.84 - 3.92 (m, 2H), 4.44 (t, 2H), 4.87 - 4.95 (m, 1H), 5.86 (s, 2H), 7.10 - 7.17 (m, 2H), 7.21 - 7.28 (m, 1H), 7.32 - 7.39 (m, 1H), 7.40 - 7.45 (m, 1H), 7.84 - 7.91 (m, 2H), 8.12 (s, 1H), 8.62 - 8.67 (m, 1H), 9.08 - 9.14 (m, 1H).

### Nicht erfindungsgemäßes Beispiel 83

### 4-Amino-2-[1-(2-fluorbenzyl)-1H-pyrazolo[3,4-b]pyridin-3-yl]-6-isopropylpteridin-7(8H)-on

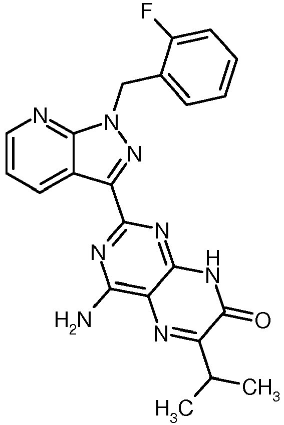

In Ethanol (50 mL) wurden 1.00 g (2.85 mmol) der Verbindung aus Beispiel 1A vorgelegt, danach wurden 453 mg (3.14 mmol) Ethyl-3-methyl-2-oxobutanoat sowie eine katalytische Menge konz. Schwefelsäure addiert und die Reaktionsmischung über Nacht zum Rückfluss erhitzt. Anschließend wurden erneut 453 mg (3.14 mmol) Ethyl-3-methyl-2-oxobutanoat zugefügt und weiter über Nacht zum Rückfluss erhitzt. Die Reaktionsmischung wurde auf RT gebracht und filtriert. Der Feststoff wurde mit Ethanol gewaschen und am Hochvakuum getrocknet. Man erhielt 592 mg (45 % d. Th.) der Titelverbindung.

LC-MS (Methode 1): Rₜ = 2.13 min

MS (ESIpos): m/z = 431 (M+H)⁺

¹H-NMR (400 MHz, DMSO-d₆): δ = 1.23 (d, 6H), 3.35 - 3.39 (m, 1H), 5.84 (s, 2H), 7.12 - 7.28 (m, 3H), 7.33 - 7.45 (m, 3H), 7.87 - 7.97 (m, 1H), 8.63 - 8.68 (m, 1H), 9.15 - 9.20 (m, 1H), 12.73 (s, 1H).

### Nicht erfindungsgemäßes Beispiel 84

### 2-[1-(2-Fluorbenzyl)-1H-pyrazolo[3,4-b]pyridin-3-yl]pyrido[4,3-d]pyrimidin-4-amin

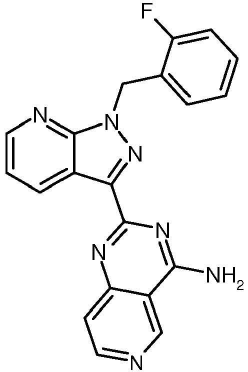

In Dimethylformamid (0.6 mL) wurden 50 mg (0.16 mmol) der Verbindung aus Beispiel 5A vorgelegt, danach 41 mg (0.18 mmol) 3-Cyano-4-iodpyridin sowie 35 mg (0.33 mmol) Natriumcarbonat addiert und die Mischung 30 min bei 200°C verrührt. Die Reaktionsmischung wurde mittels präparativer HPLC (Laufmittel: Acetonitril-Wasser mit 0.1% Ameisensäure Gradient) getrennt und die Produktfraktionen eingeengt. Man erhielt 9 mg (15 % d. Th.) der Titelverbindung als Feststoff.

LC-MS (Methode 1): Rₜ = 1.62 min

MS (ESIpos): m/z = 372 (M+H)⁺

1H-NMR (400 MHz, DMSO-d6): δ = 5.88 (s, 2H), 7.12 - 7.28 (m, 3H), 7.33 - 7.41 (m, 1H), 7.42-7.47 (m, 1H), 7.65 - 7.69 (m, 1H), 8.44 - 8.52 (m, 2H), 8.66 - 8.69 (m, 1H), 8.72 - 8.76 (m, 1H), 9.14 - 9.18 (m, 1H), 9.54 (s,1H).

### Nicht erfindungsgemäßes Beispiel 85

### 2-[1-(2-Fluorbenzyl)-1H-pyrazolo[3,4-b]pyridin-3-yl]pyrido[3,2-d]pyrimidin-4-amin

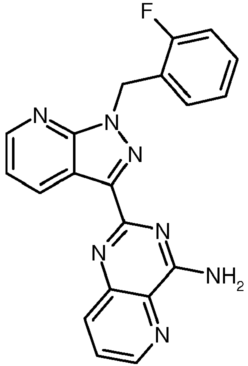

In Dimethylformamid (3.0 mL) wurden 200 mg (0.793 mmol) der Verbindung aus Beispiel 3A vorgelegt, danach 104 mg (0.872 mmol) 3-Amino-3-cyanopyridin sowie 89.0 mg (0.793 mmol) Kalium-tert.-butylat addiert und die Reaktionsmischung 30 min bei 200°C in der Mikrowelle verrührt. Die Reaktionslösung wurde auf Wasser gegeben und der entstandene Feststoff abfiltriert. Das so erhaltene Rohprodukt wurde in DMSO gelöst und mit Wasser ausgefällt. Der Feststoff wurde abfiltriert, mit Wasser gewaschen und am Hochvakuum getrocknet. Man erhielt 17.8 mg (6 % d. Th.) der Titelverbindung als Feststoff.

LC-MS (Methode 3): Rₜ = 0.98 min

MS (ESIpos): m/z = 372 (M+H)⁺

¹H-NMR (400 MHz, DMSO-d₆): δ = 5.87 (s, 2H), 7.11 - 7.30 (m, 3H), 7.33 - 7.40 (m, 1H), 7.41 - 7.47 (m, 1H), 7.82 - 7.89 (m, 1H), 8.00 - 8.09 (br. s, 1H), 8.23 (m, 2H), 8.63 - 8.70 (m, 1H), 8.77 - 8.83 (m, 1H), 9.13 - 9.21 (m, 1H).

### Nicht erfindungsgemäßes Beispiel 86

### 5-Fluor-2-[1-(2-fluorbenzyl)-1H-pyrazolo[3,4-b]pyridin-3-yl]chinazolin-4-amin

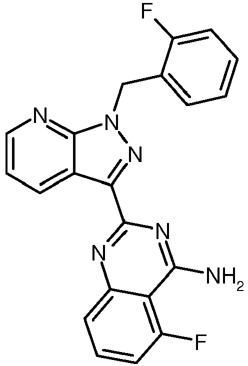

In Dimethylformamid (4.5 mL) wurden 300 mg (1.19 mmol) der Verbindung aus Beispiel 3A vorgelegt, danach 178 mg (1.31 mmol) 2-Amino-6-fluorbenzonitril sowie 66.7 mg (0.595 mmol) Kalium-tert.-butylat addiert und die Reaktionsmischung 1 h bei 200°C in der Mikrowelle verrührt. Anschließend wurden erneut 66.7 mg (0.595 mmol) Kalium-tert.-butylat zugefügt und die Mischung eine weitere 1h bei 200°C gerührt. Die Reaktionsmischung wurde anschließend mittels präparativer HPLC (Laufmittel: Acetonitril-Wasser mit 0.1% Ameisensäure Gradient) getrennt und die Produktfraktionen eingeengt. Das Rohprodukt wurde mit Acetonitril/DMSO verrührt, der Rückstand filtriert, mit Acetonitril gewaschen und am Hochvakuum getrocknet. Man erhielt 10 mg (2 % d. Th.) der Titelverbindung als Feststoff.

LC-MS (Methode 3): Rₜ = 1.02 min

MS (ESIpos): m/z = 389 (M+H)⁺

¹H-NMR (400 MHz, DMSO-d₆): δ = 5.87 (s, 2H), 7.12 - 7.17 (m, 2H), 7.21 - 7.32 (m, 2H), 7.33 - 7.47 (m, 3H), 7.63-7.70 (m, 1H), 7.74 - 7.84 (m, 1H), 8.34 (br. s, 1H), 8.60 - 8.71 (m, 1H), 9.20 (m, 1H).

### Nicht erfindungsgemäßes Beispiel 87

### 2-[1-(2-Fluorbenzyl)-1H-pyrazolo[3,4-b]pyridin-3-yl]pyrido[2,3-d]pyrimidin-4-amin

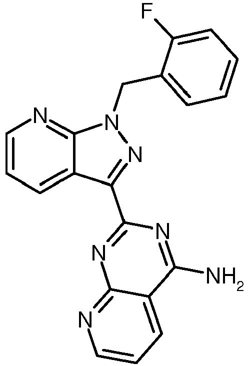

In Dimethylformamid (1.5 mL) wurden 100 mg (0.396 mmol) der Verbindung aus Beispiel 3A vorgelegt, danach 51.9 mg (0.436 mmol) 2-Amino-3-cyanopyridin sowie 44.5 mg (0.396 mmol) Kalium-tert.-butylat addiert und die Reaktionsmischung 1 h bei 200°C in der Mikrowelle verrührt. Die Reaktionslösung wurde anschließend mittels präparativer HPLC (Laufmittel: Acetonitril-Wasser mit 0.1% Ameisensäure Gradient) aufgetrennt und die Produktfraktionen eingeengt. Man erhielt 36.2 mg (25 % d. Th.) der Titelverbindung als Feststoff.

LC-MS (Methode 2): Rₜ = 0.79 min

MS (ESIpos): m/z = 372 (M+H)⁺

¹H-NMR (400 MHz, DMSO-d₆): δ = 5.87 (s, 2H), 7.12 - 7.18 (m, 1H), 7.19 - 7.28 (m, 2H), 7.33 - 7.41 (m, 1H), 7.42 - 7.48 (m, 1H), 7.49 - 7.55 (m, 1H), 8.29 (br. s, 2H), 8.65 - 8.71 (m, 2H), 9.00 - 9.04 (m, 1H), 9.13 - 9.18 (m, 1H).

### Nicht erfindungsgemäßes Beispiel 88

### 6-Amino-7-ethyl-2-[3-(2-fluorbenzyl)-1H-pyrazolo[4,3-b]pyridin-1-yl]-7,9-dihydro-8H-purin-8-on

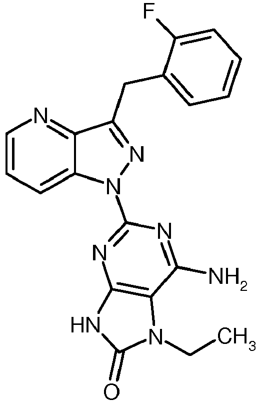

In Tetrahydrofuran (11.1 mL) wurden 200 mg (0.392 mmol) 2-[3-(2-Fluorbenzyl)-1H-pyrazolo[4,3-b]pyridin-1-yl]pyrimidin-4,5,6-triamin (Beispiel 2A) vorgelegt und die Suspension auf 0°C abgekühlt. Anschließend wurden 0.43 mL (0.43 mmol) Bis-(trimethylsilyl)natriumamid-Lösung (1.0 M in Tetrahydrofuran) zugegeben und die Mischung weitere 30 min bei 0°C gerührt. Danach wurden 31.3 µL (0.392 mmol) Iodethan zugetropft und die Reaktionsmischung bei RT über Nacht gerührt. Die Reaktionsmischung wurde erneut auf 0°C abgekühlt und weitere 0.47 mL (0.47 mmol) Bis-(Trimethylsilyl)natriumamid-Lösung zugefügt. Danach wurde über Nacht bei RT gerührt und die Reaktionsmischung weitere 2 Tage zum Rückfluss erhitzt. Anschließend wurde die Reaktionsmischung mit Essigsäureethylester verdünnt und zweimal mit gesättigter wässriger Natriumhydrogencarbonatlösung gewaschen. Die organische Phase wurde über Magnesiumsulfat getrocknet, filtriert und einrotiert. Der Rückstand wurde mittels präparativer HPLC (Laufmittel: Acetonitril-Wasser mit 0.1% Ameisensäure Gradient) aufgetrennt und die Produktfraktionen eingeengt. Das Rohprodukt wurde in Methyl-tert.-Butylether ausgerührt, der Feststoff abfiltriert und am Hochvakuum getrocknet. Man erhielt 4.6 mg (2.9 % d. Th.) der Titelverbindung als Feststoff.

LC-MS (Methode 2): Rₜ = 0.85 min

MS (ESIpos): m/z = 405 (M+H)⁺

¹H-NMR (400 MHz, DMSO-d₆): δ = 1.17 (t, 3H), 3.98 (q, 2H), 4.44 (s, 2H), 6.98 (s, 2H), 7.09 - 7.15 (m, 1H), 7.15 - 7.22 (m, 1H), 7.24 - 7.32 (m, 1H), 7.35 - 7.42 (m, 1H), 7.51 - 7.58 (m, 1H), 8.60 - 8.66 (m, 1H), 9.10 - 9.16 (m, 1H), 11.70 (s, 1H).

### Nicht erfindunsssemäßes Beispiel 89

### 6-Ammo-2-[3-(2-fluorbenzyl)-1H-pyrazolo[4,3-b]pyridm-1-yl]-7-methyl-7,9-dihydro-8H-purin-8-on

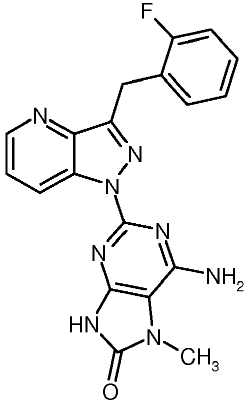

In Tetrahydrofuran (11.1 mL) wurden 200 mg (0.392 mmol) 2-[3-(2-Fluorbenzyl)-1H-pyrazolo[4,3-b]pyridin-1-yl]pyrimidin-4,5,6-triamin (Beispiel 2A) vorgelegt und die Suspension auf 0°C abgekühlt. Anschließend wurden 0.43 mL einer 1M Lösung von Bis-(Trimethylsilyl)natriumamid in Tetrahydrofuran zugegeben und die Mischung weitere 30 min bei 0°C gerührt. Danach wurden 24.4 µL (0.392 mmol) Iodmethan zugetropft und die Reaktionsmischung bei RT über Nacht gerührt. Die Reaktionsmischung wurde erneut auf 0°C abgekühlt und weitere 12.2 µL (0.196 mmol) Iodmethan (gelöst in 1.0 mL Tetrahydrofuran) zugefügt. Danach wurde 1h bei 0°C gerührt und anschließend über Nacht zum Rückfluss erhitzt. Die Reaktionsmischung wurde mit Essigsäureethylester verdünnt und zweimal mit gesättigter wässriger Natriumhydrogencarbonatlösung gewaschen. Die organische Phase wurde über Magnesiumsulfat getrocknet, filtriert und einrotiert. Der Rückstand wurde an Kieselgel chromatographiert (Laufmittel: Dichlormethan-Methanol 40:1) und die Produktfraktionen eingeengt. Man erhielt 15.6 mg (10 % d. Th.) der Titelverbindung als Feststoff.

LC-MS (Methode 2): Rₜ = 0.81 min

MS (ESIpos): m/z = 391 (M+H)⁺

¹H-NMR (400 MHz, DMSO-d₆): δ = 3.46 (s, 3H), 4.44 (s, 2H), 6.98 (s, 2H), 7.08 - 7.23 (m, 2H), 7.23 - 7.32 (m, 1H), 7.34 - 7.43 (m, 1H), 7.50 - 7.60 (m, 1H), 8.59 - 8.68 (m, 1H), 9.08 - 9.16 (m, 1H), 11.69 (s, 1H).

### Nicht erfindungsgemäßes Beispiel 90

### 6-Amino-2-[3-(2-fluorbenzyl)-1H-pyrazolo[4,3-b]pyridin-1-yl]-7,9-dihydro-8H-purin-8-on

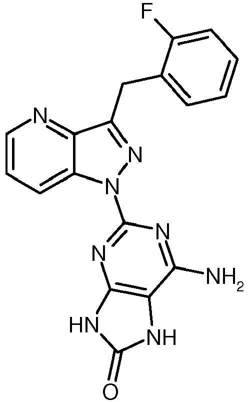

In Tetrahydrofuran (4.7 mL) wurden 235 mg (0.540 mmol) der Verbindung aus Beispiel 49A vorgelegt, 23.8 mg (0.594 mmol) Natriumhydrid (60%ig in Mineralöl) zugefügt und die Mischung 15 min bei 65°C gerührt. Die Reaktionsmischung wurde mit Essigsäureethylester verdünnt und mit gesättigter wässriger Natriumhydrogencarbonat-Lösung gewaschen. Die organische Phase wurde über Magnesiumsulfat getrocknet, filtriert und einrotiert. Der Rückstand wurde mittels präparativer HPLC (Laufmittel: Acetonitril-Wasser mit 0.1% Ameisensäure Gradient) getrennt und die Produktfraktionen eingeengt. Man erhielt 11.3 mg (5 % d. Th.) der Titelverbindung als Feststoff.

LC-MS (Methode 4): Rₜ = 1.66 min

MS (ESIpos): m/z = 377 (M+H)⁺.

¹H-NMR (400 MHz, DMSO-d₆): δ = 4.43 (s, 2H), 6.79 (br. s, 1H), 7.09 - 7.15 (m, 1H), 7.15 - 7.22 (m, 1H), 7.24 - 7.32 (m, 1H), 7.35 - 7.42 (m, 1H), 7.51 - 7.57 (m, 1H), 8.59 - 8.64 (m, 1H), 9.02 - 9.08 (m, 1H).

### Nicht erfindungsgemäßes Beispiel 91

### 6-Amino-7-(4-fluorbenzyl)-2-[3-(2-fluorbenzyl)-1H-pyrazolo[4,3-b]pyridin-1-yl]-7,9-dihydro-8H-purin-8-on

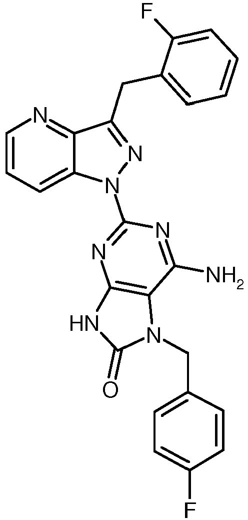

In Tetrahydrofuran (1.0 mL) wurden 21 mg (0.04 mmol) der Verbindung aus Beispiel 50A vorgelegt, 1.8 mg (0.04 mmol) Natriumhydrid (60%ig in Mineralöl) zugefügt und die Mischung 30 min bei 65°C gerührt. Die Reaktionsmischung wurde mit gesättigter wässriger Natriumhydrogencarbonat-Lösung versetzt und zweimal mit Essigsäureethylester extrahiert. Die vereinigten organischen Phasen wurden über Natriumsulfat getrocknet, filtriert und einrotiert. Der Rückstand wurde mittels präparativer HPLC (Laufmittel: Acetonitril-Wasser mit 0.1% Ameisensäure Gradient) getrennt und die Produktfraktionen eingeengt. Man erhielt 8.5 mg (44 % d. Th.) der Titelverbindung als Feststoff.

LC-MS (Methode 2): Rₜ = 1.00 min

MS (ESIpos): m/z = 485 (M+H)⁺

¹H-NMR (400 MHz, DMSO-d₆): δ = 4.43 (s, 2H), 5.20 (s, 2H), 6.92 (s, 2H), 7.08 - 7.22 (m, 4H), 7.24 - 7.32 (m, 3H), 7.35 - 7.41 (m, 1H), 7.51 - 7.56 (m, 1H), 8.60 - 8.65 (m, 1H), 9.07 - 9.13 (m, 1H),11.90 (s, 1H).

### Nicht erfindungsgemäßes Beispiel 92

### 6-Amino-2-[3-(2-fluorbenzyl)-1H-pyrazolo[4,3-b]pyridin-1-yl]-7-(2-hydroxyethyl)-7,9-dihydro-8H-purin-8-on

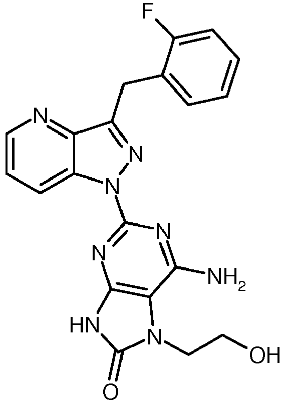

In Methanol (5.0 mL) wurden 120 mg (0.263 mmol) des Carbamats aus Beispiel 51A vorgelegt, 100 µL (0.525 mmol) Natriummethylatlösung (25%ig w/w in Methanol) zugefügt und die Mischung 30 min bei 65°C gerührt. Das Lösungsmittel wurde am im Vakuum entfernt, der Rückstand mittels präparativer HPLC getrennt und die Produktfraktionen eingeengt. Man erhielt 53.1 mg (48 % d. Th.) der Titelverbindung als Feststoff.

LC-MS (Methode 2): Rₜ = 0.80 min

MS (ESIpos): m/z = 421 (M+H)⁺

¹H-NMR (400 MHz, DMSO-d₆): δ = 3.59 - 3.66 (m, 2H), 3.93 - 3.99 (m, 2H), 4.44 (s, 2H), 5.32 - 5.36 (m, 1H), 7.00 (s, 2H), 7.09 - 7.15 (m, 1H), 7.16 - 7.22 (m, 1H), 7.25 - 7.32 (m, 1H), 7.36 - 7.42 (m, 1H), 7.53 - 7.58 (m, 1H), 8.62 - 8.65 (m, 1H), 9.09 - 9.14 (m, 1H), 11.74 (s, 1H).

### Nicht erfindungsgemäßes Beispiel 93

### 4-Amino-2-[1-(2-fluorbenzyl)-1H-pyrazolo[3,4-b]pyridin-3-yl]-6-propylpteridin-7(8H)-on

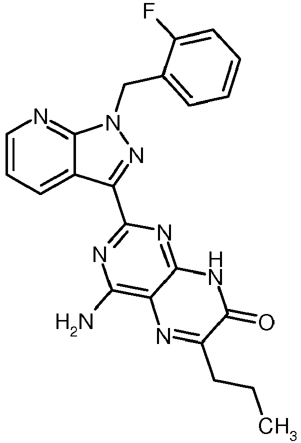

In Ethanol (3.0 mL) wurden 250 mg (0.714 mmol) der Verbindung aus Beispiel 1A vorgelegt, danach 113 mg (0.785 mmol) Ethyl-2-oxopentanoat zugefügt und die Reaktionsmischung zwei Tage zum Rückfluss erhitzt. Die Reaktionsmischung wurde auf RT gebracht und filtriert. Der Feststoff wurde mit Ethanol gewaschen, anschließend in Dimethylformamid suspendiert und die Mischung über Nacht stehen gelassen. Danach wurde die überstehende Lösung dekantiert und verworfen. Der verbliebene Rückstand wurde mehrfach mit Methanol verrührt und die überstehende Lösung jeweils verworfen. Der Rückstand wurde am Hochvakuum getrocknet und man erhielt nach Lyophilisation 69 mg (22 % d. Th.) der Titelverbindung als Feststoff.

LC-MS (Methode 2): Rₜ = 1.02 min

MS (ESIpos): m/z = 431 (M+H)⁺

¹H-NMR (400 MHz, DMSO-d₆): δ = 0.97 (t, 3H), 1.70 - 1.81 (m, 2H), 2.69 - 2.76 (m, 2H), 5.84 (s, 1H), 7.11 - 7.27 (m, 3H), 7.33 - 7.45 (m, 3H), 7.88 (br. s, 1H), 8.63 - 8.68 (m, 1H), 9.14 - 9.20 (m, 1H), 12.73 (s, 1H).

### Nicht erfindungsgemäßes Beispiel 94

### 4-Amino-6-ethyl-2-[1-(2-fluorbenzyl)-1H-pyrazolo[3,4-b]pyridin-3-yl]pteridin-7(8H)-on

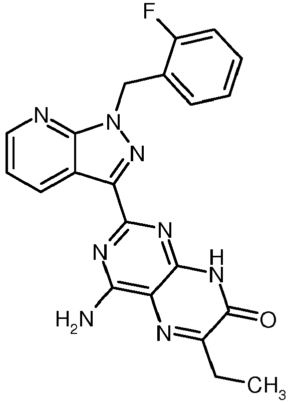

In Ethanol (3.8 mL) wurden 250 mg (0.714 mmol) der Verbindung aus Beispiel 1A vorgelegt, danach 102 mg (0.785 mmol) Methyl-3-oxopentanoat zugefügt und die Reaktionsmischung zwei Tage zum Rückfluss erhitzt. Die Reaktionsmischung wurde auf RT gebracht und filtriert. Der Feststoff wurde mit Ethanol gewaschen, anschließend in Dimethylformamid suspendiert und die Mischung über Nacht stehen gelassen. Danach wurde die überstehende Lösung dekantiert und verworfen. Der verbliebene Rückstand wurde mehrfach mit Methanol verrührt und die überstehende Lösung jeweils verworfen. Der Rückstand wurde am Hochvakuum getrocknet und man erhielt nach Lyophilisation 77 mg (25 % d. Th.) der Titelverbindung als Feststoff.

LC-MS (Methode 2): Rₜ = 0.96 min

MS (ESIpos): m/z = 417 (M+H)⁺

¹H-NMR (400 MHz, DMSO-d₆): δ = 1.24 (t, 3H), 2.76 (q, 2H), 5.84 (s, 2H), 7.12 - 7.28 (m, 3H), 7.32 - 7.47 (m, 3H), 7.89 (br. s, 1H), 8.63 - 8.68 (m, 1H), 9.14 - 9.20 (m, 1H), 12.72 (s, 1H).

### Beispiel 95

### 2-[5-Fluor-1-(2-fluorbenzyl)-1H-pyrazolo[3,4-b]pyridin-3-yl]-7-methyl-7,9-dihydro-8H-purin-8-on

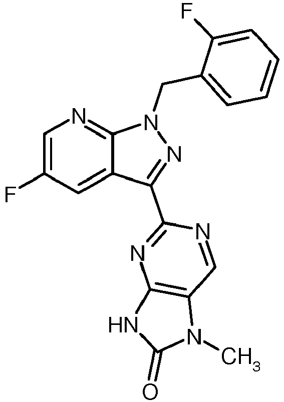

In Analogie zur Vorschrift beschrieben unter Beispiel 1 wurden 417 mg (0.686 mmol, ca. 70%-ige Reinheit) Beispiel 75A umgesetzt. Man erhielt 213 mg (79 % d. Th.) der Titelverbindung.

LC-MS (Methode 2): Rₜ = 0.96 min; MS (ESIpos): m/z = 394 (M+H)⁺

¹H-NMR (400 MHz, DMSO-d₆): δ [ppm] = 3.37 (s, 3H), 5.83 (s, 2H), 7.15-7.30 (m, 3H), 7.34-7.40 (m, 1H), 8.51 (s, 1H), 8.58 (dd, 1H), 8.72 (dd, 1H), 12.16 (s br, 1H).

### Beispiel 96

### 5-[1-(2-Fluorbenzyl)1H-pyrazolo[3,4-b]pyridin-3-yl]-1,3-dihydro-2H-imidazo[4,5-b]pyrazin-2-on

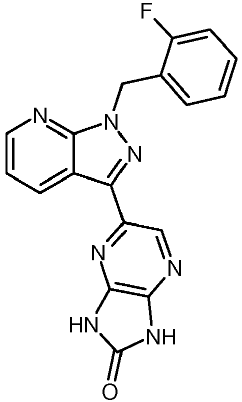

In Pyridin (5.0 mL) wurden 40 mg (0.12 mmol) der Verbindung aus Beispiel 52A vorgelegt, anschließend 39 mg (0.13 mmol) Bis(trichlormethyl)carbonat zugefügt und die Mischung 1 h bei 100°C gerührt. Die Reaktionsmischung wurde eingeengt, mit gesättigter wässriger Natriumhydrogencarbonatlösung versetzt und zweimal mit Essigsäureethylester extrahiert. Die gesammelten organischen Phasen wurden über Natriumsulfat getrocknet, filtriert und einrotiert. Der Rückstand wurde mittels präparativer HPLC (Laufmittel: Acetonitril-Wasser mit 0.1% Ameisensäure Gradient) getrennt und die Produktfraktionen eingeengt. Man erhielt 9 mg (20 % d. Th.) der Titelverbindung als Feststoff.

LC-MS (Methode 2): Rₜ = 0.87 min

MS (ESIpos): m/z = 362 (M+H)⁺

¹H-NMR (400 MHz, DMSO-d₆): δ = 5.82 (s, 2H), 7.11 - 7.17 (m, 1H), 7.18 - 7.27 (m, 2H), 7.32 - 7.42 (m, 2H), 8.55 (s, 1H), 8.64 - 8.67 (m, 1H), 8.74 - 8.78 (m, 1H), 11.89 (br. s, 1H).

### Nicht erfindungsgemäßes Beispiel 97

### 5-Fluor-1-(2-fluorbenzyl)-3-(7H-pyrrolo[2,3-d]pyrimidin-2-yl)-1H-pyrazolo[3,4-b]pyridin

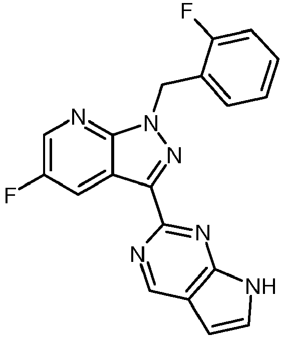

Unter einer Argonatmosphäre wurden 2.000 g (5.389 mmol) der Verbindung aus Beispiel 24A in 200 ml 1,4-Dioxan gelöst und mit 9.378 g (16.167 mmol) Hexabutyldistannan versetzt. Zum Gemisch wurden 2.000 g (1.731 mmol) Tetrakis(triphenylphosphin)palladium(0) sowie 0.910 g (5.928 mmol) 2-Chlor-7H-pyrrolo[2,3-d]pyrimidin (beschrieben in Bioorg. Med. Chem. 17(19), 6926-6936; 2009) addiert. Es wurde 60 h zum Rückfluß erhitzt. Nach dem Abkühlen wurde das Reaktionsgemisch über Celite filtriert und das Filtrat am Rotationsverdampfer eingeengt. Der Rückstand wurde nacheinander mit Essigsäureethylester, Acetonitril und Dimethylsulfoxid verrührt. Anschließend wurde der Rückstand in Trifluoressigsäure gelöst und mit Acetonitril und Wasser bis zu einer leichten Trübung versetzt. Es wurde filtriert und das Filtrat am Rotationsverdampfer eingeengt und der Rückstand am Hochvakuum getrocknet. Es wurden 161 mg (8% d. Th.) der Titelverbindung erhalten.

LC-MS (Methode 3): Rₜ = 1.20 min; MS (EIpos): m/z = 362 [M+H]⁺.

¹H-NMR (400 MHz, DMSO-d₆): δ [ppm] = 5.88 (s, 2H), 6.74-6.75 (m, 1H), 7.18 (t, 1H), 7.23-7.27 (m, 1H), 7.31 (dt, 1H), 7.36-7.41 (m, 1H), 7.74-7.76 (m, 1H), 8.72 (dd, 1H), 8.77-8.78 (m, 1H), 9.21 (s, 1H), 12.51 (s br, 1H).

### Nicht erfindungsgemäßes Beispiel 98

### Methyl-4-amino-2-[1-(2-fluorbenzyl)-1H-pyrazolo[3,4-b]pyridin-3-yl]-7-oxo-7,8-dihydropteridin-5(6H)-carboxylat

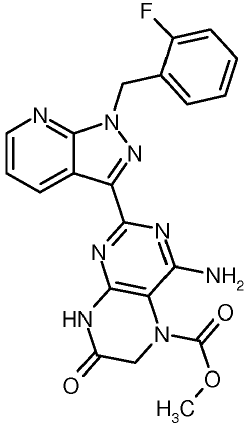

3.5 g (7.285 mmol) der Rohverbindung aus Beispiel 55A wurden in Ethanol (25 ml) und Wasser (25 ml) gelöst und mit 174 mg (7.285 mmol) Lithiumhydroxid versetzt. Nach 30 min Rühren bei RT wurde mit verdünnter Salzsäure auf pH=6 eingestellt und mit Essigsäureethylester extrahiert. Nach Trennung der Phasen wurde die organische Phase mit gesättigter wässriger Natriumchlorid-Lösung gewaschen und anschliessend mit Natriumsulfat getrocknet, filtriert und bis zur Trockene eingeengt. Der Rückstand wurde in Acetonitril und Essigsäureethylester aufgeschlämmt und unlösliche Bestandteilen abfiltriert. Das Filtrat wurde eingeengt und der Rückstand wurde mittels präparativer HPLC gereinigt (Acetonitril:Wasser (+0.05 % Ameisensäure) - Gradient). Es wurden 38 mg der Titelverbindung erhalten (1 % d. Th.).

LC-MS (Methode 2): Rₜ = 0.88 min; MS (ESIpos): m/z = 449 (M+H)⁺

¹H-NMR (400 MHz, DMSO-d₆): δ [ppm] = 3.30 (s, 2H), 3.69 (s, 3H), 5.82 (s, 2H), 7.12-7.16 (m, 2H), 7.20-7.25 (m, 1H), 7.33-7.40 (m, 2H), 8.63 (dd, 1H), 9.10 (dd, 1H), 11.17 (s, 1H).

### Beispiel 99

### 2-[1-(2-Fluorbenzyl)-1H-pyrazolo[3,4-b]pyridin-3-yl]-7-(3,3,3-trifluor-2-hydroxypropyl)-7,9-dihydro-8H-purin-8-on

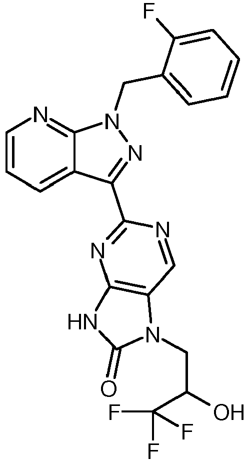

In Analogie zur Synthese von Beispiel 3 wurde Beispiel 99 aus 597 mg (1.261 mmol) Beispiel 77A hergestellt. Es wurden 42 mg der Titelverbindung erhalten (7 % d. Th.).

LC-MS (Methode 2): Rₜ = 0.96 min; MS (EIpos): m/z = 474 [M+H]⁺.

¹H-NMR (400 MHz, DMSO-d₆): δ [ppm] = 4.04-4.16 (m, 2H), 4.42-4.46 (m, 1H), 5.84 (s, 2H), 6.77 (d, 1H), 7.16 (t, 1H), 7.22-7.26 (m, 2H), 7.34-7.44 (m, 2H), 8.59 (s, 1H), 8.66 (dd, 1H), 8.87 (dd, 1H), 12.34 (s, 1H).

### Beispiel 100

### 7-(2,2-Difluorethyl)-2-[1-(2-fluorbenzyl)-1H-pyrazolo[3,4-b]pyridin-3-yl]-6-iod-7,9-dihydro-8H-purin-8-on

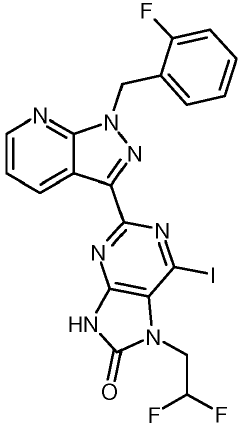

603 mg (1.369 mmol) Beispiel 4 wurden in Isopentylnitrit (3.96 ml) und Diiodmethan (10.53 ml) vorgelegt und anschliessend unter Rühren für 1h auf 85°C erhitzt. Danach wurde bis zur Trockene eingeengt und mit Acetonitril versetzt. Der unlösliche Rückstand wurde abfiltriert, mit Acetonitril nachgewaschen und getrocknet. Es wurden 227 mg der Titelverbindung erhalten (30 % d. Th.).

LC-MS (Methode 2): Rₜ = 1.12 min; MS (ESIpos): m/z = 552 (M+H)⁺

¹H-NMR (400 MHz, DMSO-d₆): δ [ppm] = 4.55 (m, 2H), 5.86 (s, 2H), 6.29-6.57 (m, 1H), 7.13-7.39 (m, 4H), 7.45-7.49 (m, 1H), 8.68 (dd, 1H), 8.81 (dd, 1H), 12.67 (s, 1H).

### Beispiel 101

### 4-Amino-6-[1-(2-fluorbenzyl)-1H-pyrazolo[3,4-b]pyridin-3-yl]-1H-pyrazolo[3,4-d]pyrimidin-3-ol

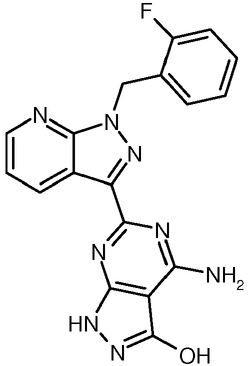

150 mg (0.357 mmol) Beispiel 21 wurden mit einem Überschuss Pyridinhydrochlorid (ca. 1g) versetzt und bei 155°C in der Schmelze über Nacht gerührt. Nach Abkühlung wurde der Rückstand mit Wasser und Acetonitril versetzt, kurz im Ultraschallbad behandelt und anschliessend abfiltriert. Der Rückstand wurde mit Wasser und Acetonitril gewaschen und im Vakuum getrocknet. Es wurden 136 mg der Titelverbindung erhalten (91 % d. Th., 90 %ige Reinheit).

LC-MS (Methode 2): Rₜ = 0.76 min; MS (ESIpos): m/z = 377 (M+H)⁺

¹H-NMR (400 MHz, DMSO-d₆): δ [ppm] = 5.90 (s, 2H), 7.14-7.27 (m, 3H), 7.35-7.40 (m, 1H), 7.48-7.52 (m, 1H), 8.72 (dd, 1H), 8.91 (dd, 1H).

### Beispiel 102

### 7-(2,2-Difluorethyl)-2-[1-(2-fluorbenzyl)-1H-pyrazolo[3,4-b]pyridin-3-yl]-7,9-dihydro-8H-purin-8-on

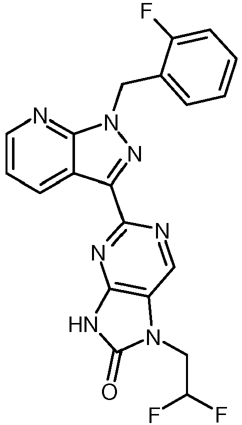

100 mg (0.181 mmol) Beispiel 100 wurden in Dimethylformamid (10 ml) mit 40 mg Palladium auf Kohle (10 %) versetzt und 4 h bei Wasserstoff-Normaldruck hydriert. Anschliessend wurde filtriert und bis zur Trockene eingeengt. Der Rückstand wurde mittels präparativer HPLC gereinigt (Acetonitril:Wasser (+0.05 % Ameisensäure) - Gradient). Es wurden 47 mg der Titelverbindung erhalten (68 % d. Th.).

LC-MS (Methode 2): Rₜ = 0.95 min; MS (ESIpos): m/z = 426 (M+H)⁺

¹H-NMR (400 MHz, DMSO-d₆): δ [ppm] = 4.39 (m, 2H), 5.84 (s, 2H), 6.26-6.56 (m, 1H), 7.14-7.44 (m, 5H), 8.57 (s, 1H), 8.66 (dd, 1H), 8.87 (dd, 1H), 12.47 (s br, 1H).

### Nicht erfindungsgemäßes Beispiel 103

### 7-(2,2-Difluorethyl)-2-[1-(2-fluorbenzyl)-1H-pyrazolo[3,4-b]pyridin-3-yl]-6-(methylamino)-7,9-dihydro-8H-purin-8-on

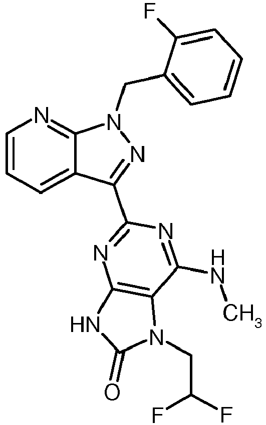

166 mg (0.301 mmol) Beispiel 100 in DMSO (13 ml) wurden mit 2.5 ml einer 2M Lösung von Methylamin in Methanol versetzt und in einem mikrowellengeeigneten Kolben mit Septum für 2.5 h bei 150°C in der Mikrowelle erhitzt. Anschliessend wurde mit Essigsäureethylester und Wasser versetzt und die Phasen getrennt. Die wässrige Phase wurde mit Essigsäureethylester zweimal extrahiert und die vereinigten organischen Phasen wurden mit Wasser und gesättigter wässriger Natriumchlorid-Lösung gewaschen. Es wurde mit Natriumsulfat getrocknet, filtriert und bis zur Trockene eingeengt. Der Rückstand wurde mittels präparativer HPLC gereinigt (Acetonitril:Wasser (+0.05 % Ameisensäure) - Gradient). Es wurden 33 mg der Titelverbindung erhalten (22 % d. Th.) werden.

LC-MS (Methode 2): Rₜ = 0.92 min; MS (ESIpos): m/z = 455 (M+H)⁺

¹H-NMR (400 MHz, DMSO-d₆): δ [ppm] = 3.10 (d, 3H), 4.50 (m, 2H), 5.82 (s, 2H), 6.09-6.38 (m, 1H), 6.67 (m, 1H), 7.12-7.25 (m, 3H), 7.31-7.42 (m, 2H), 8.64 (dd, 1H), 8.90 (dd, 1H), 11.85 (s br, 1H).

### Beispiel 104

### 7-(1-Cyclopropylpiperidin-4-yl)-2-[1-(2-fluorbenzyl)-1H-pyrazolo[3,4-b]pyridin-3-yl]-7,9-dihydro-8H-purin-8-on

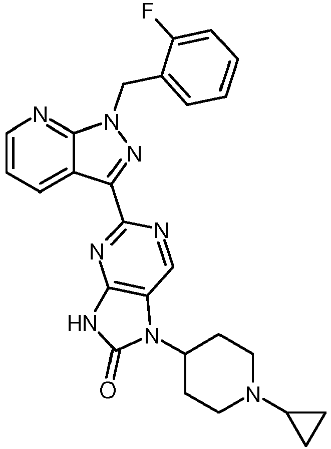

55 mg (0.12 mmol) Beispiel 34A und 27 mg (0.092 mmol) Bis(trichlormethyl)carbonat wurden in Dichlormethan (1.5 ml) vorgelegt und bei 0°C mit Pyridin (1.5 ml) versetzt. Nach 30 min Rühren bei 0°C wurde mit gesättigter wässriger Natriumhydrogencarbonat-Lösung versetzt, kurz kräftig gerührt und anschliessend dreimal mit Dichlormethan extrahiert. Die vereinigten organischen Phasen wurden über Natriumsulfat getrocknet, filtriert, eingeengt und danach mittels präparativer HPLC gereinigt (Acetonitril:Wasser (+0.05 % Ameisensäure) - Gradient). Es wurden 22 mg der Titelverbindung erhalten (37 % d. Th.).

LC-MS (Methode 2): Rₜ = 0.76 min; MS (EIpos): m/z = 485 [M+H]⁺.

¹H-NMR (400 MHz, DMSO-d₆): δ [ppm] = 0.86 (m, 2H), 1.12 (m, 2H), 2.05 (m, 2H), 2.59-2.89 (m, 5H covered by solvent), 3.65 (m, 2H), 4.63 (m, 1H), 5.84 (s, 2H), 7.15 (t, 1H), 7.21-7.26 (m, 2H), 7.35-7.38 (m, 1H), 7.42 (dd, 1H), 8.66 (dd, 1H), 8.80-8.87 (m, 2H), 12.31 (s br, 1H).

### Nicht erfindungsgemäßes Beispiel 105

### Methyl-8-amino-6-[1-(2-fluorbenzyl)-1H-pyrazolo[3,4-b]pyridin-3-yl]-3-oxo-3,4-dihydropyrimido[4,5-e][1,2,4]triazin-1 (2H)-carboxylat

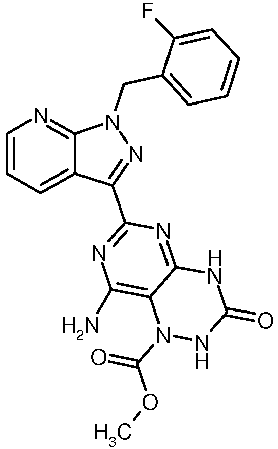

56 mg (0.132 mmol) der unter Beispiel 56A erhaltenen Verbindung wurden in 3 ml Dimethylformamid vorgelegt und mit 27.6 µl (0.159 mmol) N,N-Diisopropylethylamin und 25.7 mg (0.159 mmol) Carbonyldiimidazol versetzt. Nach 5 h bei RT wurden nochmals 27.6 µl (0.159 mmol) N,N-Diisopropylethylamin und 25.7 mg (0.159 mmol) Carbonyldiimidazol zugegeben und es wurde auf 60°C erhitzt. Am nächsten Tag wurden nochmals 27.6 µl (0.159 mmol) N,N-Diisopropylethylamin und 25.7 mg (0.159 mmol) Carbonyldiimidazol zugegeben und für 2 weitere Tage auf 60°C erhitzt. Die Reaktionsmischung wurde mittels präparativer HPLC gereinigt (Acetonitril:Wasser (+0.05 % Ameisensäure) - Gradient). Es wurden 25 mg der Titelverbindung erhalten (42 % d. Th.).

LC-MS (Methode 2): Rₜ = 0.84 min; MS (ESIpos): m/z = 450 (M+H)⁺

¹H-NMR (400 MHz, DMSO-d₆): δ [ppm] = 3.72 (s, 3H), 5.82 (s, 2H), 7.11-7.25 (m, 5H), 7.33-7.40 (m, 2H), 8.63 (dd, 1H), 9.07 (dd, 1H), 10.01 (s br, 1H), 10.36 (s br, 1H).

### Nicht erfindungsgemäßes Beispiel 106

### 7-Amino-5-[1-(2-fluorbenzyl)-1H-pyrazolo[3,4-b]pyridin-3-yl][1,3]oxazolo[4,5-d]pyrimidin-2(3H)-on

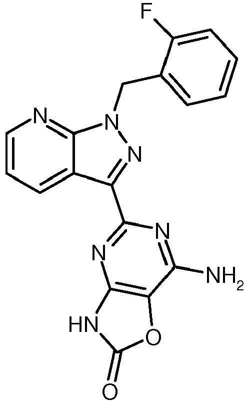

159 mg (0.453 mmol) der unter Beispiel 58A hergestellten Verbindung wurden in Dimethylformamid (10 ml) mit 94 µl (0.543 mmol) N,N-Diisopropylethylamin und 88 mg (0.543 mmol) Carbonyldiimidazol versetzt und über Nacht bei RT gerührt. Der Ansatz wurde filtriert, das Filtrat eingeengt und der Rückstand wurde mittels präparativer HPLC gereinigt (Acetonitril:Wasser (+0.05 % Ameisensäure) - Gradient). Es wurden 90 mg der Titelverbindung erhalten (52 % d. Th.).

LC-MS (Methode 2): Rₜ = 0.87 min; MS (ESIpos): m/z = 378 (M+H)⁺

¹H-NMR (400 MHz, DMSO-d₆): δ [ppm] = 5.81 (s, 2H), 7.12-7.25 (m, 3H), 7.33-7.41 (m, 4H), 8.64 (dd, 1H), 8.97 (dd, 1H), 12.38 (s br, 1H).

### Nicht erfindungsgemäßes Beispiel 107

### 2-[1-(2-Fluorbenzyl)-1H-pyrazolo[3,4-b]pyridin-3-yl]-6-iod-7-methyl-7,9-dihydro-8H-purin-8-on

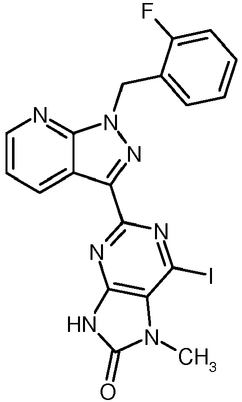

7.634 g (19.554 mmol) der unter Beispiel 41 erhaltenen Verbindung wurden in Analogie zu Beispiel 100 umgesetzt. Es wurden 5.63 g der Titelverbindung erhalten (57% d. Th.).

LC-MS (Methode 2): Rₜ = 1.08 min; MS (EIpos): m/z = 502 [M+H]⁺.

¹H-NMR (400 MHz, DMSO-d₆): δ [ppm] = 3.54 (s, 3H), 5.85 (s, 2H), 7.13-7.26 (m, 3H), 7.34-7.39 (m, 1H), 7.47 (dd, 1H), 8.67 (dd, 1H), 8.79 (dd, 1H), 12.41 (s, 1H).

### Nicht erfindungsgemäßes Beispiel 108

### 2-[1-(2-Fluorbenzyl)-1H-pyrazolo[3,4-b]pyridin-3-yl]-6-[(2-hydroxyethyl)amino]-7-methyl-7,9-dihydro-8H-purin-8-on

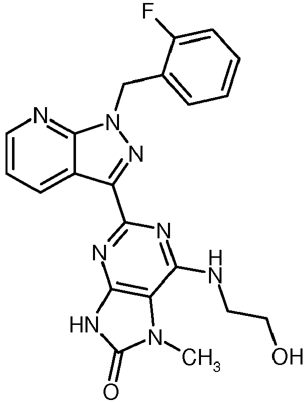

200 mg (0.399 mmol) der unter Beispiel 107 erhaltenen Verbindung wurden in N-Methylpyrrolidon (4 ml) gelöst und mit 2-Aminoethanol (1.5 ml) versetzt und anschliessend in einem mikrowellengeeigneten Kolben mit Septum für 5 h bei 150°C in der Mikrowelle erhitzt. Das Reaktionsgemisch wurde mittels präparativer HPLC gereinigt (Acetonitril:Wasser (+0.05 % Ameisensäure) - Gradient). Es wurden 31 mg der Titelverbindung erhalten (18 % d. Th.).

LC-MS (Methode 2): Rₜ = 0.83 min; MS (EIpos): m/z = 435 [M+H]⁺.

¹H-NMR (400 MHz, DMSO-d₆): δ [ppm] = 3.51 (s, 3H), 3.69 (m, 4H), 4.87 (m, 1H), 5.81 (s, 2H), 6.61 (m, 1H), 7.12-7.25 (m, 3H), 7.33-7.40 (m, 2H), 8.63 (dd, 1H), 8.84 (dd, 1H), 11.53 (br s, 1H).

### Nicht erfindungsgemäßes Beispiel 109

### 2-[1-(2-Fluorbenzyl)-1H-pyrazolo[3,4-b]pyridin-3-yl]-6-isopropoxy-7-methyl-7,9-dihydro-8H-purin-8-on

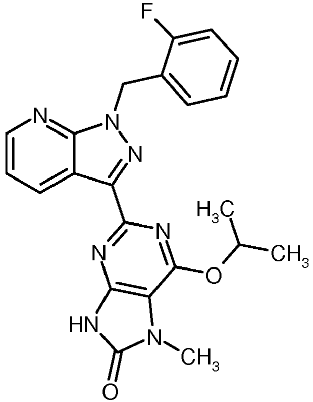

200 mg (0.399 mmol) der unter Beispiel 107 erhaltenen Verbindung wurden in einem mikrowellengeeigneten Kolben mit Isopropanol (3 ml), 260 mg (0.798 mmol) Cäsiumcarbonat, 8 mg (0.04 mmol) Kupfer-(I)-iodid und 19 mg (0.08 mmol) 3,4,7,8-Tetramethyl-1,10-Phenanthrolin versetzt. Es wurde unter Ultraschallbehandlung für 5 min mit Argon gespült und anschliessend mit einem entsprechenden Septum verschlossen. Danach wurde für 2 h bei 140°C in der Mikrowelle erhitzt. Nach Reaktionskontrolle zeigte sich geringer Umsatz. Es wurde daher mit N-methylpyrrolidon (1 ml) versetzt und nochmal für 2 h bei 140°C und anschliessend für 8 h bei 180 °C in der Mikrowelle erhitzt. Nach Abkühlen wurde das Reaktionsgemisch filtriert, eingeengt und der Rückstand wurde mittels präparativer HPLC gereinigt (Acetonitril:Wasser (+0.05 % Ameisensäure) - Gradient). Es wurden 21 mg der Titelverbindung erhalten (12 % d. Th.).

LC-MS (Methode 2): Rₜ = 1.13 min; MS (EIpos): m/z = 434 [M+H]⁺.

¹H-NMR (400 MHz, DMSO-d₆): δ [ppm] = 1.47 (d, 6H), 3.44 (s, 3H), 5.59 (sep, 1H), 5.84 (s, 2H), 7.15 (t, 1H), 7.20-7.26 (m, 2H), 7.33-7.39 (m, 1H), 7.43 (dd, 1H), 8.66 (dd, 1H), 8.80 (dd, 1H), 11.98 (s br, 1H).

### Nicht erfindungsgemäßes Beispiel 110

### 2-[1-(2-Fluorbenzyl)-1H-pyrazolo[3,4-b]pyridin-3-yl]-7-methyl-8-oxo-8,9-dihydro-7H-purin-6-carbonitril

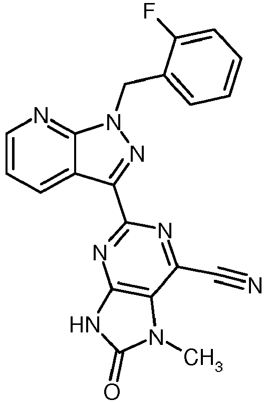

200 mg (0.399 mmol) der unter Beispiel 107 erhaltenen Verbindung in Pyridin (3 ml) wurden mit 37 mg (0.419 mmol) Kupfer-(I)-cyanid versetzt und über Nacht zum Rückfluss erhitzt. Nach Abkühlen wurde das Reaktionsgemisch filtriert, eingeengt und der Rückstand wurde mittels präparativer HPLC gereinigt (Acetonitril:Wasser (+0.05 % Ameisensäure) - Gradient). Es wurden 31 mg der Titelverbindung erhalten (18 % d. Th.).

LC-MS (Methode 2): Rₜ = 0.99 min; MS (EIpos): m/z = 401 [M+H]⁺.

¹H-NMR (400 MHz, DMSO-d₆): δ [ppm] = 3.55 (s, 3H), 5.85 (s, 2H), 7.15 (t, 1H), 7.20-7.26 (m, 2H), 7.33-7.39 (m, 1H), 7.45 (dd, 1H), 8.67 (dd, 1H), 8.82 (dd, 1H), 12.88 (s br, 1H).

### Nicht erfindungsgemäßes Beispiel 111

### 2-[1-(2-Fluorbenzyl)-1H-pyrazolo[3,4-b]pyridin-3-yl]-6-methoxy-7-methyl-7,9-dihydro-8H-purin-8-on

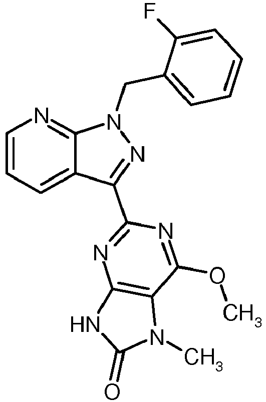

200 mg (0.399 mmol) der unter Beispiel 107 erhaltenen Verbindung wurden in einem mikrowellengeeigneten Kolben mit Methanol (3 ml), N-Methylpyrrolidon (1 ml), 260 mg (0.798 mmol) Cäsiumcarbonat, 8 mg (0.04 mmol) Kupfer-(I)-iodid und 19 mg (0.08 mmol) 3,4,7,8-Tetramethyl-1,10-Phenanthrolin versetzt. Es wurde unter Ultraschallbehandlung für 5 min mit Argon gespült und anschliessend mit einem entsprechenden Septum verschlossen. Danach wurde für 8 h bei 180 °C in der Mikrowelle erhitzt. Nach Abkühlen wurde das Reaktionsgemisch filtriert, eingeengt und der Rückstand wurde mittels präparativer HPLC gereinigt (Acetonitril:Wasser (+0.05 % Ameisensäure) - Gradient). Es wurden 17 mg der Titelverbindung erhalten (10 % d. Th.).

LC-MS (Methode 2): Rₜ = 0.98 min; MS (EIpos): m/z = 406 [M+H]⁺.

¹H-NMR (400 MHz, DMSO-d₆): δ [ppm] = 3.44 (s, 3H), 4.19 (s, 3H), 5.84 (s, 2H), 7.13-7.26 (m, 3H), 7.33-7.39 (m, 1H), 7.42 (dd, 1H), 8.66 (dd, 1H), 8.90 (dd, 1H), 12.02 (s br, 1H).

### Nicht erfindungsgemäßes Beispiel 112

### 6-(Azetidin-1-yl)-2-[1-(2-fluorbenzyl)-1H-pyrazolo[3,4-b]pyridin-3-yl]-7-methyl-7,9-dihydro-8H-purin-8-on

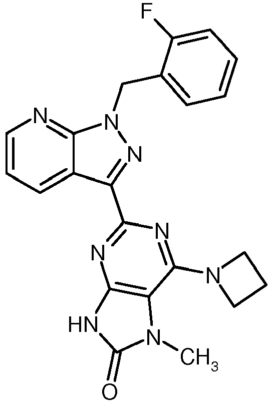

200 mg (0.399 mmol) der unter Beispiel 107 erhaltenen Verbindung wurden in N-Methylpyrrolidon (3 ml) gelöst und mit 1.00 g (17.514 mmol) Azetidin versetzt und anschliessend in einem mikrowellengeeigneten Kolben mit Septum für 5 h bei 150°C in der Mikrowelle erhitzt. Der Ansatz wurde mittels präparativer HPLC gereinigt (Acetonitril:Wasser (+0.05 % Ameisensäure) - Gradient). Es wurden 9 mg der Titelverbindung erhalten (5 % d. Th.).

LC-MS (Methode 2): Rₜ = 1.00 min; MS (EIpos): m/z = 431 [M+H]⁺.

¹H-NMR (400 MHz, DMSO-d₆): δ [ppm] = 2.42 (m, 2H), 3.38 (s, 3H), 4.37 (t, 4H), 5.81 (s, 2H), 7.12-7.25 (m, 3H), 7.33-7.40 (m, 2H), 8.63 (dd, 1H), 8.86 (dd, 1H), 11.66 (br s, 1H).

### Beispiel 113

### 2-[1-(2-Fluorbenzyl)-1H-pyrazolo[3,4-b]pyridin-3-yl]-7-(2-hydroxyethyl)-7,9-dihydro-8H-purin-8-on

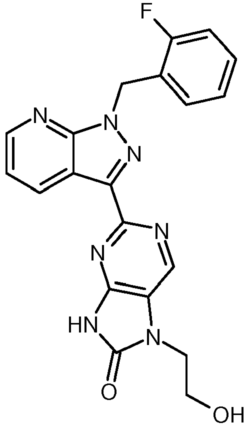

232 mg (0.359 mmol) der unter Beispiel 60A erhaltenen Verbindung wurden analog zu Beispiel 102 hydriert. Das Reaktionsgemisch wurde mittels präparativer HPLC gereinigt (Acetonitril:Wasser (+0.05 % Ameisensäure) - Gradient). Es wurden 14 mg der Titelverbindung erhalten (9 % d. Th.).

LC-MS (Methode 2): Rₜ = 0.81 min; MS (ESIpos): m/z = 406 (M+H)⁺

¹H-NMR (400 MHz, DMSO-d₆): δ [ppm] = 3.69 (br signal, 2H), 3.92 (t, 2H), 4.96 (br signal, 1H), 5.83 (s, 2H), 7.15 (t, 1H), 7.21-7.26 (m, 2H), 7.34-7.43 (m, 2H), 8.52 (s, 1H), 8.65 (dd, 1H), 8.88 (dd, 1H).

### Nicht erfindungsgemäßes Beispiel 114

### 2-[1-(2-Fluorbenzyl)-1H-pyrazolo[3,4-b]pyridin-3-yl]-6-(hydroxymethyl)-7,9-dihydro-8H-purin-8-on

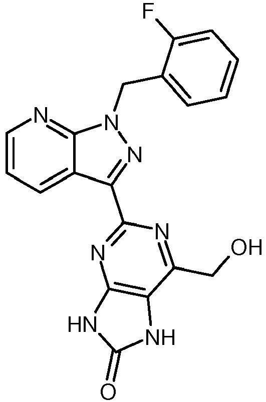

Unter einer Argon-Atmosphäre wurden 76 mg (0.18 mmol) der Verbindung aus Beispiel 118 in Tetrahydrofuran (1.8 mL) vorgelegt. Bei 0°C wurden anschließend 0.26 mL einer 1M Lösung von Lithiumaluminiumhydrid in Tetrahydrofuran zugetropft und die Mischung weitere 30 min bei 0°C gerührt. Die Reaktionsmischung wurde tropfenweise mit Wasser und schließlich mit Natronlauge (1.0 M) versetzt. Danach wurde auf RT erwärmt und weitere 30 min nachgerührt. Anschließend wurde gesättigte wässrige Natriumchlorid-Lösung zugefügt, der entstandene Niederschlag abfiltriert und mit Wasser gewaschen. Weitere Trocknung am Hochvakuum lieferte 51 mg (71% d. Th.) der Titelverbindung als Feststoff.

LC-MS (Methode 3): Rₜ = 0.90 min

MS (ESIpos): m/z = 392 (M+H)⁺

¹H-NMR (400MHz, DMSO-d₆): δ [ppm]= 4.60 (s, 2H), 5.21 (br. s, 1H), 5.80 (s, 2H), 7.08 - 7.18 (m, 2H), 7.19 - 7.28 (m, 1H), 7.29 - 7.41 (m, 2H), 8.54 - 8.63 (m, 1H), 8.96 - 9.06 (m, 1H).

### Beispiel 115

### 6-Amino-2-[1-(2-fluorbenzyl)-1H-pyrazolo[3,4-b]pyridin-3-yl]-7-isopropyl-7,9-dihydro-8H-purin-8-on

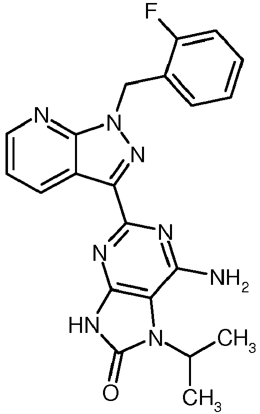

In Dimethylformamid (5 mL) wurden 520 mg (1.33 mmol) der Verbindung aus Beispiel 61A vorgelegt, anschließend 1.07 g (6.63 mmol) N,N-Carbonyldiimidazol sowie 2.31 mL (1.68 mmol) Triethylamin zugefügt und die Mischung über Nacht bei 100°C gerührt. Die Reaktionsmischung wurde anschließend mit Wasser sowie Essigsäureethylester verdünnt, die Phasen getrennt und die wässrige Phase mit Essigsäureethylester extrahiert. Die gesammelten organischen Phasen wurden über Natriumsulfat getrocknet, filtriert und eingeengt. Das Rohprodukt (540 mg, 84%-ige Reinheit) wurde nicht weiter aufgereinigt.

LC-MS (Methode 2): Rₜ = 0.90 min

MS (ESIpos): m/z = 419 (M+H)⁺

¹H-NMR (400MHz, DMSO-d₆): δ [ppm]= 1.48 (d, 6H), 4.55 - 4.65 (m, 1H), 5.80 (s, 2H), 6.66 - 6.71 (m, 2H), 7.11 - 7.27 (m, 3H), 7.34 - 7.39 (m, 2H), 8.61 - 8.65 (m, 1H), 9.05 - 9.09 (m, 1H), 11.51-11.57 (m, 1H).

### Nicht erfindungsgemäßes Beispiel 116

### 2-[1-(2-Fluorbenzyl)-1H-pyrazolo[3,4-b]pyridin-3-yl]-6-iod-7-isopropyl-7,9-dihydro-8H-purin-8-on

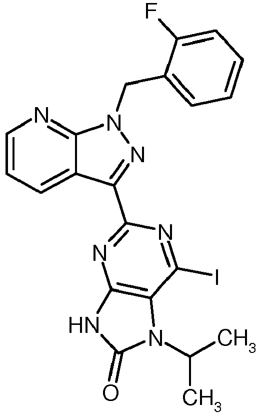

Zu einer Mischung aus 2.59 mL (32.20 mmol) Diiodmethan und 2.78 mL (20.61 mmol) Isoamylnitrit wurden 539 mg (ca. 1.08 mmol) der Verbindung aus Beispiel 115 addiert und die Reaktionsmischung über Nacht bei 85°C gerührt. Die Mischung wurde eingeengt, der Rückstand mit Dichlormethan verrührt und filtriert. Der Feststoff wurde mit Dichlormethan gewaschen und am Hochvakuum getrocknet. Es wurden 330 mg der Titelverbindung erhalten (92%-ige Reinheit, 53% d. Th.).

LC-MS (Methode 2): Rₜ = 1.21 min

MS (ESIpos): m/z = 530 (M+H)⁺

¹H-NMR (400MHz, DMSO-d₆): δ [ppm]= 1.55 (d, 6H), 5.22 - 5.32 (m, 1H), 5.85 (s, 2H), 7.11 - 7.28 (m, 3H), 7.32 - 7.40 (m, 1H), 7.43 - 7.51 (m, 1H), 8.65 - 8.71 (m, 1H), 8.77 - 8.83 (m, 1H), 12.29 - 12.35 (m, 1H).

### Beispiel 117

### 2-[1-(2-Fluorbenzyl)-1H-pyrazolo[3,4-b]pyridin-3-yl]-7-isopropyl-7,9-dihydro-8H-purin-8-on

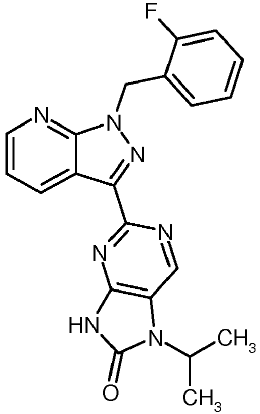

In Dimethylformamid (10.0 mL) wurden 330 mg (0.57 mmol) der Verbindung aus Beispiel 116 vorgelegt, anschließend 66 mg Palladium (10% w/w auf Kohle) zugefügt und die Mischung zwei Tage bei Wasserstoff-Normaldruck hydriert. Anschließend wurde erneut 66 mg Palladium (10% w/w auf Kohle) zugefügt und weiter über Nacht hydriert. Danach wurde die Mischung über Celite filtriert, mit Methanol nachgewaschen und das Filtrat eingeengt. Präparative HPLC (Laufmittel: Acetonitril-Wasser mit 0.1% Ameisensäure Gradient) des Rückstands lieferte 23 mg (90%-ige Reinheit, 9% d. Th.) der Titelverbindung als Feststoff.

LC-MS (Methode 2): Rₜ = 1.00 min

MS (ESIpos): m/z = 404 (M+H)⁺

¹H-NMR (400MHz, DMSO-d₆): δ [ppm]= 1.48 (d, 6H), 4.57 - 4.65 (m, 1H), 5.82 - 5.86 (m, 2H), 7.12 - 7.18 (m, 1H), 7.20 - 7.27 (m, 2H), 7.33 - 7.40 (m, 1H), 7.40 - 7.44 (m, 1H), 8.65 - 8.67 (m, 1H), 8.70 (s, 1H), 8.85 - 8.89 (m, 1H), 12.16 - 12.20 (m, 1H).

### Nicht erfindungsgemäßes Beispiel 118

### Ethyl-2-[1-(2-fluorbenzyl)-1H-pyrazolo[3,4-b]pyridin-3-yl]-8-oxo-8,9-dihydro-7H-purin-6-carboxylat

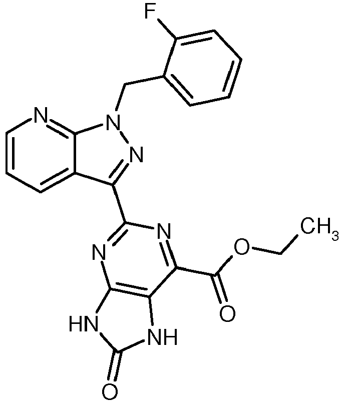

In Dimethylformamid (2.5 mL) wurden 225 mg (0.55 mmol) der Verbindung aus Beispiel 63A vorgelegt, anschließend 448 mg (2.76 mmol) N,N-Carbonyldiimidazol sowie 0.96 mL (6.90 mmol) Triethylamin zugefügt und die Mischung über Nacht bei 100°C gerührt. Die Reaktionsmischung wurde anschließend mit Wasser sowie Essigsäureethylester verdünnt und filtriert. Der Feststoff wurde mit Wasser gewaschen und lieferte nach Trocknung am Hochvakuum 112 mg (46% d. Th.) der Titelverbindung.

LC-MS (Methode 2): Rₜ = 0.95 min

MS (ESIpos): m/z = 434 (M+H)⁺

¹H-NMR (400MHz, DMSO-d₆): δ [ppm]= 1.40 (t, 3H), 4.48 (q, 2H), 5.82 - 5.88 (m, 2H), 7.12 - 7.18 (m, 1H), 7.19 - 7.27 (m, 2H), 7.33 - 7.40 (m, 1H), 7.45 (dd, 1H), 8.65 - 8.69 (m, 1H), 8.99 - 9.03 (m, 1H), 11.54 (br. s, 1H), 12.32 (br. s, 1H).

### Nicht erfindungsgemäßes Beispiel 119

### 2-[1-(2-Fluorbenzyl)-1H-pyrazolo[3,4-b]pyridin-3-yl]-6-methyl-7,9-dihydro-8H-purin-8-on

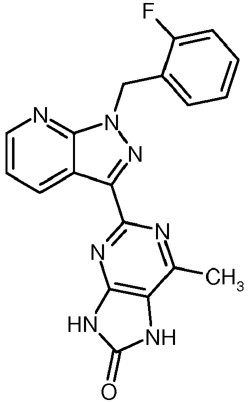

Unter einer Argon-Atmosphäre wurden 245 mg (0.60 mmol) der Verbindung aus Beispiel 69A in Tetrahydrofuran (2.6 mL) vorgelegt, auf 0°C gekühlt und mit 0.90 mL (0.90 mmol) Bis-(trimethylsilyl)natriumamid (1.0 M in Tetrahydrofuran) tropfenweise versetzt. Es wurde für 1 h bei 0°C und über Nacht bei RT gerührt. Anschließend wurde 8 h bei 60°C gerührt. Danach wurde Wasser zugefügt und die Reaktionsmischung eingeengt. Der Rückstand wurde zweimal mit Essigsäureethylester extrahiert, die gesammelten organischen Phasen mit Wasser sowie gesättigter wässriger Natriumchlorid-Lösung gewaschen, über Magnesiumsulfat getrocknet, filtriert und eingeengt. Man erhielt 110 mg (85%-ige Reinheit, 41% d. Th.) der Titelverbindung als Feststoff. Aus der wässrigen Phase fiel ebenfalls ein Feststoff aus, der abfiltriert, mit Wasser gewaschen und am Hochvakuum getrocknet wurde. Dies lieferte weitere 100 mg (44 % d. Th.) der Titelverbindung.

LC-MS (Methode 2): Rₜ = 0.87 min; MS (EIpos): m/z = 376 [M+H]⁺.

¹H-NMR (400MHz, DMSO-d₆): δ [ppm]= 2.48 (s, 3H), 5.81 (s, 2H), 7.10 - 7.27 (m, 3H), 7.32 - 7.41 (m, 2H), 8.60 - 8.64 (m, 1H), 8.90 - 8.96 (m, 1H), 11.25 (br. s, 2H).

### Beispiel 120

### 6-Amino-7-(2,2-dimethylpropyl)-2-[1-(2-fluorbenzyl)-1H-pyrazolo[3,4-b]pyridin-3-yl]-7,9-dihydro-8H-purin-8-on

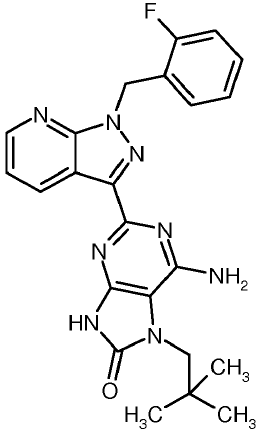

In Dimethylformamid (5 mL) wurden 447 mg (0.94 mmol) der Verbindung aus Beispiel 64A vorgelegt, anschließend 758 mg (4.68 mmol) N,N-Carbonyldiimidazol sowie 1.57 mL (11.23 mmol) Triethylamin zugefügt und die Mischung über Nacht bei 100°C gerührt. Die Reaktionsmischung wurde direkt mittels präparativer HPLC (Laufmittel: Methanol-Wasser mit 0.1% Ameisensäure Gradient) gereinigt. Man erhielt 163 mg (39% d. Th.) der Titelverbindung als Feststoff.

LC-MS (Methode 2): Rₜ = 0.99 min

MS (ESIpos): m/z = 447 (M+H)⁺

¹H-NMR (400MHz, DMSO-d₆): δ [ppm]= 0.94 (s, 9H), 3.83 (s, 2H), 5.80 (s, 2H), 6.73 (s, 2H), 7.11 - 7.26 (m, 3H), 7.32 - 7.41 (m, 2H), 8.61 - 8.65 (m, 1H), 9.04 - 9.08 (m, 1H), 11.65 (s, 1H).

### Beispiel 121

### 6-Amino-7-cyclobutyl-2-[1-(2-fluorbenzyl)-1H-pyrazolo[3,4-b]pyridin-3-yl]-7,9-dihydro-8H-purin-8-on

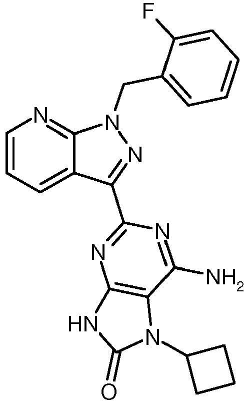

In Dimethylformamid (1.4 mL) wurden 133 mg (0.26 mmol) der Verbindung aus Beispiel 65A vorgelegt, anschließend 213 mg (1.32 mmol) N,N-Carbonyldiimidazol sowie 0.44 mL (3.16 mmol) Triethylamin zugefügt und die Mischung über Nacht bei 100°C gerührt. Danach wurden 200 mg (1.23 mmol) N,N-Carbonyldiimidazol nachgegeben und erneut über Nacht bei 100°C gerührt. Die Reaktionsmischung wurde direkt mittels präparativer HPLC (Laufmittel: Acetonitril-Wasser mit 0.1% Ameisensäure Gradient) gereinigt. Man erhielt 79 mg (68% d. Th.) der Titelverbindung als Feststoff.

LC-MS (Methode 2): Rₜ = 0.94 min

MS (ESIpos): m/z = 431 (M+H)⁺

¹H-NMR (400MHz, DMSO-d₆): δ [ppm]= 1.63 - 1.81 (m, 2H), 2.20 - 2.30 (m, 2H), 2.92 - 3.04 (m, 2H), 4.78 - 4.88 (m, 1H), 5.80 (s, 2H), 6.74 (s, 2H), 7.11 - 7.26 (m, 3H), 7.32 - 7.39 (m, 2H), 8.61 - 8.64 (m, 1H), 9.03 - 9.08 (m, 1H), 11.58 (s, 1H).

### Beispiel 122

### 7-(2,2-Dimethylpropyl)-2-[1-(2-fluorbenzyl)-1H-pyrazolo[3,4-b]pyridin-3-yl]-7,9-dihydro-8H-purin-8-on

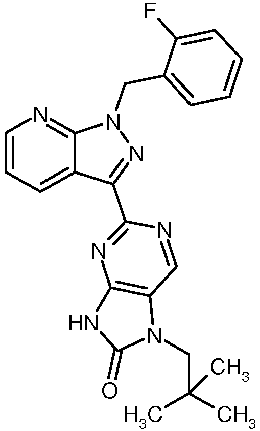

In Tetrahydrofuran (10 mL) wurden 100 mg (0.22 mmol) der Verbindung aus Beispiel 120 vorgelegt, anschließend 0.21 mL (1.57 mmol) Isoamylnitrit sowie 6.0 mg (0.05 mmol) Kupfer(II)chlorid zugefügt und die Mischung über Nacht bei RT gerührt. Die Reaktionsmischung wurde mit gesättigter wässriger Natriumhydrogencarbonat-Lösung versetzt und zweimal mit Essigsäureethylester extrahiert. Die gesammelten organischen Phasen wurden über Natriumsulfat getrocknet, filtriert und eingeengt. Der Rückstand wurde mittels präparativer HPLC (Laufmittel: Acetonitril-Wasser mit 0.1% Ameisensäure Gradient) gereinigt. Man erhielt 34 mg (34% d. Th.) der Titelverbindung als Feststoff.

LC-MS (Methode 2): Rₜ = 1.12 min

MS (ESIpos): m/z = 432 (M+H)⁺

¹H-NMR (400MHz, DMSO-d₆): δ [ppm]= 0.99 (s, 9H), 3.66 (s, 2H), 5.84 (s, 2H), 7.12 - 7.18 (m, 1H), 7.20 - 7.26 (m, 2H), 7.33 - 7.39 (m, 1H), 7.39 - 7.44 (m, 1H), 8.56 (s, 1H), 8.64 - 8.67 (m, 1H), 8.86 - 8.90 (m, 1H), 12.21 (br. s, 1H).

### Beispiel 123

### 6-Amino-7-(2-fluorbenzyl)-2-[1-(2-fluorbenzyl)-1H-pyrazolo[3,4-b]pyridin-3-yl]-7,9-dihydro-8H-purin-8-on

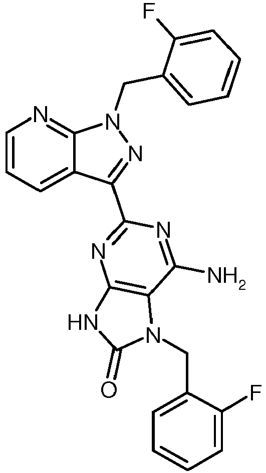

In Dimethylformamid (2.6 mL) wurden 268 mg (0.48 mmol) der Verbindung aus Beispiel 66A vorgelegt, anschließend 389 mg (2.40 mmol) N,N-Carbonyldiimidazol sowie 0.80 mL (5.75 mmol) Triethylamin zugefügt und die Mischung über Nacht bei 100°C gerührt. Anschließend wurden 380 mg (2.34 mmol) N,N-Carbonyldiimidazol zugefügt und erneut bei 100°C über Nacht gerührt. Die Reaktionsmischung wurde direkt mittels präparativer HPLC (Laufmittel: Acetonitril-Wasser mit 0.1 % Ameisensäure Gradient) gereinigt. Man erhielt 130 mg (56% d. Th.) der Titelverbindung.

LC-MS (Methode 4): Rₜ = 2.05 min; MS (ESIpos): m/z = 485 [M+H]⁺.

### Beispiel 124

### 7-(2-Fluorbenzyl)-2-[1-(2-fluorbenzyl)-1H-pyrazolo[3,4-b]pyridin-3-yl]-7,9-dihydro-8H-purin-8-on

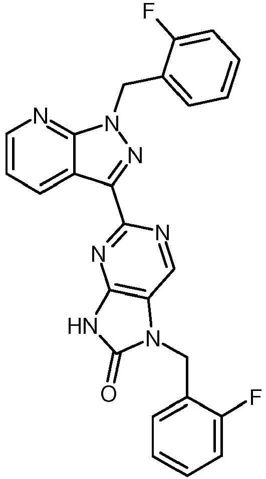

In Tetrahydrofuran (7 mL) wurden 80 mg (0.17 mmol) der Verbindung aus Beispiel 124 vorgelegt, anschließend 0.16 mL (1.16 mmol) Isoamylnitrit sowie 4 mg (0.03 mmol) Kupfer(II)chlorid zugefügt und die Mischung über Nacht bei RT gerührt. Die Reaktionsmischung wurde mit gesättigter wässriger Natriumhydrogencarbonat-Lösung versetzt und zweimal mit Essigsäureethylester extrahiert. Die gesammelten organischen Phasen wurden über Natriumsulfat getrocknet, filtriert und eingeengt. Der Rückstand wurde mittels präparativer HPLC (Laufmittel: Acetonitril-Wasser mit 0.1% Ameisensäure Gradient) gereinigt. Man erhielt 15 mg (19% d. Th.) der Titelverbindung als Feststoff.

LC-MS (Methode 2): Rₜ = 1.10 min

MS (ESIpos): m/z = 470 (M+H)⁺

¹H-NMR (400MHz, DMSO-d₆): δ [ppm]= 5.16 (s, 2H), 5.83 (s, 2H), 7.12 - 7.18 (m, 1H), 7.19 - 7.28 (m, 4H), 7.32 - 7.42 (m, 4H), 8.40 (s, 1H), 8.64 - 8.67 (m, 1H), 8.84 - 8.88 (m, 1H), 12.35 (br. s, 1H).

### Beispiel 125

### 7-Cyclobutyl-2-[1-(2-fluorbenzyl)-1H-pyrazolo[3,4-b]pyridin-3-yl]-7,9-dihydro-8H-purin-8-on

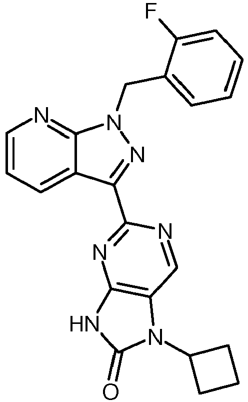

In Tetrahydrofuran (5 mL) wurden 50 mg (0.12 mmol) der Verbindung aus Beispiel 121 vorgelegt, anschließend 0.11 mL (0.81 mmol) Isoamylnitrit sowie 3 mg (0.02 mmol) Kupfer(II)chlorid zugefügt und die Mischung über Nacht bei RT gerührt. Die Reaktionsmischung wurde mit gesättigter wässriger Natriumhydrogencarbonat-Lösung versetzt und zweimal mit Essigsäureethylester extrahiert. Die gesammelten organischen Phasen wurden über Natriumsulfat getrocknet, filtriert und eingeengt. Der Rückstand wurde mittels präparativer HPLC (Laufmittel: Acetonitril-Wasser mit 0.1% Ameisensäure Gradient) gereinigt. Man erhielt 7 mg (13% d. Th.) der Titelverbindung als Feststoff.

LC-MS (Methode 2): Rₜ = 1.05 min

MS (ESIpos): m/z = 416 (M+H)⁺

¹H-NMR (400MHz, DMSO-d₆): δ [ppm]= 1.74 - 1.87 (m, 1H), 1.87 - 1.97 (m, 1H), 2.28 - 2.38 (m, 2H), 2.69 - 2.82 (m, 2H), 4.79 - 4.90 (m, 1H), 5.84 (s, 2H), 7.13 - 7.18 (m, 1H), 7.21 - 7.27 (m, 2H), 7.33 - 7.40 (m, 1H), 7.40 - 7.45 (m, 1H), 8.65 - 8.68 (m, 1H), 8.75 (s, 1H), 8.86 - 8.90 (m, 1H), 12.20 (br. s, 1H).

### Nicht erfindungsgemäßes Beispiel 126

### 6-Chlor-7-ethyl-2-[1-(2-fluorbenzyl)-1H-pyrazolo[3,4-b]pyridin-3-yl]-7,9-dihydro-8H-purin-8-on

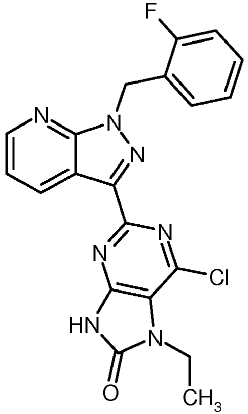

In Tetrahydrofuran (20 mL) wurden 428 mg (1.06 mmol) der Verbindung aus Beispiel 42 vorgelegt, anschließend 1.0 mL (7.41 mmol) Isoamylnitrit sowie 28 mg (0.21 mmol) Kupfer(II)chlorid zugefügt und die Mischung über Nacht bei RT gerührt. Anschließend wurden erneut 1.0 mL (7.41 mmol) Isoamylnitrit sowie 28 mg (0.21 mmol) Kupfer(II)chlorid zugefügt und die Mischung weiter über Nacht bei RT gerührt. Die Reaktionsmischung wurde mit gesättigter wässriger Natriumhydrogencarbonat-Lösung versetzt und zweimal mit Essigsäureethylester extrahiert. Die gesammelten organischen Phasen wurden über Natriumsulfat getrocknet, filtriert und eingeengt. Der Rückstand wurde an Kieselgel (Laufmittel: Dichlormethan/Methanol 40:1, 20:1) chromatographiert und mittels präparativer HPLC (Laufmittel: Acetonitril-Wasser mit 0.1% Ameisensäure Gradient) weiter gereinigt. Man erhielt 195 mg (57%-ige Reinheit, 25% d. Th.) der Titelverbindung, die ohne weitere Reinigung umgesetzt wurden.

LC-MS (Methode 3): Rₜ = 1.23 min

MS (ESIpos): m/z = 424 (M+H)⁺

### Beispiel 127

### 7-Ethyl-2-[1-(2-fluorbenzyl)-1H-pyrazolo[3,4-b]pyridin-3-yl]-7,9-dihydro-8H-purin-8-on

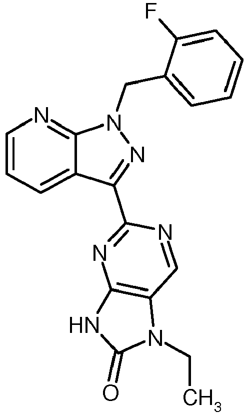

In Pyridin (2.4 mL) wurden 195 mg (0.22 mmol) der Verbindung aus Beispiel 126 vorgelegt, anschließend 24 mg (0.02 mmol) Palladium (10%ig auf Kohle) zugefügt und die Mischung über Nacht bei RT unter Wasserstoff-Normaldruck hydriert. Die Reaktionsmischung wurde über Celite filtriert, der Filterkuchen mit Methanol nachgewaschen und das Filtrat eingeengt. Der Rückstand wurde erneut in Pyridin (5.0 mL) aufgenommen, mit 59 mg (0.06 mmol) Palladium (10%ig auf Kohle) versetzt und über Nacht bei RT unter Wasserstoff-Normaldruck hydriert. Danach wurde die Reaktionsmischung durch Celite filtriert, der Filterrückstand mit Methanol nachgewaschen und das Filtrat eingeengt. Der Rückstand wurde mittels präparativer HPLC (Laufmittel: Acetonitril-Wasser mit 0.1% Ameisensäure Gradient) getrennt und die Produktfraktionen eingeengt. Das so erhaltene Rohprodukt wurde mit Dichlormethan verrührt, der Feststoff abfiltriert und am Hochvakuum getrocknet. Man erhielt 18 mg (21% d. Th.) der Titelverbindung als Feststoff.

LC-MS (Methode 2): Rₜ = 0.95 min

MS (ESIpos): m/z = 390 (M+H)⁺

¹H-NMR (400MHz, DMSO-d₆): δ [ppm]= 1.28 (t, 3H), 3.90 (q, 2H), 5.83 (s, 2H), 7.12 - 7.29 (m, 3H), 7.32 - 7.46 (m, 2H), 8.58 (s, 1H), 8.63 - 8.69 (m, 1H), 8.88 (d, 1H), 12.11 (br. s, 1H).

### Nicht erfindungsgemäßes Beispiel 128

### 6-[1-(2-Fluorbenzyl)-1H-pyrazolo[3,4-b]pyridin-3-yl]-2H-pyrazolo[3,4-b]pyrazin-3-ol

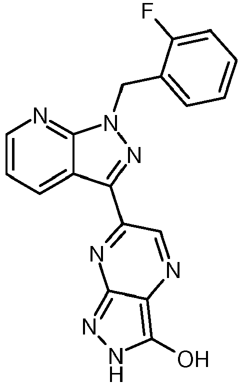

Es wurden 500 mg (1.291 mmol) der Verbindung aus Beispiel 68A mit 7 ml Essigsäure und 273 mg (1.291 mmol) 3,4-Diamino-1H-pyrazol-5-ol-Sulfat versetzt. Es wurde für 16 h bei RT gerührt und anschleißend das Gemisch am Rotationsverdampfer eingeengt. Es wurde Wasser und Essigsäureethylester addiert und verrührt. Die organische Phase wurde abgetrennt und einmal mit einer 1N Natronlauge extrahiert, über Natriumsulfat getrocknet und am Rotationsverdampfer eingeengt. Der Rückstand wurde mit präparativer HPLC gereinigt (Eluent: Wasser/Acetonitril/Wasser mit 1% Trifluoressigsäure, Verhältnis 80:15:5). Es wurden 142 mg der Titelverbindung erhalten (29 % d. Th.).

LC-MS (Methode 3): Rₜ = 1.03 min; MS (EIpos): m/z = 362 [M+H]⁺.

¹H-NMR (400 MHz, DMSO-d₆): δ [ppm] = 5.86 (s, 2H), 7.14-7.18 (m, 1H), 7.23-7.28 (m, 2H), 7.33-7.39 (m, 1H), 7.45 (dd, 1H), 8.70 (dd, 1H), 9.04 (dd, 1H), 9.20 (s, 1H), 11.39 (s br, 1H), 12.83 (s, 1H).

### Nicht erfindungsgemäßes Beispiel 129

### 2-[1-(2-Fluorbenzyl)-1H-pyrazolo[3,4-b]pyridin-3-yl]-6-iod-7-methyl-7,9-dihydro-8H-purin-8-on

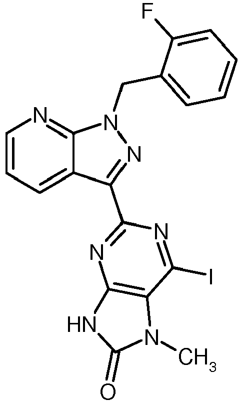

Es wurden 1.725 g (3.358 mmol) der Verbindung aus Beispiel 41 in 7 ml Diiodmethan gelöst und mit 7.19 ml (53.732 mmol) Isopentylnitrit versetzt. Es wurde für 16 h auf 85°C erhitzt und nach dem Abkühlen das Reaktionsgemisch am Rotationsverdampfer eingeengt. Der Rückstand wurde mittels präparativer HPLC (Eluent: Wasser/Acetonitril/Wasser mit 1% Trifluoressigsäure, Gradient 55:40:5 → 0:95:5). Es wurden 64 mg der Titelverbindung erhalten (4% d. Th.).

LC-MS (Methode 3): Rₜ = 1.22 min; MS (EIpos): m/z = 502 [M+H]⁺.

¹H-NMR (400 MHz, DMSO-d₆): δ [ppm] = 3.54 (s, 3H), 5.85 (s, 2H) 7.13-7.17 (m, 1H), 7.17-7.26 (m, 2H), 7.34-7.39 (m, 1H), 7.46 (dd, 1H), 8.68 (dd, 1H), 8.80 (dd, 1H), 12.41 (s, 1H).

### Beispiel 130

### 2-[1-(2-Fluorbenzyl)-1H-pyrazolo[3,4-b]pyridin-3-yl]-7-methyl-7,9-dihydro-8H-purin-8-on

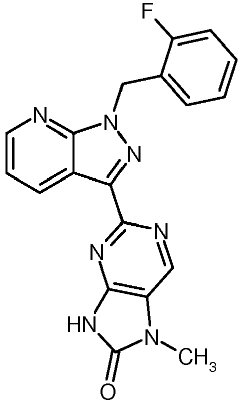

Unter einer Argonatmosphäre wurden 225 mg (ca. 0.112 mmol) der Verbindung aus Beispiel 107 in 15 ml Dimethylformamid gelöst, mit 150 mg 10% Palladium auf Kohle versetzt und unter Wasserstoff-Normaldruck über Nacht hydriert. Das Reaktionsgemisch wurde über Celite filtriert, eingeengt und der Rückstand mittels präparativer HPLC (Eluent: Wasser/Acetonitril/Wasser mit 1% Trifluoressigsäure, Gradient 60:35:5 → 35:60:5) gereinigt. Es wurden 33 mg der Titelverbindung erhalten (76% d. Th.).

LC-MS (Methode 2): Rₜ = 0.88 min; MS (EIpos): m/z = 376 [M+H]⁺.

¹H-NMR (400 MHz, DMSO-d₆): δ [ppm] = 3.38 (s, 3H), 5.84 (s, 2H) 7.13-7.17 (m, 1H), 7.21-7.26 (m, 2H), 7.34-7.39 (m, 1H), 7.42 (dd, 1H), 8.53 (s, 1H), 8.66 (dd, 1H), 8.88 (dd, 1H), 12.21 (s, 1H).

### Nicht erfindungsgemäßes Beispiel 131

### 2-[5-Fluor-1-(2-fluorbenzyl)-1H-pyrazolo[3,4-b]pyridin-3-yl]-6-iod-7-(2,2,2-trifluorethyl)-7,9-dihydro-8H-purin-8-on

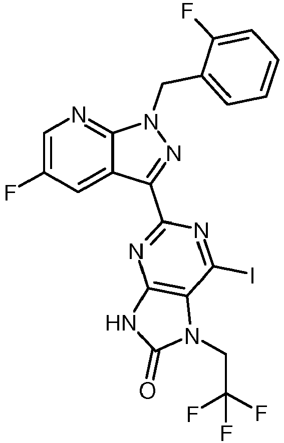

Es wurden 4.650 g (5.954 mmol) der Verbindung aus Beispiel 62 in 12 ml Diiodmethan gelöst und mit 12.76 ml (95.270 mmol) Isopentylnitrit versetzt. Es wurde für 16 h auf 85°C erhitzt und nach dem Abkühlen das Reaktionsgemisch am Rotationsverdampfer eingeengt. Es wurden 5 g des Rohprodukts (Reinheit 54%) erhalten. 1.2 g des Rückstands wurde mittels präparativer HPLC (Eluent: Acetonitril/Wasser mit 0.05% Ameisensäure, Gradient 40:60 → 95:5). Es wurden 128 mg der Titelverbindung erhalten (15% d. Th.).

LC-MS (Methode 2): Rₜ = 1.23 min; MS (EIpos): m/z = 588 [M+H]⁺.

¹H-NMR (400 MHz, DMSO-d₆): δ [ppm] = 4.95 (q, 2H), 5.86 (s, 2H), 7.14-7.18 (m, 1H), 7.21-7.27 (m, 2H), 7.35-7.41 (m, 1H), 8.47 (dd, 1H), 8.76 (dd, 1H), 12.79 (s, 1H).

### Beispiel 132

### 2-[5-Fluor-1-(2-fluorbenzyl)-1H-pyrazolo[3,4-b]pyridin-3-yl]-7-(2,2,2-trifluorethyl)-7,9-dihydro-8H-purin-8-on

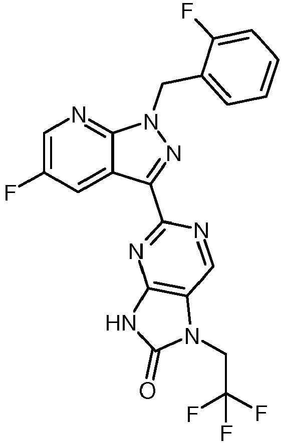

Unter einer Argonatmosphäre wurden 161 mg (0.275 mmol) der Verbindung aus Beispiel 131 in 15 ml Dimethylformamid gelöst, mit 100 mg 10% Palladium auf Kohle versetzt und unter Wasserstoff-Normaldruck über Nacht hydriert. Das Reaktionsgemisch wurde über Celite filtriert, eingeengt und der Rückstand mittels präparativer HPLC (Eluent: Wasser/Acetonitril/Wasser mit 1 % Trifluoressigsäure, Gradient 65:30:5 → 0:95:5) gereinigt. Es wurden 41 mg der Titelverbindung erhalten (33% d. Th.).

LC-MS (Methode 2): Rₜ = 1.08 min; MS (EIpos): m/z = 462 [M+H]⁺.

¹H-NMR (400 MHz, DMSO-d₆): δ [ppm] = 4.87 (q, 2H), 5.84 (s, 2H) 7.15-7.19 (m, 1H), 7.21-7.30 (m, 2H), 7.35-7.41 (m, 1H), 8.60 (dd, 1H), 8.64 (s, 1H), 8.74 (dd, 1H), 12.53 (s, 1H).

### Beispiel 133

### 5-[1-(2-Fluorbenzyl)-1H-pyrazolo[3,4-b]pyridin-3-yl]-3H-[1,2,3]triazolo[4,5-d]pyrimidin

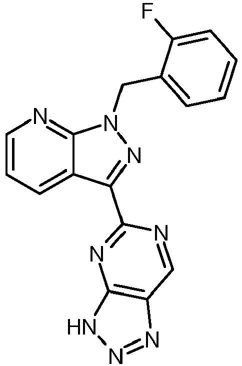

Es wurden 570 mg (5.535 mmol) *tert*.-Butylnitrit in 20 ml trockenem Dimethylformamid gelöst und bei 65°C 1000 mg (2.767 mmol) der Verbindung aus Beispiel 53 suspendiert in 15 ml Dimethylformamid mittels einer Spritzenpumpe innerhalb 1 h addiert. Nach einer weiteren Stunde Rühren bei 65°C wurden 100 ml Wasser addiert, wobei sich ein Niederschlag bildete. Der Niederschlag wurde abfiltriert und mittels präparativer HPLC gereinigt (Eluent: Wasser/Acetonitril/Wasser mit 1% Trifluoressigsäure, Gradient 64:20:16 → 0:100:0). Es wurden 10 mg der Titelverbindung erhalten (1 % d. Th.).

LC-MS (Methode 3): Rₜ = 1.02 min; MS (EIpos): m/z = 347 [M+H]⁺.

### Nicht erfindungsgemäßes Beispiel 134

### 6-Acetyl-2-[1-(2-fluorbenzyl)-1H-pyrazolo[3,4-b]pyridin-3-yl]-7-methyl-7,9-dihydro-8H-purin-8-on

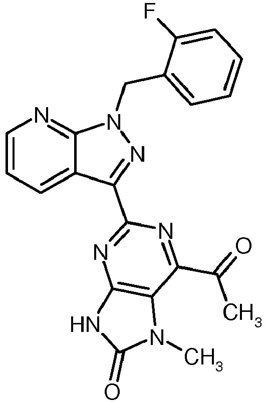

1.20 g (1.798 mmol, 60 % Reinheit) der unter Beispiel 110 erhaltenen Verbindung in Tetrahydrofuran (70 ml) wurden mit 2.997 ml (8.992 mmol) Methylmagnesiumbromid (3.0 M in Diethylether) versetzt und für 7 h zum Rückfluss erhitzt. Nach Abkühlen wurde erst Eis und dann 4N Salzsäure (20 ml) zugegeben und kräftig gerührt. Der Ansatz wurde anschliessend dreimal mit Essigsäureethylester extrahiert und die vereinigten organischen Phasen wurden mit gesättigter wässriger Natriumchlorid-Lösung gewaschen. Nach Trocknen über Natriumsulfat wurde der Ansatz filtriert, eingeengt und der Rückstand mittels präparativer HPLC gereinigt (Acetonitril:Wasser (+0.05 % Ameisensäure) - Gradient). Es wurden 236 mg der Titelverbindung erhalten (31 % d. Th.).

LC-MS (Methode 2): Rₜ = 1.05 min; MS (EIpos): m/z = 418 [M+H]⁺.

¹H-NMR (400 MHz, DMSO-d₆): δ [ppm] = 2.84 (s, 3H), 3.45 (s, 3H), 5.86 (s, 2H), 7.15 (t, 1H), 7.21-7.26 (m, 2H), 7.34-7.38 (m, 1H), 7.46 (dd, 1H), 8.68 (dd, 1H), 8.91 (dd, 1H), 12.62 (s br, 1H).

### Nicht erfindungsgemäßes Beispiel 135

### 2-[1-(2-Fluorbenzyl)-1H-pyrazolo[3,4-b]pyridin-3-yl]-6-(3-hydroxy-3-methylbut-1-in-1-yl)-7-methyl-7,9-dihydro-8H-purin-8-on

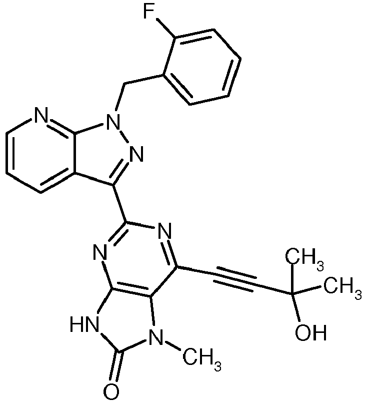

0.5 g (0.997 mmol) der unter Beispiel 107 erhaltenen Verbindung wurden in Tetrahydrofuran (10 ml) unter Argon mit 251 mg (2.992 mmol) 2-Methyl-3-butin-2-ol, 0.419 ml (2.992 mmol) Diisopropylamin, 57 mg (0.299 mmol) Kupfer-(I)-iodid und 140 mg (0.199 mmol) Dichlorbis(triphenylphosphin)palladium(II) versetzt. Es wurde anschliessend über Nacht bei RT gerührt und danach für 10 h zum Rückfluss erhitzt. Nach Abkühlen wurde der Ansatz filtriert. Das Filtrat wurde eingeengt, in Acetonitril, Wasser sowie einer geringen Menge Dimethylformamid aufgeschlämmt und abermals filtriert. Das so erhaltene Filtrat wurde mit Essigsäureethylester und Cyclohexan versetzt, kurz verrührt, vom ausgefallenen Rückstand filtriert, eingeengt und mittels präparativer HPLC gereinigt (Acetonitril: Wasser + 5 % Trifluoressigsäure) - Gradient. Es wurden 110 mg der Titelverbindung erhalten (23 % d. Th.).

LC-MS (Methode 2): Rₜ = 0.94 min; MS (EIpos): m/z = 458 [M+H]⁺.

¹H-NMR (400 MHz, DMSO-d₆): δ [ppm] = 1.55 (s, 6H), 3.56 (s, 3H), 5.80 (s, 1H), 5.84 (s, 2H), 7.15 (t, 1H), 7.21-7.26 (m, 2H), 7.34-7.39 (m, 1H), 7.44 (dd, 1H), 8.67 (dd, 1H), 8.82 (dd, 1H), 12.36 (s br, 1H).

### Nicht erfindungsgemäßes Beispiel 136

### 2-[1-(2-Fluorbenzyl)-1H-pyrazolo[3,4-b]pyridin-3-yl]-6-(2-hydroxypropan-2-yl)-7-methyl-7,9-dihydro-8H-purin-8-on

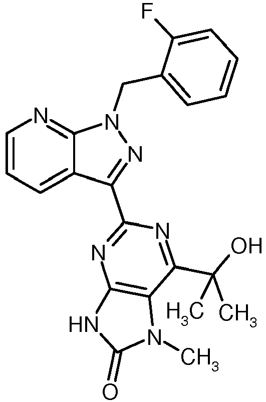

237 mg (0.568 mmol) der unter Beispiel 134 erhaltenen Verbindung in Tetrahydrofuran wurden auf 0°C abgekühlt und mit 0.568 ml einer 3M Lösung von Methylmagnesiumbromid in Diethylether versetzt und für 1h bei dieser Temperatur und anschliessend über Nacht bei RT gerührt. Dann wurde in 2 Portionen jeweils 0.379 ml einer 3M Lösung von Methylmagnesiumbromid in Diethylether zugegeben und eine weitere Nacht bei RT gerührt. Nach Abkühlen wurde erst Eis und dann 4N Salzsäure (20 ml) zugegeben und kräftig gerührt. Der Ansatz wurde anschliessend dreimal mit Essigsäureethylesterester extrahiert und die vereinigten organischen Phasen wurden mit gesättigter wässriger Natriumchlorid-Lösung gewaschen. Nach Trocknen über Natriumsulfat wurde der Ansatz filtriert, eingeengt und der Rückstand mittels präparativer HPLC gereinigt (Acetonitril:Wasser (+0.05 % Ameisensäure) - Gradient). Es wurden 29 mg der Titelverbindung erhalten (10 % d. Th.).

LC-MS (Methode 3): Rₜ = 1.09 min; MS (EIpos): m/z = 434 [M+H]⁺.

¹H-NMR (400 MHz, DMSO-d₆): δ [ppm] = 1.70 (s, 6H), 3.69 (s, 3H), 5.78 (s, 1H), 5.84 (s, 2H), 7.15 (t, 1H), 7.19-7.25 (m, 2H), 7.33-7.39 (m, 1H), 7.44 (dd, 1H), 8.66 (dd, 1H), 8.86 (dd, 1H), 12.26 (s br, 1H).

### Nicht erfindungsgemäßes Beispiel 137

### 2-[1-(2-Fluorbenzyl)-1H-pyrazolo[3,4-b]pyridin-3-yl]-7-(2-methoxyethyl)-7,9-dihydro-8H-purin-8-on

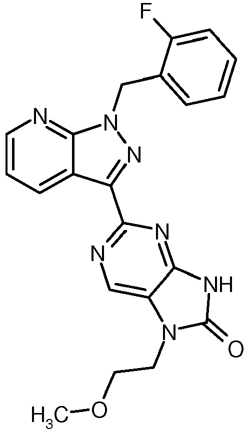

317 mg (0.501 mmol) der Verbindung aus Beispiel 83A wurden in 15 ml Trifluoressigsäure gelöst, mit 582 mg (5.009 mmol) Triethylsilan versetzt und 18h zum Rückfluss erhitzt. Es wurde zwischen Wasser und Essigsäureethylester verteilt und das Reaktionsgemisch mit gesättigter wässriger Natriumhydrogencarbonat-Lösung neutralisiert. Die organische Phase wurde über Natriumsulfat getrocknet und am Rotationsverdampfer eingeengt. Der Rückstand wurde am Hochvakuum getrocknet. und mittels präparativer HPLC gereinigt (Eluent: Methanol/Wasser mit 0.1% Trifluoressigsäure, Gradient 30:70 → 90:10). Es wurden 167 mg der Titelverbindung erhalten (79 % d. Th.).

LC-MS (Methode 3): Rₜ = 1.07 min; MS (ESIpos): m/z = 420 (M+H)⁺

¹H-NMR (400 MHz, DMSO-d₆): δ [ppm] = 3.26 (s, 3H), 3.64 (t, 2H), 4.05 (t, 2H), 5.84 (s, 2H), 7.15 (dt, 1H), 7.21-7.26 (m, 2H), 7.34-7.40 (m, 1H), 7.42 (dd, 1H), 8.55 (s, 1H), 8.66 (dd, 1H), 8.88 (dd, 1H), 12.25 (s br, 1H).

### Nicht erfindungsgemäßes Beispiel 138

### 6-[1-(2-Fluorbenzyl)-1H-pyrazolo[3,4-b]pyridin-3-yl]-1,2,3,4-tetrahydropyrido[2,3-b]pyrazin

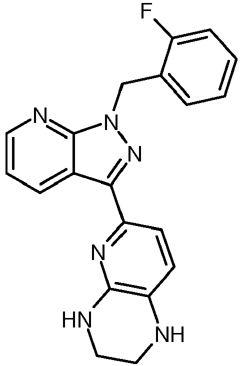

In Methanol (4 mL) wurden 80 mg (0.22 mmol) der Verbindung aus Beispiel 68 vorgelegt, anschließend 12 mg (0.045 mmol) Platin(IV)oxid zugefügt und die Reaktionsmischung über Nacht bei Normaldruck hydriert. Die Reaktionsmischung wurde durch Celite filtriert und der Filterkuchen mit Methanol nachgewaschen. Das Filtrat wurde eingeengt und man erhielt 79 mg (92%-ige Reinheit, 90 % d. Th.) der Titelverbindung, die ohne weitere Reinigung umgesetzt wurden.

LC-MS (Methode 2): Rₜ = 0.79 min

MS (ESIpos): m/z = 361 (M+H)⁺.

### Beispiel 139

### 7-(Cyclopropylmethyl)-2-[1-(2-fluorbenzyl)-1H-pyrazolo[3,4-b]pyridin-3-yl]-7,9-dihydro-8H-purin-8-on

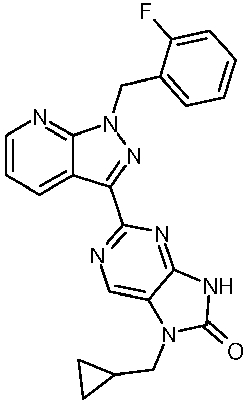

482 mg (0.70 mmol) der Verbindung aus Beispiel 84A wurden in 21 ml Trifluoressigsäure gelöst, mit 817 mg (7.02 mmol) Triethylsilan versetzt und 18h zum Rückfluss erhitzt. Das Reaktionsgemisch wurde mit Wasser und Essigsäureethylester versetzt und mit gesättigter wässriger Natriumhydrogencarbonat-Lösung neutralisiert. Die organische Phase wurde über Natriumsulfat getrocknet und am Rotationsverdampfer eingeengt. Der Rückstand wurde am Hochvakuum getrocknet. und mittels präparativer HPLC gereinigt (Eluent: Methanol/Wasser mit 0.1% Trifluoressigsäure, Gradient 30:70 → 90:10). Es wurden 156 mg der Titelverbindung erhalten (53 % d. Th.).

LC-MS (Methode2): Rₜ = 1.02 min; MS (ESIpos): m/z = 416 (M+H)⁺

¹H-NMR (400 MHz, DMSO-d₆): δ [ppm] = 0.40-0.44 (m, 2H), 0.50-0.55 (m, 2H), 1.20-1.27 (m, 1H), 3.76 (d, 2H), 5.84 (s, 2H), 7.16 (t, 1H), 7.21-7.27 (m, 2H), 7.34-7.40 (m, 1H), 7.42 (dd, 1H), 8.65 (s, 1H), 8.66 (dd, 1H), 8.88 (dd, 1H), 12.24 (s br, 1H).

### Beispiel 140

### 2-[1-(2-Fluorbenzyl)-1H-pyrazolo[3,4-b]pyridin-3-yl]-7-(2,2,3,3,3-pentafluorpropyl)-7,9-dihydro-8H-purin-8-on

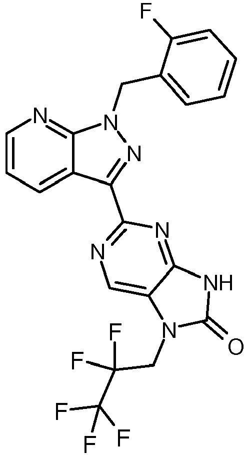

471 mg (0.60 mmol) der Verbindung aus Beispiel 85A wurden in 15 ml Trifluoressigsäure gelöst, mit 698 mg (6.01 mmol) Triethylsilan versetzt und 18h zum Rückfluss erhitzt. Das Reaktionsgemisch wurde mit Wasser und Essigsäureethylester versetzt und mit gesättigter wässriger Natriumhydrogencarbonat-Lösung neutralisiert. Die organische Phase wurde über Natriumsulfat getrocknet und am Rotationsverdampfer eingeengt. Der Rückstand wurde am Hochvakuum getrocknet. und mittels präparativer HPLC gereinigt (Eluent: Methanol/Wasser mit 0.1% Trifluoressigsäure, Gradient 30:70 → 90:10). Es wurden 252 mg der Titelverbindung erhalten (85 % d. Th.).

LC-MS (Methode 3): Rₜ = 1.29 min; MS (ESIpos): m/z = 494 (M+H)⁺

¹H-NMR (400 MHz, DMSO-d₆): δ [ppm] = 4.92 (t, 2H), 5.85 (s, 2H), 7.16 (dt, 1H), 7.21-7.26 (m, 2H), 7.34-7.40 (m, 1H), 7.43 (dd, 1H), 8.63 (s, 1H), 8.67 (dd, 1H), 8.88 (dd, 1H), 12.60 (s br, 1H).

### Beispiel 141

### 2-[1-(2-Fluorbenzyl)-1H-pyrazolo[3,4-b]pyridin-3-yl]-7-(oxetan-2-ylmethyl)-7,9-dihydro-8H-purin-8-on

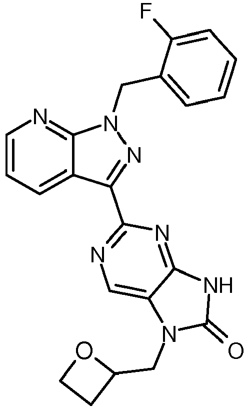

476 mg (0.82 mmol) der Verbindung aus Beispiel 86A wurden in 15 ml Trifluoressigsäure gelöst, mit 953 mg (8.20 mmol) Triethylsilan versetzt und 18h zum Rückfluss erhitzt. Das Reaktionsgemisch wurde mit Wasser und Essigsäureethylester versetzt und mit gesättigter wässriger Natriumhydrogencarbonat-Lösung neutralisiert. Die organische Phase wurde über Natriumsulfat getrocknet und am Rotationsverdampfer eingeengt. Der Rückstand wurde am Hochvakuum getrocknet. und mittels präparativer HPLC gereinigt (Eluent: Methanol/Wasser mit 0.1% Trifluoressigsäure, Gradient 30:70 → 90:10). Es wurden 173 mg der Titelverbindung erhalten (49 % d. Th.).

LC-MS (Methode 2): Rₜ = 0.91 min; MS (ESIpos): m/z = 432 (M+H)⁺

¹H-NMR (400 MHz, DMSO-d₆): δ [ppm] = 2.09-2.18 (m, 1H), 2.37-4.46 (m, 1H), 3.71 (q, 1H), 3.85 (dd, 1H), 4.07 (dd, 1H), 4.20-4.25 (m, 1H), 5.09-5.15 (m, 1H), 5.84 (dt, 1H), 5.84 (s, 2H), 7.15 (dt, 1H), 7.21-7.26 (m, 2H), 7.34-7.39 (m, 1H), 7.42 (dd, 1H), 8.51 (s, 1H), 8.66 (dd, 1H), 8.89 (dd, 1H), 12.27 (s br, 1H).

### Beispiel 142

### 2-[1-(2-Fluorbenzyl)-1H-pyrazolo[3,4-b]pyridin-3-yl]-7-[2-(morpholin-4-yl)ethyl]-7,9-dihydro-8H-purin-8-on

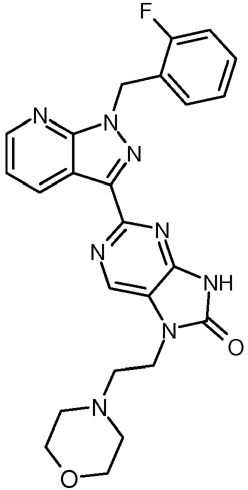

101 mg (0.16 mmol) der Verbindung aus Beispiel 87A wurden in 10 ml Trifluoressigsäure gelöst, mit 188 mg (1.62 mmol) Triethylsilan versetzt und 18h zum Rückfluss erhitzt. Das Reaktionsgemisch wurde mit Wasser und Essigsäureethylester versetzt und mit gesättigter wässriger Natriumhydrogencarbonat-Lösung neutralisiert. Die organische Phase wurde über Natriumsulfat getrocknet und am Rotationsverdampfer eingeengt. Der Rückstand wurde am Hochvakuum getrocknet. und mittels präparativer HPLC gereinigt (Eluent: Methanol/Wasser mit 0.1% Trifluoressigsäure, Gradient 30:70 → 90:10). Es wurden 53 mg der Titelverbindung erhalten (69 % d. Th.).

LC-MS (Methode 2): Rₜ = 0.72 min; MS (ESIpos): m/z = 475 (M+H)⁺

¹H-NMR (400 MHz, DMSO-d₆): δ [ppm] = 3.19-4.30 (m, 12H), 5.85 (s, 2H), 7.16 (t, 1H), 7.22-7.28 (m, 2H), 7.35-7.40 (m, 1H), 7.44 (dd, 1H), 8.67 (s, 1H), 8.68 (dd, 1H), 8.88 (dd, 1H), 12.40 (s br, 1H).

### Beispiel 143

### 2-[1-(2-Fluorbenzyl)-1H-pyrazolo[3,4-b]pyridin-3-yl]-7-[1-(2,2,2-trifluorethyl)piperidin-4-yl]-7,9-dihydro-8H-purin-8-on

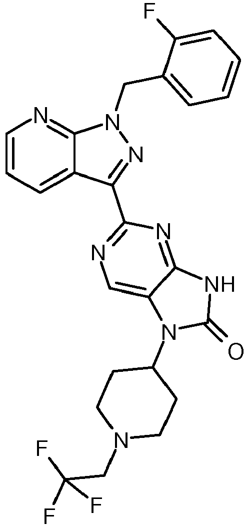

30 mg (0.06 mmol) Beispiel 88A wurden in Acetonitril (5 ml) vorgelegt und mit 12.6 mg (0.078 mmol) N,N'-Carbonyldiimidazol versetzt und anschliessend 4h zum Rückfluss erhitzt. Parallel dazu wurden 70 mg (0.14 mmol) Beispiel 88A in Acetonitril (10 ml) vorgelegt und mit 29.5 mg (0.182 mmol) N,N'-Carbonyldiimidazol versetzt und anschliessend 4h zum Rückfluss erhitzt. Beide Ansätze wurden danach vereinigt, vom Lösungsmittel befreit und anschliessend mit wenig Acetonitril behandelt. Es schied sich ein Feststoff ab, welcher abfiltriert wurde und mit wenig Acetonitril nachgewaschen wurde. Es wurden nach Trocknen im Hochvakuum 17 mg der Titelverbindung erhalten (23 % d. Th.).

LC-MS (Methode 2): Rₜ = 1.04 min; MS (EIpos): m/z = 527 [M+H]⁺.

¹H-NMR (400 MHz, DMSO-d₆): δ [ppm] = 1.76-1.79 (m, 2H), 2.24-2.33 (m, 2H), 3.06 (d, 2H), 4.19-4.25 (m, 1H), 5.84 (s, 2H), 7.15 (t, 1H), 7.21-7.26 (m, 2H), 7.34-7.39 (m, 1H), 7.42 (dd, 1H), 8.65-8.67 (m, 2H), 8.87 (d, 1H), 12.24 (s br, 1H), 4H unter Lösungsmittelpeak.

### Beispiel 144

### 2-[1-(2-Fluorbenzyl)-1H-pyrazolo[3,4-b]pyridin-3-yl]-7-[1-(2,2,2-trifluorethyl)pyrrolidin-3-yl]-7,9-dihydro-8H-purin-8-on

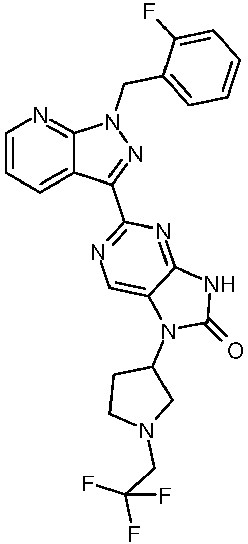

240 mg (0.493 mmol) Beispiel 89A wurden in Analogie zur Vorschrift unter Beispiel 143 umgesetzt. Man erhielt nach Reinigung mittels präparativer HPLC (Acetonitril:Wasser (+0.05 % Ameisensäure) - Gradient) 73 mg der Titelverbindung (29 % d. Th.).

LC-MS (Methode 2): Rₜ = 1.11 min; MS (EIpos): m/z = 513 [M+H]⁺.

¹H-NMR (400 MHz, DMSO-d₆): δ [ppm] = 2.00-2.08 (m, 1H), 2.30-2.35 (m, 1H), 2.87 (dd, 1H), 3.18 (dd, 1H), 5.03-5.09 (m, 1H), 5.84 (s, 2H), 7.15 (t, 1H), 7.20-7.26 (m, 2H), 7.34-7.39 (m, 1H), 7.43 (dd, 1H), 8.66 (dd, 1H), 8.78 (s, 1H), 8.86 (dd, 1H), 12.22 (s br, 1H), 4H unter Lösungsmittelpeak.

### Beispiel 145

### 7-[1-(2,2-Difluorethyl)piperidin-4-yl]-2-[1-(2-fluorbenzyl)-1H-pyrazolo[3,4-b]pyridin-3-yl]-7,9-dihydro-8H-purin-8-on

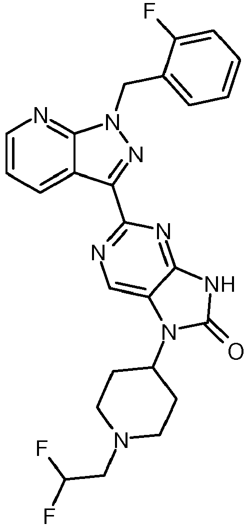

110 mg (0.228 mmol) Beispiel 90A wurden in Analogie zur Vorschrift unter Beispiel 143 umgesetzt. Man erhielt nach Reinigung mittels präparativer HPLC (Acetonitril:Wasser (+0.05 % Ameisensäure) - Gradient) 18 mg der Titelverbindung (15 % d. Th., 94%ige Reinheit).

LC-MS (Methode 2): Rₜ = 0.81 min; MS (EIpos): m/z = 509 [M+H]⁺.

¹H-NMR (400 MHz, DMSO-d₆): δ [ppm] = 2.01 (m, 2H), 4.48 (m, 1H), 5.84 (s, 2H), 6.30-6.70 (m, 1H), 7.15 (t, 1H), 7.22-7.26 (m, 2H), 7.34-7.40 (m, 1H), 7.43 (dd, 1H), 8.67 (m, 2H), 8.86 (d, 1H), 12.32 (s br, 1H), 8H unter Lösungsmittelpeak.

### Nicht erfindungsgemäßes Beispiel 146

### 2-[5-Fluor-1-(2-fluorbenzyl)-1H-pyrazolo[3,4-b]pyridin-3-yl]-7-[2-(methylsulfonyl)ethyl]-7,9-dihydro-8H-purin-8-on

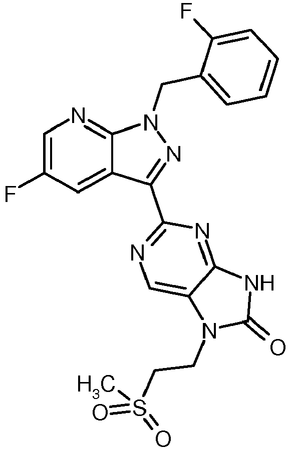

490 mg (0.567 mmol, 73 %ige Reinheit) der Verbindung aus Beispiel 94A wurden in Analogie zur Vorschrift unter Beispiel 142 umgesetzt. Es wurden 89 mg (30 % d. Th., 95 %ige Reinheit) der Titelverbindung erhalten.

LC-MS (Methode 2): Rₜ = 0.86 min; MS (EIpos): m/z = 486 [M+H]⁺.

¹H-NMR (400 MHz, DMSO-d₆): δ [ppm] = 3.09 (s, 3H), 3.64 (t, 2H), 4.33 (t, 2H), 5.84 (s, 2H), 7.16 (t, 1H), 7.21-7.29 (m, 2H), 7.35-7.40 (m, 1H), 8.58 (dd, 1H), 8.62 (s, 1H), 8.74 (dd, 1H), 12.30 (s br, 1H).

### Beispiel 147

### 7-(Azetidin-3-yl)-2-[1-(2-fluorbenzyl)-1H-pyrazolo[3,4-b]pyridin-3-yl]-7,9-dihydro-8H-purin-8-on-Hydrochlorid

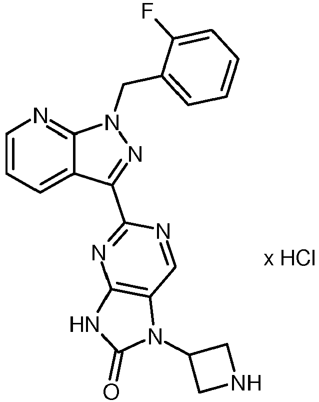

579 mg (ca. 0.887 mmol, 79 %ige Reinheit) der Verbindung aus Beispiel 98A wurden 10 ml Dioxan gelöst und anschliessend mit 4 ml einer 4N Lösung von Chlorwasserstoff in Dioxan versetzt und 0.5 h bei RT gerührt. Danach wurde bis zur Trockene eingeengt. Man erhielt nach Reinigung mittels präparativer HPLC (Acetonitril:Wasser (+0.05 % Ameisensäure) - Gradient) 168 mg (41 % d. Th.) der Titelverbindung.

LC-MS (Methode 2): Rₜ = 0.66 min; MS (EIpos): m/z = 417 [M+H]⁺.

¹H-NMR (400 MHz, DMSO-d₆): δ [ppm] = 4.03 (t, 2H), 4.23 (t, 2H), 5.21-5.29 (m, 1H), 5.84 (s, 2H), 7.16 (t, 1H), 7.22-7.26 (m, 2H), 7.34-7.40 (m, 1H), 7.42 (dd, 1H), 8.22 (s, 1H), 8.66 (dd, 1H), 8.88 (dd, 1H), 8.90 (s, 1H).

### Nicht erfindungsgemäßes Beispiel 148

### 5-[5-Fluor-1-(2-fluorbenzyl)-1H-pyrazolo[3,4-b]pyridin-3-yl]-1-methyl-1,3-dihydro [1,2,5]thiadiazolo[3,4-d]pyrimidin-2,2-dioxid

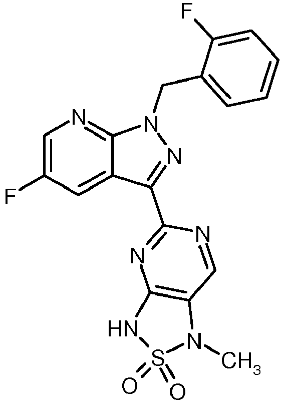

28 mg (0.050 mmol) der Verbindung aus Beispiel 100A wurden in 1.5 ml Trifluoressigsäure gelöst und anschliessend mit 79 µl (0.495 mmol) Triethylsilan versetzt und über Nacht zum Rückfluss erhitzt. Danach wurde bis zur Trockene eingeengt. Man erhielt nach Reinigung mittels präparativer HPLC (Acetonitril:Wasser (+0.05 % Ameisensäure) - Gradient) 10.4 mg (48 % d. Th.,) der Titelverbindung.

LC-MS (Methode 2): Rₜ = 0.92 min; MS (EIpos): m/z = 430 [M+H]⁺.

¹H-NMR (400 MHz, DMSO-d₆): δ [ppm] = 3.24 (s, 3H), 5.91 (s, 2H), 7.16 (t, 1H), 7.22-7.28 (m, 2H), 7.35-7.40 (m, 1H), 7.49 (s, 1H), 8.48 (dd, 1H), 8.83 (dd, 1H), 13.80 (s br, 1H).

### Nicht erfindungsgemäßes Beispiel 149

### 7-[1-(2-Fluorbenzyl)-1H-pyrazolo[3,4-b]pyridin-3-yl]-3-(2,2,2-trifluorethyl)pyrimido[4,5-d]pyrimidin-2,4(1H,3H)-dion

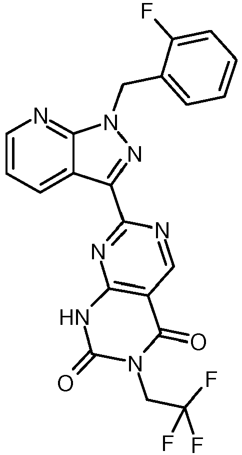

50 mg (0.11 mmol) 4-Amino-2-[1-(2-fluorbenzyl)-1H-pyrazolo[3,4-b]pyridin-3-yl]-N-(2,2,2-trifluorethyl)pyrimidin-5-carboxamid (Beispiel 103A) wurden in 5 ml THF vorgelegt und unter Eiskühlung über einen Zeitraum von 5 min mit 18.0 mg (0.45 mmol) Natriumhydrid (60%-ig in Mineralöl) versetzt. Es wurde 25 min gerührt, dann wurden 27.3 mg (0.17 mmol) 1.1'-Carbonyldiimidazol über 5 min bei Eiskühlung addiert. Es wurde 1 h bei Raumtemperatur und 90 min bei Rückflußtemperatur umgesetzt. Der Ansatz wurde nach dem Abkühlen hydrolysiert und die organische Phase dann mit 10 ml THF verdünnt. Nach dem Trocknen mit Natriumsulfat wurden die flüchtigen Komponenten mittels eines Rotationsverdampfers abgetrennt. Dann wurde das Rohmaterial mittels präparativer HPLC aufgereinigt [Säule: Reprosil C18, 10 µm, 250*30mm; Fluss: 40 ml/min; Eluent: Methanol/0.05 %-ige Ameisensäure; Gradient: 50% Methanol → 95% Methanol)]. Die flüchtigen Komponenten der Produktfraktionen wurden am Rotationsverdampfer abgetrennt. So wurden 26 mg (48% d. Th.) der Zielverbindung erhalten.

LC-MS (Methode 2): Rₜ = 1.02 min; MS (ESIpos): m/z = 472 (M+H)⁺

¹H-NMR (400 MHz, DMSO-d₆): δ [ppm] = 4.72 (q, 2H), 5.91 (s, 2H), 7.17 (t, 1H), 7.21-7.31 (m, 2H), 7.38 (m, 1H), 7.52 (dd, 1H), 8.73 (dd, 1H), 9.07 (dd, 1H), 9.28 (s, 1H), 12.86 (s, 1H).

### Nicht erfindungsgemäßes Beispiel 150

### 7-[1-(2-Fluorbenzyl)-1H-pyrazolo[3,4-b]pyridin-3-yl]pyrimido[4,5-d]pyrimidin-2,4(1H,3H)-dion

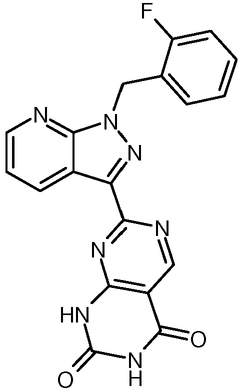

70 mg (0.19 mmol) 4-Amino-2-[1-(2-fluorbenzyl)-1H-pyrazolo[3,4-b]pyridin-3-yl]pyrimidin-5-carboxamid (Beispiel 104A) wurden in 7 ml THF vorgelegt und unter Eiskühlung über einen Zeitraum von 5 min mit 30.8 mg (0.77 mmol) Natriumhydrid (60%-ig in Mineralöl) versetzt. Es wurde 25 min gerührt, dann wurden 46.9 mg (0.29 mmol) 1.1'-Carbonyldiimidazol über 5 min bei Eiskühlung addiert. Es wurde 1 h bei Raumtemperatur und 90 min bei Rückflußtemperatur umgesetzt. Der Ansatz wurde nach dem Abkühlen mit 2 ml Wasser hydrolysiert. Der entstandene Niederschlag wurde abfiltriert und dann in 7 ml Methanol/Wasser (5/2) verrührt. Der verbliebene Niederschlag wurde abfiltriert und am Hochvakuum getrocknet. So wurden 26 mg (35% d. Th.) der Zielverbindung erhalten.

LC-MS (Methode 2): Rₜ = 0.80 min; MS (ESIpos): m/z = 390 (M+H)⁺

¹H-NMR (400 MHz, DMSO-d₆): δ [ppm] = 5.85 (s, 2H), 7.15 (t, 1H), 7.19-7.28 (m, 2H), 7.36 (t, 1H), 7.41 (dd, 1H), 8.65 (d, 1H), 8.78 (s, 1H), 8.97 (d, 1H), 10.10 (s br, 1H), 1xNH nicht zugeordnet.

### Nicht erfindungsgemäßes Beispiel 151

### 7-[1-(2-Fluorbenzyl)-1H-pyrazolo[3,4-b]pyridin-3-yl]pyrimido[4,5-d]pyrimidin-4(1H)-on

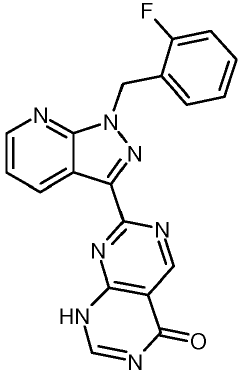

150 mg (0.38 mmol) Methyl-4-chlor-2-[1-(2-fluorbenzyl)-1H-pyrazolo[3,4-b]pyridin-3-yl]pyrimidin-5-carboxylat (Beispiel 106A) und 78.5 mg (0.75 mmol) Formamidin-Hydrochlorid wurden in 1.5 ml DMF vorgelegt. Anschließend wurden 260 µl (1.89 mmol) Triethylamin addiert und über Nacht bei 80°C umgesetzt. Der ausgefallene Niederschlag wurde nach dem Abkühlen abfiltriert und die Mutterlauge anschließend mittels präparativer HPLC aufgereinigt [Säule: Reprosil C18, 10 µm, 250*40mm; Eluent: Acetonitril/0.05 %-ige Ameisensäure; Gradient: 10% Acetonitril → 90% Acetonitril)]. So wurden 65 mg (46% d. Th.) der Zielverbindung erhalten.

LC-MS (Methode 2): Rₜ = 0.83 min; MS (ESIpos): m/z = 374 (M+H)⁺

¹H-NMR (400 MHz, DMSO-d₆): δ [ppm] = 5.92 (s, 2H), 7.17 (dt, 1H), 7.21-7.32 (m, 2H), 7.38 (m, 1H), 7.51 (dd, 1H), 8.56 (s, 1H), 8.73 (dd, 1H), 8.98 (dd, 1H), 9.56 (s, 1H), 12.94 (s br, 1H).

### Nicht erfindungsgemäßes Beispiel 152

### 7-[1-(2-Fluorbenzyl)-1H-pyrazolo[3,4-b]pyridin-3-yl]-2-methylpyrimido[4,5-d]pyrimidin-4(1H)-on

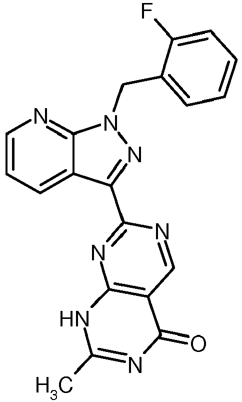

In Analogie zum Herstellungsverfahren von 7-[1-(2-Fluorbenzyl)-1H-pyrazolo[3,4-b]pyridin-3-yl]pyrimido[4,5-d]pyrimidin-4(1H)-on (Beispiel 152) wurden ausgehend von 100 mg (0.25 mmol) Methyl-4-chlor-2-[1-(2-fluorbenzyl)-1H-pyrazolo[3,4-b]pyridin-3-yl]pyrimidin-5-carboxylat (Beispiel 106A), 47.5 mg (0.50 mmol) Acetamidin-Hydrochlorid und 175 µl (1.26 mmol) Triethylamin 32 mg (31% d. Th.) der Zielverbindung erhalten.

LC-MS (Methode 2): Rₜ = 0.85 min; MS (ESIpos): m/z = 388 (M+H)⁺

¹H-NMR (400 MHz, DMSO-d₆): δ [ppm] = 2.47 (s, 3H), 5.91 (s, 2H), 7.17 (t, 1H), 7.24 (t, 1H), 7.30 (dt, 1H), 7.38 (m, 1H), 7.51 (dd, 1H), 8.72 (dd, 1H), 8.98 (dd, 1H), 9.49 (s, 1H), 12.85 (s br, 1H).

### Beispiel 153

### 2-Ethyl-7-[1-(2-fluorbenzyl)-1H-pyrazolo[3,4-b]pyridin-3-yl]pyrimido[4,5-d]pyrimidin-4(1H)-on

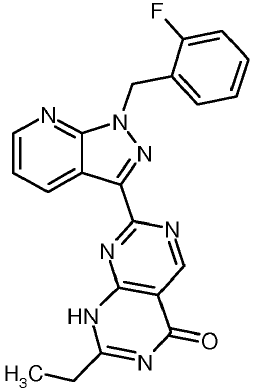

In Analogie zum Herstellungsverfahren von 7-[1-(2-Fluorbenzyl)-1H-pyrazolo[3,4-b]pyridin-3-yl]pyrimido[4,5-d]pyrimidin-4(1H)-on (Beispiel 152) wurden ausgehend von 150 mg (0.38 mmol) Methyl-4-chlor-2-[1-(2-fluorbenzyl)-1H-pyrazolo[3,4-b]pyridin-3-yl]pyrimidin-5-carboxylat (Beispiel 106A), 81.9 mg (0.75 mmol) Propionamidin-Hydrochlorid und 260 µl (1.89 mmol) Triethylamin 42 mg (25% d. Th.) der Zielverbindung erhalten.

LC-MS (Methode 5): Rₜ = 0.89 min; MS (ESIpos): m/z = 402 (M+H)⁺

¹H-NMR (400 MHz, DMSO-d₆): δ [ppm] = 1.29 (t, 3H), 2.70 (q, 2H), 5.90 (s, 2H), 7.17 (t, 1H), 7.21-7.33 (m, 2H), 7.38 (m, 1H), 7.50 (dd, 1H), 8.72 (dd, 1H), 8.99 (dd, 1H), 9.45 (s, 1H), 12.81 (s br, 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| **Beispiel Nr.** | **IC₅₀ [nM]** | **Beispiel Nr.** | **IC₅₀ [nM]** |
|---|---|---|---|
| 2 | 49 | 85 | 164 |
| 7 | 1090 | 89 | 146 |
| 12 | 284 | 90 | 5980 |
| 13 | 246 | 91 | 475 |
| 15 | 730 | 92 | 535 |
| 21 | 233 | 95 | 44 |
| 23 | 197 | 97 | 464 |
| 28 | 800 | 98 | 71 |
| 29 | 31 | 103 | 658 |
| 44 | 83 | 104 | 40 |
| 45 | 41 | 105 | 402 |
| 50 | 1390 | 112 | 1350 |
| 52 | 126 | 119 | 735 |
| 53 | 424 | 122 | 42 |
| 57 | 39 | 124 | 87 |
| 60 | 199 | 135 | 264 |
| 61 | 759 | 136 | 114 |
| 62 | 114 | 142 | 98 |
| 63 | 139 | 145 | 171 |
| 71 | 86 | 146 | 421 |
| 74 | 713 | 147 | 580 |
| 76 | 23 | 149 | 462 |
| 83 | 27 | 150 | 479 |
| 84 | 155 | 153 | 1300 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| **Beispiel Nr**. | **IC₅₀ [µM**] | **Beispiel Nr**. | **IC₅₀ [µM]** | **Beispiel Nr.** | **IC₅₀ [µM]** |
|---|---|---|---|---|---|
| 1 | 0.003 | 49 | 0.01 | 97 | 0.1 |
| 2 | 0.01 | 50 | 0.001 | 98 | 0.1 |
| 3 | 0.01 | 51 | 0.3 | 99 | 0.03 |
| 4 | 0.03 | 52 | 0.03 | 100 | 0.3 |
| 5 | 1.0 | 53 | 0.03 | 101 | 0.3 |
| 6 | 10.0 | 54 | 0.3 | 103 | 0.1 |
| 7 | 3.0 | 55 | 0.3 | 105 | 0.1 |
| 8 | 10.0 | 56 | 0.3 | 106 | 0.3 |
| 9 | 10.0 | 57 | 10.0 | 108 | 0.3 |
| 10 | 0.3 | 58 | 0.003 | 109 | 0.3 |
| 11 | 3.0 | 59 | 1.0 | 110 | 0.1 |
| 12 | 0.3 | 60 | 0.3 | 111 | 3.0 |
| 13 | 0.3 | 61 | 0.3 | 112 | 0.3 |
| 14 | 0.3 | 62 | 0.003 | 113 | 0.1 |
| 15 | 0.1 | 63 | 0.1 | 114 | 0.3 |
| 16 | 0.03 | 64 | 0.3 | 117 | 0.01 |
| 17 | 1.0 | 65 | 1.0 | 118 | 0.3 |
| 18 | 0.03 | 66 | 3.0 | 119 | 0.1 |
| 19 | 0.01 | 67 | 1.0 | 120 | 0.01 |
| 20 | 0.01 | 68 | 3.0 | 121 | 0.003 |
| 21 | 0.1 | 69 | 0.03 | 122 | 0.03 |
| 22 | 0.01 | 70 | 1.0 | 124 | 0.03 |
| 23 | 0.1 | 71 | 0.01 | 125 | 0.1 |
| 24 | 0.1 | 72 | 0.3 | 127 | 0.01 |
| 25 | 0.03 | 73 | 1.0 | 128 | 0.3 |
| 26 | 0.1 | 74 | 1.0 | 129 | 0.1 |
| 27 | 0.1 | 75 | 0.03 | 130 | 0.1 |
| 28 | 0.03 | 76 | 0.03 | 131 | 0.3 |
| 29 | 0.01 | 77 | 0.03 | 132 | 0.03 |
| 30 | 1.0 | 78 | 1.0 | 133 | 0.3 |
| 31 | 0.003 | 79 | 0.1 | 134 | 1.0 |
| 32 | 0.003 | 80 | 0.03 | 136 | 0.03 |
| 33 | 0.03 | 81 | 0.3 | 137 | 0.03 |
| 34 | 10.0 | 82 | 1.0 | 139 | 0.03 |
| 35 | 1.0 | 83 | 0.01 | 140 | 0.03 |
| 36 | 0.1 | 84 | 0.3 | 141 | 0.1 |
| 37 | 0.1 | 85 | 0.3 | 142 | 0.3 |
| 38 | 0.3 | 86 | 0.1 | 143 | 0.1 |
| 39 | 1.0 | 87 | 0.3 | 144 | 0.1 |
| 40 | 10.0 | 88 | 0.03 | 145 | 0.03 |
| 41 | 0.03 | 89 | 0.1 | 146 | 0.3 |
| 42 | 0.01 | 90 | 0.1 | 147 | 3.0 |
| 43 | 0.01 | 91 | 0.03 | 148 | 1.0 |
| 44 | 0.0003 | 92 | 0.03 | 149 | 0.1 |
| 45 | 0.003 | 93 | 0.03 | 150 | 0.3 |
| 46 | 0.3 | 94 | 0.01 | 151 | 1.0 |
| 47 | 0.003 | 95 | 0.1 | 152 | 3.0 |
| 48 | 0.03 | 96 | 0.01 | 153 | 3.0 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix) mit einem
Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
Systolischer Blutdruck (SBP)
Diastolischer Blutdruck (DBP)
Arterieller Mitteldruck (MAP)
Herzfrequenz (HR)
Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial (β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel® (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 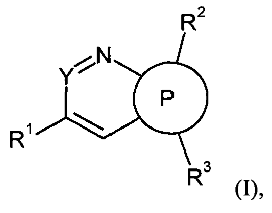 in welcher
der Ring P für eine Gruppe der Formel 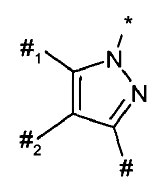 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
Y für CH steht,
R¹ für Wasserstoff oder Fluor steht,
R² für 2-Fluorbenzyl steht,
R³ für eine Gruppe der Formel 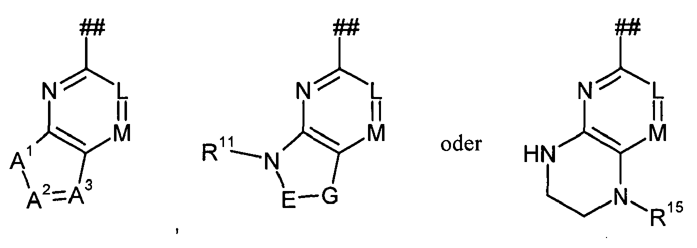 steht, wobei
## für die Anknüpfstelle an den Ring P steht,
L für N oder CH steht,
M für N oder CR⁴ steht,
worin
R⁴ für Wasserstoff oder Amino steht,
mit der Maßgabe, dass nur eine der Gruppen L und M für N steht,
A¹ für NR⁵ steht,
worin
R⁵ für Wasserstoff steht,
A² für N steht,
A³ für N oder CR⁷ steht,
worin
R⁷ für Wasserstoff, Fluor, Trifluormethyl, (C₁-C₄)-Alkyl, (C₁-C₄)-Alkoxy, Amino, Methylamino, Ethylamino, Dimethylamino oder Diethylamino steht,
worin (C₁-C₄)-Alkyl und (C₁-C₄)-Alkoxy ihrerseits mit 1 oder 2 Substituenten unabhängig voneinander ausgewählt aus der Gruppe Fluor, Trifluormethyl, Hydroxy, Methoxy und Ethoxy substituiert sein können,
E für C=O steht,
G für NR¹² steht,
worin
R¹² für Trideuteromethyl, (C₁-C₆)-Alkyl, Cyclopropyl, Cyclobutyl, Azetidin-3-yl, Pyrrolidin-3-yl oder Piperidin-4-yl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-1-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl, Cyclopropyl und Cyclobutyl substituiert sind,
R¹¹ für Wasserstoff steht
R¹⁵ für Wasserstoff oder (C₁-C₃)-Alkoxycarbonyl steht,
worin (C₁-C₃)-Alkoxycarbonyl mit 1 oder 2 Substituenten unabhängig voneinander ausgewählt aus der Gruppe Fluor, Trifluormethyl, Hydroxy, Methoxy und Ethoxy substituiert sein können,
sowie ihre Salze, Solvate und Solvate der Salze.

2. Verbindung der Formel (I) nach Anspruch 1, in welcher
der Ring P für eine Gruppe der Formel 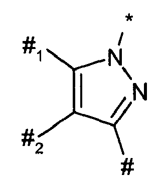 steht, wobei
* für die Anknüpfstelle an R² steht,
# für die Anknüpfstelle an R³ steht,
#₁ für die Anknüpfstelle an das Stickstoffatom steht,
#₂ für die Anknüpfstelle an das Kohlenstoffatom steht,
Y für CH steht,
R¹ für Wasserstoff oder Fluor steht,
R² für 2-Fluorbenzyl steht,
R³ für eine Gruppe der Formel 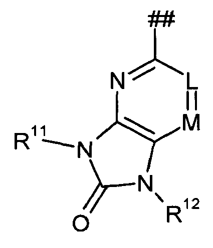 steht, wobei
## für die Anknüpfstelle an den Ring P steht,
L für CH oder N steht,
M für N oder CR⁴ steht,
wobei
R⁴ für Wasserstoff oder Amino steht,
mit der Maßgabe, dass nur eine der Gruppen L und M für N steht,
R¹¹ für Wasserstoff steht,
R¹² für Trideuteromethyl, (C₁-C₆)-Alkyl, Cyclopropyl, Cyclobutyl, Azetidin-3-yl, Pyrrolidin-3-yl oder Piperidin-4-yl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-1-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl, Cyclopropyl und Cyclobutyl substituiert sind,
sowie ihre Salze, Solvate und Solvate der Salze.

3. Verfahren zur Herstellung von Verbindungen der Formel (I), wie in den Ansprüchen 1 und 2 definiert, **dadurch gekennzeichnet, dass** man
[A] eine Verbindung der Formel (II-1) bzw. (II-2) 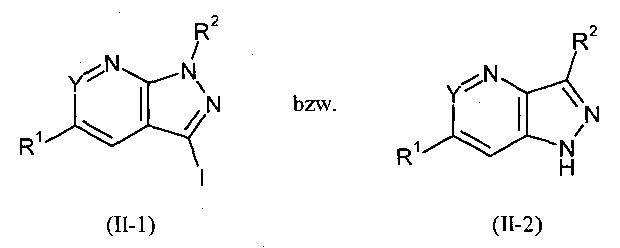 in welchen Y, R¹ und R² jeweils die in den Ansprüchen 1 und 2 angegebenen Bedeutungen haben,
in einem inerten Lösungsmittel in Gegenwart eines geeigneten Übergangsmetall-Katalysators mit einer Verbindung der Formel (III)
R ³-x¹ (III),
in welcher R³ die im Anspruch 1 angegebene Bedeutung hat und
X¹ für eine geeignete Abgangsgruppe wie beispielsweise Halogen, Mesylat, Tosylat oder Triflat steht,
zu einer Verbindung der Formel (I-A-1) bzw. (I-A-2) 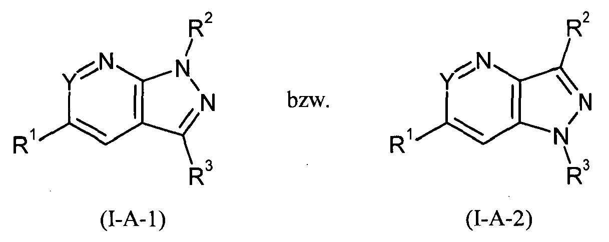 in welchen Y, R¹ und R² jeweils die in den Ansprüchen 1 und 2 angegebenen Bedeutungen haben,
umsetzt,
oder
[B] eine Verbindung der Formel (IV) 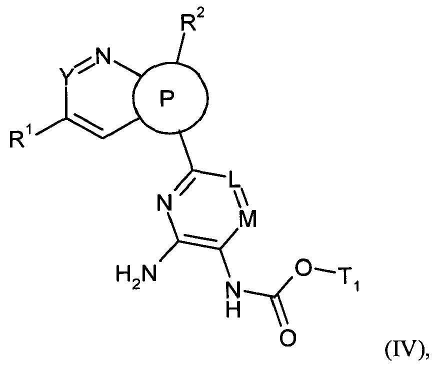 in welcher L, M, P, Y, R¹ und R² jeweils die in den Ansprüchen 1 und 2 angegebenen Bedeutungen haben und
T¹ für (C₁-C₄)-Alkyl steht,
in einem inerten Lösungsmittel in Gegenwart einer geeigneten Base zu einer Verbindung der Formel (I-B) 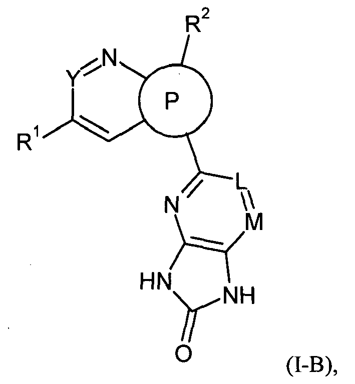 in welcher L, M, P, Y, R¹ und R² jeweils die in den Ansprüchen 1 und 2 angegebenen Bedeutungen haben,
umsetzt,
oder
[C] eine Verbindung der Formel (IV) zunächst in einem inerten Lösungsmittel in Gegenwart einer geeigneten Base mit einer Verbindung der Formel (V)
R^{12A}-X² (V),
in welcher
R^{12A} für Trideuteromethyl, (C₁-C₆)-Alkyl, (C₂-C₆)-Alkenyl, (C₃-C₇)-Cycloalkyl, Azetidinyl, Pyrrolidinyl, Piperidinyl oder Benzyl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Methoxy, Ethoxy, Methylsulfonyl, Ethylsulfonyl, Azetidinyl, Oxetanyl, Pyrrolidinyl, Piperidinyl und Morpholinyl substituiert sein kann,
worin Azetidinyl, Oxetanyl, Pyrrolidinyl, Piperidinyl und Morpholinyl wiederum ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl substituiert sein können,
worin Azetidinyl, Pyrrolidinyl und Piperidinyl ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
worin Methyl und Ethyl wiederum ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
und
worin Benzyl seinerseits mit 1 oder 2 Substituenten Fluor, Chlor, Trifluormethyl, Methyl, Ethyl, Methylsulfonyl und Ethylsulfonyl substituiert sein kann,
und
X² für eine geeignete Abgangsgruppe, wie beispielsweise Halogen, insbesondere Chlor oder Brom, Meyslat oder Tosylat steht
zu einer Verbindung der Formel (VI) 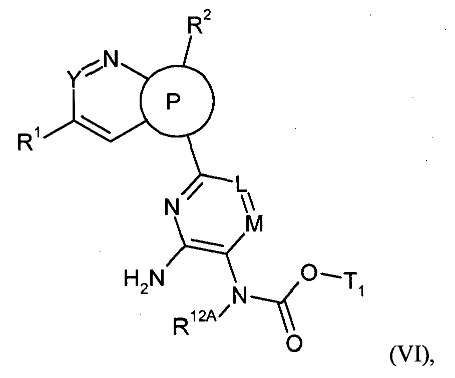 in welcher L, M, P, Y, R¹, R², R^{12A} und T¹ jeweils die in den Ansprüchen 1 und 2 angegebenen Bedeutungen haben,
umsetzt, und diese anschliessend in einem inerten Lösungsmittel in Gegenwart einer geeigneten Base zu einer Verbindung der Formel (I-C) 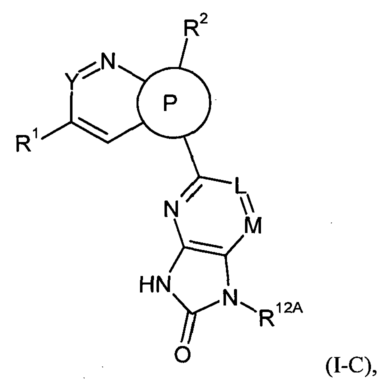 in welcher L, M, P, Y, R¹, R² und R^{12A} jeweils die in den Ansprüchen 1 und 2 angegebenen Bedeutungen haben,
cyclisiert,
oder
[D] eine Verbindung der Formel (VII) 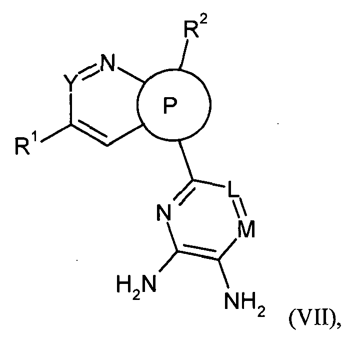 in welcher L, M, P, Y, R¹ und R² jeweils die in den Ansprüchen 1 und 2 angegebenen Bedeutungen haben,
mit einer Verbindung der Formel (VIII) 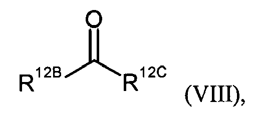 in welcher
R^{12B} für Trifluormethyl, (C₁-C₅)-Alkyl, (C₂-C₆)-Alkenyl, Cyclopropyl, Cyclobutyl oder Phenyl steht,
worin (C₁-C₅)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
und
worin Phenyl seinerseits mit 1 oder 2 Substituenten Fluor, Chlor, Trifluormethyl, Methyl, Ethyl, Methylsulfonyl und Ethylsulfonyl substituiert sein kann,
R^{12C} für Wasserstoff oder (C₁-C₅)-Alkyl steht, worin (C₁-C₅)-Alkyl seinerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
oder worin
R^{12B} und R^{12C} zusammen mit dem Kohlenstoffatom, an das sie gebunden sind einen Cyclobutyl-, Azetidinyl-, Pyrrolidinyl- oder Piperidinyl-Ring bilden,
worin der Azetidinyl-, Pyrrolidinyl- und Piperidinyl-Ring ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
worin Methyl und Ethyl ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
reduktiv zu einer Verbindung der Formel (IX) 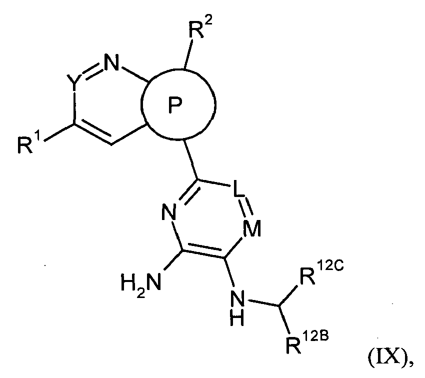 in welcher L, M, P, Y, R¹, R², R^{12B} und R^{12C} jeweils die in den Ansprüchen 1 und 2 angegebenen Bedeutungen haben,
aminiert, und diese in einem inerten Lösungsmittel in Gegenwart einer geeigneten Base mit Phosgen, einem Phosgen-Derivat oder einem Phosgen-Äquivalent zu einer Verbindung der Formel (I-D) 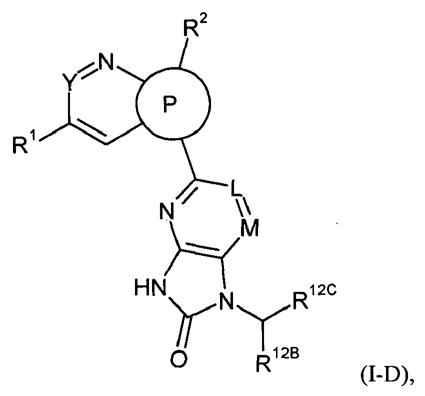 in welcher L, M, P, Y, R¹, R², R^{12B} und R^{12C} jeweils die in den Ansprüchen 1 und 2 angegebenen Bedeutungen haben,
cyclisiert,
oder
[E] eine Verbindung der Formel (X) 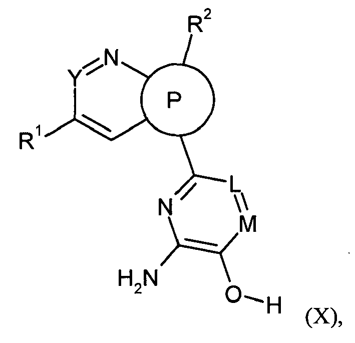 in welcher L, M, P, Y, R¹ und R² jeweils die in den Ansprüchen 1 und 2 angegebenen Bedeutungen haben,
in einem inerten Lösungsmittel in Gegenwart einer geeigneten Base mit Phosgen, einem Phosgen-Derivat oder einem Phosgen-Äquivalent zu einer Verbindung der Formel (I-E) 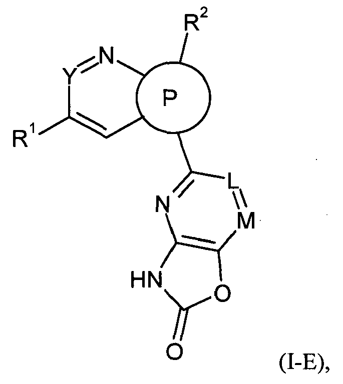 in welcher L, M, P, Y, R¹ und Jeweils die in den Ansprüchen 1 und 2 angegebenen Bedeutungen haben,
cyclisiert,
oder
[F] eine Verbindung der Formel (VII) in einem inerten Lösungsmittel unter sauren Bedingungen mit einem geeigneten Nitrit zu einer Verbindung der Formel (I-F) 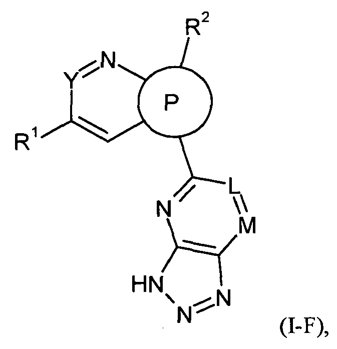 in welcher L, M, P, Y, R¹ und Jeweils die in den Ansprüchen 1 und 2 angegebenen Bedeutungen haben,
umsetzt,
oder
[G] eine Verbindung der Formel (VII) in einem inerten Lösungsmittel mit einer Verbindung der Formel (XI)
R^{5C}-X³ (XI),
in welcher
R^{5C} für Trifluormethyl oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
und
X³ für eine geeignete Abgangsgruppe, wie beispielsweise Halogen, insbesondere Chlor oder Brom, Meyslat oder Tosylat steht,
zu einer Verbindung der Formel (XII) 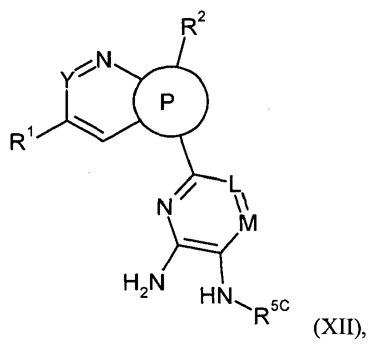 in welcher L, M, P, Y, R¹, R² und R^{5C} jeweils die in den Ansprüchen 1 und 2 angegebenen Bedeutungen haben,
umsetzt, und diese anschliessend in einem inerten Lösungsmittel mit einer Verbindung der Formel (XIII) 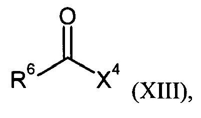 in welcher R⁶ die in den Ansprüchen 1 und 2 angegebene Bedeutung hat und
X⁴ für Chlor, Hydroxy, (C₁-C₄)-Alkoxycarbonyl oder eine Gruppe der Formel O(C=O)R⁶ steht,
zu einer Verbindung der Formel (I-G) 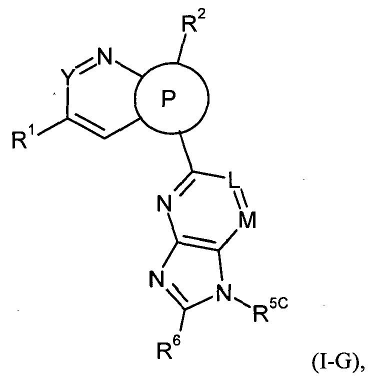 in welcher L, M, P, Y, R¹, R², R^{5C} und R⁶ jeweils die in den Ansprüchen 1 und 2 angegebenen Bedeutungen haben,
cyclisiert,
oder
[H] eine Verbindung der Formel (XIV) 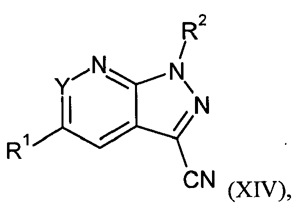 in welcher Y, R¹ und R² jeweils die in den Ansprüchen 1 und 2 angegebenen Bedeutungen haben,
in einem inerten Lösungsmittel in Gegenwart einer geeigneten Base mit einer Verbindung der Formel (XV-1) bzw. (XV-2) 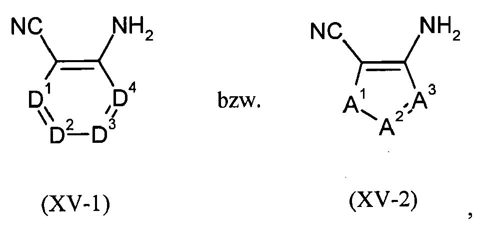 in welcher D¹, D², D³, D⁴, A¹, A² und A³ jeweils die in den Ansprüchen 1 und 2 angegebenen Bedeutungen haben,
zu einer Verbindung der Formel (I-H-1) bzw- (I-H-2) 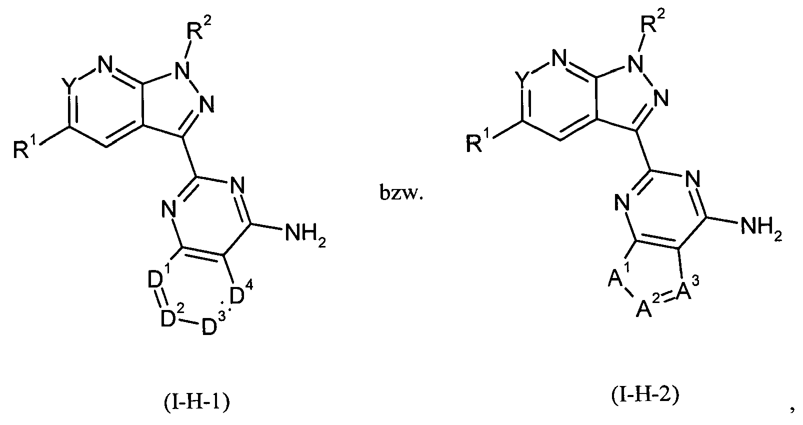 in welcher Y, R¹, R², D¹, D², D³, D⁴, A¹, A² und A³ jeweils die in den Ansprüchen 1 und 2 angegebenen Bedeutungen haben,
umsetzt,
oder
[I] eine Verbindung der Formel (I-I-1) oder (I-I-2) 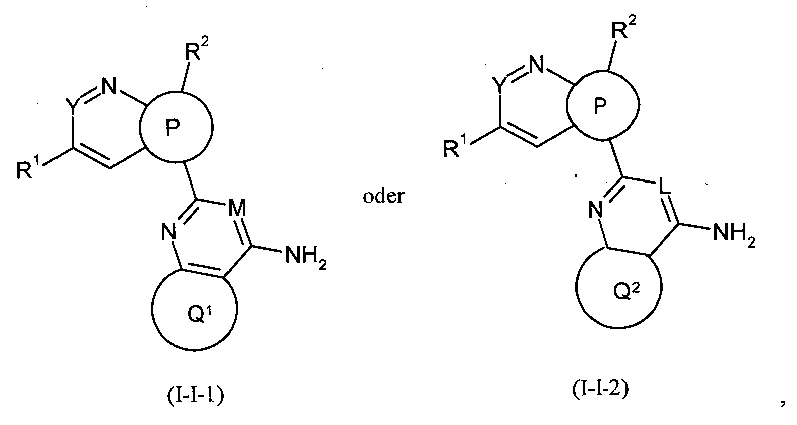 in welcher M, P, Y, R¹, R², Q¹ und Q² jeweils die in den Ansprüchen 1 und 2 angegebenen Bedeutungen haben,
in einem inerten Lösungsmittel mit einem geeigneten Nitrit zu einer Verbindung der Formel (I-I-3) oder (I-I-4), 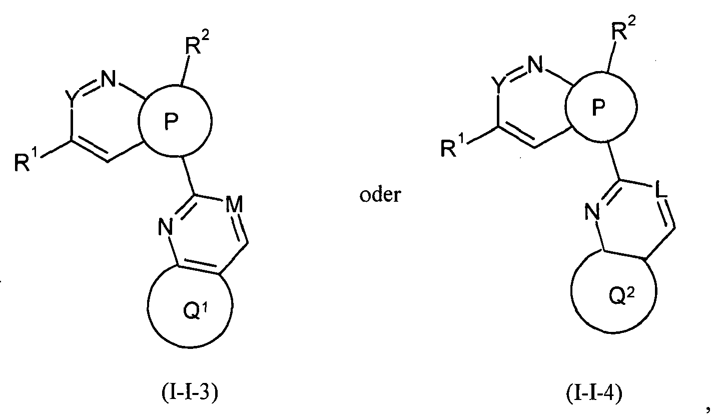 in welcher M, P, Y, R¹, R², Q¹ und Q² jeweils die in den Ansprüchen 1 und 2 angegebenen Bedeutungen haben,
umsetzt,
oder
[J] eine Verbindung der Formel (I-I-1) oder (I-I-2) in einem inerten Lösungsmittel mit Isopentylnitrit und Düodmethan zu einer Verbindung der Formel (I-J-1) oder (I-J-2) 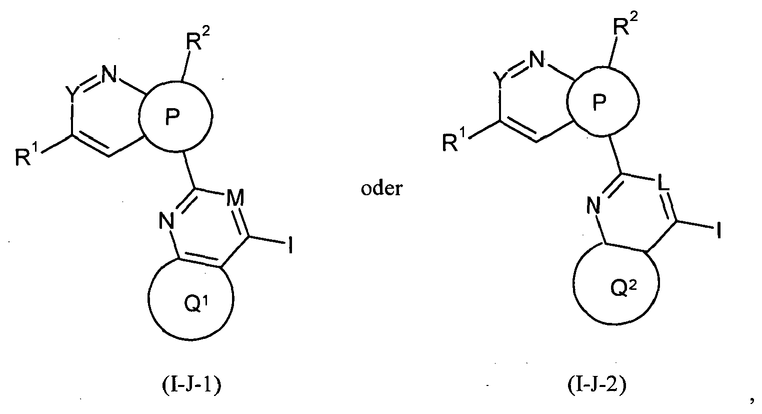 in welcher M, P, Y, R¹, R², Q¹ und Q² jeweils die in den Ansprüchen 1 und 2 angegebenen Bedeutungen haben,
oder
[K] eine Verbindung der Formel (I-J-1) oder (I-J-2) in einem inerten Lösungsmittel mit einer Verbindung der Formel (XVI)
R^{4A}-X⁵ (XVI),
in welcher
R^{4A} für Cyano, (C₁-C₄)-Alkyl, (C₂-C₄)-Alkinyl, Hydroxy, (C₁-C₄)-Alkoxy, (C₁-C₄)-Alkylcarbonyl, (C₁-C₄)-Alkoxycarbonyl, Amino, Mono-(C₁-C₄)-alkylamino, Di-(C₁-C₄)-Alkylamino oder Azetidinyl steht,
worin (C₁-C₄)-Alkyl, (C₂-C₄)-Alkinyl, (C₁-C₄)-Alkoxy, Mono-(C₁-C₄)-alkylamino und Di-(C₁-C₄)-Alkylamino ihrerseits mit 1 oder 2 Substituenten unanhangig voneinander ausgewählt aus der Gruppe Fluor, Hydroxy und Amino substituiert sein können,
und
X⁵ für Wasserstoff, Halogen, Tosylat, Mesylat, oder ein geeignetes Kation steht,
zu einer Verbindung der Formel (I-K-1) oder (I-K-2) 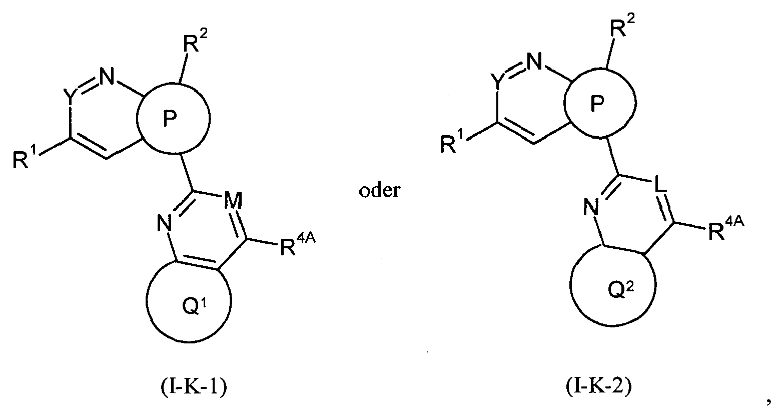 in welcher M, P, Y, R¹, R², R^{4A}, Q¹ und Q² jeweils die in den Ansprüchen 1 und 2 angegebenen Bedeutungen haben,
umsetzt,
und gegebenenfalls vorhandene Schutzgruppen nach den dem Fachmann bekannten Methoden abspaltet, und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

4. Verbindung der Formel (I), wie in einem der Ansprüche 1 und 2 definiert, zur Behandlung und/oder Prophylaxe von Krankheiten.

5. Verbindung der Formel (I), wie in einem der Ansprüche 1 und 2 definiert, zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

6. Verwendung einer Verbindung der Formel (I), wie in einem der Ansprüche 1 und 2 definiert, zur Herstellung eines Arzneimittels zur Behandlung und/oder Prophylaxe von Herzinsuffizienz, Angina pectoris, Hypertonie, pulmonaler Hypertonie, Ischämien, Gefäβ-erkrankungen, Niereninsuffizienz, thromboembolischen Erkrankungen, fibrotischen Erkrankungen und Arteriosklerose.

7. Arzneimittel enthaltend eine Verbindung der Formel (I), wie in einem der Ansprüche 1 und 2 definiert, in Kombination mit einem inerten, nicht-toxischen, pharmazeutisch geeigneten Hilfsstoff.

8. Arzneimittel enthaltend eine Verbindung der Formel (I), wie in einem der Ansprüche 1 und 2 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

9. Arzneimittel nach Anspruch 8 oder 9 zur Behandlung und/oder Prophylaxe von Herzinsuffizienz, Angina pectoris, Hypertonie, pulmonaler Hypertonie, Ischämien, Gefäß-erkrankungen, Niereninsuffizienz, thromboembolischen Erkrankungen, fibrotischen Erkrankungen und Arteriosklerose.

## Claims

1. Compound of the formula (I) 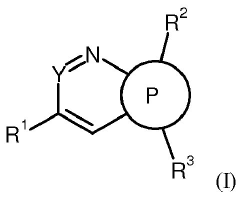 in which
the ring P is a group of the formula 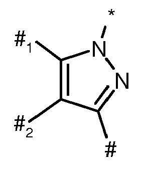 where
* is the attachment site to R²,
# is the attachment site to R³,
#₁ is the attachment site to the nitrogen atom,
#₂ is the attachment site to the carbon atom,
Y is CH,
R¹ is hydrogen or fluorine,
R² is 2-fluorobenzyl,
R³ is a group of the formula 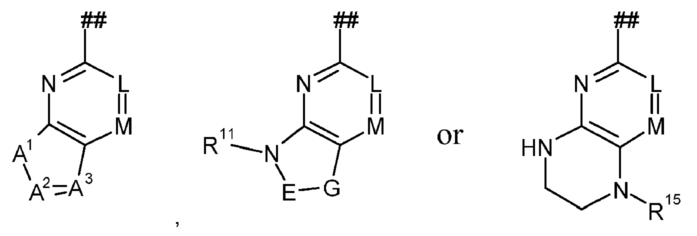 where
## is the attachment site to the ring P,
L is N or CH,
M is N or CR⁴,
in which
R⁴ is hydrogen or amino,
with the proviso that only one of the L and M groups is N,
A¹ is NR⁵,
in which
R⁵ is hydrogen,
A² is N,
A³ is N or CR⁷,
in which
R⁷ is hydrogen, fluorine, trifluoromethyl, (C₁-C₄)-alkyl, (C₁-C₄)-alkoxy, amino, methylamino, ethylamino, dimethylamino or diethylamino,
in which (C₁-C₄)-alkyl and (C₁-C₄)-alkoxy may themselves be substituted by 1 or 2 substituents each independently selected from the group of fluorine, trifluoromethyl, hydroxyl, methoxy and ethoxy,
E is C=O,
G is NR¹²,
in which
R¹² is trideuteromethyl, (C₁-C₆)-alkyl, cyclopropyl, cyclobutyl, azetidin-3-yl, pyrrolidin-3-yl or piperidin-4-yl,
in which (C₁-C₆)-alkyl may itself be substituted by 1 to 3 substituents each independently selected from the group of fluorine, trifluoromethyl, cyclopropyl, cyclobutyl, hydroxyl, oxetanyl and morpholin-1-yl,
and
in which azetidin-3-yl, pyrrolidin-3-yl and piperidin-4-yl are themselves substituted by 1 or 2 substituents each independently selected from the group of fluorine, trifluoromethyl, 2,2-difluoroethyl, 2,2,2-trifluoroethyl, methyl and ethyl, cyclopropyl and cyclobutyl,
R¹¹ is hydrogen,
R¹⁵ is hydrogen or (C₁-C₃)-alkoxycarbonyl,
in which (C₁-C₃)-alkoxycarbonyl may be substituted by 1 or 2 substituents each independently selected from the group of fluorine, trifluoromethyl, hydroxyl, methoxy and ethoxy,
and the salts, solvates and solvates of the salts thereof.

2. Compound of the formula (I) according to Claim 1, in which
the ring P is a group of the formula 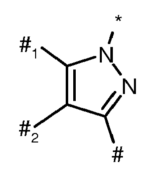 where
.* is the attachment site to R²,
# is the attachment site to R³,
#₁ is the attachment site to the nitrogen atom,
#₂ is the attachment site to the carbon atom,
Y is CH,
R¹ is hydrogen or fluorine,
R² is 2-fluorobenzyl,
R³ is a group of the formula 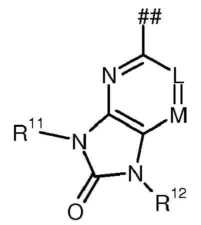 where
## is the attachment site to the ring P,
L is CH or N,
M is N or CR⁴,
where
R⁴ is hydrogen or amino,
with the proviso that only one of the L and M groups is N,
R¹¹ is hydrogen,
R ¹² is trideuteromethyl, (C₁-C₆)-alkyl, cyclopropyl, cyclobutyl, azetidin-3-yl, pyrrolidin-3-yl or piperidin-4-yl,
in which (C₁-C₆)-alkyl may itself be substituted by 1 to 3 substituents each independently selected from the group of fluorine, trifluoromethyl, cyclopropyl, cyclobutyl, hydroxyl, oxetanyl and morpholin-1-yl,
and
in which azetidin-3-yl, pyrrolidin-3-yl and piperidin-4-yl are themselves substituted by 1 or 2 substituents each independently selected from the group of fluorine, trifluoromethyl, 2,2-difluoroethyl, 2,2,2-trifluoroethyl, methyl and ethyl, cyclopropyl and cyclobutyl,
and the salts, solvates and solvates of the salts thereof.

3. Process for preparing compounds of the formula (I) as defined in Claims 1 and 2, **characterized in that**
[A] a compound of the formula (II-1) or (II-2) 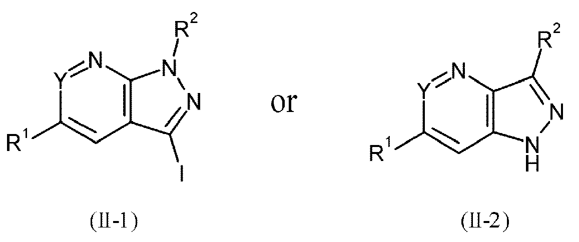 in which Y, R¹ and R² are each as defined in Claims 1 and 2,
is reacted in an inert solvent in the presence of a suitable transition metal catalyst with a compound of the formula (III)
R³-X¹ (III)
in which R³ is as defined in Claim 1 and
X¹ is a suitable leaving group, for example halogen, mesylate, tosylate or triflate,
to give a compound of the formula (I-A-1) or (I-A-2) 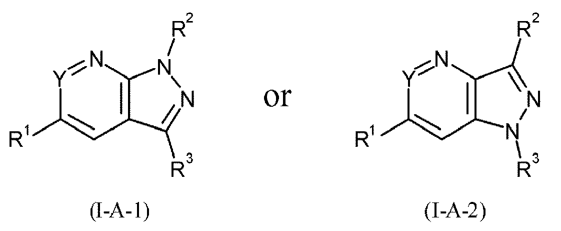 in which Y, R¹ and R² are each as defined in Claims 1 and 2,
or
[B] a compound of the formula (IV) 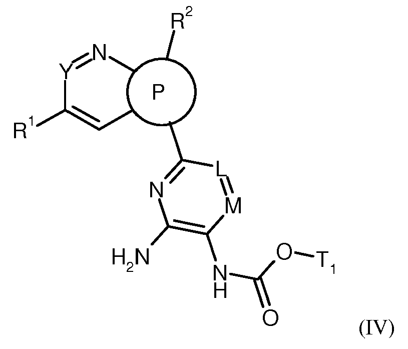 in which L, M, P, Y, R¹ and R² are each as defined in Claims 1 and 2 and
T¹ is (C₁-C₄)-alkyl
is converted in an inert solvent in the presence of a suitable base to a compound of the formula (I-B) 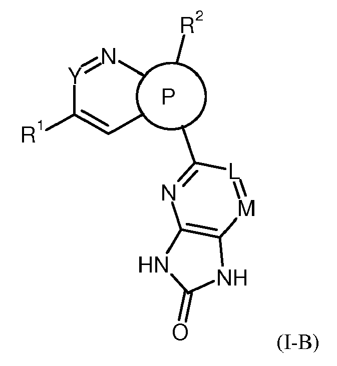 in which L, M, P, Y, R¹ and R² are each as defined in Claims 1 and 2,
or
[C] a compound of the formula (IV) is first reacted in an inert solvent in the presence of a suitable base with a compound of the formula (V)
R^{12A}-X² (V)
in which
R^{12A} is trideuteromethyl, (C₁-C₆)-alkyl, (C₂-C₆)-alkenyl, (C₃-C₇)-cycloalkyl, azetidinyl, pyrrolidinyl, piperidinyl or benzyl,
in which (C₁-C₆)-alkyl may itself be substituted by 1 to 3 substituents each independently selected from the group of fluorine, trifluoromethyl, cyclopropyl, cyclobutyl, hydroxyl, methoxy, ethoxy, methylsulfonyl, ethylsulfonyl, azetidinyl, oxetanyl, pyrrolidinyl, piperidinyl and morpholinyl,
in which azetidinyl, oxetanyl, pyrrolidinyl, piperidinyl and morpholinyl in turn may themselves be substituted by 1 or 2 substituents each independently selected from the group of fluorine, trifluoromethyl, 2,2-difluoroethyl, 2,2,2-trifluoroethyl, methyl and ethyl,
in which azetidinyl, pyrrolidinyl and piperidinyl may themselves be substituted by 1 or 2 substituents each independently selected from the group of fluorine, trifluoromethyl, methyl, ethyl, cyclopropyl and cyclobutyl,
in which methyl and ethyl in turn may themselves be substituted by 1 or 2 substituents each independently selected from the group of fluorine, trifluoromethyl, cyclopropyl, cyclobutyl, hydroxyl, methoxy and ethoxy,
and
in which benzyl may itself be substituted by 1 or 2 fluorine, chlorine, trifluoromethyl, methyl, ethyl, methylsulfonyl and ethylsulfonyl substituents,
and
X² is a suitable leaving group, for example halogen, especially chlorine or bromine, mesylate or tosylate,
to give a compound of the formula (VI) 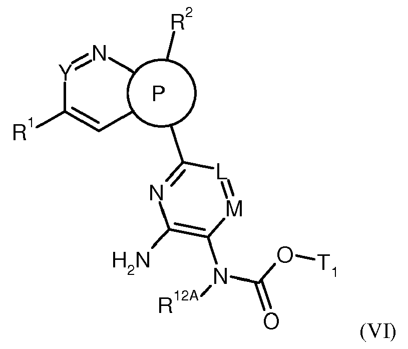 in which L, M, P, Y, R¹, R², R^{12A} and T¹ are each as defined in Claims 1 and 2,
and this is then cyclized in an inert solvent, in the presence of a suitable base, to give a compound of the formula (I-C) 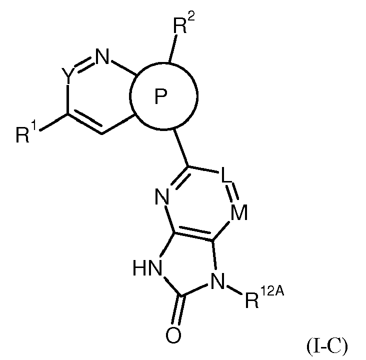 in which L, M, P, Y, R¹, R² and R^{12A} are each as defined in Claims 1 and 2,
or
[D] a compound of the formula (VII) 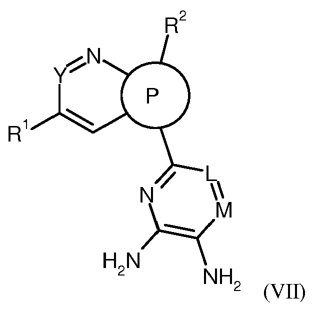 in which L, M, P, Y, R¹ and R² are each as defined in Claims 1 and 2,
is reductively aminated with a compound of the formula (VIII) 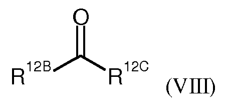 in which
R^{12B} is trifluoromethyl, (C₁-C₅)-alkyl, (C₂-C₆)-alkenyl, cyclopropyl, cyclobutyl or phenyl,
in which (C₁-C₅)-alkyl may itself be substituted by 1 or 2 substituents each independently selected from the group of fluorine, trifluoromethyl, hydroxyl, methoxy and ethoxy,
and
in which phenyl may itself be substituted by 1 or 2 fluorine, chlorine, trifluoromethyl, methyl, ethyl, methylsulfonyl and ethylsulfonyl substituents,
R^{12C} is hydrogen or (C₁-C₅)-alkyl,
in which (C₁-C₅)-alkyl may itself be substituted by 1 or 2 substituents each independently selected from the group of fluorine, trifluoromethyl, cyclopropyl, cyclobutyl, hydroxyl, methoxy and ethoxy,
or where
R^{12B} and R^{12C} together with the carbon atom to which they are bonded form a cyclobutyl, azetidinyl, pyrrolidinyl or piperidinyl ring,
in which the azetidinyl, pyrrolidinyl and piperidinyl ring may itself be substituted by 1 or 2 substituents each independently selected from the group of fluorine, trifluoromethyl, methyl, ethyl, cyclopropyl and cyclobutyl,
in which methyl and ethyl may themselves be substituted by 1 or 2 substituents each independently selected from the group of fluorine, trifluoromethyl, cyclopropyl, cyclobutyl, hydroxyl, methoxy and ethoxy,
to give a compound of the formula (IX) 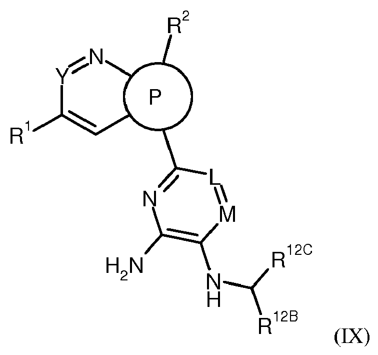 in which L, M, P, Y, R¹, R², R^{12B} and R^{12C} are each as defined in Claims 1 and 2,
and this is cyclized in an inert solvent in the presence of a suitable base with phosgene, a phosgene derivative or a phosgene equivalent to give a compound of the formula (I-D) 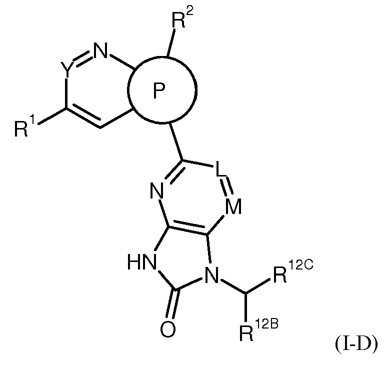 in which L, M, P, Y, R¹, R², R^{12B} and R^{12C} are each as defined in Claims 1 and 2,
or
[E] a compound of the formula (X) 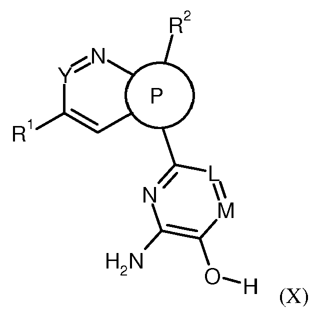 in which L, M, P, Y, R¹ and R² are each as defined in Claims 1 and 2,
is cyclized in an inert solvent in the presence of a suitable base with phosgene, a phosgene derivative or a phosgene equivalent to give a compound of the formula (I-E) 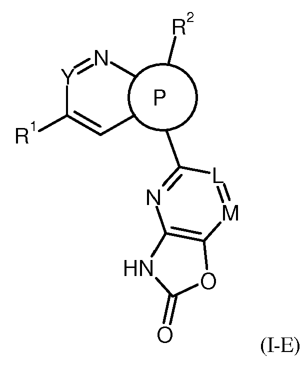 in which L, M, P, Y, R¹ and R² are each as defined in Claims 1 and 2,
or
[F] a compound of the formula (VII) is reacted in an inert solvent under acidic conditions with a suitable nitrite to give a compound of the formula (I-F) 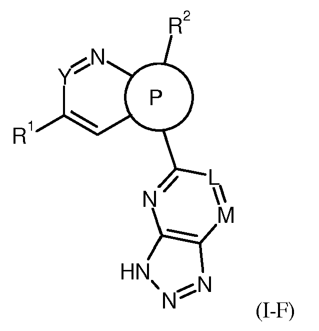 in which L, M, P, Y, R¹ and R² are each as defined in Claims 1 and 2,
or
[G] a compound of the formula (VII) is reacted in an inert solvent with a compound of the formula (XI)
R^{5C}-X³ (XI)
in which
R^{5C} is trifluoromethyl or (C₁-C₄)-alkyl,
in which (C₁-C₄)-alkyl may itself be substituted by 1 or 2 substituents each independently selected from the group of fluorine, trifluoromethyl, hydroxyl, methoxy and ethoxy,
and
X³ is a suitable leaving group, for example halogen, especially chlorine or bromine, mesylate or tosylate,
to give a compound of the formula (XII) 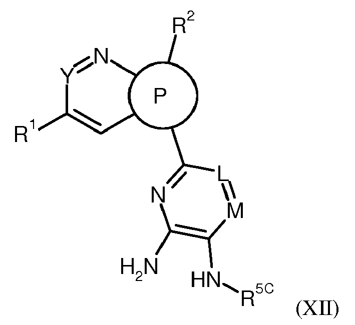 in which L, M, P, Y, R¹, R² and R^{5C} are each as defined in Claims 1 and 2,
and this is then cyclized in an inert solvent with a compound of the formula (XIII) 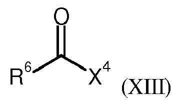 in which R⁶ is as defined in Claims 1 and 2 and
X⁴ is chlorine, hydroxyl, (C₁-C₄)-alkoxycarbonyl or a group of the formula O(C=O)R⁶
to give a compound of the formula (I-G) 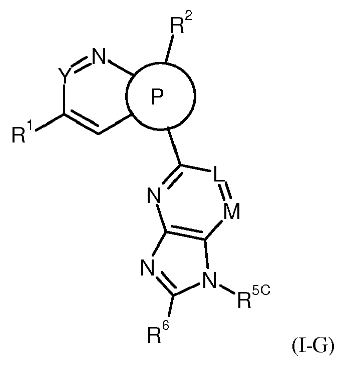 in which L, M, P, Y, R¹, R², R^{5C} and R⁶ are each as defined in Claims 1 and 2,
or
[H] a compound of the formula (XIV) 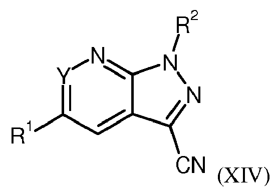 in which Y, R¹ and R² are each as defined in Claims 1 and 2
is reacted in an inert solvent in the presence of a suitable base with a compound of the formula (XV-1) or (XV-2) 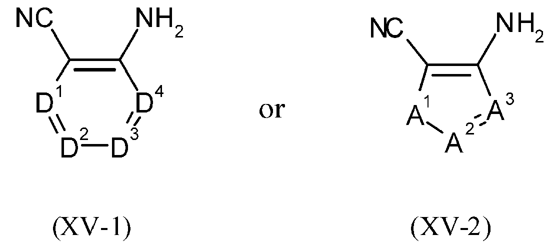 in which D¹, D², D³, D⁴, A¹, A² and A³ are each as defined in Claims 1 and 2,
to give a compound of the formula (I-H-1) or (I-H-2) 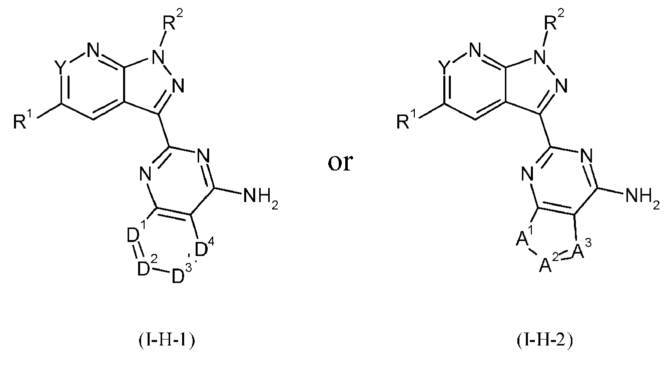 in which Y, R¹, R², D¹, D², D³, D⁴, A¹, A² and A³ are each as defined in Claims 1 and 2,
or
[I] a compound of the formula (I-I-1) or (I-I-2) 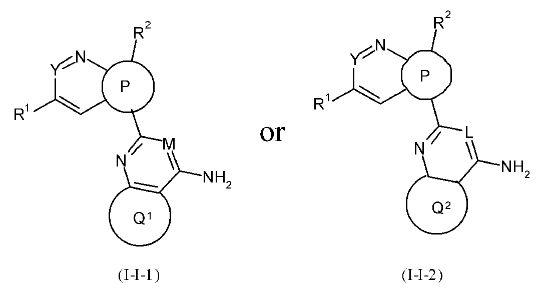 in which M, P, Y, R¹, R², Q¹ and Q² are each as defined in Claims 1 and 2
is reacted in an inert solvent with a suitable nitrite to give a compound of the formula (I-I-3) or (I-I-4) 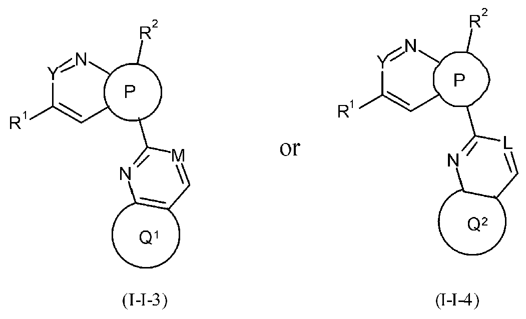 in which M, P, Y, R¹, R², Q¹ and Q² are each as defined in Claims 1 and 2,
or
[J] a compound of the formula (I-I-1) or (I-I-2) is reacted in an inert solvent with isopentyl nitrite and diiodomethane to give a compound of the formula (I-J-1) or (I-J-2) 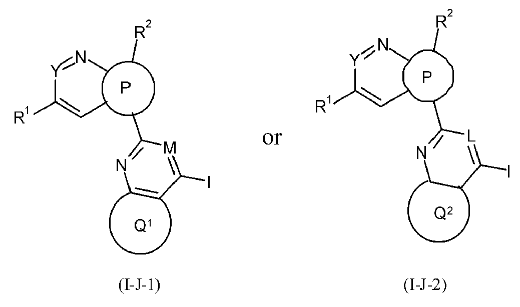 in which M, P, Y, R¹, R², Q¹ and Q² are each as defined in Claims 1 and 2,
or
[K] a compound of the formula (I-J-1) or (I-J-2) is reacted in an inert solvent with a compound of the formula (XVI)
R^{4A}-X⁵ (XVI)
in which
R^{4A} is cyano, (C₁-C₄)-alkyl, (C₂-C₄)-alkynyl, hydroxyl, (C₁-C₄)-alkoxy, (C₁-C₄)-alkylcarbonyl, (C₁-C₄)-alkoxycarbonyl, amino, mono-(C₁-C₄)-alkylamino, di-(C₁-C₄)-alkylamino or azetidinyl,
in which (C₁-C₄)-alkyl, (C₂-C₄)-alkynyl, (C₁-C₄)-alkoxy, mono-(C₁-C₄)-alkylamino and di-(C₁-C₄)-alkylamino may themselves be substituted by 1 or 2 substituents each independently selected from the group of fluorine, hydroxyl and amino,
and
X⁵ is hydrogen, halogen, tosylate, mesylate, or a suitable cation,
to give a compound of the formula (I-K-1) or (I-K-2) 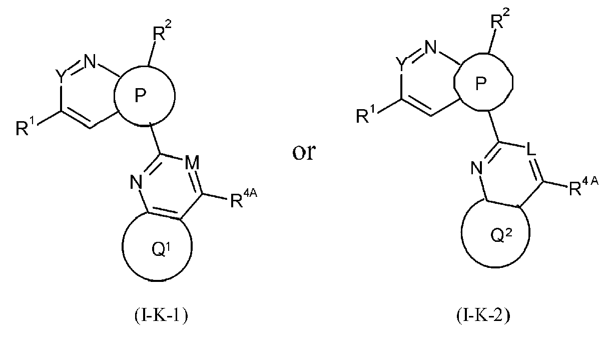 in which M, P, Y, R¹, R², R^{4A}, Q¹ and Q² are each as defined in Claims 1 and 2,
and any protecting groups present are detached by methods known to those skilled in the art, and the resulting compounds of the formula (I) are optionally converted with the appropriate (i) solvents and/or (ii) acids or bases to the solvates, salts and/or solvates of the salts thereof.

4. Compound of the formula (I) as defined in either of Claims 1 and 2 for treatment and/or prophylaxis of diseases.

5. Compound of the formula (I) as defined in either of Claims 1 and 2 for use in a method for treatment and/or prophylaxis of heart failure, angina pectoris, hypertension, pulmonary hypertension, ischemia, vascular disorders, kidney failure, thromboembolic disorders, fibrotic disorders and arteriosclerosis.

6. Use of a compound of the formula (I) as defined in either of Claims 1 and 2 for production of a medicament for treatment and/or prophylaxis of heart failure, angina pectoris, hypertension, pulmonary hypertension, ischemia, vascular disorders, kidney failure, thromboembolic disorders, fibrotic disorders and arteriosclerosis.

7. Medicament comprising a compound of the formula (I) as defined in either of Claims 1 and 2 in combination with an inert, nontoxic, pharmaceutically suitable excipient.

8. Medicament comprising a compound of the formula (I) as defined in either of Claims 1 and 2 in combination with a further active ingredient selected from the group consisting of organic nitrates, NO donors, cGMP-PDE inhibitors, antithrombotic agents, hypotensive agents and lipid metabolism modifiers.

9. Medicament according to Claim 8 or 9 for treatment and/or prophylaxis of heart failure, angina pectoris, hypertension, pulmonary hypertension, ischemia, vascular disorders, kidney failure, thromboembolic disorders, fibrotic disorders and arteriosclerosis.

## Revendications

1. Composé de formule (I) 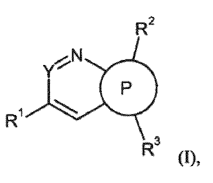 dans lequel
le cycle P représente un groupe de formule 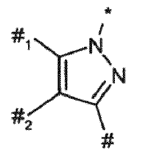 dans laquelle
* représente l'emplacement de liaison à R²,
# représente l'emplacement de liaison à R³,
#₁ représente l'emplacement de liaison à l'atome d'azote,
#₂ représente l'emplacement de liaison à l'atome de carbone,
Y représente CH,
R¹ représente hydrogène ou fluor,
R² représente 2-fluorobenzyle,
R³ représente un groupe de formule 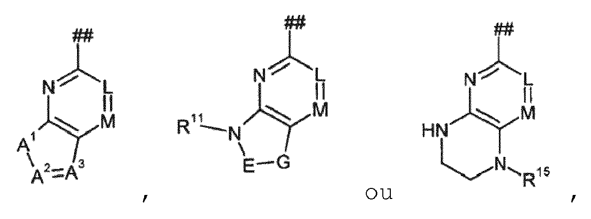 dans lesquelles
## représente l'emplacement de liaison au cycle P,
L représente N ou CH,
M représente N ou CR⁴,
R⁴ représentant hydrogène ou amino,
à condition qu'uniquement un des groupes L et M représente N,
A¹ représente NR⁵,
R⁵ représentant hydrogène,
A² représente N,
A³ représente N ou CR⁷,
R⁷ représentant hydrogène, fluor, trifluorométhyle, alkyle en (C₁-C₄), alcoxy en (C₁-C₄), amino, méthylamino, éthylamino, diméthylamino ou diéthylamino,
alkyle en (C₁-C₄) et alcoxy en (C₁-C₄) pouvant de leur côté être substitués avec 1 ou 2 substituants choisis indépendamment l'un de l'autre dans le groupe constitué par fluor, trifluorométhyle, hydroxy, méthoxy et éthoxy,
E représente C=O,
G représente NR¹²,
R¹² représentant trideutérométhyle, alkyle en (C₁-C₆), cyclopropyle, cyclobutyle, azétidin-3-yle, pyrrolidin-3-yle ou pipéridin-4-yle,
alkyle en (C₁-C₆) pouvant de son côté être substitué avec 1 à 3 substituants choisis indépendamment les uns des autres dans le groupe constitué par fluor, trifluorométhyle, cyclopropyle, cyclobutyle, hydroxy, oxétanyle et morpholin-1-yle,
et
azétidin-3-yle, pyrrolidin-3-yle et pipéridin-4-yle étant de leur côté substitués avec 1 ou 2 substituants choisis indépendamment l'un de l'autre dans le groupe constitué par fluor, trifluorométhyle, 2,2-difluoroéthyle, 2,2,2-trifluoroéthyle, méthyle et éthyle, cyclopropyle et cyclobutyle,
R¹¹ représente hydrogène,
R¹⁵ représente hydrogène ou alcoxycarbonyle en (C₁-C₃), alcoxycarbonyle en (C₁-C₃) pouvant être substitué avec 1 ou 2 substituants choisis indépendamment l'un de l'autre dans le groupe constitué par fluor, trifluorométhyle, hydroxy, méthoxy et éthoxy,
ainsi que leurs sels, solvates et solvates des sels.

2. Composé de formule (I) selon la revendication 1, dans lequel
le cycle P représente un groupe de formule 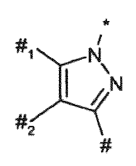 dans laquelle
* représente l'emplacement de liaison à R²,
# représente l'emplacement de liaison à R³,
#₁ représente l'emplacement de liaison à l'atome d'azote,
#₂ représente l'emplacement de liaison à l'atome de carbone,
Y représente CH,
R¹ représente hydrogène ou fluor,
R² représente 2-fluorobenzyle,
R³ représente un groupe de formule 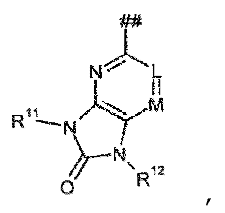 dans laquelle
## représente l'emplacement de liaison au cycle P,
L représente CH ou N,
M représente N ou CR⁴,
R⁴ représentant hydrogène ou amino,
à condition qu'uniquement un des groupes L et M représente N,
R¹¹ représente hydrogène,
R¹² représente trideutérométhyle, alkyle en (C₁-C₆), cyclopropyle, cyclobutyle, azétidin-3-yle, pyrrolidin-3-yle ou pipéridin-4-yle,
alkyle en (C₁-C₆) pouvant de son côté être substitué avec 1 à 3 substituants choisis indépendamment les uns des autres dans le groupe constitué par fluor, trifluorométhyle, cyclopropyle, cyclobutyle, hydroxy, oxétanyle et morpholin-1-yle,
et
azétidin-3-yle, pyrrolidin-3-yle et pipéridin-4-yle étant de leur côté substitués avec 1 ou 2 substituants choisis indépendamment l'un de l'autre dans le groupe constitué par fluor, trifluorométhyle, 2,2-difluoroéthyle, 2,2,2-trifluoroéthyle, méthyle et éthyle, cyclopropyle et cyclobutyle,
ainsi que leurs sels, solvates et solvates des sels.

3. Procédé de fabrication de composés de formule (I), tels que définis dans les revendications 1 et 2, **caractérisé en ce que**
[A] un composé de formule (II-1) ou (II-2) 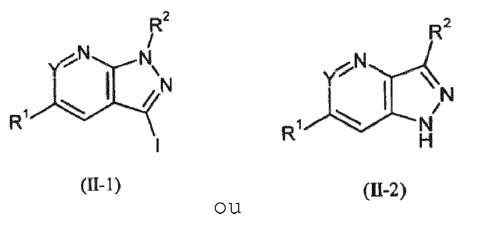 dans lesquelles Y, R¹ et R² ont chacun les significations indiquées dans les revendications 1 et 2,
est mis en réaction dans un solvant inerte en présence d'un catalyseur de métal de transition approprié avec un composé de formule (III)
R³-X¹ (III)
dans laquelle R³ a la signification indiquée dans la revendication 1, et
X¹ représente un groupe partant approprié tel que par exemple halogène, mésylate, tosylate ou triflate,
pour former un composé de formule (I-A-1) ou (I-A-2) 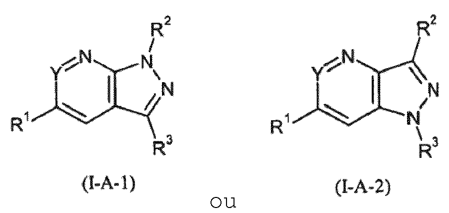 dans lesquelles Y, R¹ et R² ont chacun les significations indiquées dans les revendications 1 et 2,
ou
[B] un composé de formule (IV) 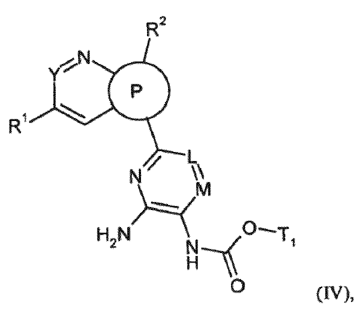 dans laquelle L, M, P, Y, R¹ et R² ont chacun les significations indiquées dans les revendications 1 et 2, et
T¹ représente alkyle en (C₁-C₄),
est mis en réaction dans un solvant inerte en présence d'une base approprié pour former un composé de formule (I-B) 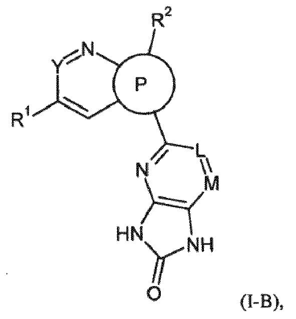 dans laquelle L, M, P, Y, R¹ et R² ont chacun les significations indiquées dans les revendications 1 et 2,
ou
[C] un composé de formule (IV) est tout d'abord mis en réaction dans un solvant inerte en présence d'une base appropriée avec un composé de formule (V)
R^{12A}-X² (V)
dans laquelle
R^{12A} représente trideutérométhyle, alkyle en (C₁-C₆), alcényle en (C₂-C₆), cycloalkyle en (C₃-C₇), azétidinyle, pyrrolidinyle, pipéridinyle ou benzyle,
alkyle en (C₁-C₆) pouvant de son côté être substitué avec 1 à 3 substituants choisis indépendamment les uns des autres dans le groupe constitué par fluor, trifluorométhyle, cyclopropyle, cyclobutyle, hydroxy, méthoxy, éthoxy, méthylsulfonyle, éthylsulfonyle, azétidinyle, oxétanyle, pyrrolidinyle, pipéridinyle et morpholinyle,
azétidinyle, oxétanyle, pyrrolidinyle, pipéridinyle et morpholinyle pouvant eux-mêmes de leur côté être substitués avec 1 ou 2 substituants choisis indépendamment l'un de l'autre dans le groupe constitué par fluor, trifluorométhyle, 2,2-difluoroéthyle, 2,2,2-trifluoroéthyle, méthyle et éthyle,
azétidinyle, pyrrolidinyle et pipéridinyle pouvant de leur côté être substitués avec 1 ou 2 substituants choisis indépendamment l'un de l'autre dans le groupe constitué par fluor, trifluorométhyle, méthyle, éthyle, cyclopropyle et cyclobutyle,
méthyle et éthyle pouvant eux-mêmes de leur côté être substitués avec 1 ou 2 substituants choisis indépendamment l'un de l'autre dans le groupe constitué par fluor, trifluorométhyle, cyclopropyle, cyclobutyle, hydroxy, méthoxy et éthoxy,
et
benzyle pouvant de son côté être substitué avec 1 ou 2 substituants choisis indépendamment l'un de l'autre dans le groupe constitué par fluor, chlore, trifluorométhyle, méthyle, éthyle, méthylsulfonyle et éthylsulfonyle,
et
X² représente un groupe partant approprié, tel que par exemple halogène, notamment chlore ou brome, mésylate ou tosylate,
pour former un composé de formule (VI) 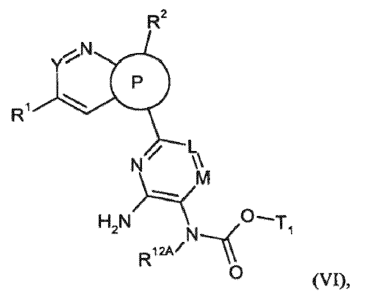 dans laquelle L, M, P, Y, R¹, R², R^{12A} et T¹ ont chacun les significations indiquées dans les revendications 1 et 2,
et celui-ci est ensuite cyclisé dans un solvant inerte en présence d'une base appropriée pour former un composé de formule (I-C) 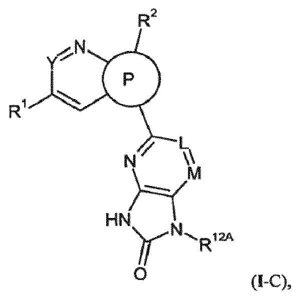 dans laquelle L, M, P, Y, R¹, R² et R^{12A} ont chacun les significations indiquées dans les revendications 1 et 2,
ou
[D] un composé de formule (VII) 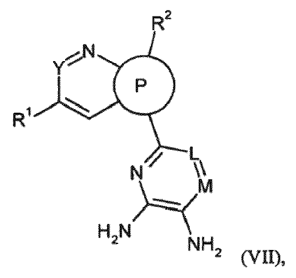 dans laquelle L, M, P, Y, R¹ et R² ont chacun les significations indiquées dans les revendications 1 et 2,
est aminé avec un composé de formule (VIII) 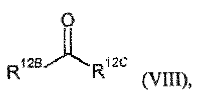 dans laquelle
R^{12B} représente trifluorométhyle, alkyle en (C₁-C₅), alcényle en (C₂-C₆), cyclopropyle, cyclobutyle ou phényle,
alkyle en (C₁-C₅) pouvant de son côté être substitué avec 1 ou 2 substituants choisis indépendamment l'un de l'autre dans le groupe constitué par fluor, trifluorométhyle, hydroxy, méthoxy et éthoxy,
et
phényle pouvant de son côté être substitué avec 1 ou 2 substituants choisis indépendamment l'un de l'autre dans le groupe constitué par fluor, chlore, trifluorométhyle, méthyle, éthyle, méthylsulfonyle et éthylsulfonyle,
R^{12C} représente hydrogène ou alkyle en (C₁-C₅),
alkyle en (C₁-C₅) pouvant de son côté être substitué avec 1 ou 2 substituants choisis indépendamment l'un de l'autre dans le groupe constitué par fluor, trifluorométhyle, cyclopropyle, cyclobutyle, hydroxy, méthoxy et éthoxy,
ou
R^{12B} et R^{12C} forment ensemble avec l'atome de carbone auquel ils sont reliés un cycle cyclobutyle, azétidinyle, pyrrolidinyle ou pipéridinyle,
les cycles azétidinyle, pyrrolidinyle et pipéridinyle pouvant de leur côté être substitués avec 1 ou 2 substituants choisis indépendamment l'un de l'autre dans le groupe constitué par fluor, trifluorométhyle, méthyle, éthyle, cyclopropyle et cyclobutyle,
méthyle et éthyle pouvant de leur côté être substitués avec 1 ou 2 substituants choisis indépendamment l'un de l'autre dans le groupe constitué par fluor, trifluorométhyle, cyclopropyle, cyclobutyle, hydroxy, méthoxy et éthoxy,
par réduction pour former un composé de formule (IX) 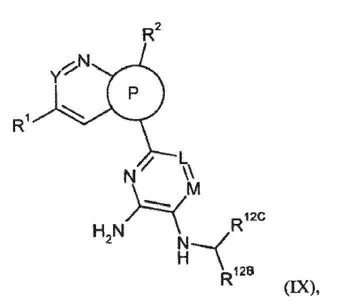 dans laquelle L, M, P, Y, R¹, R², R^{12B} et R^{12C} ont chacun les significations indiquées dans les revendications 1 et 2,
et celui-ci est cyclisé dans un solvant inerte en présence d'une base appropriée avec du phosgène, un dérivé de phosgène ou un équivalent de phosgène pour former un composé de formule (I-D) 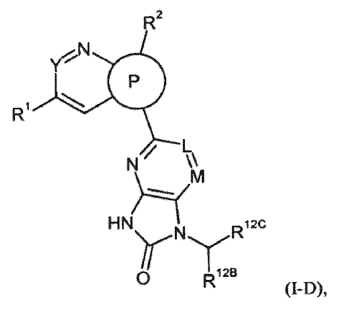 dans laquelle L, M, P, Y, R¹, R², R^{12B} et R^{12C} ont chacun les significations indiquées dans les revendications 1 et 2,
ou
[E] un composé de formule (X) 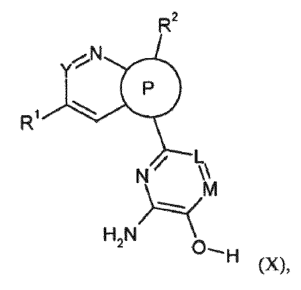 dans laquelle L, M, P, Y, R¹ et R² ont chacun les significations indiquées dans les revendications 1 et 2,
est cyclisé dans un solvant inerte en présence d'une base appropriée avec du phosgène, un dérivé de phosgène ou un équivalent de phosgène pour former un composé de formule (I-E) 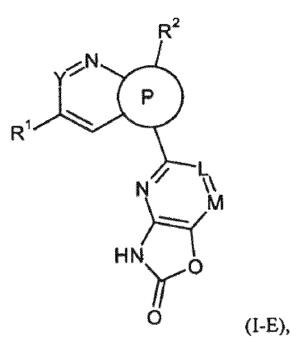 dans laquelle L, M, P, Y, R¹ et R² ont chacun les significations indiquées dans les revendications 1 et 2,
ou
[F] un composé de formule (VII) est mis en réaction dans un solvant inerte en conditions acides avec un nitrite approprié pour former un composé de formule (I-F) 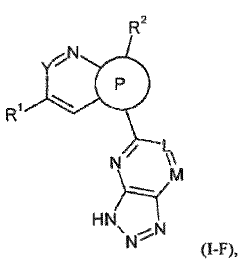 dans laquelle L, M, P, Y, R¹ et R² ont chacun les significations indiquées dans les revendications 1 et 2,
ou
[G] un composé de formule (VII) est mis en réaction dans un solvant inerte avec un composé de formule (XI)
R^{5C}-X³ (XI)
dans laquelle
R^{5C} représente trifluorométhyle ou alkyle en (C₁-C₄),
alkyle en (C₁-C₄) pouvant de son côté être substitué avec 1 ou 2 substituants choisis indépendamment l'un de l'autre dans le groupe constitué par fluor, trifluorométhyle, hydroxy, méthoxy et éthoxy,
et
X³ représente un groupe partant approprié, tel que par exemple halogène, notamment chlore ou brome, mésylate ou tosylate,
pour former un composé de formule (XII) 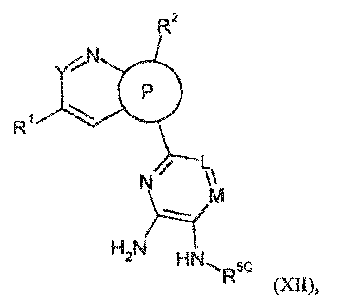 dans laquelle L, M, P, Y, R¹, R² et R^{5C} ont chacun les significations indiquées dans les revendications 1 et 2,
et celui-ci est ensuite cyclisé dans un solvant inerte avec un composé de formule (XIII) 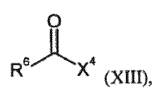 dans laquelle R⁶ a la signification indiquée dans les revendications 1 et 2, et
X⁴ représente chlore, hydroxy, alcoxycarbonyle en (C₁-C₄) ou un groupe de formule O(C=O)R⁶,
pour former un composé de formule (I-G) 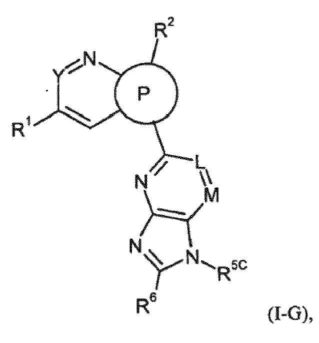 dans laquelle L, M, P, Y, R¹, R², R^{5C} et R⁶ ont chacun les significations indiquées dans les revendications 1 et 2,
ou
[H] un composé de formule (XIV) 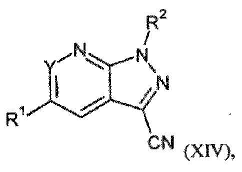 dans laquelle Y, R¹ et R² ont chacun les significations indiquées dans les revendications 1 et 2,
est mis en réaction dans un solvant inerte en présence d'une base appropriée avec un composé de formule (XV-1) ou (XV-2) 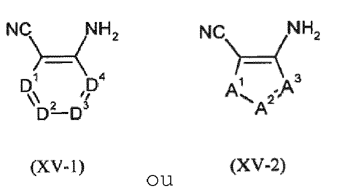 dans lesquelles D¹, D², D³, D⁴, A¹, A² et A³ ont chacun les significations indiquées dans les revendications 1 et 2,
pour former un composé de formule (I-H-1) ou (I-H-2) 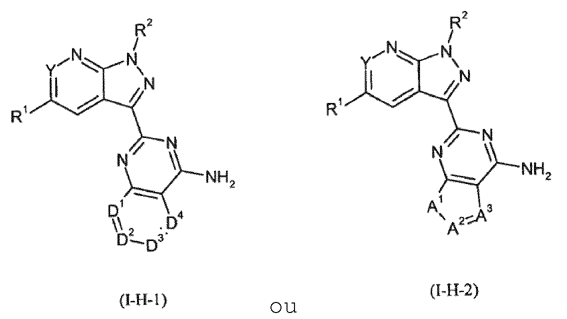 dans lesquelles Y, R¹, R², D¹, D², D³, D⁴, A¹, A² et A³ ont chacun les significations indiquées dans les revendications 1 et 2,
ou
[I] un composé de formule (I-I-1) ou (I-I-2) 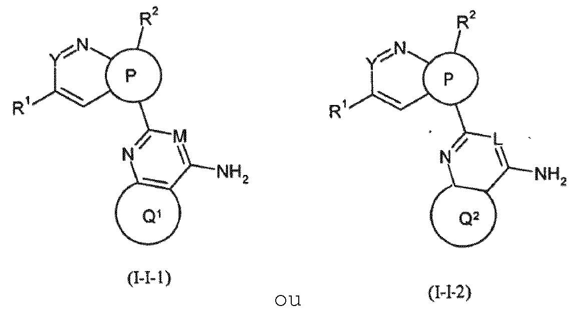 dans lesquelles M, P, Y, R¹, R², Q¹ et Q² ont chacun les significations indiquées dans les revendications 1 et 2,
est mis en réaction dans un solvant inerte avec un nitrite approprié pour former un composé de formule (I-I-3) ou (I-I-4) 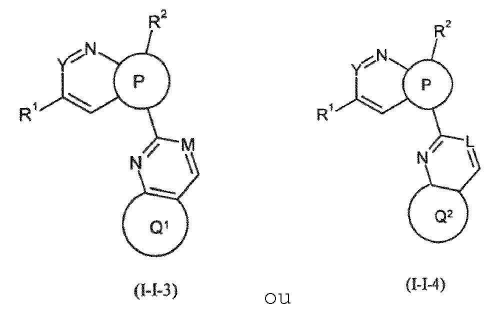 dans lesquelles M, P, Y, R¹, R², Q¹ et Q² ont chacun les significations indiquées dans les revendications 1 et 2,
ou
[J] un composé de formule (I-I-1) ou (I-I-2) est mis en réaction dans un solvant inerte avec de l'isopentylnitrite et du diiodométhane pour former un composé de formule (I-J-1) ou (I-J-2) 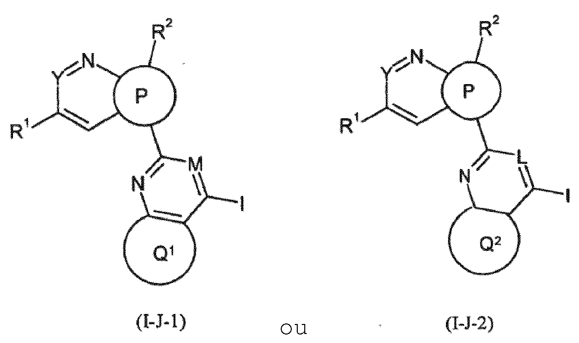 dans lesquelles M, P, Y, R¹, R², Q¹ et Q² ont chacun les significations indiquées dans les revendications 1 et 2,
ou
[K] un composé de formule (I-J-1) ou (I-J-2) est mis en réaction dans un solvant inerte avec un composé de formule (XVI)
R^{4A}-X⁵ (XVI)
dans laquelle
R^{4A} représente cyano, alkyle en (C₁-C₄), alcynyle en (C₂-C₄), hydroxy, alcoxy en (C₁-C₄), alkylcarbonyle en (C₁-C₄), alcoxycarbonyle en (C₁-C₄), amino, mono-alkylamino en (C₁-C₄), di-alkylamino en (C₁-C₄) ou azétidinyle,
alkyle en (C₁-C₄), alcynyle en (C₂-C₄), alcoxy en (C₁-C₄), mono-alkylamino en (C₁-C₄) et di-alkylamino en (C₁-C₄) pouvant de leur côté être substitués avec 1 ou 2 substituants choisis indépendamment l'un de l'autre dans le groupe constitué par fluor, hydroxy et amino, et
X⁵ représente hydrogène, halogène, tosylate, mésylate ou un cation approprié,
pour former un composé de formule (I-K-1) ou (I-K-2) 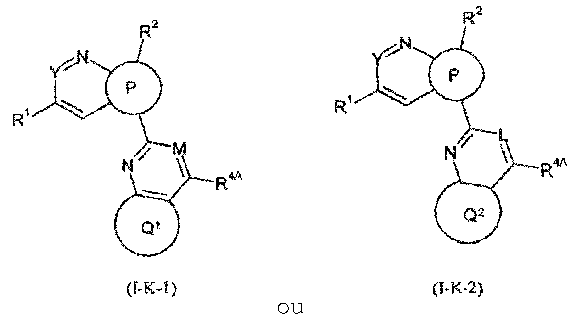 dans lesquelles M, P, Y, R¹, R², R^{4A}, Q¹ et Q² ont chacun les significations indiquées dans les revendications 1 et 2,
et les groupes protecteurs éventuellement présents sont clivés par les méthodes connues de l'homme du métier, et les composés de formule (I) résultants sont éventuellement transformés avec (i) les solvants et/ou (ii) les acides ou les bases appropriés en leurs solvates, sels et/ou solvates des sels.

4. Composé de formule (I), tel que défini dans l'une quelconque des revendications 1 et 2, pour le traitement et/ou la prophylaxie de maladies.

5. Composé de formule (I), tel que défini dans l'une quelconque des revendications 1 et 2, destiné à une utilisation dans un procédé de traitement et/ou de prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

6. Utilisation d'un composé de formule (I), tel que défini dans l'une quelconque des revendications 1 et 2, pour la fabrication d'un médicament pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

7. Médicament contenant un composé de formule (I), tel que défini dans l'une quelconque des revendications 1 et 2, en combinaison avec un adjuvant inerte, non toxique, pharmaceutiquement approprié.

8. Médicament contenant un composé de formule (I), tel que défini dans l'une quelconque des revendications 1 et 2, en combinaison avec un autre agent actif choisi dans le groupe constitué par les nitrates organiques, les donneurs de NO, les inhibiteurs de cGMP-PDE, les agents antithrombotiques, les agents abaissant la tension artérielle et les agents modifiant le métabolisme des lipides.

9. Médicament selon la revendication 8 ou 9 pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.
